# EUROPEAN PATENT APPLICATION

(11) **EP 4 803 530 A1**
(43) Date of publication of application: **09.09.2026**
(21) Application number: 24884274.2
(22) Date of filing: 13.09.2024
(51) Int. Cl.: C07H 21/02, C07H 1/00, A61K 31/713, A61P 31/20, A61P 3/06

(54) **NUCLEOSIDE AND NUCLEOTIDE ANALOG, DOUBLE-STRANDED OLIGONUCLEOTIDE AND CONJUGATE CONTAINING NUCLEOTIDE ANALOG, AND USE THEREOF**

(30) Priority: 30.10.2023 CN 202311421634
(71) Applicant: Rigerna Therapeutics (Beijing) Co., Ltd., Beijing 102629 (CN)
(72) Inventor: HUANG, Yuanyu, Beijing 102629 (CN); LI, Haitao, Beijing 102629 (CN); GAO, Yongxin, Beijing 102629 (CN); SONG, Hao, Beijing 102629 (CN); HAN, Xiaofeng, Beijing 102629 (CN); LIU, Zhichao, Beijing 102629 (CN)
(74) Representative: Cabinet Beau de Loménie
(86) International application number: PCT/CN2024/118746
(87) International publication number: WO 2025/092267

(57) **Abstract**

The present disclosure provides a nucleotide analog, a double-stranded oligonucleotide and a conjugate containing the nucleotide analog, and a use thereof, belonging to the technical field of small nucleic acid drugs. The nucleotide analog set forth in the present disclosure has a structure as shown in formula (200), or a stereoisomer thereof, or a pharmaceutically acceptable salt thereof. The double-stranded oligonucleotide comprises a sense strand and an antisense strand, each strand having 17-35 nucleotides in the direction from the 5' end to the 3' end, at least one nucleotide in the 2nd-8th positions of the antisense strand is substituted by the nucleotide analog. The double-stranded oligonucleotide and conjugate containing the nucleotide analog provided in the present disclosure can effectively treat and/or prevent disease symptoms related to the mRNA expression level of a target gene, while also having a low off-target effect, thereby reducing toxic reactions caused by off-target effects.

## Description

### CROSS-REFERENCE TO RELATED APPLICATION(S)

The present application claims the priority of Chinese Patent Application No. 202311421634.X, filed with the China National Intellectual Property Administration on October 30, 2023, entitled "Nucleoside and Nucleotide Analog, Double-stranded Oligonucleotide and Conjugate Containing Nucleotide Analog, and Use Thereof", the entire content of which is incorporated herein by reference.

### TECHNICAL FIELD

The present disclosure relates to the technical field of small nucleic acid drugs, and specifically to nucleosides and nucleotide analogs, double-stranded oligonucleotides comprising the nucleotide analogs, double-stranded oligonucleotide conjugates, and use thereof.

### BACKGROUND

Double-stranded oligonucleotides are a class of synthetically derived drugs, which act on mRNA through complementary base pairing, and interfere with various processes such as gene unwinding, replication, transcription, mRNA splicing, processing, and even export and translation, thereby disabling genes with abnormal coding, preventing the expression of "wrong" proteins, and exerting a unique mechanism for regulating the transcriptional and translational processes of disease genes at the genetic level.

Due to the unique chemical structure, oligonucleotide drugs exhibit poor druggability: large molecular weight, strong hydrophilicity, high electronegativity, non-compliance with Lipinski's rule, as well as poor pharmacokinetic profiles, inability to cross biological membranes, and off-target effects.

The field has been developing double-stranded oligonucleotides with both favorable activity and low off-target effects. To develop double-stranded oligonucleotides possessing both favorable pharmaceutical activity and low off-target effects, the inventors have found that a double-stranded oligonucleotide comprising a nucleotide analog can have lower off-target effects.

### SUMMARY OF THE INVENTION

The present disclosure provides a nucleoside analog, a nucleotide analog, a double-stranded oligonucleotide comprising the nucleotide analog, and use thereof. By incorporating a nucleotide analog into a double-stranded oligonucleotide, the present disclosure enhances anti-off-target effects of the double-stranded oligonucleotide, achieving significant toxicological improvement while substantially not reducing the activity of the double-stranded oligonucleotide.

In a first aspect of the present disclosure, the present disclosure provides a nucleotide analog selected from the structure shown in formula (II-i), or a stereoisomer thereof, or a pharmaceutically acceptable salt thereof: 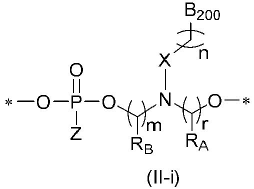
wherein, B₂₀₀ is selected from a base or a modified base;
Z is selected from hydroxyl or sulfhydryl (SH);
n is selected from 1, 2 or 3;
X is selected from 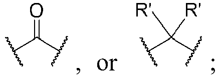 each R' is independently selected from optionally substituted C₁₋₃ alkyl, and when the optionally substituted C₁₋₃ alkyl contains substituent(s), the substituent(s) are independently selected from halogen, C₁₋₃ alkoxy, hydroxyl, and amino;
m is selected from 1, 2, 3 or 4;
r is selected from 1, 2, 3 or 4;
each R_{A} and each R_{B} are independently selected from H or optionally substituted C₁₋₃ alkyl, and there is at least one optionally substituted C₁₋₃ alkyl among R_{A} and R_{B}; wherein, if the optionally substituted C₁₋₃ alkyl contains substituent(s), the substituent(s) are independently selected from halogen, C₁₋₃ alkoxy, hydroxyl or amino.

Each * independently represents a site of covalent attachment.

In some optional embodiments of the present disclosure, each R_{A} and each R_{B} are independently selected from H or optionally substituted C₁₋₃ alkyl; and there is at least one optionally substituted C₁₋₃ alkyl among R_{A} and R_{B}; if the optionally substituted C₁₋₃ alkyl contains substituent(s), the substituent(s) are independently selected from halogen, C₁₋₃ alkoxy, hydroxyl or amino. For example, one R_{A} is selected from optionally substituted C₁₋₃ alkyl, and the remaining R_{A} and all R_{B} are selected from H; or, one R_{B} is selected from optionally substituted C₁₋₃ alkyl, and the remaining R_{B} and all R_{A} are selected from H.

In some optional embodiments of the present disclosure, each R_{A} and each R_{B} are independently selected from H or C₁₋₃ alkyl; and any one of each R_{A} and R_{B} is selected from C₁₋₃ alkyl. For example, one R_{A} is selected from C₁₋₃ alkyl, and the remaining R_{A} and all R_{B} are selected from H; or, one R_{B} is selected from C₁₋₃ alkyl, and the remaining R_{B} and all R_{A} are selected from H.

In some optional embodiments of the present disclosure, the C₁₋₃ alkyl is selected from methyl, ethyl, n-propyl and isopropyl.

In some specific embodiments of the present disclosure, the C₁₋₃ alkyl is selected from methyl.

In some optional embodiments of the present disclosure, each R_{A} and each R_{B} are independently selected from H or methyl; and any one of each R_{A} and R_{B} is selected from methyl. For example, one R_{A} is selected from methyl, and the remaining R_{A} and all R_{B} are selected from H; or, one R_{B} is selected from methyl, and the remaining R_{B} and all R_{A} are selected from H.

In some specific embodiments of the present disclosure, m is selected from 2, and r is selected from 2.

In some optional embodiments of the present disclosure, the nucleotide analog is selected from the structure set forth in formula (II-ii), or a stereoisomer thereof, or a pharmaceutically acceptable salt thereof: 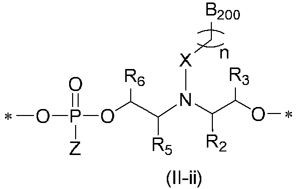
wherein, B₂₀₀ is selected from a base or a modified base;
Z is selected from hydroxyl or sulfhydryl (SH);
n is selected from 1, 2 or 3;
X is selected from 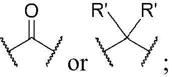 each R' is independently selected from optionally substituted C₁₋₃ alkyl, and the optionally substituted C₁₋₃ alkyl contains substituent(s) independently selected from halogen, C₁₋₃ alkoxy, hydroxyl or amino;
Each * independently represents a site of covalent attachment.

R₂, R₃, R₅ and R₆ are each independently selected from H or optionally substituted C₁₋₃ alkyl; and at least one of R₂, R₃, R₅ and R₆ is selected from optionally substituted C₁₋₃ alkyl; if the optionally substituted C₁₋₃ alkyl contains substituent(s), the substituent(s) are independently selected from halogen, C₁₋₃ alkoxy, hydroxyl or amino.

In some optional embodiments of the present disclosure, R₂, R₃, R₅ and R₆ are each independently selected from H or optionally substituted C₁₋₃ alkyl; and any one of R₂, R₃, R₅ and R₆ is selected from optionally substituted C₁₋₃ alkyl. For example, R₂ is selected from optionally substituted C₁₋₃ alkyl, and R₃, R₅ and R₆ are selected from H; or, R₃ is selected from optionally substituted C₁₋₃ alkyl, and R₂, R₅ and R₆ are selected from H; or, R₅ is selected from optionally substituted C₁₋₃ alkyl, and R₂, R₃ and R₆ are selected from H; or, R₆ is selected from optionally substituted C₁₋₃ alkyl, and R₂, R₃ and R₅ are selected from H.

In some optional embodiments of the present disclosure, R₂, R₃, R₅ and R₆ are each independently selected from H or C₁₋₃ alkyl; and any one of R₂, R₃, R₅ and R₆ is selected from C₁₋₃ alkyl. For example, R₂ is selected from C₁₋₃ alkyl, and R₃, R₅ and R₆ are selected from H; or, R₃ is selected from C₁₋₃ alkyl, and R₂, R₅ and R₆ are selected from H; or, R₅ is selected from C₁₋₃ alkyl, and R₂, R₃ and R₆ are selected from H; or, R₆ is selected from C₁₋₃ alkyl, and R₂, R₃ and R₅ are selected from H.

In some optional embodiments of the present disclosure, the C₁₋₃ alkyl is selected from methyl, ethyl, n-propyl and isopropyl.

In some specific embodiments of the present disclosure, the C₁₋₃ alkyl is selected from methyl.

In some specific embodiments of the present disclosure, R₂, R₃, R₅ and R₆ are each independently selected from H or methyl; and any one of R₂, R₃, R₅ and R₆ is selected from methyl. For example, R₂ is selected from methyl, and R₃, R₅ and R₆ are selected from H; or, R₃ is selected from methyl, and R₂, R₅ and R₆ are selected from H; or, R₅ is selected from methyl, and R₂, R₃ and R₆ are selected from H; or, R₆ is selected from methyl, and R₂, R₃ and R₅ are selected from H.

In another aspect of the present disclosure, the present disclosure provides a nucleotide analog having the structure set forth in formula (200), or a stereoisomer thereof, or a pharmaceutically acceptable salt thereof: 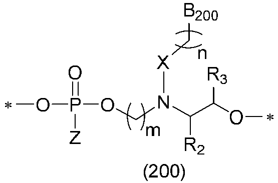
wherein, B₂₀₀ is selected from a base or a modified base;
Z is selected from hydroxyl or sulfhydryl (SH);
n is selected from 1, 2 or 3;
X is selected from 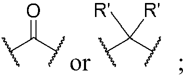 each R' is independently selected from optionally substituted C₁₋₃ alkyl, and the optionally substituted C₁₋₃ alkyl contains substituent(s) independently selected from halogen, C₁₋₃ alkoxy, hydroxyl or amino;
m is selected from 1, 2, 3 or 4;
R₂ and R₃ are independently selected from H or optionally substituted C₁₋₃ alkyl; and at least one of R₂ and R₃ is selected from optionally substituted C₁₋₃ alkyl; if the optionally substituted C₁₋₃ alkyl contains substituent(s), the substituent(s) are independently selected from halogen, C₁₋₃ alkoxy, hydroxyl or amino;
Each * independently represents a site of covalent attachment.

The present disclosure also provides a nucleotide analog having the structure set forth in formula (500), or a stereoisomer thereof, or a pharmaceutically acceptable salt thereof: 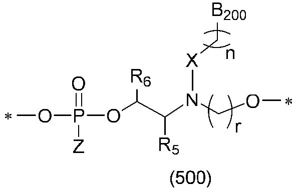
wherein, B₂₀₀ is selected from a base or a modified base;
Z is selected from hydroxyl or sulfhydryl (SH);
n is selected from 1, 2 or 3;
X is selected from 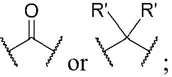 each R' is independently selected from optionally substituted C₁₋₃ alkyl, and the optionally substituted C₁₋₃ alkyl contains substituent(s) independently selected from halogen, C₁₋₃ alkoxy, hydroxyl or amino;
r is selected from 1, 2, 3 or 4;
R₅ and R₆ are independently selected from H or optionally substituted C₁₋₃ alkyl; and at least one of R₅ and R₆ is selected from optionally substituted C₁₋₃ alkyl ; if the optionally substituted C₁₋₃ alkyl contains substituent(s), the substituent(s) are independently selected from halogen, C₁₋₃ alkoxy, hydroxyl or amino;
Each * independently represents a site of covalent attachment. In one aspect of the present disclosure, the present disclosure provides a double-stranded oligonucleotide comprising a sense strand and an antisense strand, each strand having 17-35 nucleotides; in the direction from the 5' end to the 3' end, at least one nucleotide at positions 2-8 of the antisense strand is substituted by the nucleotide analog described herein above.

In one aspect of the present disclosure, the present disclosure also provides a double-stranded oligonucleotide conjugate comprising the double-stranded oligonucleotide provided by the present disclosure, and one or more ligands capable of binding to a cell surface receptor.

In one aspect of the present disclosure, the present disclosure provides a composition comprising the double-stranded oligonucleotide and/or the double-stranded oligonucleotide conjugate described in the present disclosure.

In one aspect of the present disclosure, the present disclosure also provides a pharmaceutical composition comprising the double-stranded oligonucleotide, and/or the double-stranded oligonucleotide conjugate, and/or the composition described in the present disclosure.

In one aspect of the present disclosure, the present disclosure provides a nucleoside analog characterized by having the structure set forth in formula (100), or a stereoisomer thereof, or a pharmaceutically acceptable salt thereof: 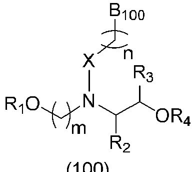
wherein, B₁₀₀ is selected from a base or a modified base, if B₁₀₀ comprises amino, the amino is protected by an amino protecting group;
n is selected from 1, 2 or 3;
X is selected from 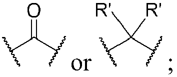 each R' is independently selected from optionally substituted C₁₋₃ alkyl, and the optionally substituted C₁₋₃ alkyl contains substituent(s) independently selected from halogen, C₁₋₃ alkoxy, hydroxyl or amino;
m is selected from 1, 2, 3 or 4;
R₁ is selected from H or a hydroxyl protecting group;
R₂ and R₃ are independently selected from H or optionally substituted C₁₋₃ alkyl; and at least one of R₂ and R₃ is selected from optionally substituted C₁₋₃ alkyl; if the optionally substituted C₁₋₃ alkyl contains substituent(s), the substituent(s) are independently selected from halogen, C₁₋₃ alkoxy, hydroxyl or amino;
R₄ is selected from H or 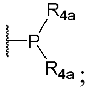 each R₄ₐ is independently selected from 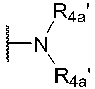 or C₁₋₆ alkoxy having a cyano substituent, and at least one R₄ₐ is selected from 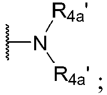 each R₄ₐ' is independently selected from optionally substituted C₁₋₆ alkyl.

In one aspect of the present disclosure, the present disclosure also provides use of the nucleotide analog, and/or the double-stranded oligonucleotide, and/or the double-stranded oligonucleotide conjugate, and/or the composition, and/or the pharmaceutical composition, and/or the nucleoside analog in the preparation of a medicament for treating and/or preventing a disease or symptom associated to the expression level of mRNA of a target gene.

In one aspect of the present disclosure, the present disclosure also provides a method for treating and/or preventing a disease or symptom associated to the expression level of mRNA of a target gene, comprising administering the double-stranded oligonucleotide, and/or the double-stranded oligonucleotide conjugate, and/or the composition, and/or the pharmaceutical composition of the present disclosure to a subject in need.

In one aspect of the present disclosure, the present disclosure also provides a method of regulating expression level of a target gene in a cell, comprising contacting the contact with an effective amount of the double-stranded oligonucleotide, and/or the double-stranded oligonucleotide conjugate, and/or the composition, and/or the pharmaceutical composition of the present disclosure.

In one aspect of the present disclosure, the present disclosure also provides a kit comprising the double-stranded oligonucleotide, and/or the double-stranded oligonucleotide conjugate, and/or the composition, and/or the pharmaceutical composition of the present disclosure.

The double-stranded oligonucleotide, double-stranded oligonucleotide conjugate, composition and/or pharmaceutical composition provided by the present disclosure can effectively treat and/or prevent a disease or symptom associated to expression level of mRNA of a target gene, while having low off-target effects and reducing toxic reactions caused by off-target effects.

### BRIEF DESCRIPTION OF THE DRAWINGS

Figure 1 shows relative expression level of the target gene in mice after administration of the siRNA conjugate in Example 1.
Figure 2 shows relative expression level of the target gene in mice after administration of the siRNA conjugate in Example 2.
Figure 3 shows serum ALT in ICR mice after administration of the siRNA conjugate in Example 3.
Figure 4 shows serum AST in ICR mice after administration of the siRNA conjugate in Example 3.
Figure 5 shows pathological scores of mouse liver tissue after administration of the siRNA conjugate in Example 3.
Figure 6 shows pathological sections of mouse liver tissue after administration of the siRNA conjugate in Example 3.
Figure 7 shows IC50 curve of the target gene ANGPTL3 in HEK 293T cells after administration of RZ597002 in Example 4.
Figure 8 shows IC50 curve of the target gene ANGPTL3 in HEK 293T cells after administration of RZ597113 in Example 4.
Figure 9 shows IC50 curve of the target gene ANGPTL3 in HEK 293T cells after administration of RZ597007 in Example 4.
Figure 10 shows IC50 curve of the target gene ANGPTL3 in HEK 293T cells after administration of RZ597112 in Example 4.
Figure 11 shows IC50 curve of the target gene ANGPTL3 in HEK 293T cells after administration of RZ597102 in Example 4.
Figure 12 shows IC50 curve of the target gene ANGPTL3 in HEK 293T cells after administration of RZ597103 in Example 4.
Figure 13 shows IC75 curve of the target gene ANGPTL3 in HEK 293T cells after administration of RZ597002 in Example 4.
Figure 14 shows IC75 curve of the target gene ANGPTL3 in HEK 293T cells after administration of RZ597113 in Example 4.
Figure 15 shows IC75 curve of the target gene ANGPTL3 in HEK 293T cells after administration of RZ597007 in Example 4.
Figure 16 shows IC75 curve of the target gene ANGPTL3 in HEK 293T cells after administration of RZ597112 in Example 4.
Figure 17 shows IC75 curve of the target gene ANGPTL3 in HEK 293T cells after administration of RZ597102 in Example 4.
Figure 18 shows IC75 curve of the target gene ANGPTL3 in HEK 293T cells after administration of RZ597103 in Example 4.
Figure 19 shows IC50 curve of the target gene ANGPTL3 in HEK 293T cells after administration of RZ597136 in Example 5.
Figure 20 shows IC50 curve of the target gene ANGPTL3 in HEK 293T cells after administration of RZ597157 in Example 5.
Figure 21 shows IC50 curve of the target gene ANGPTL3 in HEK 293T cells after administration of RZ597161 in Example 5.
Figure 22 shows IC60 curve of the target gene ANGPTL3 in HEK 293T cells after administration of RZ597136 in Example 5.
Figure 23 shows IC60 curve of the target gene ANGPTL3 in HEK 293T cells after administration of RZ597157 in Example 5.
Figure 24 shows IC60 curve of the target gene ANGPTL3 in HEK 293T cells after administration of RZ597161 in Example 5.

### EMBODIMENTS OF THE INVENTION

Hereinafter, the specific embodiments of the present disclosure are described in details. It should be understood that the specific embodiments described herein are only used to illustrate and explain the present disclosure, and are not intended to limit the present disclosure.

### Term Explanation

Herein, the term "comprising" or "including" is an open-ended expression, which includes the content specified in the present disclosure but does not exclude the content from other aspects.

Herein, the terms "optionally", "optional" or "option" generally mean that the subsequently described event or condition may but not necessarily occur, and the description includes cases where the event or condition occurs and cases where the event or condition does not occur.

Herein, the term "optionally substituted" is used to define a variable, which can be unsubstituted or substituted.

Herein, the term "unsubstituted" means that the specified group has no substituents.

Herein, the terms "substituted", "being substituted" and "substituting" are used interchangeably, and indicate that any one or more hydrogen atoms in the given structure are replaced by specific substituents (e.g., C₁₋₃ alkyl, C₁₋₃ alkoxyor halogen), provided that the normal valence of the specified atoms is not exceeded and the substitution results in a stable compound. Unless otherwise indicated, a substituted group may have a substituent at each substitutable position of the group. When more than one position in the given structural formula can be substituted by one or more substituents selected from a specific group, the substituents may be the same or different at each substitutable position.

Herein, the terms "each...independently selected from", "...each independently selected from" and "...independently selected from" are interchangeable and shall be understood in a broad sense. They can mean either that the specific options represented by the same symbols do not affect each other in different groups, or that the specific options represented by the same symbols do not affect each other in the same group.

Herein, the term "stereoisomer" refers to a compound having the same chemical structure but differing in the spatial arrangement of atoms or groups. Stereoisomers include enantiomers, diastereomers, conformers (rotamers), geometric (cis/trans) isomers, atropisomers, and the like.

Herein, the term "chiral" refers to a molecule that is non-superimposable on its mirror images; whereas "achiral" refers to a molecule that is superimposable on its mirror images.

Herein, the term "enantiomer" refers to one of two isomers of a compound that are non-superimposable mirror images of each other.

Herein, the term "diastereomer" refers to a stereoisomer having two or more chiral centers and whose molecules are not mirror images of each other. Diastereomers have different physical properties, such as melting point, boiling point, spectral properties and reactivity. The mixture of diastereomers can be separated by high-resolution analytical operations such as electrophoresis and chromatography, e.g., HPLC.

Herein, the term "conjugation" means that two or more chemical moieties each with a specific function are linked with each other through covalent linkage; correspondingly, a "conjugate" refers to a compound formed by covalent linkage between two or more chemical moieties. Further, a "drug conjugate" refers to a compound formed by covalent linking one or more chemical moieties with specific functions to an active pharmaceutical agent. Herein, sometimes, particularly in the Examples, the "drug conjugate" of the present disclosure is also referred to as "conjugate", "double-stranded oligonucleotide conjugate" or "siRNA conjugate". A drug conjugate should, be understood, depending on the context, as a general term for drug conjugates or a specific drug conjugate represented by a particular structural formula.

Herein, the term "small interfering RNA (siRNA)" refers to a double-stranded RNA of 17 to 25 nucleotides in length, comprising a sense strand and an antisense strand. By forming an RNA-induced silencing complex (RISC), siRNA mediates targeted cleavage of RNA transcripts via the RISC pathway. Specifically, the siRNA directs the sequence-specific degradation of mRNA through the known RNA interference (RNAi) process, inhibiting the translation of the mRNA into amino acids and conversion into proteins.

Herein, the term "antisense strand (also referred to as the guide strand)" comprises a region substantially complementary to a target sequence. The term "sense strand (also referred to as the passenger strand)" refers to an iRNA strand that is substantially complementary to the antisense strand. The term "substantially complementary" refers to completely complementary or at least partially complementary; for example, the antisense strand is completely complementary or at least partially complementary to the target sequence. In the case of partial complementarity, mismatches may exist in the interior or terminal regions of the molecule, wherein the most tolerated mismatches exist in the terminal regions, e.g., within 5, 4, 3 or 2 nucleotides at the 5'- and/or 3' end of the iRNA. It should be noted that the "at least partially substantially complementary" as used in connection with the antisense strand and the mRNA means that the antisense strand comprises a polynucleotide substantially complementary to a continuous portion of the mRNA of interest.

Herein, the term "substantially reverse complementary" means that there are no more than 3 base mismatches between the two nucleotide sequences concerned; "essentially reverse complementary" means that there are no more than 1 base mismatches between the two nucleotide sequences concerned; "completely reverse complementary" means that there are no base mismatches between the two nucleotide sequences concerned.

Herein, the terms "treat", "alleviate" or "ameliorate" can be used interchangeably here. These terms refer to methods of obtaining a beneficial or desired result, including but not limited to a therapeutic benefit. A "therapeutic benefit" means the eradication or amelioration of the underlying disorder being treated. Here, a therapeutic benefit is obtained by eradicating or ameliorating one or more physiological symptoms associated with the underlying disorder, such that an improvement is observed in the subject, even though the subject may still suffer from the underlying disorder.

Herein, the terms "prevent" and "prevention" can be used interchangeably, and refer to methods of obtaining a beneficial or desired result, including but not limited to a prophylactic benefit. To obtain a "prophylactic benefit", a conjugate, RNAi reagent or composition may be administered to a subject at risk of developing a particular disease, or to a subject reporting one or more physiological symptoms of the disease, even though a diagnosis of the disease may not have been made.

Herein, the term "ligand" generally refers to any compound or molecule capable of binding to a biologically active substance (such as an oligonucleotide), either covalently or by otherwise chemical means. In certain embodiments, the ligand is capable of directly or indirectly interacting with another compound, such as a receptor. The receptor that interacts with the ligand may be present on the cell surface, or alternatively may be an intracellular and/or intercellular receptor. The interaction between the ligand and the receptor may lead to a biochemical reaction, or may merely be a physical interaction or binding.

Herein, the term "administration" generally refers to introduction of a pharmaceutical preparation of the present disclosure into a body of a subject via any route of introduction or delivery. Any method known to those skill in the art for bringing a cell, organ or tissue into contact with the drug may be employed. The administration may include, but is not limited to, intravenous, intraarterial, intranasal, intraperitoneal, intramuscular, subcutaneous or oral administration. The daily dose may be divided into one, two or more doses in suitable form for administration at one, two or more times during a certain period.

Herein, the term "pharmaceutical composition" may refer to a composition for the treatment of a disease or for in vitro cell culture experiments. When used for the treatment of a disease, the term "pharmaceutical composition" generally refers to a unit dosage form, and may be prepared by any method well known in the pharmaceutical field. All such methods include the step of combining the active ingredient with excipients that constitute one or more accessory ingredients. Generally, the composition is prepared by uniformly and thoroughly combining the active siRNA with liquid excipients, finely divided solid excipients, or both.

Herein, the term "pharmaceutically acceptable" means that a substance or composition must be chemically and/or toxicologically compatible with the other components contained in the formulation and/or with the mammal being treated therewith. Preferably, the "pharmaceutically acceptable" as described in the present disclosure refers to those approved by federal regulatory agency or national governments, or listed in the United States Pharmacopeia or other generally recognized pharmacopeias for use in animals, particularly in humans.

Herein, the term "pharmaceutically acceptable excipient" may include any solvents, solid excipients, diluents, or other liquid excipients, and the like, suitable for the particular intended dosage form. Except the scope that any conventional excipient is incompatible with the siRNA of the present disclosure, such as by producing any undesirable biological effects or interacting in a deleterious manner with any other components of the pharmaceutically acceptable composition, the use thereof is also contemplated within the scope of the present disclosure.

Except the scope that any conventional excipient is incompatible with the siRNA of the present disclosure, such as by producing any undesirable biological effects or interacting in a deleterious manner with any other components of the pharmaceutically acceptable composition, the use thereof is also contemplated within the scope of the present disclosure.

### Nucleotide Analog

In a first aspect of the present disclosure, the present disclosure provides a nucleotide analog selected from the structure set forth in formula (II-i), or a stereoisomer thereof, or a pharmaceutically acceptable salt thereof: 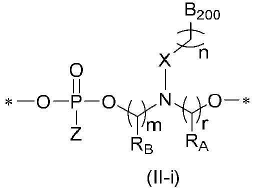
wherein, B₂₀₀ is selected from a base or a modified base;
Z is selected from hydroxyl or sulfhydryl (SH);
n is selected from 1, 2 or 3;
X is selected from 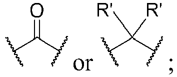 each R' is independently selected from optionally substituted C₁₋₃ alkyl, and the optionally substituted C₁₋₃ alkyl contains substituent(s) independently selected from halogen, C₁₋₃ alkoxy, hydroxyl or amino;
m is selected from 1, 2, 3 or 4;
r is selected from 1, 2, 3 or 4;
each R_{A} and each R_{B} are independently selected from H or optionally substituted C₁₋₃ alkyl; and there is at least one optionally substituted C₁₋₃ alkyl in R_{A} and R_{B}; if the optionally substituted C₁₋₃ alkyl contains substituent(s), the substituent(s) are independently selected from halogen, C₁₋₃ alkoxy, hydroxyl or amino.

Each * independently represents a site of covalent bond attachment.

In some optional embodiments of the present disclosure, each R_{A} and each R_{B} are independently selected from H or optionally substituted C₁₋₃ alkyl; and any one of each R_{A} and R_{B} is selected from optionally substituted C₁₋₃ alkyl; if the optionally substituted C₁₋₃ alkyl contains substituent(s), the substituent(s) are independently selected from halogen, C₁₋₃ alkoxy, hydroxyl or amino. For example, one R_{A} is selected from optionally substituted C₁₋₃ alkyl, and the remaining R_{A} and all R_{B} are selected from H; or, one R_{B} is selected from optionally substituted C₁₋₃ alkyl, and the remaining R_{B} and all R_{A} are selected from H.

In some optional embodiments of the present disclosure, each R_{A} and each R_{B} are independently selected from H or C₁₋₃ alkyl; and any one of each R_{A} and R_{B} is selected from C₁₋₃ alkyl. For example, one R_{A} is selected from C₁₋₃ alkyl, and the remaining R_{A} and all R_{B} are selected from H; or, one R_{B} is selected from C₁₋₃ alkyl, and the remaining R_{B} and all R_{A} are selected from H.

In some optional embodiments of the present disclosure, the C₁₋₃ alkyl is selected from methyl, ethyl, *n*-propyl and isopropyl.

In some particular embodiments of the present disclosure, the C₁₋₃ alkyl is selected from methyl.

In some optional embodiments of the present disclosure, each R_{A} and each R_{B} are independently selected from H or methyl; and any one of each R_{A} and R_{B} is selected from methyl. For example, one R_{A} is selected from methyl, and the remaining R_{A} and all R_{B} are selected from H; or, one R_{B} is selected from methyl, and the remaining R_{B} and all R_{A} are selected from H.

In some particular embodiments of the present disclosure, m is selected from 2, and r is selected from 2.

In some optional embodiments of the present disclosure, the nucleotide analog is selected from the structure set forth in formula (II-ii), or a stereoisomer thereof, or a pharmaceutically acceptable salt thereof: 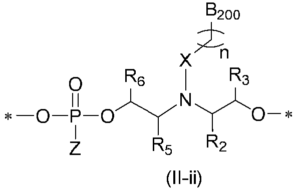
wherein, B₂₀₀ is selected from a base or a modified base;
Z is selected from hydroxyl or sulfhydryl (SH);
n is selected from 1, 2 or 3;
X is selected from 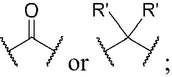 each R' is independently selected from optionally substituted C₁₋₃ alkyl, and the optionally substituted C₁₋₃ alkyl contains substituent(s) independently selected from halogen, C₁₋₃ alkoxy, hydroxyl or amino;
Each * independently represents a site of covalent bond attachment.

R₂, R₃, R₅ and R₆ are each independently selected from H or optionally substituted C₁₋₃ alkyl; and at least one of R₂, R₃, R₅ and R₆ is selected from optionally substituted C₁₋₃ alkyl; if the optionally substituted C₁₋₃ alkyl contains substituent(s), the substituent(s) are independently selected from halogen, C₁₋₃ alkoxy, hydroxyl or amino.

In some optional embodiments of the present disclosure, R₂, R₃, R₅ and R₆ are each independently selected from H or optionally substituted C₁₋₃ alkyl; and any one of R₂, R₃, R₅ and R₆ is selected from optionally substituted C₁₋₃ alkyl. For example, R₂ is selected from optionally substituted C₁₋₃ alkyl, and R₃, R₅ and R₆ are selected from H; or, R₃ is selected from optionally substituted C₁₋₃ alkyl, and R₂, R₅ and R₆ are selected from H; or, R₅ is selected from optionally substituted C₁₋₃ alkyl, and R₂, R₃ and R₆ are selected from H; or, R₆ is selected from optionally substituted C₁₋₃ alkyl, and R₂, R₃ and R₅ are selected from H.

In some optional embodiments of the present disclosure, R₂, R₃, R₅ and R₆ are each independently selected from H or C₁₋₃ alkyl; and any one of R₂, R₃, R₅ and R₆ is selected from C₁₋₃ alkyl. For example, R₂ is selected from C₁₋₃ alkyl, and R₃, R₅ and R₆ are selected from H; or, R₃ is selected from C₁₋₃ alkyl, and R₂, R₅ and R₆ are selected from H; or, R₅ is selected from C₁₋₃ alkyl, and R₂, R₃ and R₆ are selected from H; or, R₆ is selected from C₁₋₃ alkyl, and R₂, R₃ and R₅ are selected from H.

In some optional embodiments of the present disclosure, the C₁₋₃ alkyl is selected from methyl, ethyl, *n*-propyl and isopropyl.

In some specific embodiments of the present disclosure, the C₁₋₃ alkyl is selected from methyl.

In some specific embodiments of the present disclosure, R₂, R₃, R₅ and R₆ are each independently selected from H or methyl; and any one of R₂, R₃, R₅ and R₆ is selected from methyl. For example, R₂ is selected from methyl, and R₃, R₅ and R₆ are selected from H; or, R₃ is selected from methyl, and R₂, R₅ and R₆ are selected from H; or, R₅ is selected from methyl, and R₂, R₃ and R₆ are selected from H; or, R₆ is selected from methyl, and R₂, R₃ and R₅ are selected from H.

Further, the nucleotide analog has the structure set forth in formula (200), or a stereoisomer thereof, or a pharmaceutically acceptable salt thereof: 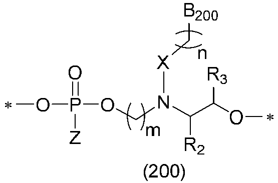
wherein, B₂₀₀ is selected from a base or a modified base;
Z is selected from hydroxyl or sulfhydryl (SH);
n is selected from 1, 2 or 3;
X is selected from 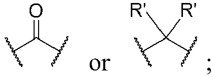 each R' is independently selected from optionally substituted C₁₋₃ alkyl, and the optionally substituted C₁₋₃ alkyl contains substituent(s) independently selected from halogen, C₁₋₃ alkoxy, hydroxyl or amino;
m is selected from 1, 2, 3 or 4;
R₂ and R₃ are independently selected from H or optionally substituted C₁₋₃ alkyl; and at least one of R₂ and R₃ is selected from optionally substituted C₁₋₃ alkyl; if the optionally substituted C₁₋₃ alkyl contains substituent(s), the substituent(s) are independently selected from halogen, C₁₋₃ alkoxy, hydroxyl or amino;
Each * independently represents a site of covalent bond attachment.

In some embodiments of the present disclosure, the base is selected from uracil U, thymine T, cytosine C, adenine A or guanine G.

In some embodiments of the present disclosure, the modified base is selected from

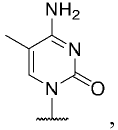

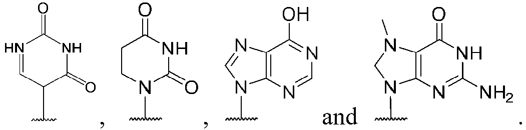

In some embodiments of the present disclosure, B₂₀₀ is selected from any one of the following structures:

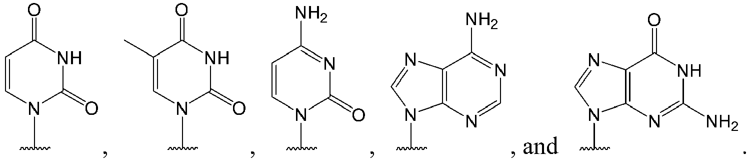

In some embodiments of the present disclosure, B₂₀₀ is selected from

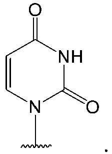

In some embodiments of the present disclosure, B₂₀₀ is selected from

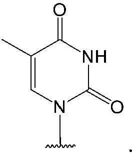

In some embodiments of the present disclosure, B₂₀₀ is selected from

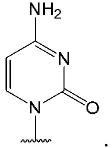

In some embodiments of the present disclosure, B₂₀₀ is selected from

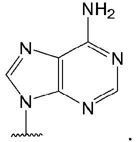

In some embodiments of the present disclosure, B₂₀₀ is selected from

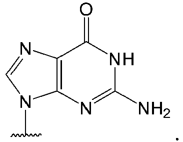

In some embodiments of the present disclosure, in formula (200), n is selected from 1 or 2.

In some embodiments of the present disclosure, n is selected from 1.

In some embodiments of the present disclosure, in formula (200), m is selected from 1, 2 or 3.

In some embodiments of the present disclosure, m is selected from 2.

In some embodiments of the present disclosure, X in the above formula is selected from

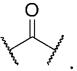

In some embodiments of the present disclosure, in formula (200), R₂ and R₃ are independently selected from H or optionally substituted C₁₋₃ alkyl; and R₂ and R₃ are not simultaneously selected from H and optionally substituted C₁₋₃ alkyl (i.e., R₂ is selected from H and R₃ is selected from optionally substituted C₁₋₃ alkyl; or, R₂ is selected from optionally substituted C₁₋₃ alkyl and R₃ is selected from H).

In some embodiments of the present disclosure, R₂ and R₃ are independently selected from H or C₁₋₃ alkyl; and R₂ and R₃ are not simultaneously selected from H and C₁₋₃ alkyl.

In some embodiments of the present disclosure, R₂ and R₃ are independently selected from H or methyl; and R₂ and R₃ are not simultaneously selected from H and methyl.

In some embodiments of the present disclosure, the nucleotide analog has the structure set forth in formula (201), or a stereoisomer thereof, or a pharmaceutically acceptable salt thereof:

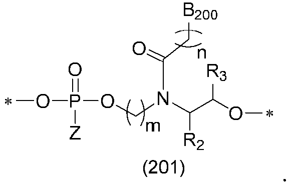

In some embodiments of the present disclosure, the nucleotide analog has any one of the following structures, or a stereoisomer thereof, or a pharmaceutically acceptable salt thereof: 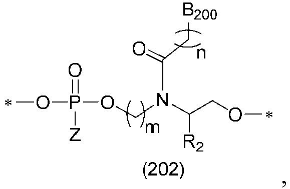 wherein R₂ is selected from optionally substituted C₁₋₃ alkyl; 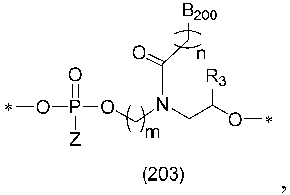 wherein R₃ is selected from optionally substituted C₁₋₃ alkyl.

In some embodiments of the present disclosure, the nucleotide analog has the structure set forth in formula (202), or a stereoisomer thereof, or a pharmaceutically acceptable salt thereof: 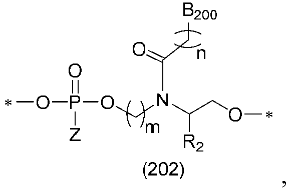 wherein R₂ is selected from optionally substituted C₁₋₃ alkyl.

In some embodiments of the present disclosure, the nucleotide analog has the structure set forth in formula (203), or a stereoisomer thereof, or a pharmaceutically acceptable salt thereof: 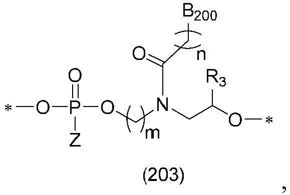 wherein R₃ is selected from optionally substituted C₁₋₃ alkyl.

In some embodiments of the present disclosure, the nucleotide analog has any one of the following structures, or a stereoisomer thereof, or a pharmaceutically acceptable salt thereof: 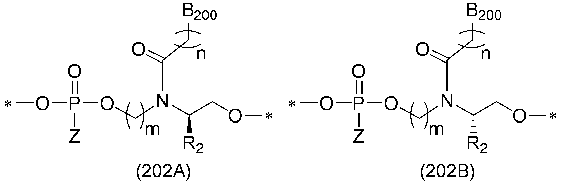 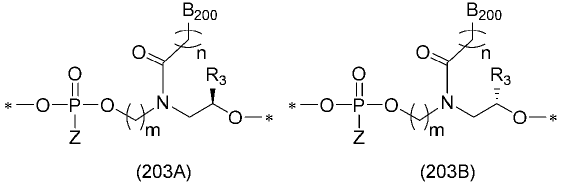 wherein R₂ and R₃ are independently selected from optionally substituted C₁₋₃ alkyl.

In some embodiments of the present disclosure, the nucleotide analog has the structure set forth in formula (202A), or a stereoisomer thereof, or a pharmaceutically acceptable salt thereof: 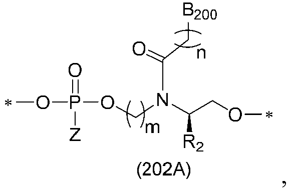 wherein R₂ is selected from optionally substituted C₁₋₃ alkyl.

In some embodiments of the present disclosure, the nucleotide analog has the structure set forth in formula (202B), or a stereoisomer thereof, or a pharmaceutically acceptable salt thereof: 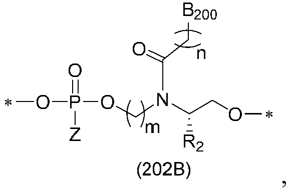 wherein R₂ is selected from optionally substituted C₁₋₃ alkyl.

In some embodiments of the present disclosure, the nucleotide analog has the structure set forth in formula (203A), or a stereoisomer thereof, or a pharmaceutically acceptable salt thereof: 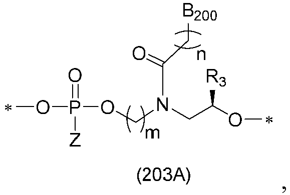 wherein R₃ is selected from optionally substituted C₁₋₃ alkyl.

In some embodiments of the present disclosure, the nucleotide analog has the structure set forth in formula (203B), or a stereoisomer thereof, or a pharmaceutically acceptable salt thereof: 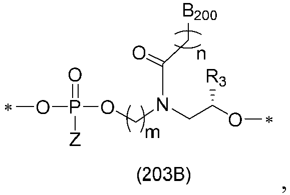 wherein R₃ is selected from optionally substituted C₁₋₃ alkyl.

In some embodiments of the present disclosure, the nucleotide analog is selected from any one of the following structures, or a stereoisomer thereof, or a pharmaceutically acceptable salt thereof:

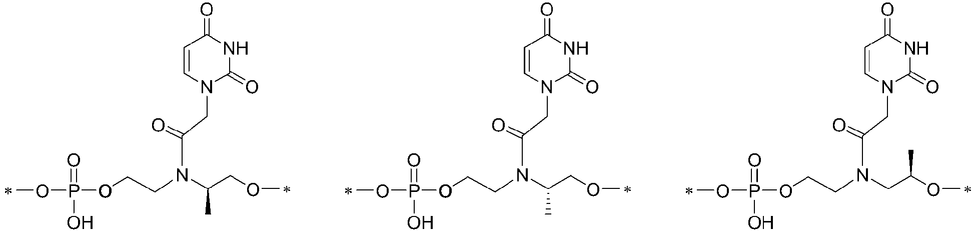

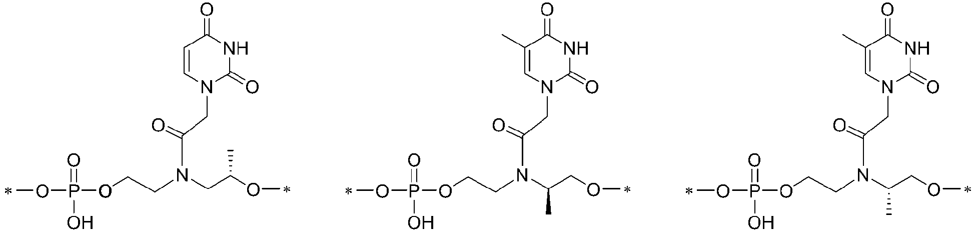

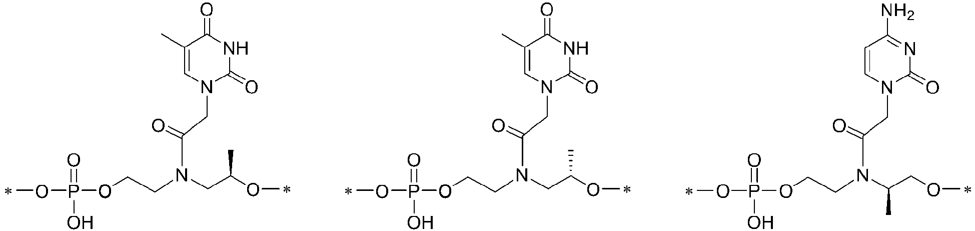

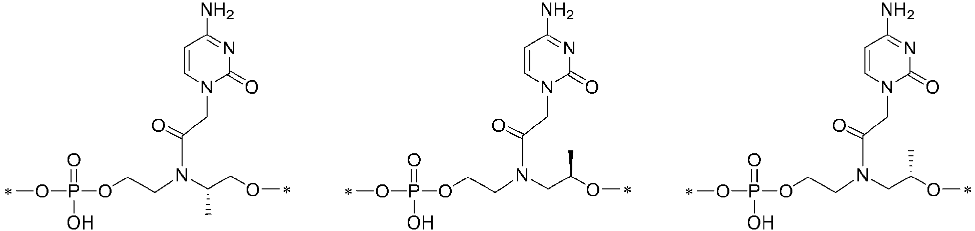

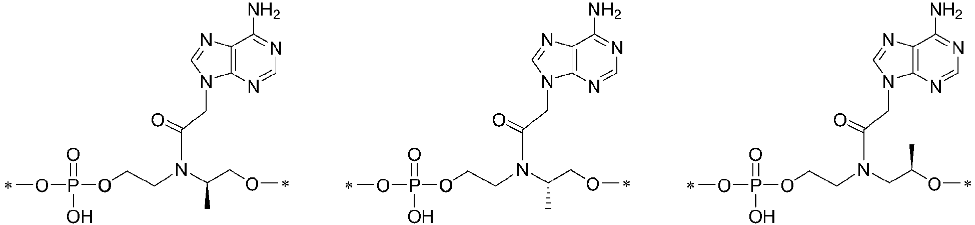

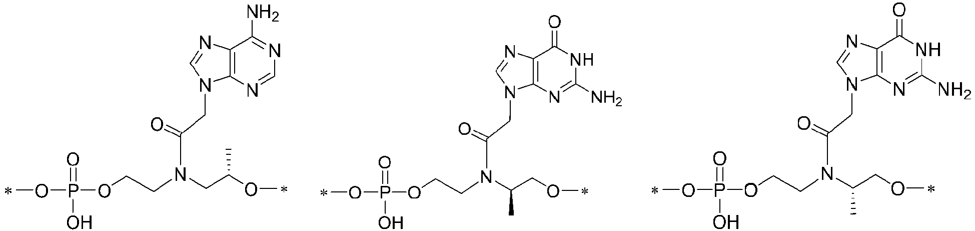

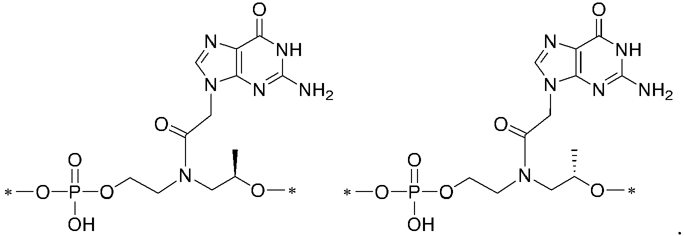

Further, the nucleotide analog has the structure set forth in formula (500), or a stereoisomer thereof, or a pharmaceutically acceptable salt thereof: 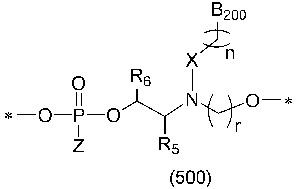
wherein B₂₀₀, Z, n, X and * are as defined above;
r is selected from 1, 2, 3 or 4;
R₅ and R₆ are independently selected from H or optionally substituted C₁₋₃ alkyl; and at least one of R₅ and R₆ is selected from optionally substituted C₁₋₃ alkyl; if the optionally substituted C₁₋₃ alkyl contains substituent(s), the substituent(s) are independently selected from halogen, C₁₋₃ alkoxy, hydroxyl or amino.

In some embodiments of the present disclosure, r is selected from 1, 2 or 3.

In some embodiments of the present disclosure, r is selected from 2.

In some embodiments of the present disclosure, R₅ and R₆ are independently selected from H or optionally substituted C₁₋₃ alkyl; and, R₅ and R₆ are not simultaneously H or optionally substituted C₁₋₃ alkyl.

In some embodiments of the present disclosure, R₅ and R₆ are independently selected from H or C₁₋₃ alkyl; and, R₅ and R₆ are not simultaneously H or C₁₋₃ alkyl.

In some embodiments of the present disclosure, R₅ and R₆ are independently selected from H or methyl; and, R₅ and R₆ are not simultaneously H or methyl.

In some embodiments of the present disclosure, the nucleotide analog has the structure set forth in formula (501), or a stereoisomer thereof, or a pharmaceutically acceptable salt thereof:

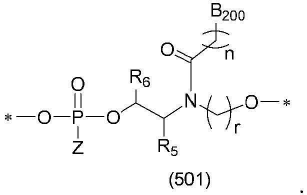

In some embodiments of the present disclosure, the nucleotide analog has any one of the following structures, or a stereoisomer thereof, or a pharmaceutically acceptable salt thereof: 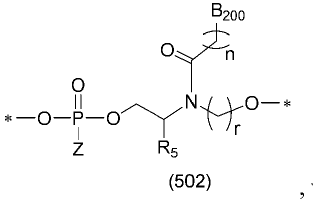 wherein R₅ is selected from optionally substituted C₁₋₃ alkyl; 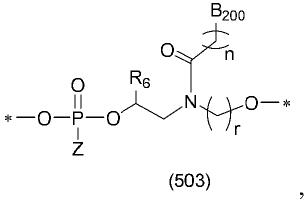 wherein R₆ is selected from optionally substituted C₁₋₃ alkyl.

In some embodiments of the present disclosure, the nucleotide analog has any one of the following structures, or a stereoisomer thereof, or a pharmaceutically acceptable salt thereof: 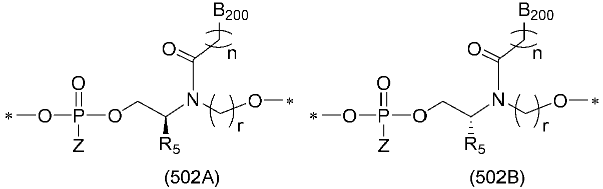 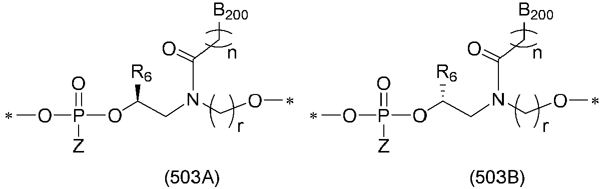 wherein R₅ and R₆ are independently selected from optionally substituted C₁₋₃ alkyl.

In some embodiments of the present disclosure, the nucleotide analog is selected from any one of the following structures, or a stereoisomer thereof, or a pharmaceutically acceptable salt thereof:

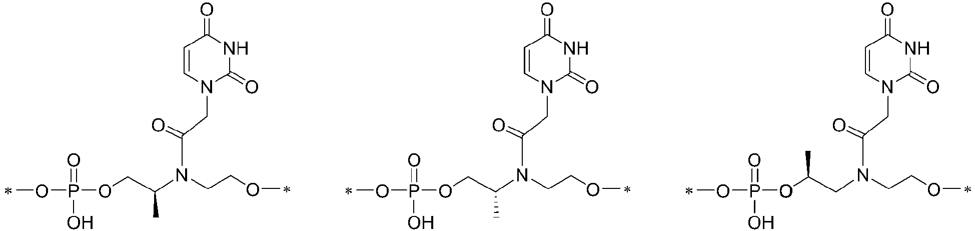

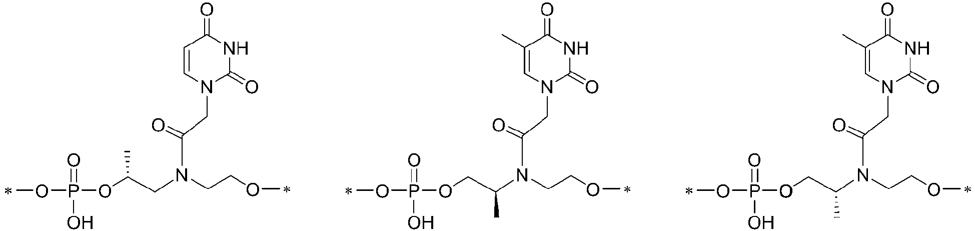

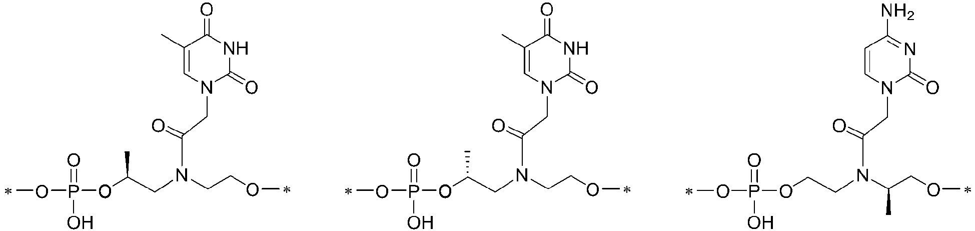

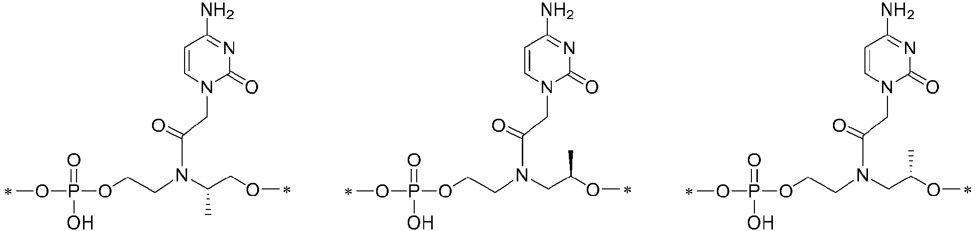

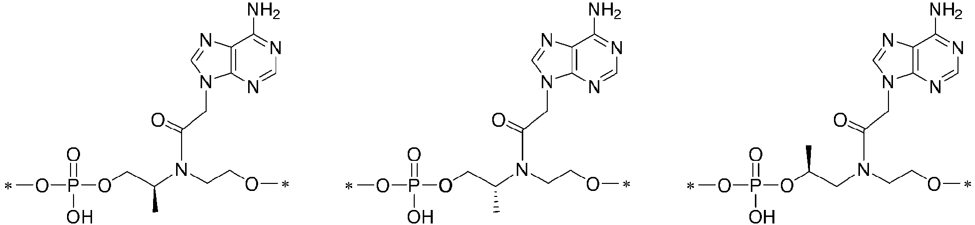

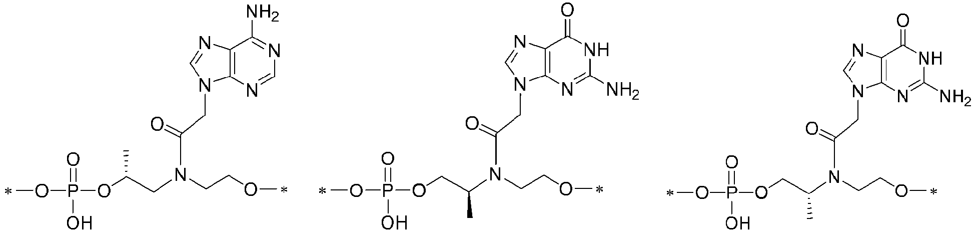

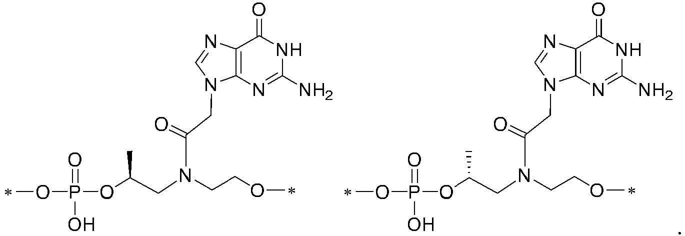

### Double-stranded Oligonucleotide

In a second aspect of the present disclosure, the present disclosure provides a double-stranded oligonucleotide comprising a sense strand and an antisense strand, each strand having 17-35 nucleotides; in the direction from the 5' end to the 3' end, at least one nucleotide at positions 2-8 of the antisense strand is substituted by the above-mentioned nucleotide analog of the present disclosure.

In some embodiments of the present disclosure, the present disclosure provides a double-stranded oligonucleotide, wherein in the direction from the 5' end to the 3' end, at least one nucleotide at positions 3-8 of the antisense strand is substituted by the nucleotide analog.

In some embodiments, in the direction from the 5' end to the 3' end, any one of the nucleotides at positions 3, 4, 5, 6, 7 and 8 of the antisense strand is substituted by the nucleotide analog.

In some embodiments, in the direction from the 5' end to the 3' end, the nucleotide at position 5, 6, 7 or 8 of the antisense strand is substituted by the nucleotide analog.

In some embodiments, in the direction from the 5' end to the 3' end, the nucleotide at position 3 of the antisense strand is substituted by the nucleotide analog.

In some embodiments, in the direction from the 5' end to the 3' end, the nucleotide at position 4 of the antisense strand is substituted by the nucleotide analog.

In some embodiments, in the direction from the 5' end to the 3' end, the nucleotide at position 5 of the antisense strand is substituted by the nucleotide analog.

In some embodiments, in the direction from the 5' end to the 3' end, the nucleotide at position 6 of the antisense strand is substituted by the nucleotide analog.

In some embodiments, in the direction from the 5' end to the 3' end, the nucleotide at position 7 of the antisense strand is substituted by the nucleotide analog.

In some embodiments, in the direction from the 5' end to the 3' end, the nucleotide at position 8 of the antisense strand is substituted by the nucleotide analog.

In some embodiments, for the double-stranded oligonucleotide provided by the present disclosure, Base in each of the nucleotide analog is the same as the base of the corresponding nucleotide it substitutes.

In some embodiments, for the double-stranded oligonucleotide provided by the present disclosure, in the nucleotide sequence of the sense strand and in the direction from the 5' end to the 3' end, the nucleotide at position 5 is selected from 2'-O-methyl modified nucleotides or 2'-O-methoxyethyl modified nucleotides, at least three nucleotides at positions 7-10 are 2'-fluoro modified nucleotides, and the nucleotides at the remaining positions are independently selected from 2'-O-methyl modified nucleotides; and/or
in some embodiments, for the double-stranded oligonucleotide provided by the present disclosure, in the nucleotide sequence of the antisense strand and in the direction from the 5' end to the 3' end, at least one nucleotide at positions 3-8 is independently selected from the nucleotide analogs, at least four nucleotides at positions 2, 6, 9, 12, 14 and 16 are selected from 2'-fluoro modified nucleotides, the nucleotide at position 15 is selected from 2'-O-methoxyethyl modified nucleotides or 2'-O-methyl modified nucleotides, and the nucleotides at the remaining positions are independently selected from 2'-O-methyl modified nucleotides; wherein, when the nucleotide at position 6 is selected from the nucleotide analogs, the nucleotides at positions 2, 9, 12, 14 and 16 are selected from 2'-fluoro modified nucleotides; and, when the nucleotide at position 6 is selected from 2'-fluoro modified nucleotides, at least one nucleotide at positions 3-5 and 7-8 is independently selected from the nucleotide analogs;
in some embodiments, for the double-stranded oligonucleotide provided by the present disclosure, in the nucleotide sequence of the sense strand and in the direction from the 5' end to the 3' end, at least three nucleotides at positions 7-10 are 2'-fluoro modified nucleotides, and the nucleotides at the remaining positions are independently selected from 2'-O-methyl modified nucleotides.

In some embodiments, for the double-stranded oligonucleotide provided by the present disclosure, in the nucleotide sequence of the antisense strand and in the direction from the 5' end to the 3' end, at least one nucleotide at positions 3-8 is independently selected from the nucleotide analogs, at least four nucleotides at positions 2, 6, 9, 12, 14 and 16 are selected from 2'-fluoro modified nucleotides, the nucleotide at position 15 is selected from 2'-O-methoxyethyl modified nucleotides or 2'-O-methyl modified nucleotides, and the nucleotides at the remaining positions are independently selected from 2'-O-methyl modified nucleotides; wherein, when the nucleotide at position 6 is selected from the nucleotide analogs, the nucleotides at positions 2, 9, 12, 14 and 16 are selected from 2'-fluoro modified nucleotides; and, when the nucleotide at position 6 is selected from 2'-fluoro modified nucleotides, at least one nucleotide at positions 3-5 and 7-8 is independently selected from the nucleotide analogs.

In some embodiments, for the double-stranded oligonucleotide provided by the present disclosure, in the nucleotide sequence of the sense strand and in the direction from the 5' end to the 3' end, at least three nucleotides at positions 7-10 are 2'-fluoro modified nucleotides, and the nucleotides at the remaining positions are independently selected from 2'-O-methyl modified nucleotides.

In some embodiments, for the double-stranded oligonucleotide provided by the present disclosure, in the nucleotide sequence of the antisense strand and in the direction from the 5' end to the 3' end, at least one nucleotide at positions 3-8 is independently selected from the nucleotide analogs, at least three nucleotides at positions 2, 6, 14 and 16 are selected from 2'-fluoro modified nucleotides, and the nucleotide at position 9 or 12 is selected from 2'-fluoro modified nucleotides, the nucleotide at position 15 is selected from 2'-O-methoxyethyl modified nucleotides or 2'-O-methyl modified nucleotides, and the nucleotides at the remaining positions are independently selected from 2'-O-methyl modified nucleotides; wherein, when the nucleotide at position 6 is selected from the nucleotide analogs, the nucleotides at positions 2, 9, 12, 14 and 16 are selected from 2'-fluoro modified nucleotides; and, when the nucleotide at position 6 is selected from 2'-fluoro modified nucleotides, at least one nucleotide at positions 3-5 and 7-8 is independently selected from the nucleotide analogs.

In some embodiments, for the double-stranded oligonucleotide provided by the present disclosure, the modifications of the sense and antisense strands in the double-stranded oligonucleotide are selected from one of the following (1) to (24):
(1) in the nucleotide sequence of the sense strand and in the direction from the 5' end to the 3' end, the nucleotides at positions 7-10 are independently selected from 2'-fluoro modified nucleotides, and the nucleotides at the remaining positions are independently selected from 2'-O-methyl modified nucleotides;
   in the nucleotide sequence of the antisense strand and in the direction from the 5' end to the 3' end, the nucleotide at position 3 is selected from the nucleotide analogs, the nucleotides at positions 2, 6, 9, 14 and 16 are independently selected from 2'-fluoro modified nucleotides, and the nucleotides at the remaining positions are independently selected from 2'-O-methyl modified nucleotides;
(2) in the nucleotide sequence of the sense strand and in the direction from the 5' end to the 3' end, the nucleotides at positions 7-10 are independently selected from 2'-fluoro modified nucleotides, and the nucleotides at the remaining positions are independently selected from 2'-O-methyl modified nucleotides;
   in the nucleotide sequence of the antisense strand and in the direction from the 5' end to the 3' end, the nucleotide at position 4 is selected from the nucleotide analogs, the nucleotides at positions 2, 6, 9, 14 and 16 are independently selected from 2'-fluoro modified nucleotides, and the nucleotides at the remaining positions are independently selected from 2'-O-methyl modified nucleotides;
(3) in the nucleotide sequence of the sense strand and in the direction from the 5' end to the 3' end, the nucleotides at positions 7-10 are independently selected from 2'-fluoro modified nucleotides, and the nucleotides at the remaining positions are independently selected from 2'-O-methyl modified nucleotides;
   in the nucleotide sequence of the antisense strand and in the direction from the 5' end to the 3' end, the nucleotide at position 5 is selected from the nucleotide analogs, the nucleotides at positions 2, 6, 9, 14 and 16 are independently selected from 2'-fluoro modified nucleotides, and the nucleotides at the remaining positions are independently selected from 2'-O-methyl modified nucleotides;
(4) in the nucleotide sequence of the sense strand and in the direction from the 5' end to the 3' end, the nucleotides at positions 7-10 are independently selected from 2'-fluoro modified nucleotides, and the nucleotides at the remaining positions are independently selected from 2'-O-methyl modified nucleotides;
   in the nucleotide sequence of the antisense strand and in the direction from the 5' end to the 3' end, the nucleotide at position 6 is selected from the nucleotide analogs, the nucleotides at positions 2, 9, 14 and 16 are independently selected from 2'-fluoro modified nucleotides, and the nucleotides at the remaining positions are independently selected from 2'-O-methyl modified nucleotides;
(5) in the nucleotide sequence of the sense strand and in the direction from the 5' end to the 3' end, the nucleotides at positions 7-10 are independently selected from 2'-fluoro modified nucleotides, and the nucleotides at the remaining positions are independently selected from 2'-O-methyl modified nucleotides;
   in the nucleotide sequence of the antisense strand and in the direction from the 5' end to the 3' end, the nucleotide at position 7 is selected from the nucleotide analogs, the nucleotides at positions 2, 6, 9, 14 and 16 are independently selected from 2'-fluoro modified nucleotides, and the nucleotides at the remaining positions are independently selected from 2'-O-methyl modified nucleotides;
(6) in the nucleotide sequence of the sense strand and in the direction from the 5' end to the 3' end, the nucleotides at positions 7-10 are independently selected from 2'-fluoro modified nucleotides, and the nucleotides at the remaining positions are independently selected from 2'-O-methyl modified nucleotides;
   in the nucleotide sequence of the antisense strand and in the direction from the 5' end to the 3' end, the nucleotide at position 8 is selected from the nucleotide analogs, the nucleotides at positions 2, 6, 9, 14 and 16 are independently selected from 2'-fluoro modified nucleotides, and the nucleotides at the remaining positions are independently selected from 2'-O-methyl modified nucleotides;
(7) in the nucleotide sequence of the sense strand and in the direction from the 5' end to the 3' end, the nucleotides at positions 7-10 are independently selected from 2'-fluoro modified nucleotides, and the nucleotides at the remaining positions are independently selected from 2'-O-methyl modified nucleotides;
   in the nucleotide sequence of the antisense strand and in the direction from the 5' end to the 3' end, the nucleotide at position 3 is selected from the nucleotide analogs, the nucleotides at positions 2, 6, 9, 14 and 16 are independently selected from 2'-fluoro modified nucleotides, the nucleotide at position 15 is selected from 2'-O-methoxyethyl modified nucleotides, and the nucleotides at the remaining positions are independently selected from 2'-O-methyl modified nucleotides;
(8) in the nucleotide sequence of the sense strand and in the direction from the 5' end to the 3' end, the nucleotides at positions 7-10 are independently selected from 2'-fluoro modified nucleotides, and the nucleotides at the remaining positions are independently selected from 2'-O-methyl modified nucleotides;
   in the nucleotide sequence of the antisense strand and in the direction from the 5' end to the 3' end, the nucleotide at position 4 is selected from the nucleotide analogs, the nucleotides at positions 2, 6, 9, 14 and 16 are independently selected from 2'-fluoro modified nucleotides, the nucleotide at position 15 is selected from 2'-O-methoxyethyl modified nucleotides, and the nucleotides at the remaining positions are independently selected from 2'-O-methyl modified nucleotides;
(9) in the nucleotide sequence of the sense strand and in the direction from the 5' end to the 3' end, the nucleotides at positions 7-10 are independently selected from 2'-fluoro modified nucleotides, the nucleotide at position 15 is selected from 2'-O-methoxyethyl modified nucleotides, and the nucleotides at the remaining positions are independently selected from 2'-O-methyl modified nucleotides;
   in the nucleotide sequence of the antisense strand and in the direction from the 5' end to the 3' end, the nucleotide at position 5 is selected from the nucleotide analogs, the nucleotides at positions 2, 6, 9, 14 and 16 are independently selected from 2'-fluoro modified nucleotides, the nucleotide at position 15 is selected from 2'-O-methoxyethyl modified nucleotides, and the nucleotides at the remaining positions are independently selected from 2'-O-methyl modified nucleotides;
(10) in the nucleotide sequence of the sense strand and in the direction from the 5' end to the 3' end, the nucleotides at positions 7-10 are independently selected from 2'-fluoro modified nucleotides, the nucleotide at position 15 is selected from 2'-O-methoxyethyl modified nucleotides, and the nucleotides at the remaining positions are independently selected from 2'-O-methyl modified nucleotides;
   in the nucleotide sequence of the antisense strand and in the direction from the 5' end to the 3' end, the nucleotide at position 6 is selected from the nucleotide analogs, the nucleotides at positions 2, 9, 14 and 16 are independently selected from 2'-fluoro modified nucleotides, the nucleotide at position 15 is selected from 2'-O-methoxyethyl modified nucleotides, and the nucleotides at the remaining positions are independently selected from 2'-O-methyl modified nucleotides;
(11) in the nucleotide sequence of the sense strand and in the direction from the 5' end to the 3' end, the nucleotides at positions 7-10 are independently selected from 2'-fluoro modified nucleotides, the nucleotide at position 15 is selected from 2'-O-methoxyethyl modified nucleotides, and the nucleotides at the remaining positions are independently selected from 2'-O-methyl modified nucleotides;
   in the nucleotide sequence of the antisense strand and in the direction from the 5' end to the 3' end, the nucleotide at position 7 is selected from the nucleotide analogs, the nucleotides at positions 2, 6, 9, 14 and 16 are independently selected from 2'-fluoro modified nucleotides, the nucleotide at position 15 is selected from 2'-O-methoxyethyl modified nucleotides, and the nucleotides at the remaining positions are independently selected from 2'-O-methyl modified nucleotides;
(12) in the nucleotide sequence of the sense strand and in the direction from the 5' end to the 3' end, the nucleotides at positions 7-10 are independently selected from 2'-fluoro modified nucleotides, and the nucleotides at the remaining positions are independently selected from 2'-O-methyl modified nucleotides;
   in the nucleotide sequence of the antisense strand and in the direction from the 5' end to the 3' end, the nucleotide at position 8 is selected from the nucleotide analogs, the nucleotides at positions 2, 6, 9, 14 and 16 are independently selected from 2'-fluoro modified nucleotides, the nucleotide at position 15 is selected from 2'-O-methoxyethyl modified nucleotides, and the nucleotides at the remaining positions are independently selected from 2'-O-methyl modified nucleotides;
(13) in the nucleotide sequence of the sense strand and in the direction from the 5' end to the 3' end, the nucleotides at positions 7-10 are independently selected from 2'-fluoro modified nucleotides, and the nucleotides at the remaining positions are independently selected from 2'-O-methyl modified nucleotides;
   in the nucleotide sequence of the antisense strand and in the direction from the 5' end to the 3' end, the nucleotide at position 3 is selected from the nucleotide analogs, the nucleotides at positions 2, 6, 12, 14 and 16 are independently selected from 2'-fluoro modified nucleotides, and the nucleotides at the remaining positions are independently selected from 2'-O-methyl modified nucleotides;
(14) in the nucleotide sequence of the sense strand and in the direction from the 5' end to the 3' end, the nucleotides at positions 7-10 are independently selected from 2'-fluoro modified nucleotides, and the nucleotides at the remaining positions are independently selected from 2'-O-methyl modified nucleotides;
   in the nucleotide sequence of the antisense strand and in the direction from the 5' end to the 3' end, the nucleotide at position 4 is selected from the nucleotide analogs, the nucleotides at positions 2, 6, 12, 14 and 16 are independently selected from 2'-fluoro modified nucleotides, and the nucleotides at the remaining positions are independently selected from 2'-O-methyl modified nucleotides;
(15) in the nucleotide sequence of the sense strand and in the direction from the 5' end to the 3' end, the nucleotides at positions 7-10 are independently selected from 2'-fluoro modified nucleotides, and the nucleotides at the remaining positions are independently selected from 2'-O-methyl modified nucleotides;
   in the nucleotide sequence of the antisense strand and in the direction from the 5' end to the 3' end, the nucleotide at position 5 is selected from the nucleotide analogs, the nucleotides at positions 2, 6, 12, 14 and 16 are independently selected from 2'-fluoro modified nucleotides, and the nucleotides at the remaining positions are independently selected from 2'-O-methyl modified nucleotides;
(16) in the nucleotide sequence of the sense strand and in the direction from the 5' end to the 3' end, the nucleotides at positions 7-10 are independently selected from 2'-fluoro modified nucleotides, and the nucleotides at the remaining positions are independently selected from 2'-O-methyl modified nucleotides;
   in the nucleotide sequence of the antisense strand and in the direction from the 5' end to the 3' end, the nucleotide at position 6 is selected from the nucleotide analogs, the nucleotides at positions 2, 12, 14 and 16 are independently selected from 2'-fluoro modified nucleotides, and the nucleotides at the remaining positions are independently selected from 2'-O-methyl modified nucleotides;
(17) in the nucleotide sequence of the sense strand and in the direction from the 5' end to the 3' end, the nucleotides at positions 7-10 are independently selected from 2'-fluoro modified nucleotides, and the nucleotides at the remaining positions are independently selected from 2'-O-methyl modified nucleotides;
   in the nucleotide sequence of the antisense strand and in the direction from the 5' end to the 3' end, the nucleotide at position 7 is selected from the nucleotide analogs, the nucleotides at positions 2, 6, 12, 14 and 16 are independently selected from 2'-fluoro modified nucleotides, and the nucleotides at the remaining positions are independently selected from 2'-O-methyl modified nucleotides;
(18) in the nucleotide sequence of the sense strand and in the direction from the 5' end to the 3' end, the nucleotides at positions 7-10 are independently selected from 2'-fluoro modified nucleotides, and the nucleotides at the remaining positions are independently selected from 2'-O-methyl modified nucleotides;
   in the nucleotide sequence of the antisense strand and in the direction from the 5' end to the 3' end, the nucleotide at position 8 is selected from the nucleotide analogs, the nucleotides at positions 2, 6, 12, 14 and 16 are independently selected from 2'-fluoro modified nucleotides, and the nucleotides at the remaining positions are independently selected from 2'-O-methyl modified nucleotides;
(19) in the nucleotide sequence of the sense strand and in the direction from the 5' end to the 3' end, the nucleotides at positions 7-10 are independently selected from 2'-fluoro modified nucleotides, and the nucleotides at the remaining positions are independently selected from 2'-O-methyl modified nucleotides;
   in the nucleotide sequence of the antisense strand and in the direction from the 5' end to the 3' end, the nucleotide at position 3 is selected from the nucleotide analogs, the nucleotides at positions 2, 6, 12, 14 and 16 are independently selected from 2'-fluoro modified nucleotides, the nucleotide at position 15 is selected from 2'-O-methoxyethyl modified nucleotides, and the nucleotides at the remaining positions are independently selected from 2'-O-methyl modified nucleotides;
(20) in the nucleotide sequence of the sense strand and in the direction from the 5' end to the 3' end, the nucleotides at positions 7-10 are independently selected from 2'-fluoro modified nucleotides, and the nucleotides at the remaining positions are independently selected from 2'-O-methyl modified nucleotides;
   in the nucleotide sequence of the antisense strand and in the direction from the 5' end to the 3' end, the nucleotide at position 4 is selected from the nucleotide analogs, the nucleotides at positions 2, 6, 12, 14 and 16 are independently selected from 2'-fluoro modified nucleotides, the nucleotide at position 15 is selected from 2'-O-methoxyethyl modified nucleotides, and the nucleotides at the remaining positions are independently selected from 2'-O-methyl modified nucleotides;
(21) in the nucleotide sequence of the sense strand and in the direction from the 5' end to the 3' end, the nucleotides at positions 7-10 are independently selected from 2'-fluoro modified nucleotides, the nucleotide at position 15 is selected from 2'-O-methoxyethyl modified nucleotides, and the nucleotides at the remaining positions are independently selected from 2'-O-methyl modified nucleotides;
   in the nucleotide sequence of the antisense strand and in the direction from the 5' end to the 3' end, the nucleotide at position 5 is selected from the nucleotide analogs, the nucleotides at positions 2, 6, 12, 14 and 16 are independently selected from 2'-fluoro modified nucleotides, the nucleotide at position 15 is selected from 2'-O-methoxyethyl modified nucleotides, and the nucleotides at the remaining positions are independently selected from 2'-O-methyl modified nucleotides;
(22) in the nucleotide sequence of the sense strand and in the direction from the 5' end to the 3' end, the nucleotides at positions 7-10 are independently selected from 2'-fluoro modified nucleotides, the nucleotide at position 15 is selected from 2'-O-methoxyethyl modified nucleotides, and the nucleotides at the remaining positions are independently selected from 2'-O-methyl modified nucleotides;
   in the nucleotide sequence of the antisense strand and in the direction from the 5' end to the 3' end, the nucleotide at position 6 is selected from the nucleotide analogs, the nucleotides at positions 2, 12, 14 and 16 are independently selected from 2'-fluoro modified nucleotides, the nucleotide at position 15 is selected from 2'-O-methoxyethyl modified nucleotides, and the nucleotides at the remaining positions are independently selected from 2'-O-methyl modified nucleotides;
(23) in the nucleotide sequence of the sense strand and in the direction from the 5' end to the 3' end, the nucleotides at positions 7-10 are independently selected from 2'-fluoro modified nucleotides, the nucleotide at position 15 is selected from 2'-O-methoxyethyl modified nucleotides, and the nucleotides at the remaining positions are independently selected from 2'-O-methyl modified nucleotides;
   in the nucleotide sequence of the antisense strand and in the direction from the 5' end to the 3' end, the nucleotide at position 7 is selected from the nucleotide analogs, the nucleotides at positions 2, 6, 12, 14 and 16 are independently selected from 2'-fluoro modified nucleotides, the nucleotide at position 15 is selected from 2'-O-methoxyethyl modified nucleotides, and the nucleotides at the remaining positions are independently selected from 2'-O-methyl modified nucleotides;
(24) in the nucleotide sequence of the sense strand and in the direction from the 5' end to the 3' end, the nucleotides at positions 7-10 are independently selected from 2'-fluoro modified nucleotides, and the nucleotides at the remaining positions are independently selected from 2'-O-methyl modified nucleotides;
   in the nucleotide sequence of the antisense strand and in the direction from the 5' end to the 3' end, the nucleotide at position 8 is selected from the nucleotide analogs, the nucleotides at positions 2, 6, 12, 14 and 16 are independently selected from 2'-fluoro modified nucleotides, the nucleotide at position 15 is selected from 2'-O-methoxyethyl modified nucleotides, and the nucleotides at the remaining positions are independently selected from 2'-O-methyl modified nucleotides.

In some embodiments of the present disclosure, in the double-stranded oligonucleotide provided by the present disclosure, the double-stranded oligonucleotide is selected from siRNA.

In some embodiments of the present disclosure, in the double-stranded oligonucleotide provided by the present disclosure, at least one of the phosphate ester groups in the phosphate-sugar backbone of at least one single strand of the sense and antisense strands is a phosphate ester group having a modification group. In some embodiments, the phosphate ester group having a modification group is a phosphorothioate group formed by substituting at least one oxygen atom in the phosphodiester bond of the phosphate ester group with a sulfur atom. In some embodiments, in the double-stranded oligonucleotide, the phosphate ester group having a modification group is present at at least one internucleotide position selected from the group consisting of the following positions:
between the 1st and 2nd nucleotides from the 5' terminal end of the sense strand;
between the 2nd and 3rd nucleotides from the 5' terminal end of the sense strand;
between the 1st and 2nd nucleotides from the 3' terminal end of the sense strand;
between the 2nd and 3rd nucleotides from the 3' terminal end of the sense strand;
between the 1st and 2nd nucleotides from the 5' terminal end of the antisense strand;
between the 2nd and 3rd nucleotides from the 5' terminal end of the antisense strand;
between the 1st and 2nd nucleotides from the 3' terminal end of the antisense strand; and between the 2nd and 3rd nucleotides from the 3' terminal end of the antisense strand.

### Double-Stranded Oligonucleotide Conjugate (siRNA Conjugate)

In a third aspect of the present disclosure, the present disclosure provides a double-stranded oligonucleotide conjugate, comprising the double-stranded oligonucleotide described above in the present disclosure, and one or more ligands capable of binding to cell surface receptors.

In some embodiments of the present disclosure, the ligand is selected from asialoglycoprotein receptor ligands (ASGPR ligands).

In some embodiments of the present disclosure, the ASGPR ligands include galactose, galactose derivatives or galactose clusters.

In some embodiments of the present disclosure, the galactose derivatives include those having an affinity for the asialoglycoprotein receptor equal to or exceeding that of galactose.

In some embodiments of the present disclosure, the galactose cluster comprises a molecule having 2-4 terminal galactoses and/or galactose derivatives.

In some embodiments of the present disclosure, the galactose derivatives are selected from galactosamine, N-formylgalactosamine, N-acetylgalactosamine, N-propionylgalactosamine, N-n-butyrylgalactosamine and N-isobutyrylgalactosamine.

In some embodiments of the present disclosure, the number of the ligands is selected from one, and one said ligand is conjugated to the 3' end of the sense strand of the double-stranded oligonucleotide.

In some embodiments of the present disclosure, in the conjugate described in the present disclosure, each said ligand is selected from the structure set forth in formula (300), or a stereoisomer thereof, or a pharmaceutically acceptable salt thereof: 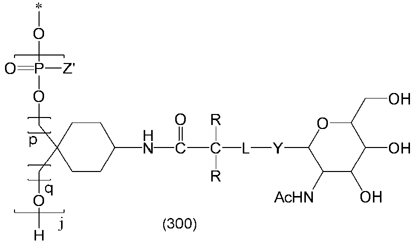
wherein, * represents the site of covalent attachment of the ligand to the sense strand or the antisense strand;
j is selected from 1, 2, 3 or 4;
each Z' is independently selected from hydroxyl or sulfhydryl;
each p is independently selected from 1, 2 or 3;
each q is independently selected from 1, 2 or 3;
each R is independently selected from H, optionally substituted C₁₋₆ alkyl or optionally substituted C₁₋₆ alkoxy;
each L is independently selected from optionally substituted C₂₋₂₀ alkylene or 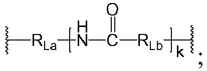 R_{La} and R_{Lb} are independently selected from optionally substituted C₁₋₁₀ alkylene, and k is selected from 1, 2, 3, 4 or 5;
each Y is independently selected from O, S or NH.

In some embodiments of the present disclosure, m is selected from 1, 2 or 3;

In some embodiments of the present disclosure, m is selected from 3.

In some embodiments of the present disclosure, Z' is selected from hydroxyl.

In some embodiments of the present disclosure, p is selected from 1.

In some embodiments of the present disclosure, q is selected from 1.

In some embodiments of the present disclosure, R is selected from H.

In some embodiments of the present disclosure, each L is independently selected from an optionally substituted C₂₋₁₀ alkylene or 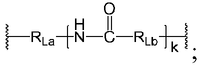 wherein R_{La} and R_{Lb} are independently selected from optionally substituted C₁₋₁₀ alkylene, and k is selected from 1, 2 or 3.

In some embodiments of the present disclosure, k is selected from 1.

In some embodiments of the present disclosure, each L is independently selected from

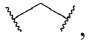

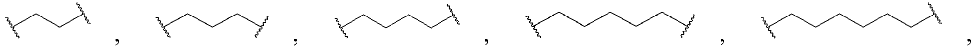

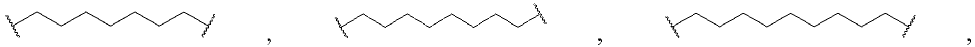

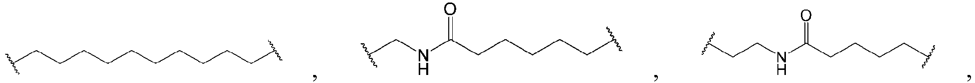

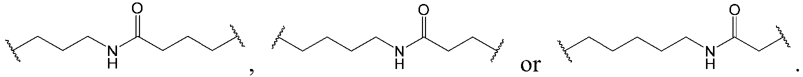

In some embodiments of the present disclosure, each L is independently selected from

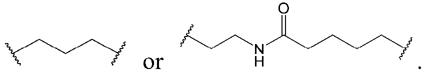

In some embodiments of the present disclosure, each L is independently selected from

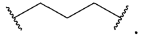

In some embodiments of the present disclosure, each L is independently selected from

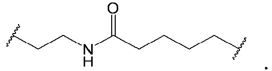

In some embodiments of the present disclosure, Y is selected from O.

In some embodiments of the present disclosure, for the conjugate described in the present disclosure, each of the ligands is independently selected from the structure set forth in formula (302), or a stereoisomer thereof, or a pharmaceutically acceptable salt thereof:

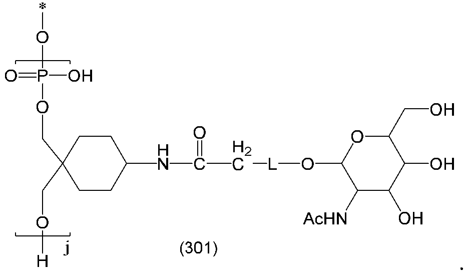

In some embodiments of the present disclosure, for the conjugate described in the present disclosure, each of the ligand is independently selected from any one of the following structures, or a stereoisomer thereof, or a pharmaceutically acceptable salt thereof:

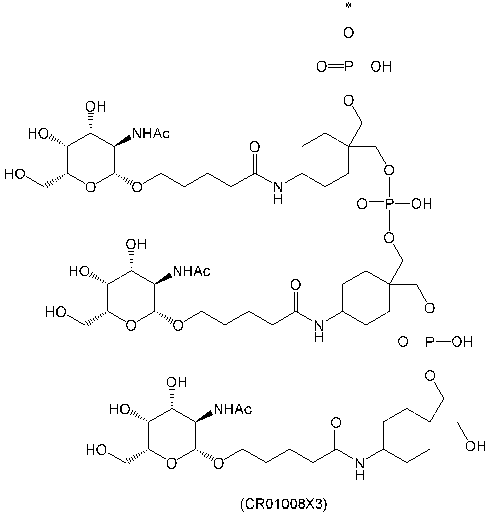

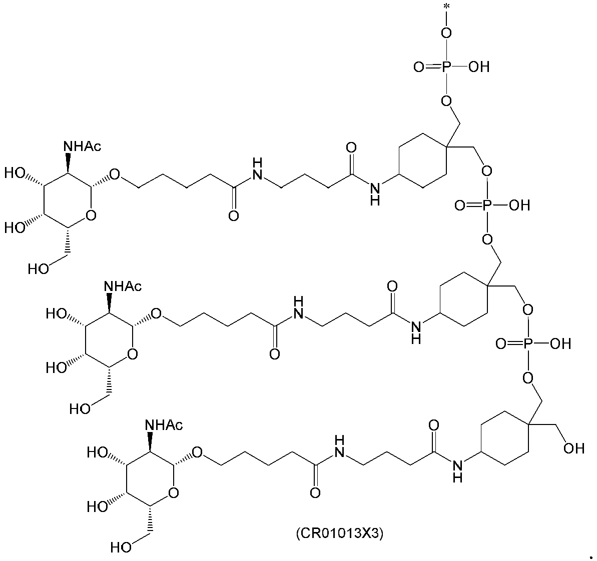

In some embodiments of the present disclosure, for the conjugate described in the present disclosure, each of the ligand is independently selected from the structure set forth in formula (CR01008×3), or a stereoisomer thereof, or a pharmaceutically acceptable salt thereof:

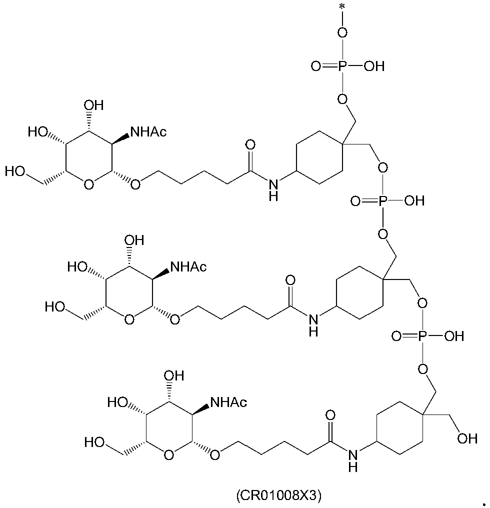

### Composition

In a fourth aspect of the present disclosure, the present disclosure provides a composition, comprising the double-stranded oligonucleotide and/or the double-stranded oligonucleotide conjugate described in the present disclosure.

### Pharmaceutical Composition

In a fifth aspect of the present disclosure, the present disclosure also provides a pharmaceutical composition, comprising the double-stranded oligonucleotide, and/or the double-stranded oligonucleotide conjugate, and/or the composition described in the present disclosure.

In some embodiments of the present disclosure, the pharmaceutical composition also comprises a pharmaceutically acceptable excipient or adjuvant.

The excipient or adjuvant may be one or more of various formulations or compounds conventionally used in the art. For example, the pharmaceutically acceptable excipient or adjuvant includes at least one of a pH buffer, a protective agent and an osmotic pressure regulator.

The pH buffer may be selected from a tris(hydroxymethyl)aminomethane hydrochloride buffer with a pH value of 7.5-8.5 and/or a phosphate buffer with a pH value of 5.5-8.5, for example, a phosphate buffer with a pH value of 5.5-8.5.

The protective agent may be at least one of inositol, sorbitol, sucrose, trehalose, mannose, maltose, lactose and glucose. Based on the total weight of the pharmaceutical composition, the content of the protective agent may be 0.01-30% by weight.

The osmotic pressure regulator may be sodium chloride and/or potassium chloride. The content of the osmotic pressure regulator is such that the osmotic pressure of the pharmaceutical composition is 200-700 milliosmoles/kilogram (mOsm/kg). The skilled in the art can easily determine the content of the osmotic pressure regulator according to the required osmotic pressure. In some embodiments, the dosage of the preparation made from the pharmaceutical composition will be adjusted according to different administration routes during administration.

In some embodiments of the present disclosure, the pharmaceutical composition may be a liquid preparation, such as an injection; it may also be a lyophilized powder for injection, which is mixed with liquid excipient(s) during administration to prepare a liquid preparation. The liquid preparation may be used for, but is not limited to, administration by subcutaneous, intramuscular or intravenous injection, and may also be administered by, but is not limited to, spray delivery to the lung, or spray delivery via the lung to other organ tissues (such as the liver); or the pharmaceutical composition can be delivered through oropharyngeal inhalation or nasal administration.

### Nucleoside Analog

In a sixth aspect of the present disclosure, the present disclosure provides a nucleoside analog selected from the structure set forth in formula (I-i), or a stereoisomer thereof, or a pharmaceutically acceptable salt thereof: 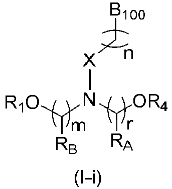
wherein B₁₀₀ is selected from a base or a modified base; if B₁₀₀ comprises amino, the amino is protected by an amino protecting group;
n is selected from 1, 2 or 3;
X is selected from 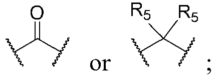 each R₅ is independently selected from optionally substituted C₁₋₃ alkyl, and the optionally substituted C₁₋₃ alkyl contains substituent(s) independently selected from halogen, C₁₋₃ alkoxy, hydroxyl or amino;
R₁ is selected from H or a hydroxyl protecting group;
R₄ is selected from H or 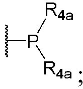 each R₄ₐ is independently selected from 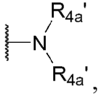 or C₁₋₆ alkoxy comprising a cyano substituent, and at least one R₄ₐ is selected from 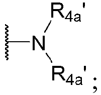 each R₄ₐ' is independently selected from optionally substituted C₁₋₆ alkyl;
m is selected from 1, 2, 3 or 4;
r is selected from 1, 2, 3 or 4;
each R_{A} and each R_{B} are independently selected from H or optionally substituted C₁₋₃ alkyl; and at least one of each R_{A} and each R_{B} is selected from optionally substituted C₁₋₃ alkyl; if the optionally substituted C₁₋₃ alkyl contains substituent(s), the substituent(s) are independently selected from halogen, C₁₋₃ alkoxy, hydroxyl or amino.

In some optional embodiments of the present disclosure, each R_{A} and each R_{B} are independently selected from H or optionally substituted C₁₋₃ alkyl; and any one of each R_{A} and R_{B} is selected from optionally substituted C₁₋₃ alkyl; if the optionally substituted C₁₋₃ alkyl contains substituent(s), the substituent(s) are independently selected from halogen, C₁₋₃ alkoxy, hydroxyl or amino. For example, one R_{A} is selected from optionally substituted C₁₋₃ alkyl, and the remaining R_{A} and all R_{B} are selected from H; or, one R_{B} is selected from optionally substituted C₁₋₃ alkyl, and the remaining R_{B} and all R_{A} are selected from H.

In some specific embodiments of the present disclosure, m is selected from 2 and r is selected from 2.

In some specific embodiments of the present disclosure, the nucleoside analog is selected from the structure set forth in formula (I-ii), or a stereoisomer thereof, or a pharmaceutically acceptable salt thereof: 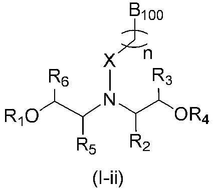 wherein, R₂, R₃, R₅ and R₆ are each independently selected from H or optionally substituted C₁₋₃ alkyl; and at least one of R₂, R₃, R₅ and R₆ is selected from optionally substituted C₁₋₃ alkyl; if the optionally substituted C₁₋₃ alkyl contains substituent(s), the substituent(s) are independently selected from halogen, C₁₋₃ alkoxy, hydroxyl or amino.

In some optional embodiments of the present disclosure, R₂, R₃, R₅ and R₆ are each independently selected from H or optionally substituted C₁₋₃ alkyl; and any one of R₂, R₃, R₅ and R₆ is selected from optionally substituted C₁₋₃ alkyl. For example, R₂ is selected from optionally substituted C₁₋₃ alkyl, and R₃, R₅ and R₆ are selected from H; or, R₃ is selected from optionally substituted C₁₋₃ alkyl, and R₂, R₅ and R₆ are selected from H; or, R₅ is selected from optionally substituted C₁₋₃ alkyl, and R₂, R₃ and R₆ are selected from H; or, R₆ is selected from optionally substituted C₁₋₃ alkyl, and R₂, R₃ and R₅ are selected from H.

In some optional embodiments of the present disclosure, R₂, R₃, R₅ and R₆ are each independently selected from H or C₁₋₃ alkyl; and any one of R₂, R₃, R₅ and R₆ is selected from C₁₋₃ alkyl. For example, R₂ is selected from C₁₋₃ alkyl, and R₃, R₅ and R₆ are selected from H; or, R₃ is selected from C₁₋₃ alkyl, and R₂, R₅ and R₆ are selected from H; or, R₅ is selected from C₁₋₃ alkyl, and R₂, R₃ and R₆ are selected from H; or, R₆ is selected from C₁₋₃ alkyl, and R₂, R₃ and R₅ are selected from H.

In some optional embodiments of the present disclosure, the C₁₋₃ alkyl is selected from methyl, ethyl, *n*-propyl or isopropyl.

In some specific embodiments of the present disclosure, the C₁₋₃ alkyl is selected from methyl.

In some specific embodiments of the present disclosure, R₂, R₃, R₅ and R₆ are each independently selected from H or methyl; and any one of R₂, R₃, R₅ and R₆ is selected from methyl. For example, R₂ is selected from methyl, and R₃, R₅ and R₆ are selected from H; or, R₃ is selected from methyl, and R₂, R₅ and R₆ are selected from H; or, R₅ is selected from methyl, and R₂, R₃ and R₆ are selected from H; or, R₆ is selected from methyl and R₂, R₃ and R₅ are selected from H.

Further, the nucleoside analog has the structure set forth in formula (100), or a stereoisomer thereof, or a pharmaceutically acceptable salt thereof: 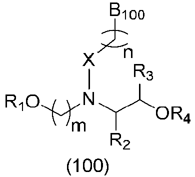
wherein, B₁₀₀ is selected from a base or a modified base; if B₁₀₀ contains amino, the amino is protected by an amino protecting group;
n is selected from 1, 2 or 3;
X is selected from 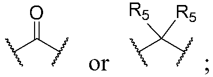 each R₅ is independently selected from optionally substituted C₁₋₃ alkyl, and the optionally substituted C₁₋₃ alkyl contains substituent(s) independently selected from halogen, C₁₋₃ alkoxy, hydroxyl or amino;
m is selected from 1, 2, 3 or 4;
R₁ is selected from H or a hydroxyl protecting group;
R₂ and R₃ are independently selected from H or optionally substituted C₁₋₃ alkyl; and at least one of R₂ and R₃ is selected from optionally substituted C₁₋₃ alkyl; if the optionally substituted C₁₋₃ alkyl contains substituent(s), the substituent(s) are independently selected from halogen, C₁₋₃ alkoxy, hydroxyl or amino;
R₄ is selected from H or 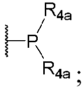 each R₄ₐ is independently selected from 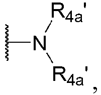 or C₁₋₆ alkoxy comprising a cyano substituent, and at least one R₄ₐ is selected from 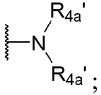 each R₄ₐ' is independently selected from optionally substituted C₁₋₆ alkyl.

In some optional embodiments of the present disclosure, the hydroxyl protecting group is selected from trityl, 4-methoxytrityl, 4,4'-dimethoxytrityl or 4,4',4"-trimethoxytrityl.

In some optional embodiments of the present disclosure, the hydroxyl protecting group is selected from 4,4'-dimethoxytrityl.

In some optional embodiments of the present disclosure, R₄ₐ' is selected from isopropyl.

In some optional embodiments of the present disclosure, the C₁₋₆ alkoxy comprising a cyano substituent is selected from

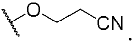

In some optional embodiments of the present disclosure, 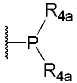 is selected from 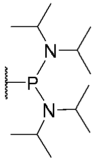 or

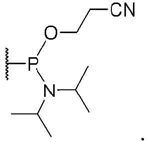

In some optional embodiments of the present disclosure, 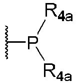 is selected from

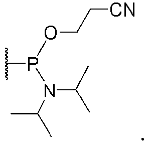

In some optional embodiments of the present disclosure, 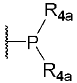 is selected from

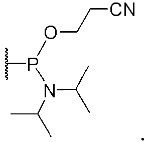

In some optional embodiments of the present disclosure, the base is selected from uracil U, thymine T, cytosine C, adenine A or guanine G.

In some optional embodiments of the present disclosure, the modified base is selected from

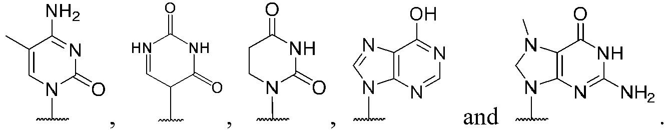

In some optional embodiments of the present disclosure, B₁₀₀ is selected from the bases, uracil U, thymine T, cytosine C, adenine A or guanine G; if B₁₀₀ contains amino, the amino is protected by an amino protecting group.

In some optional embodiments of the present disclosure, the amino protecting group is selected from alkoxycarbonyl amino protecting groups, acyl amino protecting groups or alkyl amino protecting groups.

In some optional embodiments of the present disclosure, the amino protecting group is selected from acyl amino protecting groups.

In some optional embodiments of the present disclosure, the acyl amino protecting group is selected from phthaloyl, *p*-toluenesulfonyl, trifluoroacetyl, *o*-nitrobenzenesulfonyl, *p*-nitrobenzenesulfonyl, pivaloyl, isobutyryl, acetyl (Ac) or benzoyl (Bz).

In some optional embodiments of the present disclosure, the amino protecting group is selected from isobutyryl, acetyl (Ac) or benzoyl (Bz).

In some optional embodiments of the present disclosure, B₁₀₀ is selected from any one of the following structures:

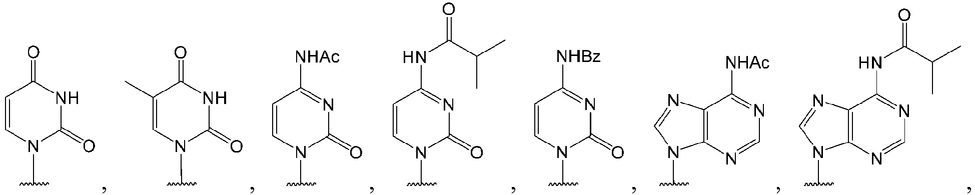

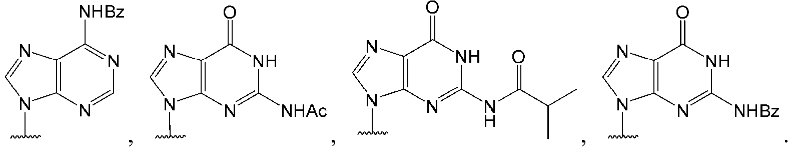

In some optional embodiments of the present disclosure, B₁₀₀ is selected from

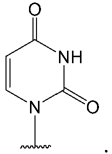

In some optional embodiments of the present disclosure, B₁₀₀ is selected from

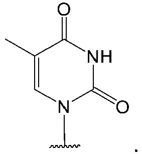

In some optional embodiments of the present disclosure, B₁₀₀ is selected from

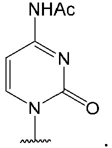

In some optional embodiments of the present disclosure, B₁₀₀ is selected from

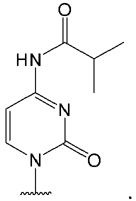

In some optional embodiments of the present disclosure, B₁₀₀ is selected from

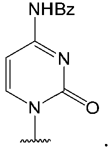

In some optional embodiments of the present disclosure, B₁₀₀ is selected from

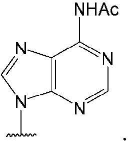

In some optional embodiments of the present disclosure, B₁₀₀ is selected from

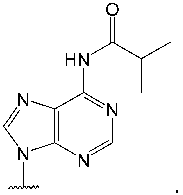

In some optional embodiments of the present disclosure, B₁₀₀ is selected from

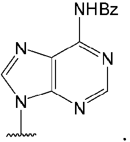

In some optional embodiments of the present disclosure, B₁₀₀ is selected from

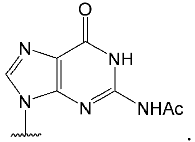

In some optional embodiments of the present disclosure, B₁₀₀ is selected from

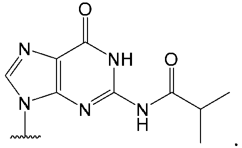

In some optional embodiments of the present disclosure, B₁₀₀ is selected from

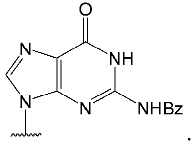

In some optional embodiments of the present disclosure, in formula (100), n is selected from 1 or 2.

In some optional embodiments of the present disclosure, n is selected from 1.

In some optional embodiments of the present disclosure, in formula (100), m is selected from 1, 2 or 3.

In some optional embodiments of the present disclosure, m is selected from 2.

In some optional embodiments of the present disclosure, in formula (100), X is selected from

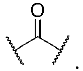

In some optional embodiments of the present disclosure, in formula (100), R₂ and R₃ are independently selected from H or optionally substituted C₁₋₃ alkyl; and R₂ and R₃ are not simultaneously selected from H and optionally substituted C₁₋₃ alkyl (i.e., R₂ is selected from H and R₃ is selected from optionally substituted C₁₋₃ alkyl; or R₂ is selected from optionally substituted C₁₋₃ alkyl and R₃ is selected from H).

In some optional embodiments of the present disclosure, R₂ and R₃ are independently selected from H or C₁₋₃ alkyl; and R₂ and R₃ are not simultaneously selected from H and C₁₋₃ alkyl.

In some optional embodiments of the present disclosure, R₂ and R₃ are independently selected from H or methyl; and R₂ and R₃ are not simultaneously selected from H and methyl.

In some optional embodiments of the present disclosure, the nucleoside analog has the structure shown in formula (101), or a stereoisomer thereof, or a pharmaceutically acceptable salt thereof:

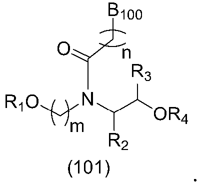

In some embodiments of the present disclosure, the nucleoside analog has any one of the following structures, or a stereoisomer thereof, or a pharmaceutically acceptable salt thereof: 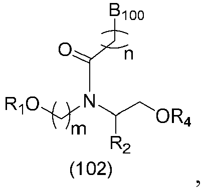 wherein R₂ is selected from optionally substituted C₁₋₃ alkyl; 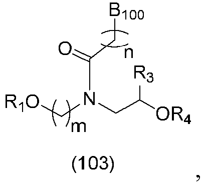 wherein R₃ is selected from optionally substituted C₁₋₃ alkyl.

In some optional embodiments of the present disclosure, the nucleoside analog has the structure shown in formula (102), or a stereoisomer thereof, or a pharmaceutically acceptable salt thereof: 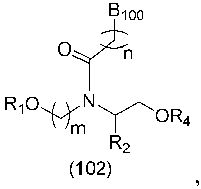 wherein R₂ is selected from optionally substituted C₁₋₃ alkyl.

In some optional embodiments of the present disclosure, the nucleoside analog has the structure shown in formula (103), or a stereoisomer thereof, or a pharmaceutically acceptable salt thereof: 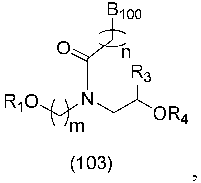 wherein R₃ is selected from optionally substituted C₁₋₃ alkyl.

In some embodiments of the present disclosure, the nucleoside analog has any one of the following structures, or a stereoisomer thereof, or a pharmaceutically acceptable salt thereof: 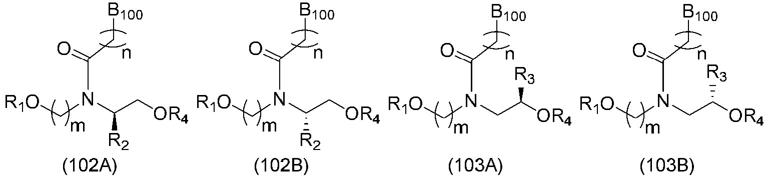 wherein R₂ and R₃ are independently selected from optionally substituted C₁₋₃ alkyl.

In some optional embodiments of the present disclosure, the nucleoside analog has the structure shown in formula (102A), or a stereoisomer thereof, or a pharmaceutically acceptable salt thereof: 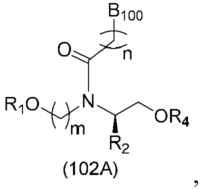 wherein R₂ is selected from optionally substituted C₁₋₃ alkyl.

In some optional embodiments of the present disclosure, the nucleoside analog has the structure shown in formula (102B), or a stereoisomer thereof, or a pharmaceutically acceptable salt thereof: 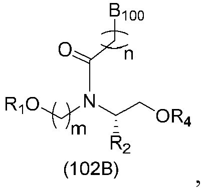 wherein R₂ is selected from optionally substituted C₁₋₃ alkyl.

In some optional embodiments of the present disclosure, the nucleoside analog has the structure shown in formula (103A), or a stereoisomer thereof, or a pharmaceutically acceptable salt thereof: 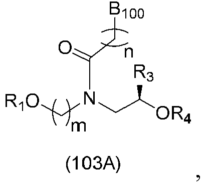 wherein R₃ is selected from optionally substituted C₁₋₃ alkyl.

In some optional embodiments of the present disclosure, the nucleoside analog has the structure shown in formula (103B), or a stereoisomer thereof, or a pharmaceutically acceptable salt thereof: 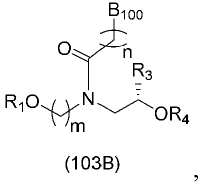 wherein R₃ is selected from optionally substituted C₁₋₃ alkyl.

In some embodiments of the present disclosure, the nucleoside analog is selected from any one of the following structures, or a stereoisomer thereof, or a pharmaceutically acceptable salt thereof:

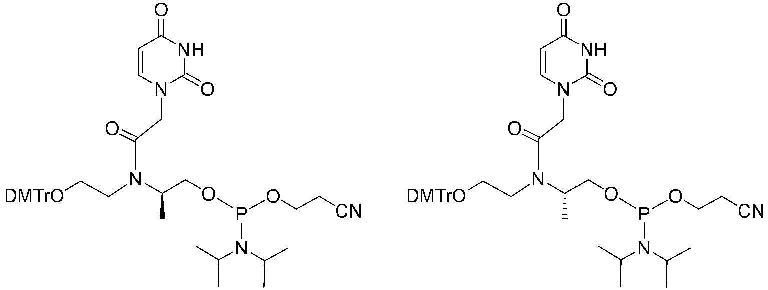

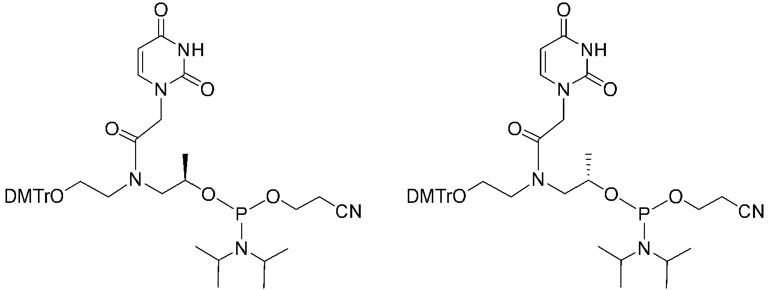

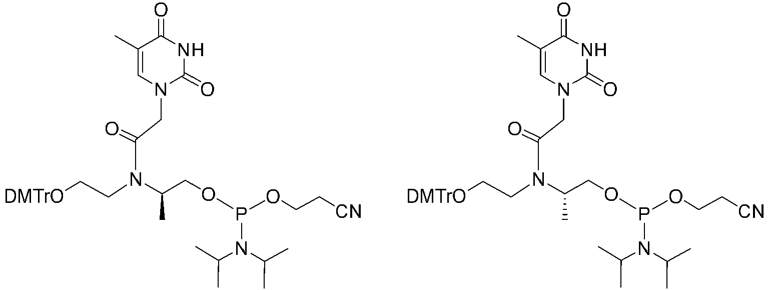

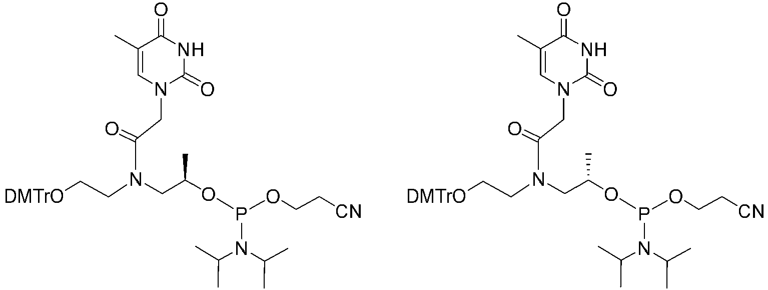

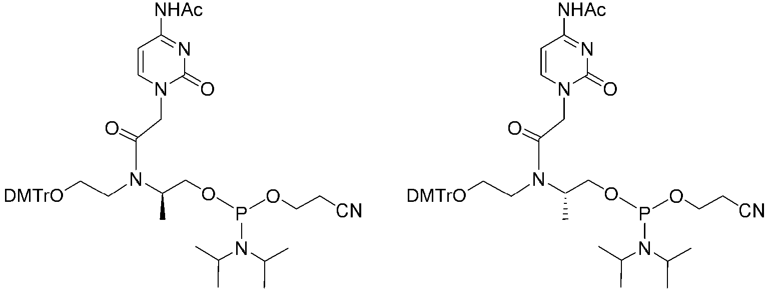

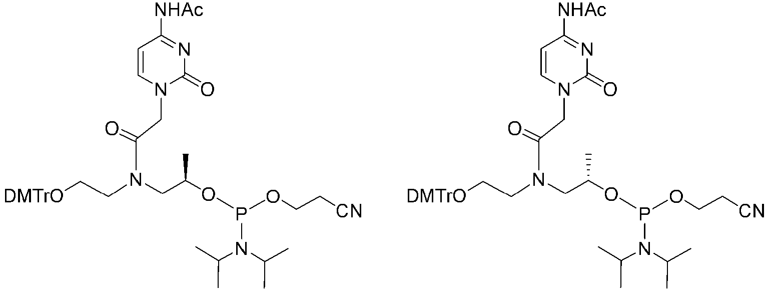

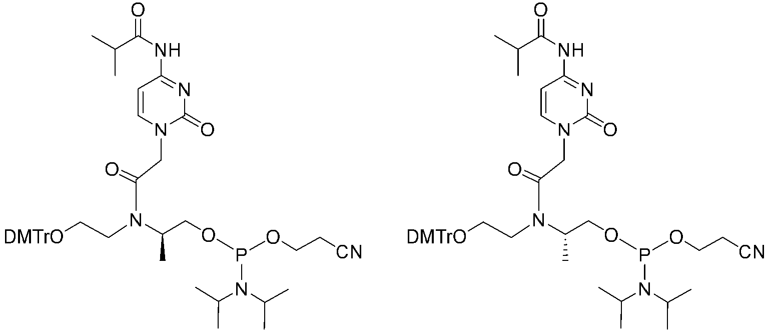

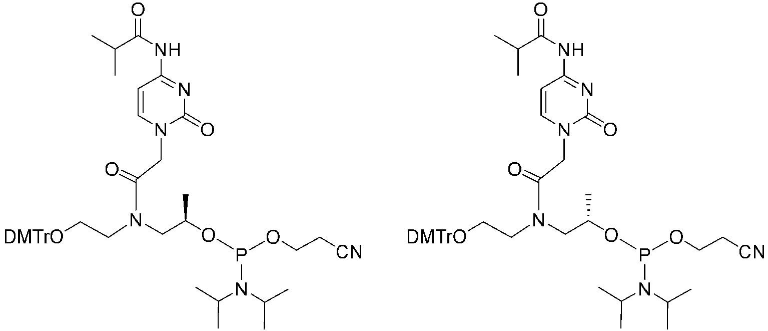

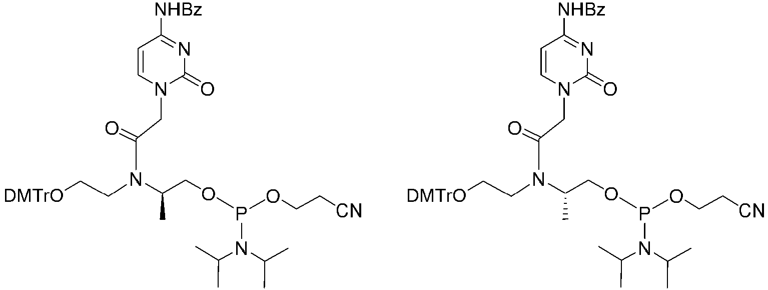

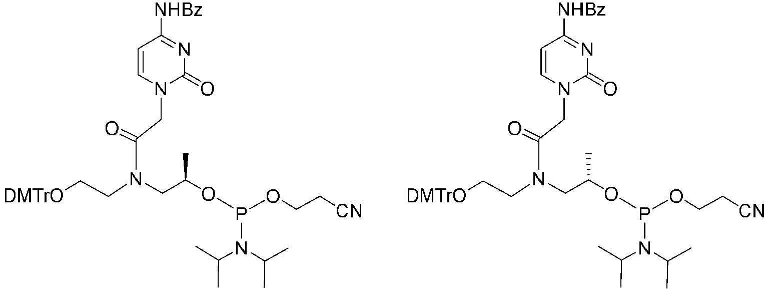

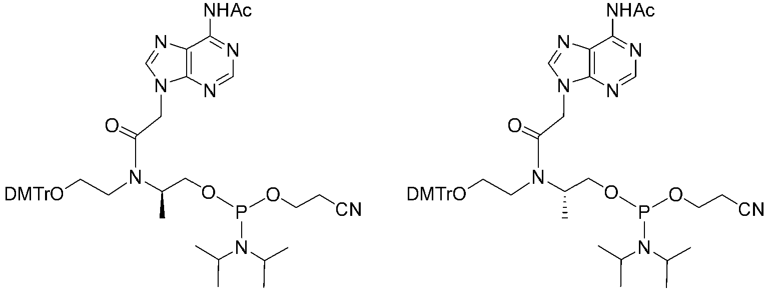

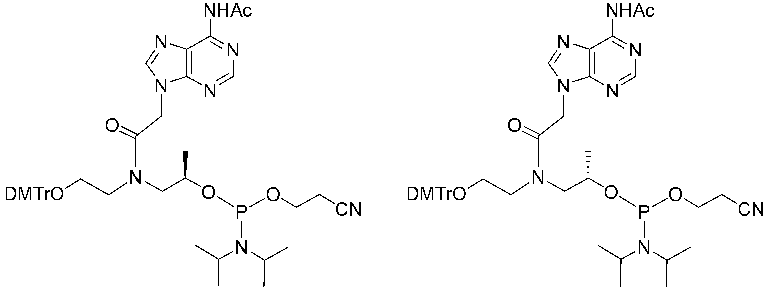

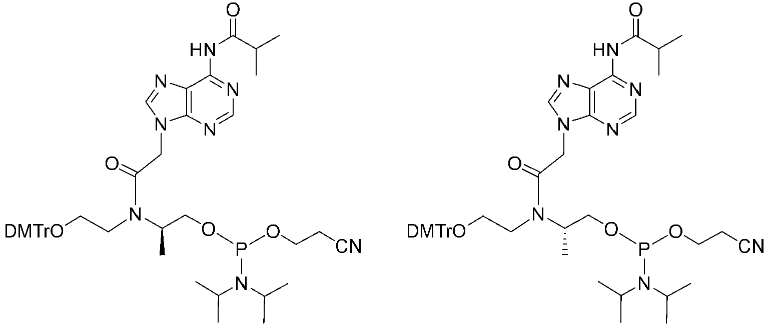

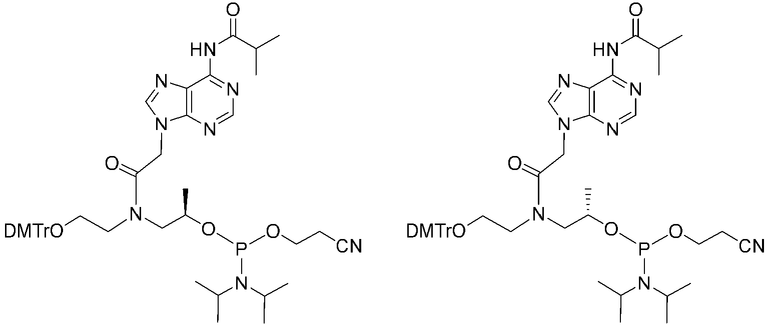

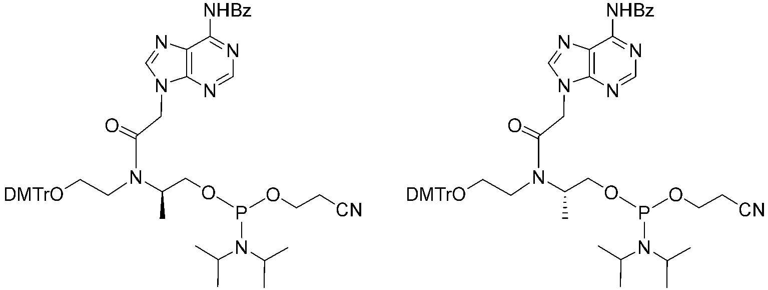

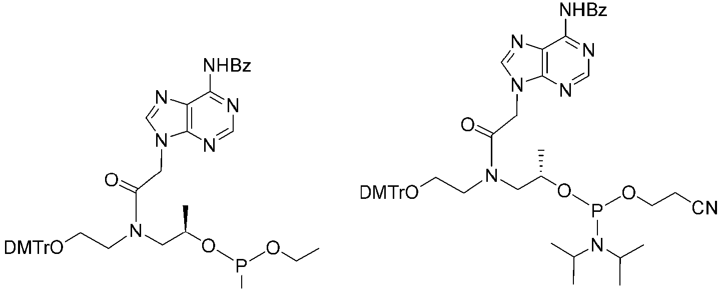

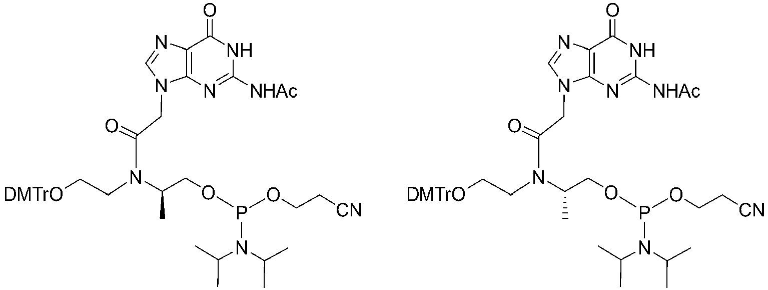

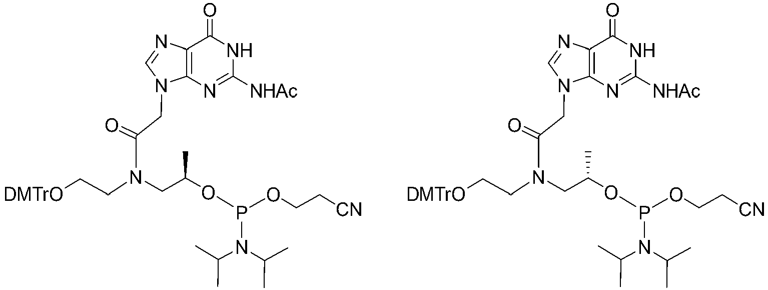

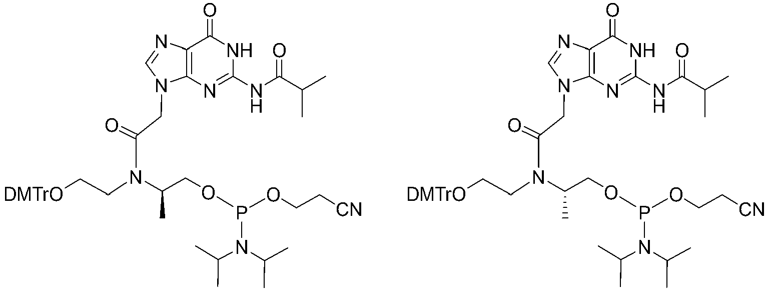

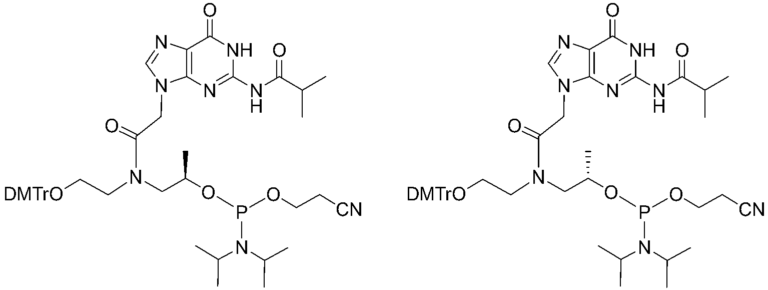

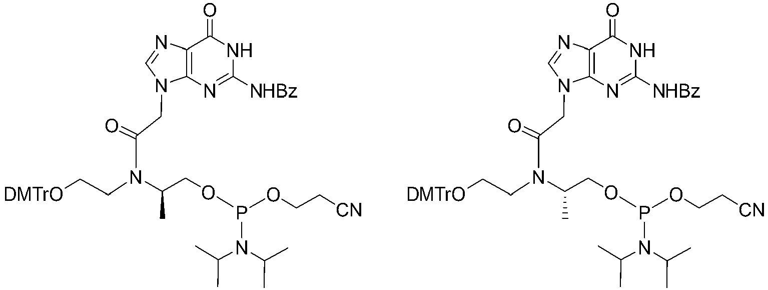

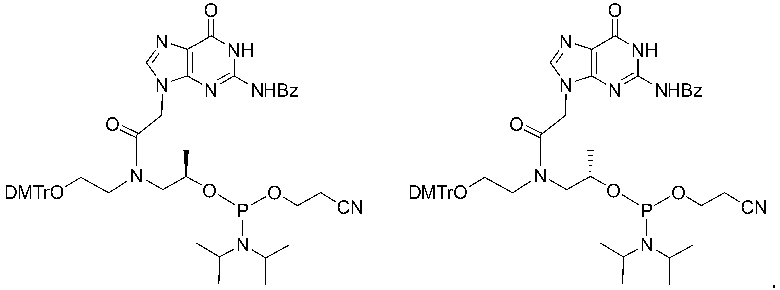

Further, the nucleoside analog has the structure shown in formula (400), or a stereoisomer thereof, or a pharmaceutically acceptable salt thereof: 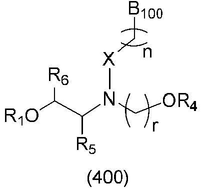
wherein R₁, R₄, B₁₀₀, n, X and * are as defined above;
r is selected from 1, 2, 3 or 4;
R₅ and R₆ are independently selected from H or optionally substituted C₁₋₃ alkyl; and at least one of R₅ and R₆ is selected from optionally substituted C₁₋₃ alkyl; if the optionally substituted C₁₋₃ alkyl contains substituent(s), the substituent(s) are independently selected from a halogen, C₁₋₃ alkoxy, hydroxyl or amino.

In some optional embodiments of the present disclosure, r is selected from 1, 2 or 3.

In some specific embodiments of the present disclosure, r is selected from 2.

In some optional embodiments of the present disclosure, R₅ and R₆ are independently selected from H or optionally substituted C₁₋₃ alkyl; and R₅ and R₆ are not simultaneously H or optionally substituted C₁₋₃ alkyl.

In some optional embodiments of the present disclosure, R₅ and R₆ are independently selected from H or C₁₋₃ alkyl; and R₅ and R₆ are not simultaneously H or C₁₋₃ alkyl.

In some optional embodiments of the present disclosure, R₅ and R₆ are independently selected from H or methyl; and R₅ and R₆ are not simultaneously H or methyl. For example, R₅ is selected from H and R₆ is selected from methyl, or R₅ is selected from methyl and R₆ is selected from H.

In some optional embodiments of the present disclosure, the nucleoside analog has the structure shown in formula (401), or a stereoisomer thereof, or a pharmaceutically acceptable salt thereof:

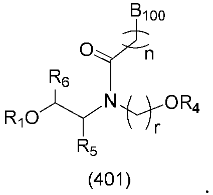

In some optional embodiments of the present disclosure, the nucleoside analog has any one of the following structures, or a stereoisomer thereof, or a pharmaceutically acceptable salt thereof: 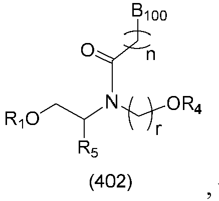 wherein R₅ is selected from optionally substituted C₁₋₃ alkyl; 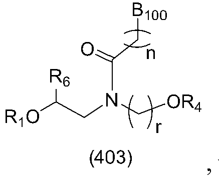 wherein R₆ is selected from optionally substituted C₁₋₃ alkyl.

In some optional embodiments of the present disclosure, the nucleoside analog has any one of the following structures, or a stereoisomer thereof, or a pharmaceutically acceptable salt thereof: 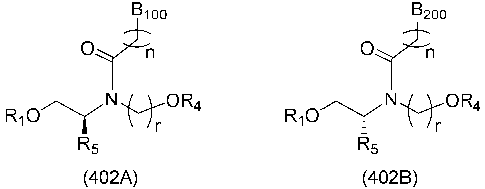 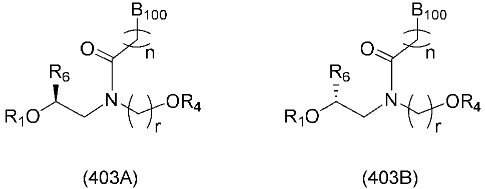 wherein R₅ and R₆ are independently selected from optionally substituted C₁₋₃ alkyl.

In some optional embodiments of the present disclosure, the nucleoside analog is selected from any one of the following structures, or a stereoisomer thereof, or a pharmaceutically acceptable salt thereof:

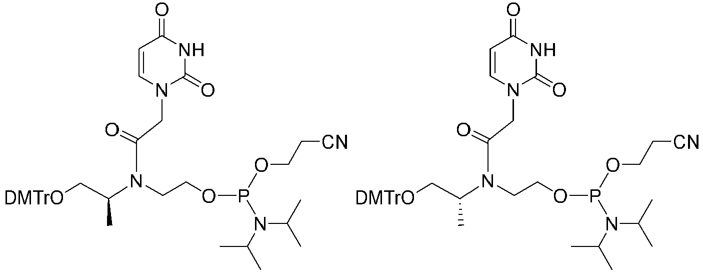

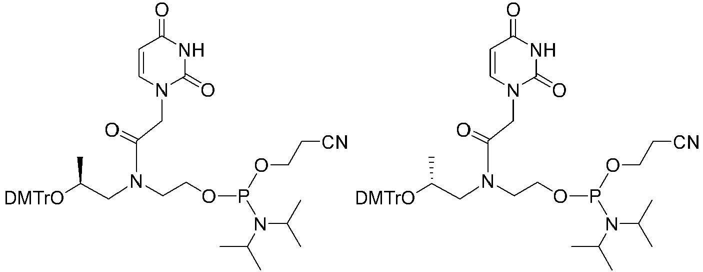

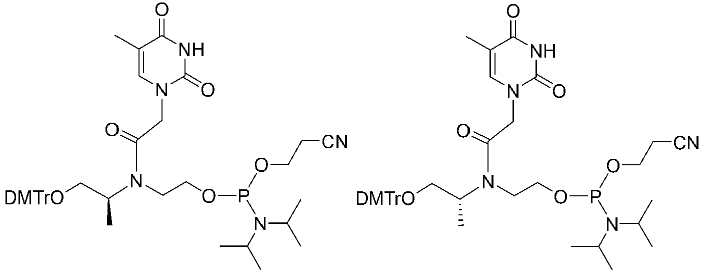

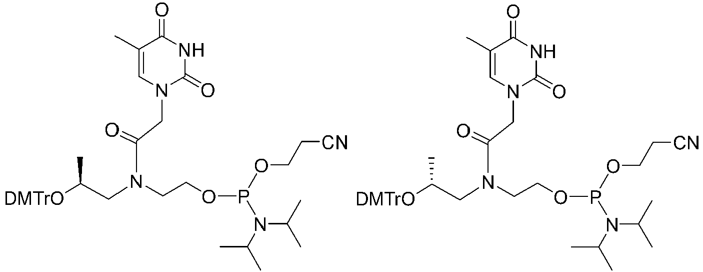

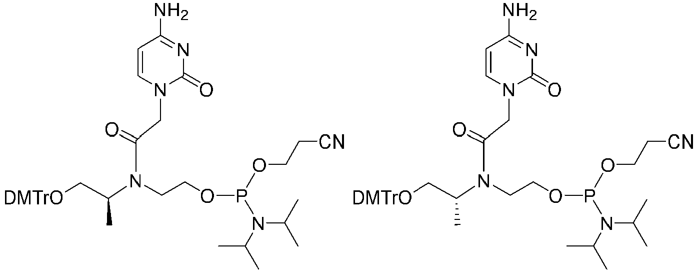

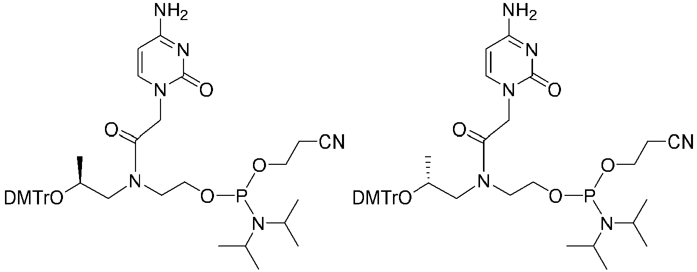

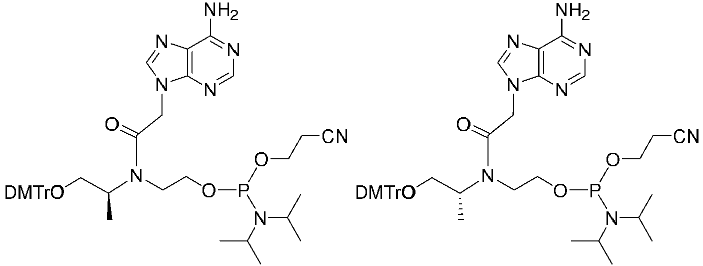

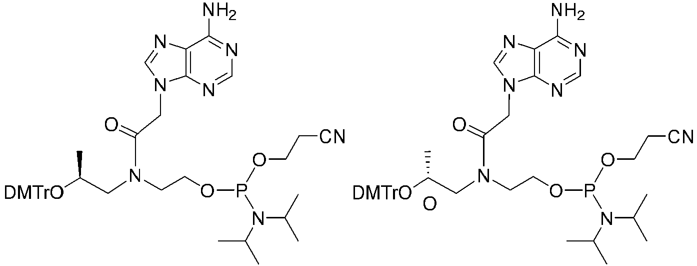

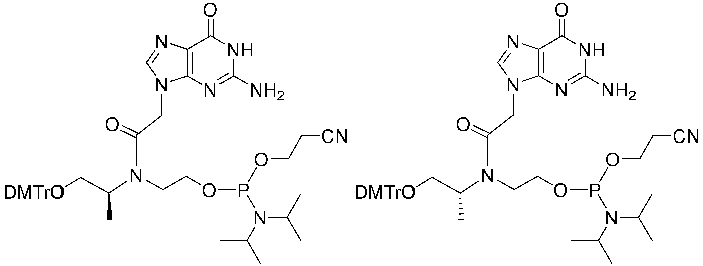

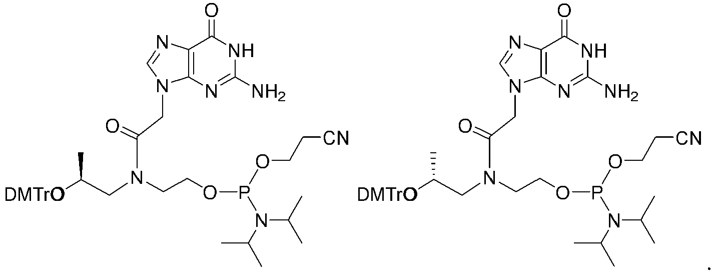

### Application

In a seventh aspect of the present disclosure, the present disclosure also provides the use of the nucleoside analog, and/or the nucleotide analog, and/or the double-stranded oligonucleotide, and/or the double-stranded oligonucleotide conjugate, and/or the composition, and/or the pharmaceutical composition in the preparation of a medicament for treating and/or preventing a disease or condition associated with dysregulation of the mRNA expression level of a specific gene.

In some embodiments of the present disclosure, the specific gene is a gene abnormally expressed in hepatocytes. In some embodiments, the specific gene is an endogenous gene expressed in the liver. In some embodiments, the specific gene is the gene of a pathogen that proliferates in the liver. In some embodiments, the specific gene is a gene expressed in lung epithelial cells. In some embodiments, the specific gene is a gene expressed in the central nervous system. In some embodiments, the specific gene is a gene expressed in tumor cells.

Exemplarily, the mRNA expressed by the specific gene includes, but is not limited to, one selected from the mRNAs transcribed from the following genes: ACE2, ANGPTL3, ApoA, ApoB, ApoC, AR, ASK1, C5, Col1A1, CTGF, Ebola, FOXO1, FTO, FVII, FXI, FXII, GCGR, HBV, HCV, HSD17B13, p53, PCSK9, PNP, PLG, PKK, KNG, SARS-CoV-2, SCD1, SCNN1A, SOD1, STAT3, TIMP-1, TMPRSS6, XO, INSR, SREBF1, HDV, RPTOR, TLK2, LPA, C3, AGT.

Further, the mRNA expressed by the specific gene is selected from the mRNA expressed by a hepatitis B virus (HBV) gene, the mRNA expressed by the angiopoietin-like protein 3 (ANGPTL3) gene, or the mRNA expressed by the apolipoprotein C3 (ApoC3) gene.

In some embodiments of the present disclosure, the disease or condition associated with the mRNA expression level of a specific gene is chronic liver disease, hepatitis, hepatic fibrosis disease, hepatic proliferative disease and/or dyslipidemia. In some embodiments, the disease or condition associated with the mRNA expression level of a specific gene is hepatitis B or dyslipidemia. In some embodiments, the dyslipidemia is hypercholesterolemia, hypertriglyceridemia or atherosclerosis.

### Method of Regulating the Expression of a Specific Gene in a Target Cell

In an eighth aspect of the present disclosure, the present disclosure also provides a method of regulating the expression of a specific gene in a target cell, comprising: contacting the double-stranded oligonucleotide, and/or the double-stranded oligonucleotide conjugate, and/or the composition, and/or the pharmaceutical composition provided by the present disclosure with the target cell.

In some embodiments, the regulation refers to inhibiting the expression of a specific gene in cells.

By introducing the double-stranded oligonucleotide, and/or the double-stranded oligonucleotide conjugate, and/or the composition, and/or the pharmaceutical composition of the present disclosure into a cell with abnormal expression of the specific gene, the purpose of inhibiting the expression of the specific gene in the cell can also be achieved through the mechanism of gene expression regulation. In some embodiments, the cell is hepatocytes. In some embodiments, the hepatocytes may be the cells selected from liver cancer cell lines such as Hep3B, HepG2, Huh7, or isolated primary hepatocytes. In some embodiments, the hepatocytes are primary hepatocytes.

For inhibiting the expression of a specific gene in cells using the method provided by the present disclosure, the dosage of the double-stranded oligonucleotide, and/or the double-stranded oligonucleotide conjugate, and/or the composition, and/or the pharmaceutical composition provided is readily determined by the skilled in the art according to the desired effect. For example, in some embodiments, the dosage of the double-stranded oligonucleotide conjugate is an amount sufficient to reduce the expression of the target gene and result in an extracellular concentration of 1 pM to 1 µM, or 0.01 nM to 100 nM, or 0.05 nM to 50 nM, or to about 5 nM at the surface of the target cells. The amount required to achieve such a local concentration will depend on various factors, including the delivery method, the delivery site, the number of cell layers between the delivery site and the target cells or tissues, and whether the delivery is local or systemic. The concentration at the delivery site can be significantly higher than that at the surface of the target cells or tissues.

### Method of Preventing and/or Treating Diseases

In a ninth aspect of the present disclosure, the present disclosure also provides a method of preventing and/or treating a disease or condition associated with dysregulation of the mRNA expression level of a specific gene in a target cell in a subject, characterized in that the method comprises: administering to the subject a pharmaceutically acceptable dose of the double-stranded oligonucleotide, and/or the double-stranded oligonucleotide conjugate, and/or the composition, and/or the pharmaceutical composition described in the present disclosure.

By administering the double-stranded oligonucleotide, and/or the double-stranded oligonucleotide conjugate, and/or the composition, and/or the pharmaceutical composition provided by the present disclosure to a subject in need thereof, the purpose of preventing and/or treating pathological conditions or diseases resulting from expression of a specific gene in cells can be achieved through a mechanism of regulating gene expression.

The administration refers to placing the double-stranded oligonucleotide, and/or the double-stranded oligonucleotide conjugate, and/or the composition, and/or the pharmaceutical composition into the body of a subject by a method or route that at least partially localizes the double-stranded oligonucleotide, and/or the double-stranded oligonucleotide conjugate, and/or the composition, and/or the pharmaceutical composition at a desired site to produce a desired effect. Administration routes suitable for the method of the present disclosure include local administration and systemic administration. In general, local administration results in delivery of a graet amount of the double-stranded oligonucleotide, and/or the double-stranded oligonucleotide conjugate, and/or the composition, and/or the pharmaceutical composition to a specific site relative to the entire body of the subject; whereas systemic administration results in delivery of the double-stranded oligonucleotide, and/or the double-stranded oligonucleotide conjugate, and/or the composition, and/or the pharmaceutical composition to substantially the entire body of the subject.

Administration to a subject may be performed via any suitable route known in the art, including but not limited to oral or parenteral routes, such as intravenous, intramuscular, subcutaneous, transdermal, airway (aerosol), pulmonary, nasal, rectal, and local administration (including buccal and sublingual administration). The administration frequency may be once or multiple times daily, weekly, every two weeks, every three weeks, monthly, or yearly.

The dosage of the double-stranded oligonucleotide, and/or the double-stranded oligonucleotide conjugate, and/or the composition, and/or the pharmaceutical composition described in the present disclosure may be a conventional dosage in the art, and the dosage may be determined according to various parameters, particularly the age, body weight, and gender of the subject. Toxicity and efficacy may be determined by standard pharmaceutical procedures in cell cultures or experimental animals, such as determining the LD50 (the dose lethal to 50% of a population) and the ED50 (in a graded response, it refers to the dose capable of eliciting 50% of the maximum response intensity; in a quantal response, it refers to the dose at which 50% of the experimental subjects show a positive response). A range of dosage for human use may be extrapolated from the data obtained from cell culture assays and animal studies.

When administering the double-stranded oligonucleotide, and/or the double-stranded oligonucleotide conjugate, and/or the composition, and/or the pharmaceutical composition described in the present disclosure, for example, to male or female C57BL/6J or C3H/HeNCrlVr mice aged 6-12 weeks with a body weight of 18-25 g, based on the amount of the double-stranded oligonucleotide, and/or the double-stranded oligonucleotide in the double-stranded oligonucleotide conjugate, and/or the composition, and/or the pharmaceutical composition: for the oligonucleotide conjugate formed from the double-stranded oligonucleotide and a pharmaceutically acceptable conjugate molecule, the dosage of the double-stranded oligonucleotide may be 0.001-100 mg/kg body weight; in some embodiments, 0.01-50 mg/kg body weight; in further embodiments, 0.05-20 mg/kg body weight; in still further embodiments, 0.1-15 mg/kg body weight; and in yet further embodiments, 0.1-10 mg/kg body weight. The above dosages may be preferred when administering the double-stranded oligonucleotide, and/or the double-stranded oligonucleotide conjugate, and/or the composition, and/or the pharmaceutical composition described in the present disclosure.

### Kit

In a tenth aspect of the present disclosure, the present disclosure provides a kit comprising the double-stranded oligonucleotide, and/or the double-stranded oligonucleotide conjugate, and/or the composition, and/or the pharmaceutical composition provided by the present disclosure.

In some embodiments, the kit of the present disclosure may provide the double-stranded oligonucleotide, and/or the double-stranded oligonucleotide conjugate, and/or the composition, and/or the pharmaceutical composition in a single container. In some embodiments, the kit of the present disclosure may comprise a container providing a pharmaceutically acceptable excipient. In some embodiments, the kit may further comprise other components, such as stabilizers or preservatives. In some embodiments, the kit of the present disclosure may comprise at least one other therapeutic agent in one or more containers other than the container(s) providing the double-stranded oligonucleotide, and/or the double-stranded oligonucleotide conjugate, and/or the composition, and/or the pharmaceutical composition described herein. In some embodiments, the kit may comprise instructions for mixing the double-stranded oligonucleotide, and/or the double-stranded oligonucleotide conjugate, and/or the composition, and/or the pharmaceutical

composition with a pharmaceutically acceptable excipient or adjuvant or other ingredient(s) (if any).

In the kit of the present disclosure, the double-stranded oligonucleotide, and/or the double-stranded oligonucleotide conjugate, and/or the composition, and/or the pharmaceutical composition, and the pharmaceutically acceptable excipient or adjuvant may be provided in any form, such as a liquid form, a dry form or a lyophilized form. In some embodiments, the double-stranded oligonucleotide, and/or the double-stranded oligonucleotide conjugate, and/or the composition, and/or the pharmaceutical composition, and optionally a pharmaceutically acceptable excipient or adjuvant are substantially pure and/or sterile. In some embodiments, sterile water may be provided in the kit of the present disclosure.

### Examples

Unless otherwise specified, the ratios of the reagents used in the present disclosure are all calculated by volume ratio (v/v).

Unless otherwise specified, in the synthesis of nucleoside analogs and the synthesis of ligands/carriers, all reagents used in the Examples of the present disclosure are purchased from Beijing Ouhe Technology Co., Ltd. Among them, the main reagents are shown in Table 1.

**Table 1 Main Reagents**

| Reagent Name | Abbreviation | CAS No. |
|---|---|---|
| N2-Isobutyrylguanine | Compound NM-g | 21047-89-2 |
| N4-Benzoylcytosine | Compound NM-c | 26661-13-2 |
| 2-(6-Benzamido-9H-purin-9-yl)acetic acid | Compound NM-A | 171486-04-7 |
| L-Alanine ethyl ester hydrochloride | - | 1115-59-9 |
| 2-(7-Azobenzotriazole)-N,N,N',N'-Tetramethylurea hexafluorophosphate | HATU | 148893-10-1 |
| N,N-Diisopropylethylamine | DIEA | 7087-68-5 |
| tert-Butyl bromoacetate | | 5292-43-3 |
| Bis(diisopropylamino)(2-cyanoethoxy)phosphine | - | 102691-36-1 |
| 4,5-Dicyanoimidazole | DCI | 1122-28-7 |
| 4,4'-Dimethoxytrityl chloride/4,4'-Dimethoxytrityl chloride | DMTrCl | 40615-36-9 |
| 2M Lithium aluminum hydride in tetrahydrofuran solution | LiAlH₄/THF | - |
| 4M Hydrochloric acid in 1,4-dioxane solution | HCl/1,4-Dioxane | - |
| N-(Methoxymethyl)-N-(trimethylsilylmethyl)benzyl amine | - | 93102-05-7 |
| Wet palladium on carbon (10 wt% loading) | Pd/C | - |
| Palladium hydroxide on carbon (10 wt% loading) | Pd(OH)₂/C | - |
| Benzotriazole-N,N,N',N'-tetramethylurea hexafluorophosphate | HBTU | 94790-37-1 |
| Amino CPG (Aminoalkyl-CPG, Model C3006-1000) | 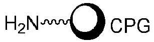 | - |
| trans-4-(Boc-amino)cyclohexylcarbaldehyde | - | 181308-57-6 |
| N-Benzyloxycarbonyl-4-aminobutyric acid | - | 5105-78-2 |
| 5-[[(2R,3R,4R,5R,6R)-3-Acetamido-4,5-diacetoxy-6 -(acetoxymethyl)-2-tetrahydropyranyl]oxy]pentanoic acid | Compound 4 | 1159408-54-4 |

Wherein, CPG represents Controlled Pore Glass support.

Unless otherwise specified, the reagents, consumables, instruments and equipment used in the biological detection experiments of the present disclosure are all commercially available products. Among them, the main reagents and consumables are shown in Table 2, and the main instruments and equipment are shown in Table 3.

**Table 2 Main Reagents and Consumables**

| Name | Manufacturer |
|---|---|
| 1×PBS | Zhongke Maichen (Beijing) Technology Co., Ltd. |
| Hanwei RNA Extraction Kit | Zhejiang Hanwei Technology Co., Ltd. |
| Reverse Transcription System | Promega Corporation |
| SYBR Select Master Mix | ABI |
| RNALater | Thermo Fisher Scientific |
| Tissue and Cell Fixative (4%) | Beijing Biodee Biotechnology Co., Ltd. |

**Table 3 Main Instruments and Equipment**

| Name | Manufacturer |
|---|---|
| Automatic Nucleic Acid Extractor | Zhejiang Hanwei Technology Co., Ltd. |
| High-Speed Refrigerated Centrifuge | Eppendorf |
| NANODROP OneC | Thermo Fisher Scientific |
| Gradient PCR Amplifier | Eppendorf |
| Real-Time Quantitative PCR Instrument | ABI StepOne Plus |
| Gel Imaging System | Shanghai Tanon Life Science Co., Ltd. |
| Electrophoresis System | Beijing Liuyi Instrument Factory |
| Tissuelyser II Automatic Tissue Homogenizer | Shanghai Jingxin Industrial Development Co., Ltd. |
| R540IE Small Animal Anesthesia Machine | Shenzhen RWD Life Science Co., Ltd. |
| Mindray BS-430 Automatic Biochemical Analyzer | Shenzhen Mindray Animal Medical Technology Co., Ltd. |

### Synthesis of Nucleoside Analogs

### Preparation Example 1: Synthesis of Compound NM022

In this preparation example, the synthetic scheme of compound NM022 is shown as follows:

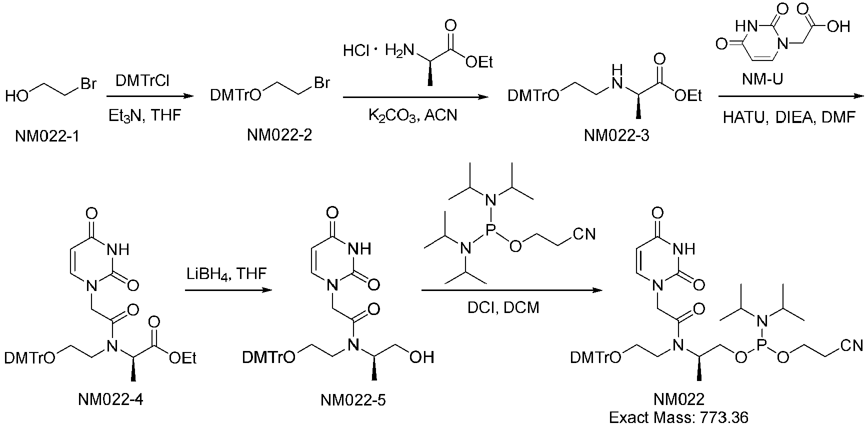

### (1-1) Synthesis of Compound NM-U

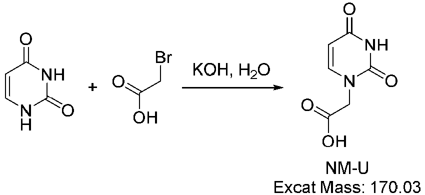

KOH (36.04 g, 642.35 mmol, 4.0 eq) was dissolved in 180 mL of water, uracil (18 g, 160.59 mmol, 1.0 eq) was slowly added to the KOH aqueous solution. It was raised to the temperature of 80 °C, stirred and reacted at 80 °C for 30 min. Bromoacetic acid (24.54 g, 176.65 mmol, 1.1 eq) was added thereto, and the reaction mixture was stirred and reacted at 80 °C for 6 h. After the reaction was completed, the reaction solution was added with 4M hydrogen chloride aqueous solution to adjust the pH to 2, and then filtered to give a solid substance. The solid substance was first washed with water (2 × 50 mL) and then with saturated aqueous ethyl acetate solution (2 × 50 mL) to give compound NM-U as a white solid (12 g, 70.58 mmol, yield 44.4%, CAS No. 4113-97-7). MS-ESI (m/z) = 171 [M + H]⁺.

### (1-2) Synthesis of Compound NM022-2

2-Bromoethanol (20 g, 160.045 mmol, 1.0 eq.), triethylamine (40.49 g, 400.112 mmol, 2.5 eq.) and DMTrCl (58.93 g, 192.054 mmol, 1.2 eq.) were dissolved in tetrahydrofuran (200 mL), stirred at 25 °C for 6 hours under an argon atmosphere. After the reaction was completed, the reaction solution was concentrated to dryness under vacuum. The residue was purified by silica gel column chromatography (eluent: ethyl acetate/petroleum ether = 40/60, v/v) to give Compound NM022-2 as a white solid (50 g, yield 73.11%). MS ESI (m/z) = 427.2 [M + H]⁺.

¹H NMR (300 MHz, Chloroform-*d*) δ: 7.48 - 7.43 (m, 2H), 7.37 - 7.15 (m, 7H), 6.85 - 6.80 (m, 4H), 3.78 (s, 6H), 3.43 - 3.37 (m, 4H).

### (1-3) Synthesis of Compound NM022-3

Ethyl-(2R)-2-aminopropionate hydrochloride (5.03 g, 32.76 mmol, 1.0 eq.) and K₂CO₃ (6.79 g, 49.14 mmol, 1.5 eq.) were dissolved in acetonitrile (70 mL), stirred at 25 °C for 30 min, and then added with Compound NM022-2 (14 g, 32.761 mmol, 1.0 eq.). After stirring at 80 °C for 16 h, the reaction solution was cooled to 0 °C and quenched with a saturated aqueous solution of NaHCO₃ (300 mL). After the reaction was completed, the reaction solution was extracted with ethyl acetate (500 mL × 3), and the organic phase was combined. The organic phase was washed with saturated aqueous sodium chloride solution (500 mL), dried over anhydrous sodium sulfate, filtered with suction and concentrated to dryness under vacuum. The residue was purified by silica gel column chromatography (eluent: ethyl acetate/petroleum ether = 20/80, v/v) to give Compound NM022-3 as a yellow semi-solid (11 g, yield 72.43%). MS ESI (m/z) = 464.2 [M + H]⁺.

¹H NMR (300 MHz, Chloroform-d) δ: 7.45 - 7.41 (m, 2H), 7.34 - 7.16 (m, 7H), 6.84 - 6.79 (m, 4H), 4.24 - 4.14 (m, 2H), 3.78 (s, 6H), 3.39 - 3.32 (m, 1H), 3.21 - 3.17 (m, 2H), 2.89 - 2.81 (m, 1H), 2.70 - 2.63 (m, 1H), 1.31 - 1.25 (m, 6H).

### (1-4) Synthesis of Compound NM022-4

(2,4-dioxy-3H-pyrimidin-1-yl) acetic acid (4.04 g, 23.747 mmol, 1.0 eq.) and HATU (18.04 g, 47.458 mmol, 2.0 eq.) were dissolved in N,N-dimethylformamide (80 mL), stirred at 25 °C for 20 min, and then Compound NM022-3 (11 g, 23.729 mmol, 1.0 eq.) and DIEA (10.73 g, 83.052 mmol, 3.5 eq.) were added. The reaction solution was stirred at 25 °C for 2 hours, and then quenched by adding saturated NaHCO₃ aqueous solution (400 mL) at 0 °C. After the reaction was completed, the reaction solution was extracted with ethyl acetate (500 mL × 3), and the organic phase was combined. The organic phase was washed with saturated aqueous sodium chloride solution (400 mL × 3), dried over anhydrous sodium sulfate, filtered and concentrated to dryness under vacuum. The residue was purified by silica gel column chromatography (eluent: ethyl acetate/petroleum ether = 65/35, v/v) to give Compound NM022-4 as a yellow semi-solid (10.2 g, yield 69.82%). MS ESI (m/z) = 614.20 [M - H]⁻.

¹H NMR (400 MHz, Chloroform-d) δ: 7.40 - 7.35 (m, 2H), 7.32 - 7.18 (m, 7H), 6.90 - 6.79 (m, 5H), 5.63 (d, J = 7.6 Hz, 1H), 4.67 - 4.46 (m, 2H), 4.33 - 4.28 (m, 1H), 4.13 - 4.04 (m, 2H), 3.78 - 3.75 (m, 7H), 3.58 - 3.54 (m, 1H), 3.47 - 3.40 (m, 2H), 1.34 - 1.30 (m, 3H), 1.20 - 1.15 (m, 3H).

### (1-5) Synthesis of Compound NM022-5

Under an argon atmosphere, LiBH₄ (546.7 mg, 24.851 mmol, 1.5 equiv) was added to tetrahydrofuran (120 mL) at 0 °C. Then Compound NM022-4 (10.2 g, 16.567 mmol, 1.0 equiv) was added with stirring, followed by stirring at 0 °C for 1 hour. The reaction system was quenched by adding water (500 mL) at 0 °C. After the reaction was completed, the reaction solution was extracted with ethyl acetate (500 mL × 3), and the organic phase was combined. The organic phase was washed with saturated aqueous sodium chloride solution (500 mL), dried over anhydrous sodium sulfate, filtered and concentrated to dryness under vacuum. The residue was purified by reversed-phase column chromatography (column: AQ-C18; eluent: acetonitrile/water = 5/95, v/v; detector: UV-254) to give Compound NM022-5 as an off-white semi-solid (6.6 g, yield 69.45%). MS ESI (m/z) = 572.15 [M - H]⁻.

¹H NMR (400 MHz, Acetonitrile-*d₃*) δ: 7.48 - 7.43 (m, 2H), 7.37 - 7.05 (m, 8H), 6.92 - 6.87 (m, 4H), 5.57 - 5.54 (m, 1H), 4.69 - 4.49 (m, 2H), 3.96 - 3.85 (m, 1H), 3.79 (s, 6H), 3.55 - 3.11 (m, 6H), 1.09 - 1.03 (m, 3H).

### (1-6) Synthesis of Compound NM022

Compound NM022-5 (1.5 g, 2.615 mmol, 1.0 eq.) was dissolved in dry dichloromethane (15 mL). Bis(diisopropylamino)(2-cyanoethoxy)phosphine (1.18 g, 3.923 mmol, 1.5 eq.) was added, and 4,5-dicyanoimidazole (0.25 g, 2.092 mmol, 0.8 eq.) was added to the reaction system under an argon atmosphere. The reaction system was allowed to react at 25 °C for 3 hours, and was then quenched by adding cold saturated aqueous NaHCO₃ solution (100 mL). After the reaction was completed, the reaction solution was extracted with dichloromethane (100 mL × 3), and the organic phase was combined. The organic phase was first washed with saturated aqueous sodium chloride solution (100 mL × 2), then dried over anhydrous sodium sulfate, filtered and concentrated to dryness under reduced pressure. The residue was purified by reversed-phase column chromatography (column type: spherical C18; eluent: acetonitrile/water = 10/90, v/v; detector: UV-254) to give Compound NM022 as a colorless oil (1.2912 g, yield 61.38%). MS ESI (m/z) = 772.30 [M - H]⁻.

¹H NMR ((300 MHz, Acetonitrile-*d₃*) δ: 9.01 (br s, 1H), 7.43 - 7.37 (m, 2H), 7.31 - 7.21 (m, 7H), 7.15 - 6.95 (m, 1H), 6.87 - 6.81 (m, 4H), 5.54 - 5.47 (m, 1H), 4.61 - 4.38 (m, 2H), 4.16 - 3.84 (m, 1H), 3.77 - 3.13 (m, 16H), 2.61 - 2.52 (m, 2H), 1.20 - 0.90 (m, 15H).

### Preparation Example 2: Synthesis of Compound NM023

In this preparation example, the synthetic scheme of Compound NM023 is shown as follows:

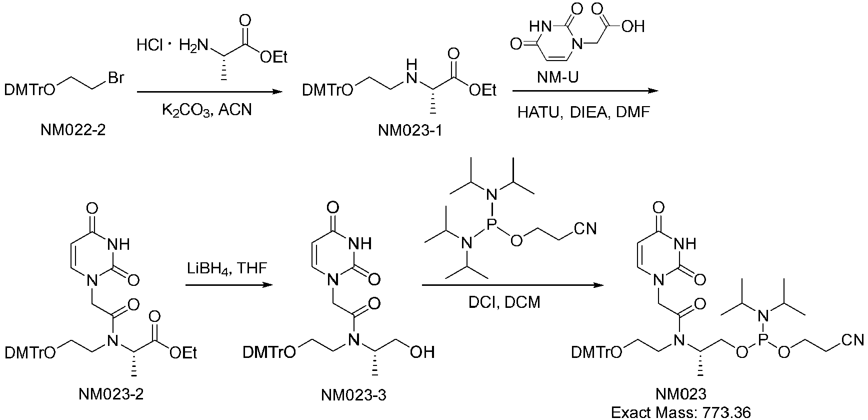

### (2-1) Synthesis of Compound NM023-1

Ethyl-(2S)-2-aminopropionate hydrochloride (6.47 g, 42.14 mmol, 1.0 eq.) and K₂CO₃ (8.73 g, 63.19 mmol, 1.5 eq.) were stirred in acetonitrile (90 mL) for 30 min. Then Compound NM022-2 (18 g, 42.121 mmol, 1.0 eq.) was added. The reaction mixture was stirred at 80 °C for 16 h, and quenched by adding saturated aqueous NaHCO₃ solution (300 mL) at 0 °C. After the reaction was completed, the reaction solution was extracted with ethyl acetate (500 mL × 3), and the organic phase was combined. The organic phase was first washed with saturated aqueous sodium chloride solution (500 mL), then dried over anhydrous sodium sulfate, filtered and concentrated to dryness under vacuum. The residue was purified by silica gel column chromatography (eluent: ethyl acetate/petroleum ether = 40/60, v/v) to give Compound NM023-1 as a yellow semi-solid (12.4 g, yield 63.50%).

¹H NMR (400 MHz, Chloroform-d) δ: 7.45 - 7.42 (m, 2H), 7.34 - 7.17 (m, 7H), 6.83 - 6.79 (m, 4H), 4.23 - 4.13 (m, 2H), 3.78 (s, 6H), 3.37 - 3.32 (m, 1H), 3.20 - 3.17 (m, 2H), 2.86 - 2.84 (m, 1H), 2.69 - 2.66 (m, 1H), 1.33 - 1.29 (m, 6H).

### (2-2) Synthesis of Compound NM023-2

Compound NM-U (4.55 g, 26.749 mmol, 1.0 eq.) and HATU (20.34 g, 53.498 mmol, 2.0 eq.) were added to N,N-dimethylformamide (80 mL), and then stirred at 25 °C for 20 min. Compound NM023-1 (12.4 g, 26.749 mmol, 1.0 eq.) and DIEA (12.10 g, 93.621 mmol, 3.5 eq.) were added. The reaction solution was stirred at 25 °C for 2 hours, and saturated aqueous NaHCO₃ solution (400 mL) at 0 °C was added for quenching. After the reaction was completed, the reaction solution was extracted with ethyl acetate (500 mL × 3), and the organic phase was combined. The organic phase was first washed with saturated aqueous sodium chloride solution (400 mL × 3), then dried over anhydrous Na₂SO₄, filtered and concentrated to dryness under vacuum. The residue was purified by silica gel column chromatography (eluent: ethyl acetate/petroleum ether = 60/40, v/v) to give Compound NM023-2 as a yellow semi-solid (12 g, yield 72.87%). MS ESI *(m*/*z)* = 614.15 [M - H]⁻.

### (2-3) Synthesis of Compound NM023-3

Under a nitrogen atmosphere at 0 °C, LiBH₄ (583.69 mg, 26.799 mmol, 1.5 eq.) was added to tetrahydrofuran (120 mL), and Compound NM023-2 (11 g, 17.866 mmol, 1.0 eq.) was added to the above solution with stirring. The reaction system was stirred at 0 °C for 1 hour and quenched by adding water (500 mL) at 0 °C. After the reaction was completed, the reaction solution was extracted with ethyl acetate (500 mL × 3), and the organic phase was combined. The organic phase was first washed with saturated aqueous sodium chloride solution (500 mL), then dried over anhydrous Na₂SO₄ and filtered. The filtrate was concentrated to dryness under vacuum. The residue was purified using the RP-Combi-flash method (chromatographic column: AQ-C18; eluent: acetonitrile/water = 5/95, v/v; detector: UV-254) to give Compound NM023-3 (6.2 g, yield 60.49%). MS ESI (m/z) = 572.20 [M - H]⁻.

¹H NMR (400 MHz, DMSO-d6) δ: 11.25 (br s, 1H), 7.42 - 7.13 (m, 10H), 6.87 - 6.83 (m, 4H), 5.50 - 5.48 (m, 1H), 4.92 - 4.62 (m, 2H), 3.96 - 3.74 (m, 1H), 3.69 - 3.68 (m, 6H), 3.43 - 3.29 (m, 3H), 3.26 - 2.98 (m, 2H), 1.04 - 0.85 (m, 3H).

### (2-4) Synthesis of Compound NM023

Compound NM023-3 (2.7 g, 4.707 mmol, 1.0 eq.) was dissolved in dry dichloromethane (15 mL). Bis(diisopropylamino)(2-cyanoethoxy)phosphine (2.13 g, 7.06 mmol, 1.5 eq.) was added, and 4,5-dicyanoimidazole (389.10 mg, 3.295 mmol, 0.7 eq.) was added under an argon atmosphere. The mixture was allowed to react at 25 °C for 3 hours, and saturated aqueous NaHCO₃ solution (100 mL) at 0 °C was added for quenching. After the reaction was completed, the reaction solution was extracted with dichloromethane (100 mL × 3), and the organic phase was combined. The organic phase was first washed with saturated aqueous sodium chloride solution (100 mL × 2), then dried over anhydrous sodium sulfate and filtered. The filtrate was concentrated to dryness under reduced pressure. The residue was purified using the RP-Flash method (column type: spherical C18; eluent: acetonitrile/water = 10/90, v/v; detector: UV-254) to give NM023 as a colorless oil (1.88 g, yield 51.88%). MS ESI (m/z) = 772.30 [M - H]⁻.

¹H NMR (400 MHz, Acetonitrile-*d₃*) δ: 8.97 (br s, 1H), 7.38 - 7.32 (m, 2H), 7.26 - 7.13 (m, 7H), 7.05 - 6.92 (m, 1H), 6.83 - 6.75 (m, 4H), 5.51 - 5.41 (m, 1H), 4.60 - 4.26 (m, 2H), 4.15 - 3.82 (m, 1H), 3.68 (s, 6H), 3.64 - 3.56 (m, 2H), 3.52 - 3.36 (m, 4H), 3.33 - 3.20 (m, 3H), 3.17 - 3.04 (m, 1H), 2.54 - 2.48 (m, 2H), 1.13 - 0.97 (m, 15H).

### Preparation Example 3: Synthesis of Compound NM036

In this preparation example, the synthetic scheme of Compound NM036 is shown as follows:

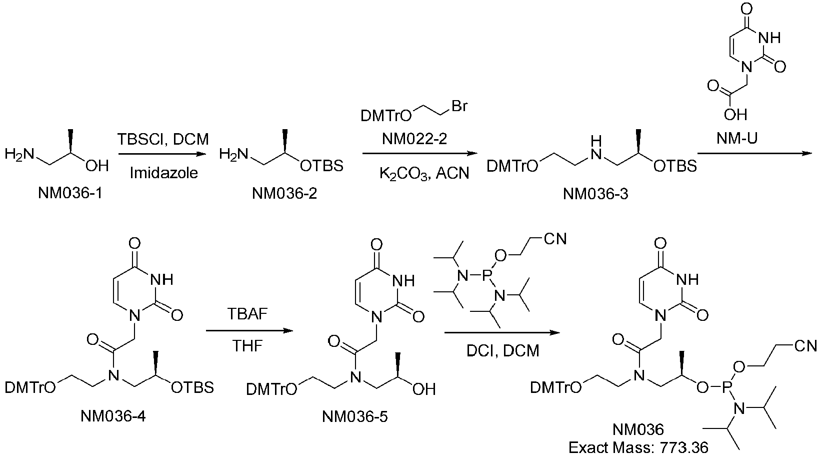

### (3-1) Synthesis of Compound NM036-2

Compound NM036-1 (3.0 g, 39.96 mmol, 1.0 eq, (R)-1-aminopropan-2-ol, CAS No. 2799-17-8) was dissolved in 30 mL of dichloromethane, imidazole (4.08 g, 59.94 mmol, 1.5 eq) was added. The reaction mixture was cooled to 0 °C in an ice bath. tert-butyldimethylchlorosilane (7.84 g, 51.95 mmol, 1.3 eq, TBSCl) was then added, and the mixture was allowed to react at 25 °C for 16 hours with stirring. After the reaction was completed, dichloromethane (30 mL) was added to the reaction solution for dilution, then washed with saturated aqueous ammonium chloride solution (2×50 mL) and saturated aqueous sodium chloride solution (2×50 mL), dried over anhydrous sodium sulfate, filtered and concentrated to give Compound NM036-2 as a yellow oil (7.5 g), which was directly used for the next reaction without purification. MS-ESI (m/z) = 190 [M + H]⁺.

### (3-2) Synthesis of Compound NM036-3

Compound NM036-2 (3.88 g, 22.65 mmol, 1.0 eq) was dissolved in 100 mL of acetonitrile, K₂CO₃ (4.68 g, 33.98 mmol, 1.5 eq) and Compound NM022-2 (9.65 g, 22.65 mmol, 1.0 eq) were added. The mixture was allowed to react at 80 °C for 16 hours with stirring. After the reaction was completed, saturated aqueous sodium bicarbonate solution (100 mL) was added to the reaction solution, extracted with ethyl acetate (2×100 mL), and the organic phase was combined. The organic phase was first washed with saturated aqueous sodium chloride solution (2×50 mL), then dried over anhydrous sodium sulfate, filtered, concentrated and purified by column chromatography (eluent: petroleum ether/ethyl acetate = 5/1, v/v) to give Compound NM036-3 as a white solid (3.48 g, 6.50 mmol, yield 28.70%). MS-ESI (m/z) = 536 [M + H]⁺.

### (3-3) Synthesis of Compound NM036-4

Compound NM036-3 (1.91 g, 11.2 mmol, 2.0 eq) was dissolved in N,N-dimethylformamide (30 mL), and cooled to 0 °C in an ice bath. EDCI (1.61 g, 8.4 mmol, 1.5 eq) and triethylamine (1.98 g, 19.6 mmol, 3.5 eq) were added at 0 °C, and stirred at 0 °C for 10 minutes. Then 1-hydroxybenzotriazole (1.14 g, 8.4 mmol, 1.5 eq) was added at 0 °C. The reaction mixture was slowly raised to 25 °C, and Compound NM-U (3.0 g, 5.6 mmol, 1.0 eq) was added at 25 °C, followed by reaction at 25 °C for 16 hours with stirring. After the reaction was completed, the reaction solution was added with saturated aqueous sodium bicarbonate solution (200 mL) and extracted with ethyl acetate (2×100 mL), and the organic phase was combined. The organic phase was first washed with saturated aqueous sodium chloride solution (2×50 mL), then dried over anhydrous sodium sulfate, filtered, concentrated and purified by column chromatography (eluent: petroleum ether/ethyl acetate = 2/3, v/v) to give Compound NM036-4 as a white solid (3.83 g, 5.57 mmol, yield 99.5%). MS-ESI (m/z) = 688 [M + H]⁺.

### (3-4) Synthesis of Compound NM036-5

Compound NM036-4 (3.34 g, 4.86 mmol, 1.0 eq) was dissolved in 30 mL of tetrahydrofuran, and a tetrahydrofuran solution of tetrabutylammonium fluoride (1 M, 14.6 mL, 14.6 mmol, 3.0 eq) was added, followed by reaction at 25 °C for 16 hours with stirring. After the reaction was completed, the reaction solution was added with saturated aqueous sodium bicarbonate solution (100 mL) and extracted with ethyl acetate (2×100 mL), and the organic phase was combined. The organic phase was first washed with saturated brine (2×50 mL), then dried over anhydrous sodium sulfate, filtered, concentrated and purified by column chromatography (eluent: dichloromethane/methanol = 20/1, v/v) to give Compound NM036-5 as a white solid (2.56 g, 4.46 mmol, yield 91.77%). MS-ESI (m/z) = 574 [M + H]⁺.

### (3-5) Synthesis of Compound NM036

Compound NM036-5 (2.33 g, 4.06 mmol, 1.0 eq) was dissolved in 30 mL of anhydrous dichloromethane, and DCI (384 mg, 3.25 mmol, 0.8 eq) and bis(diisopropylamino)(2-cyanoethoxy)phosphine (1.48 g, 4.87 mmol, 1.2 eq) were added respectively. After purging with nitrogen 3 times, the reaction solution was allowed to react at 25 °C for 1 hour with stirring. After the reaction was completed, the reaction solution was first washed with saturated aqueous sodium bicarbonate solution (2×20 mL), then washed with saturated brine (20 mL), and the organic phase was separated. The organic phase was dried over anhydrous sodium sulfate, filtered, concentrated and purified by column chromatography (eluent: water/acetonitrile = 5/95, v/v) to give Compound NM036 as a white solid (2.7 g, 3.49 mmol, yield 85.96%). MS-ESI (m/z) = 774 [M + H]⁺.

¹H NMR (400 MHz, DMSO-*d₆*) δ: 11.30 (d, J = 11.3 Hz, 1H), 7.34 (dt, J = 16.1, 8.0 Hz, 4H), 7.22 (ddt, J = 12.1, 9.1, 4.4 Hz, 5H), 6.89 (d, J = 8.7 Hz, 4H), 4.59 (q, J = 10.9, 8.8 Hz, 2H), 3.73 (s, 6H), 3.71 - 3.36 (m, 7H), 3.36 - 3.20 (m, 3H), 3.15 - 3.03 (m, 1H), 2.72 (ddt, J = 24.1, 11.9, 5.8 Hz, 2H), 1.32 - 0.99 (m, 15H).

### Preparation Example 4: Synthesis of Compound NM037

In this preparation example, the synthetic scheme of Compound NM037 is shown as follows:

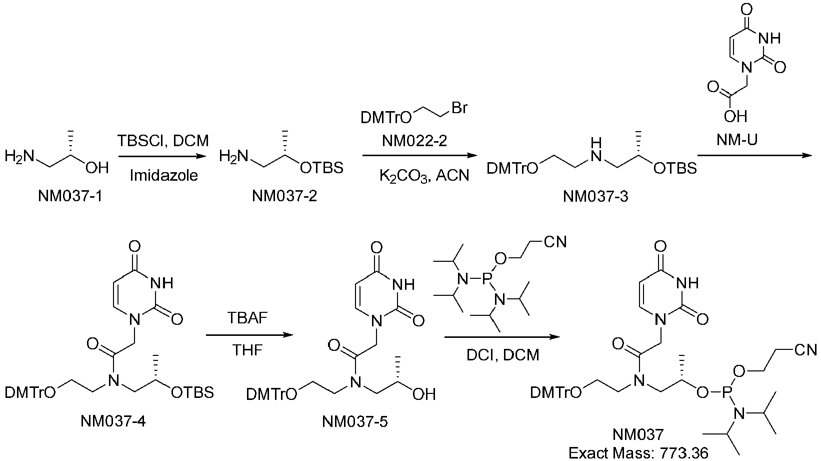

### (4-1) Synthesis of Compound NM037-2

Compound NM037-1 (3 g, 39.96 mmol, 1.0 eq, (S)-1-aminopropan-2-ol, CAS No. 2799-17-9) was dissolved in 30 mL of dichloromethane, imidazole (4.08 g, 59.94 mmol, 1.5 eq) was added. The mixture was cooled to 0 °C in an ice bath, and tert-butyldimethylchlorosilane (7.84 g, 51.95 mmol, 1.3 eq) was added at 0 °C, followed by reaction at 25 °C for 16 hours with stirring. After the reaction was completed, the reaction solution was added with dichloromethane (30 mL) for dilution, washed with saturated aqueous ammonium chloride solution (2×50 mL) and saturated aqueous sodium chloride solution (2×50 mL), dried over anhydrous sodium sulfate, filtered and concentrated to give Compound NM037-2 as a yellow oil (5 g). MS-ESI (m/z) = 190 [M + H]⁺.

### (4-2) Synthesis of Compound NM037-3

Compound NM037-2 (3 g, 15.84 mmol, 1.0 eq) was dissolved in 100 mL of acetonitrile, K₂CO₃ (3.28 g, 23.76 mmol, 1.5 eq) and Compound NM022-2 (6.77 g, 15.84 mmol, 1.0 eq) were added, followed by reacting at 80 °C for 30 hours with stirring. After the reaction was completed, the reaction solution was added with saturated aqueous sodium bicarbonate solution (100 mL), extracted with ethyl acetate (2×100 mL), and the organic phase was combined. The organic phase was washed with saturated aqueous sodium chloride solution (2×50 mL), dried over anhydrous sodium sulfate, filtered, concentrated and purified by column chromatography (eluent: petroleum ether/ethyl acetate = 5/1, v/v) to give Compound NM037-3 as a white solid (3.5 g, 6.50 mmol, yield 41.2%). MS-ESI (m/z) = 536 [M + H]⁺.

### (4-3) Synthesis of Compound NM037-4

Compound NM037-3 (0.95 g, 6.16 mmol, 1.1 eq) was dissolved in 30 mL of N,N-dimethylformamide, cooled to 0 °C in an ice bath and stirred at 0 °C for 10 minutes. HATU (3.19 g, 8.4 mmol, 1.5 eq) and DIEA (2.05 g, 16.8 mmol, 3 eq) were then added at 0 °C. After slowly warming up to 25 °C, Compound NM-U (3.0 g, 5.6 mmol, 1.0 eq) was added at 25 °C, followed by reaction at 25 °C for 16 hours with stirring. After the reaction was completed, the reaction solution was added with saturated aqueous sodium bicarbonate solution (200 mL), and extracted with ethyl acetate (2×100 mL), and the organic phase was combined. The organic phase was washed with saturated aqueous sodium chloride solution (2×50 mL), dried over anhydrous sodium sulfate, filtered, concentrated and purified by column chromatography (eluent: petroleum ether/ethyl acetate = 2/3, v/v) to give Compound NM037-4 as a white solid (1.5 g, 2.18 mmol, yield 56.5%). MS-ESI (m/z) = 688 [M + H]⁺.

### (4-4) Synthesis of Compound NM037-5

Compound NM037-4 (1.4 g, 2.03 mmol, 1.0 eq) was dissolved in 30 mL of tetrahydrofuran, and a tetrahydrofuran solution of tetrabutylammonium fluoride (1 M, 6.09 mL, 6.09 mmol, 3.0 eq) was added, followed by reaction at 25 °C for 16 hours with stirring. After the reaction was completed, the reaction solution was added with saturated aqueous sodium bicarbonate solution (100 mL) and extracted with ethyl acetate (2×100 mL), and the organic phase was combined. The organic phase was washed with saturated brine (2×50 mL), dried over anhydrous sodium sulfate, filtered, concentrated and purified by column chromatography (eluent: dichloromethane/methanol = 20/1, v/v) to give Compound NM037-5 as a white solid (1 g, 1.74 mmol, yield 71.4%). MS-ESI (m/z) = 574 [M + H]⁻.

### (4-5) Synthesis of Compound NM037

Compound NM037-5 (1 g, 1.74 mmol, 1.0 eq) was dissolved in 30 mL of anhydrous dichloromethane, and DCI (161 mg, 1.39 mmol, 0.8 eq) and bis(diisopropylamino)(2-cyanoethoxy)phosphine (629 mg, 2.08 mmol, 1.2 eq) were added respectively. The reaction system was purged with nitrogen 3 times, and the reaction solution was allowed to react at 25 °C for 1 hour with stirring. After the reaction was completed, the reaction solution was washed with saturated aqueous sodium bicarbonate solution (2×20 mL) and saturated aqueous sodium chloride solution (20 mL), dried over anhydrous sodium sulfate, filtered, concentrated and purified by column chromatography (eluent: water/acetonitrile = 5/95, v/v) to give Compound NM037 as a white solid (670 mg, 3.49 mmol, yield 50%). MS-ESI (m/z) = 774 [M + H]⁺.

¹H NMR (400 MHz, DMSO-*d₆*) δ: 11.30 (d, J = 11.3 Hz, 1H), 7.45 (dd, J = 7.7, 4.5 Hz, 2H), 7.39-7.15 (m, 7H), 6.98-6.85 (m, 5H), 5.69-5.47 (m, 1H), 4.89-4.44 (m, 2H), 3.63-3.5 (ddt, J - 19.9, 13.6, 6.5 Hz, 10H), 3.47- 3.15 (m, 4H), 2.64 (ddt, J = 17.7, 12.6, 5.9 Hz, 2H), 2.17 (s, 2H), 1.39-1.04 (m, 15H).

### Preparation Example 5: Synthesis of Compound NM084

In this preparation example, the synthetic scheme of Compound NM084 is shown as follows:

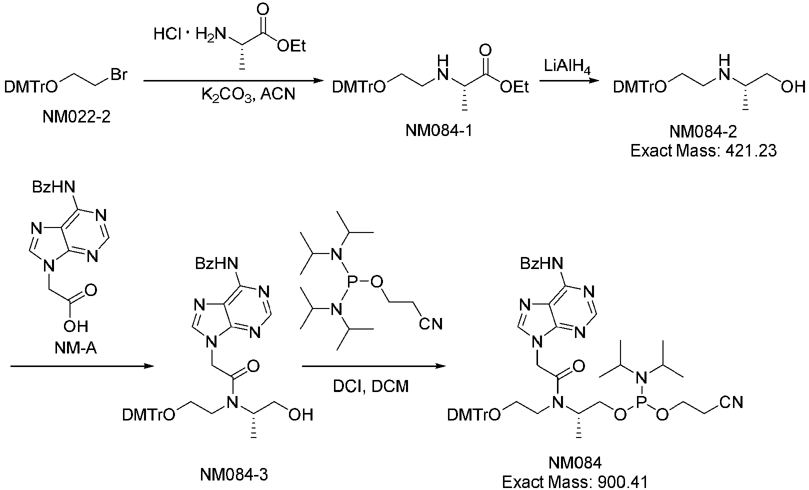

### (5-1) Synthesis of Compound NM084-1

Compound NM022-2 (64 g, 0.15 mol) and L-alanine ethyl ester hydrochloride (23 g, 0.15 mol) were added to a 2000 mL reactor, and potassium carbonate (62 g, 0.45 mol) and acetonitrile (640 mL) were added. The reaction solution was warmed to 80 °C, and stirred at 80 °C for 16 hours. After the reaction was completed, the reaction solution was filtered, and the filtrate was concentrated, and purified by column chromatography to give Compound NM084-1 as a yellow oil (31 g, yield 44.5%). MS ESI (m/z) = 464.0 [M + H]⁻.

### (5-2) Synthesis of Compound NM084-2

Compound NM084-1 (7 g, 0.015 mol) and tetrahydrofuran (42 mL) were added to a 100 mL reactor. The temperature was cooled down to 0 °C, and a 2 M tetrahydrofuran solution of lithium aluminum hydride (15 mL, 0.0486 mol) was added dropwise at 0 °C. The reactor was purged with nitrogen 3 times, and the reaction solution was stirred at 25 °C for 1 hour, and slowly added with 20 mL of purified water. Ethyl acetate was added for extraction, and the organic phase was separated. The organic phase was dried over anhydrous sodium sulfate, filtered with suction and concentrated to give Compound NM084-2 as a white solid (5 g, yield 78.6%). MS ESI (m/z) = 422 [M + H]⁺.

### (5-3) Synthesis of Compound NM084-3

Compound NM084-2 (3 g, 6.5 mmol), Compound NM-A (2.12 g, 7.1 mmol), HATU (3.69 g, 9.7 mmol), DIEA (1.67 g, 13 mmol) and N,N-dimethylformamide (30 mL) were added to a 250 mL reactor. The mixture was purged with nitrogen 3 times, and stirred at 25 °C for 3 hours. After the reaction was completed, the reaction solution was added with purified water (30 mL) and ethyl acetate (30 mL) for extraction, and the organic phase was separated. The organic phase was dried over anhydrous sodium sulfate, filtered with suction, concentrated and purified by column chromatography to give Compound NM084-3 as a white solid (2 g, yield 40.2%). MS ESI (m/z) = 701 [M + H]⁺.

### (5-4) Synthesis of Compound NM084

Compound NM084-3 (1.6 g, 2.3 mmol) was added to a 100 mL reactor and cooled down to 0 °C. Bis(diisopropylamino)(2-cyanoethoxy)phosphine (0.83 g, 2.77 mmol), 4,5-dicyanoimidazole (0.215 g, 1.8 mmol) and dichloromethane (16 mL) were added in batches at 0 °C. The mixture was purged with nitrogen 3 times, and stirred at 25 °C for 3 hours. After the reaction was completed, 20 mL of saturated aqueous sodium bicarbonate solution was added to the reaction solution, and the organic phase was separated. The organic phase was dried over anhydrous sodium sulfate, filtered with suction, concentrated and purified by reversed-phase column chromatography (eluent: acetonitrile/water = 80/20, v/v) to give Compound NM084 as a white solid (1.6 g, yield 77.7%). MS ESI (m/z) = 901 [M + H]⁺.

¹H NMR (400 MHz, DMSO-d6) δ 11.13 (s, 1H), 8.72 - 8.56 (m, 1H), 8.28 - 8.15 (m, 1H), 8.05 (d, J = 7.6 Hz, 2H), 7.70 - 7.61 (m, 1H), 7.61 - 7.49 (m, 2H), 7.49 - 7.12 (m, 9H), 6.93 - 6.79 (m, 4H), 5.18 (d, J = 17.8 Hz, 2H), 3.71 (d, J = 4.3 Hz, 7H), 3.57 (dt, J = 19.4, 9.1 Hz, 5H), 3.34 (s, 2H), 3.14 (s, 1H), 2.70 (q, J = 8.0, 6.1 Hz, 2H), 2.07 (d, J = 1.4 Hz, 2H), 1.39 - 0.84 (m, 15H).

### Preparation Example 6: Synthesis of Compound NM085

In this preparation example, the synthetic scheme of Compound NM085 is shown as follows:

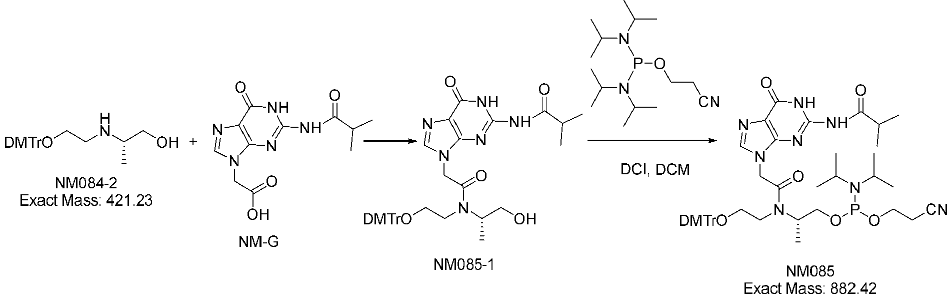

### (6-1) Synthesis of Compound NM-G

In this preparation example, the synthetic scheme of Compound NM-G is shown as follows:

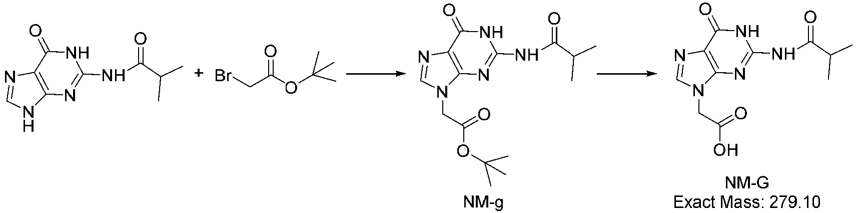

### (6-1-1) Synthesis of Compound NM-g

N2-isobutyrylguanine (15 g, 0.0679 mol), tert-butyl bromoacetate (14.55 g, 0.0746 mol), potassium carbonate (18.73 g, 0.136 mol) and N,N-dimethylformamide (150 mL) were added to a 250 mL reactor. The mixture was purged with nitrogen 3 times, and stirred at 25 °C for 6 hours. After the reaction was completed, the reaction solution was filtered. Purified water (300 mL) and ethyl acetate (300 mL) were added to the filtrate for extraction, and the organic phase was separated. The organic phase was dried over anhydrous sodium sulfate, filtered with suction, concentrated and purified by column chromatography to give Compound NM-g (7.7 g, yield 34%). MS ESI (m/z) = 336 [M + H]⁻.

### (6-1-2) Synthesis of Compound NM-G

Compound NM-g (7.7 g, 0.023 mol), trifluoroacetic acid (77 mL) and water (0.77 mL) were added to a 250 mL reactor, and stirred at 25 °C for 1 hour. After the reaction was completed, the reaction solution was concentrated, slurried with ethyl acetate and filtered with suction to give Compound NM-G (4 g, yield 62.5%). MS ESI (m/z) = 280 [M + H]⁺.

### (6-2) Synthesis of Compound NM085-1

Compound NM084-2 (5 g, 0.012 mol), Compound NM-G (2.77 g, 0.01 mol), HATU (5.64 g, 0.015 mol), DIEA (2.55 g, 0.02 mol) and N,N-dimethylformamide (28 mL) were added to a 250 mL reactor. The reaction solution was purged with nitrogen 3 times, and stirred at 25 °C for 3 hours. After the reaction was completed, purified water (80 mL) and ethyl acetate (80 mL) were added to the reaction solution for extraction, and the organic phase was separated. The organic phase was dried over anhydrous sodium sulfate, filtered with suction, concentrated and purified by column chromatography to give Compound NM085-1 (3.1 g, yield 45.8%). MS ESI (m/z) = 683 [M + H]⁺.

### (6-3) Synthesis of Compound NM085

Compound NM085-1 (3.1 g, 0.0045 mol) was added to a 100 mL reactor. The temperature was cooled down to 0 °C, and bis(diisopropylamino)(2-cyanoethoxy)phosphine (1.78 g, 0.0055 mol), 4,5-dicyanoimidazole (0.428 g, 0.0036 mol) and dichloromethane (31 mL) were added in batches at 0 °C. The reaction solution was purged with nitrogen 3 times, and stirred at 25 °C for 3 hours. After the reaction was completed, saturated aqueous sodium bicarbonate solution (30 mL) was added to the reaction solution, and the organic phase was separated. The organic phase was dried over anhydrous sodium sulfate, filtered with suction, concentrated and purified by reversed-phase column chromatography (eluent: can acetonitrile/water = can 85/15, v/v) to give Compound NM085 (2.2 g, yield 55%). MS ESI (m/z) = 884 [M + H]⁺.

¹H NMR (400 MHz, Chloroform-d) δ 11.31 - 11.07 (s, 1H), δ 7.44 - 7.15 (m, 9H), 6.88 (dd, J - 18.4, 8.1 Hz, 5H), 5.14 - 4.85 (m, 2H), 3.71 (s, 8H), 3.60 - 3.33 (m, 5H), 3.10 (s, 1H), 2.83 - 2.64 (m, 2H), 2.50 (s, 2H), 1.25 - 0.99 (m, 21H).

### Preparation Example 7: Synthesis of Compound NM086

In this preparation example, the synthetic scheme of Compound NM086 is shown as follows:

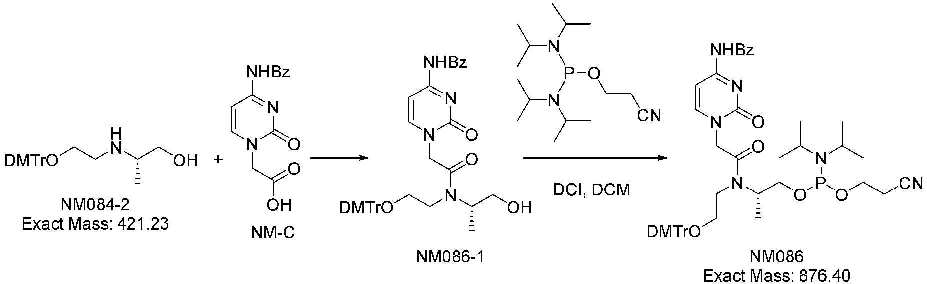

### (7-1) Synthesis of Compound NM-C

In this preparation example, the synthetic scheme of Compound NM-C is shown as follows:

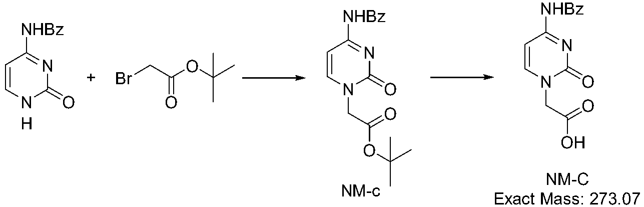

### (7-1-1) Synthesis of Compound NM-c

N4-benzoylcytosine (9.5 g, 0.0442 mol), tert-butyl bromoacetate (9.04 g, 0.0464 mol), potassium carbonate (12.2 g, 0.0884 mol) and N,N-dimethylformamide (95 mL) were added to a 250 mL reactor. The reaction solution was purged with nitrogen 3 times, and stirred at 25 °C for 3 hours. After the reaction was completed, the reaction solution was filtered. Purified water (100 mL) and ethyl acetate (100 mL) were added for extraction, and the organic phase was separated. The organic phase was dried over anhydrous sodium sulfate, filtered with suction, concentrated and purified by column chromatography to give Compound NM-c (8.5 g, yield 58.6%). MS ESI (m/z) - 330 [M + H]⁺.

### (7-1-2) Synthesis of Compound NM-C

Compound NM-c (8.5 g, 0.0258 mol), trifluoroacetic acid (85 mL) and water (0.85 mL) were added to a 250 mL reactor, and stirred at 25 °C for 1 hour. After the reaction was completed, the reaction solution was concentrated to give Compound NM-C (5.4 g, yield 77.1%). MS ESI (m/z) = 274 [M + H]⁺.

### (7-2) Synthesis of Compound NM086-1

Compound NM084-2 (4.68 g, 0.0111 mol), Compound NM-C (2.33 g, 0.0085 mol), HATU (4.86 g, 0.0128 mol), DIEA (2.2 g, 0.017 mol) and N,N-dimethylformamide (47 mL) were added to a 250 mL reactor. The reaction solution was purged with nitrogen 3 times, and stirred at 25 °C for 3 hours. After the reaction was completed, purified water (80 mL) and ethyl acetate (80 mL) were added to the reaction solution for extraction, and the organic phase was separated. The organic phase was dried over anhydrous sodium sulfate, filtered with suction, concentrated and purified by reversed-phase column chromatography (eluent: acetonitrile/water = 70/30, v/v) to give Compound NM086-1 (2.5 g, yield 43.3%). MS ESI (m/z) = 677 [M + H]⁺.

### (7-3) Synthesis of Compound NM086

Compound NM086-1 (2.5 g, 0.0037 mol) was added to a 100 mL reactor. The temperature was cooled down to 0 °C, and bis(diisopropylamino)(2-cyanoethoxy)phosphine (1.4 g, 0.0046 mol), 4,5-dicyanoimidazole (0.334 g, 0.0028 mol) and dichloromethane (25 mL) were added in batches at 0 °C. The reaction solution was purged with nitrogen 3 times, and stirred at 25 °C for 3 hours. After the reaction was completed, saturated aqueous sodium bicarbonate solution (30 mL) was added to the reaction solution, and the organic phase was separated. The organic phase was dried over anhydrous sodium sulfate, filtered with suction, concentrated and purified by reversed-phase column chromatography (eluent: acetonitrile/water = 85/15) to give Compound NM086 (1.4 g, yield 38.9%). MS ESI (m/z) = 977 [M + H]⁺.

¹H NMR (400 MHz, DMSO-*d₆*) δ 11.31 - 11.07 (s, 1H), 8.07 - 8.00 (d, J = 7.7 Hz, 2H), 7.94 - 7.81 (d, J = 7.2 Hz, 1H), 7.71 - 7.61 (t, J = 7.4 Hz, 1H), 7.56 - 7.49 (d, J = 7.6 Hz, 2H), 7.48 - 7.20 (m, 10H), 6.97 - 6.85 (t, J = 8.1 Hz, 4H), 4.77 - 4.60 (m, 1H), 4.22 - 4.03 (tt, J = 13.5, 7.2 Hz, 1H), 3.82 - 3.69 (s, 7H), 3.68 - 3.44 (m, 7H), 3.44 - 3.36 (q, J = 11.2, 7.4 Hz, 2H), 3.19 - 3.10 (d, J = 8.3 Hz, 1H), 2.84 - 2.67 (dt, J = 23.5, 5.6 Hz, 2H), 1.21 - 1.02 (m, 15H).

### Preparation Example 8: Synthesis of Compound NM102

In this preparation example, the synthetic scheme of Compound NM102 is shown as follows:

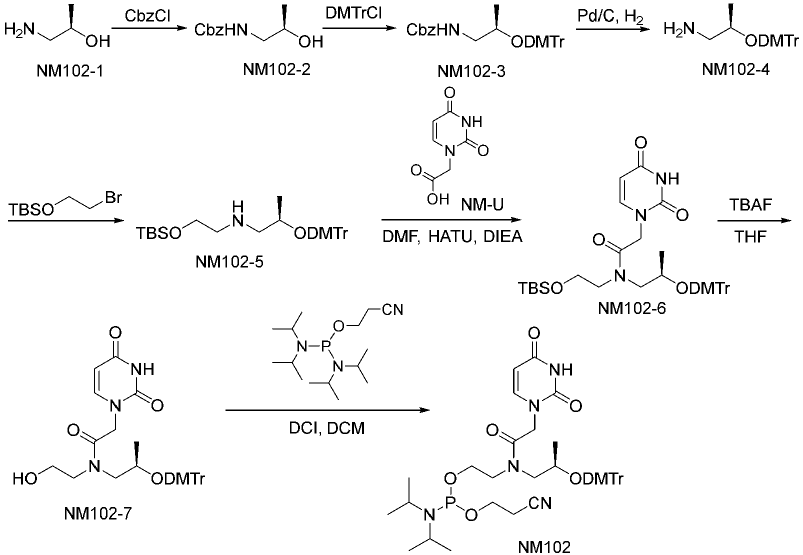

### (8-1) Synthesis of Compound NM102-2

Compound NM102-1 (10 g, 0.13 mol, 1 eq) was dissolved in 100 mL of a mixed solution of tetrahydrofuran and water (volume ratio of tetrahydrofuran to water of 1:4). Sodium bicarbonate (21.8 g, 0.26 mol, 2 eq) and benzyl chloroformate (23.3 g, 0.14 mol, 1.05 eq) were added sequentially at 0 °C. After the addition, the reaction solution was warmed to 25 °C, and was allowed to react for 2 hours with stirring. After the reaction was completed, the reaction solution was extracted with ethyl acetate (3×100 mL), and the organic phase was combined. The organic phase was washed with saturated aqueous sodium chloride solution (2×50 mL), dried over anhydrous sodium sulfate, filtered, concentrated and purified by column chromatography to give Compound NM102-2 as a white solid (20 g, yield 69.4%). MS-ESI (m/z) = 210.2 [M + H]⁺.

### (8-2) Synthesis of Compound NM102-3

Compound NM102-2 (20 g, 95.7 mmol, 1.0 eq) was dissolved in 200 mL of pyridine and cooled to 0 °C in an ice bath. DMTrCl (4,4'-dimethoxytrityl chloride, 48.7 g, 143.6 mmol, 1.5 eq) was added in batches, and stirred at 0 °C for 1 hour. Methanol was added to quench. After the reaction was completed, the reaction solution was concentrated to remove pyridine. Saturated aqueous ammonium chloride solution (150 mL) was added. The mixture was extracted with ethyl acetate (2×250 mL), and the organic phase was combined. The organic phase was washed with saturated brine (150 mL), dried over anhydrous sodium sulfate, filtered, concentrated and purified by column chromatography (eluent: petroleum ether/ethyl acetate = 1/1, v/v) to give Compound NM102-3 as a yellow solid (35 g, yield 71.7%). MS-ESI (m/z) = 512.2 [M + H]⁺.

### (8-3) Synthesis of Compound NM102-4

Compound NM102-3 (35 g, 68.5 mmol, 1.0 eq) was dissolved in 350 mL of methanol, and 3.5 g of wet palladium on carbon (10%, 0.1 g/g) was added. The reaction solution was purged with hydrogen 3 times and was then stirred at 25 °C for 6 hours. After the reaction was completed, the reaction solution was filtered with suction, and the filtrate was concentrated to give Compound NM102-4 as a yellow solid (20 g, yield 77.5%). MS-ESI (m/z) = 378.3 [M + H]⁺.

### (8-4) Synthesis of Compound NM102-5

Compound NM102-4 (20 g, 52.9 mmol, 1 eq) was dissolved in 200 mL of acetonitrile. Potassium carbonate (14.6 g, 105.8 mmol, 2 eq) and (2-bromoethoxy)-tert-butyldimethylsilane (19.0 g, 79.4 mmol, 1.5 eq) were added sequentially, and stirred at 80 °C for 12 hours. After the reaction was completed, the reaction solution was cooled down to 25 °C, added with 100 mL of water, and extracted with ethyl acetate (3×150 mL). The organic phase was combined. The organic phase was washed with saturated brine (2×50 mL), dried over anhydrous sodium sulfate, filtered, concentrated and purified by column chromatography to give Compound NM102-5 as a white solid (25 g, yield 88.4%). MS-ESI (m/z) = 536.2 [M + H]⁺.

### (8-5) Synthesis of Compound NM102-6

Compound NM102-5 (10 g, 18.7 mmol, 1 eq) was dissolved in 100 mL of DMF. HATU (10.6 g, 28.1 mmol, 1.5 eq), Compound NM-U (4.8 g, 28.1 mmol, 1.5 eq) and DIEA (6.03 g, 46.75 mmol, 2.5 eq) were added sequentially, and stirred at 25 °C for 2 hours. After the reaction was completed, the reaction solution was added with 100 mL of water and extracted with ethyl acetate (3×100 mL), and the organic phase was combined. The organic phase was washed with saturated brine (5×50 mL), dried over anhydrous sodium sulfate, filtered, concentrated and purified by column chromatography to give Compound NM102-6 as a white solid (10 g, yield 78.1%). MS-ESI (m/z) = 688.2 [M - H]⁺.

### (8-6) Synthesis of Compound NM102-7

Compound NM102-6 (10 g, 14.5 mmol, 1 eq) was dissolved in 100 mL of tetrahydrofuran, a 1 M tetrahydrofuran solution of tetrabutylammonium fluoride (21.8 mL, 21.8 mmol, 1.5 eq) was added, and the mixture was stirred at 25 °C for 2 hours. After the reaction was completed, the reaction solution was added with 100 mL of water and extracted with ethyl acetate (3×100 mL), and the organic phase was combined. The organic phase was washed with water (4×50 mL), dried over anhydrous sodium sulfate, filtered, concentrated and purified by column chromatography to give Compound NM102-7 as a white solid (6.5 g, yield 78.3%). MS-ESI (m/z) - 574.2 [M + H]⁺.

### (8-7) Synthesis of Compound NM102

Compound NM102-7 (3.0 g, 5.23 mmol, 1.0 eq) was dissolved in 30 mL of anhydrous dichloromethane. DCI (493 mg, 4.18 mmol, 0.8 eq) and bis(diisopropylamino)(2-cyanoethoxy)phosphine (1.65 g, 5.49 mmol, 1.05 eq) were added respectively. The reaction solution was purged with nitrogen 3 times, and stirred at 25 °C for 1 hour. After the reaction was completed, the reaction solution was washed by adding saturated aqueous sodium bicarbonate solution (2×30 mL). The organic phase was washed with saturated brine (20 mL), dried over anhydrous sodium sulfate, filtered, concentrated and purified by column chromatography (eluent: water/acetonitrile = 10/90, v/v) to give Compound NM102 as a white solid (2.5 g, yield 62.5%). MS-ESI (m/z) = 774 [M + H]⁺.

### Preparation Example 9: Synthesis of Compound NM103

In this preparation example, the synthetic scheme of Compound NM103 is shown as follows:

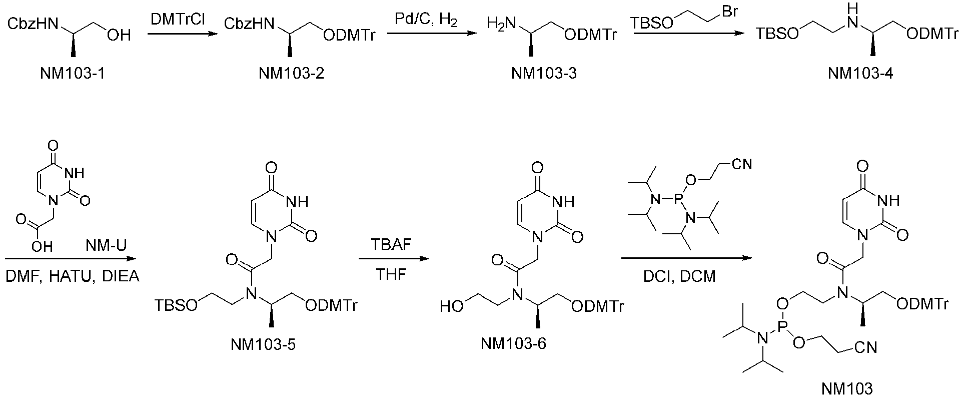

### (9-1) Synthesis of Compound NM103-2

Compound NM103-1 (20 g, 95.7 mmol, 1.0 eq) was dissolved in 200 mL of pyridine, and cooled to 0 °C in an ice bath. DMTrCl (4,4'-dimethoxytrityl chloride, 48.7 g, 143.6 mmol, 1.5 eq) was added in batches. The mixture was stirred at 0 °C for 1 hour, and quenched by adding methanol. After the reaction was completed, the reaction solution was concentrated to remove pyridine, added with saturated aqueous ammonium chloride solution (150 mL), and extracted with ethyl acetate (2×250 mL). The organic phase was combined. The organic phase was washed with saturated brine (150 mL), dried over anhydrous sodium sulfate, filtered, concentrated and purified by column chromatography (eluent: petroleum ether/ethyl acetate = 1/1, v/v) to give Compound NM103-2 as a yellow solid (30 g, yield 61.3%). MS-ESI (m/z) = 512.2 [M + H]⁺.

### (9-2) Synthesis of Compound NM 103-3

Compound NM103-2 (30 g, 58.6 mmol, 1.0 eq) was dissolved in 300 mL of methanol, 3.0 g of wet palladium on carbon (10%, 0.1 g/g) was added, and the mixture was purged with hydrogen 3 times. The mixture was then stirred at 25 °C for 6 hours. After the reaction was completed, the reaction solution was filtered with suction, and the filtrate was concentrated to give Compound NM103-3 as a yellow solid (20 g, yield 90.9%). MS-ESI (m/z) = 378.3 [M + H]⁺.

### (9-3) Synthesis of Compound NM 103-4

Compound NM103-3 (20 g, 52.9 mmol, 1 eq) was dissolved in 200 mL of acetonitrile. Potassium carbonate (14.6 g, 105.8 mmol, 2 eq) and (2-bromoethoxy)-tert-butyldimethylsilane (19.0 g, 79.4 mmol, 1.5 eq) were added sequentially, and then stirred at 80 °C for 12 hours. After the reaction was completed, the reaction solution was cooled down to 25 °C, added with 100 mL of water, and extracted with ethyl acetate (3×150 mL). The organic phase was combined. The organic phase was washed with saturated brine (2×50 mL), dried over anhydrous sodium sulfate, filtered, concentrated and purified by column chromatography to give Compound NM103-4 as a white solid (25 g, yield 88.4%). MS-ESI (m/z) = 536.2 [M + H]⁺.

### (9-4) Synthesis of Compound NM103-5

Compound NM103-4 (10 g, 18.7 mmol, 1 eq) was dissolved in 100 mL of DMF. HATU (10.6 g, 28.1 mmol, 1.5 eq), Compound NM-U (4.8 g, 28.1 mmol, 1.5 eq) and DIEA (6.03 g, 46.75 mmol, 2.5 eq) were added sequentially, and stirred at 25 °C for 2 hours. After the reaction was completed, the reaction solution was added with 100 mL of water and extracted with ethyl acetate (3×100 mL), and the organic phase was combined. The organic phase was washed with saturated brine (5×50 mL), dried over anhydrous sodium sulfate, filtered, concentrated and purified by column chromatography to give Compound NM103-5 as a white solid (10 g, yield 78.1%). MS-ESI (m/z) = 688.2 [M - H]⁺.

### (9-5) Synthesis of Compound NM103-6

Compound NM103-5 (10 g, 14.5 mmol, 1 eq) was dissolved in 100 mL of tetrahydrofuran, and a 1 M tetrahydrofuran solution of tetrabutylammonium fluoride (21.8 mL, 21.8 mmol, 1.5 eq) was added. The reaction solution was stirred at 25 °C for 2 hours. After the reaction was completed, the reaction solution was added with 100 mL of water and extracted with ethyl acetate (3×100 mL), and the organic phase was combined. The organic phase was washed with water (4×50 mL), dried over anhydrous sodium sulfate, filtered, concentrated and purified by column chromatography to give Compound NM103-6 as a white solid (6.5 g, yield 78.3%). MS-ESI (m/z) = 574.2 [M + H]⁺.

### (9-6) Synthesis of Compound NM103

Compound NM103-6 (3.0 g, 5.23 mmol, 1.0 eq) was dissolved in 30 mL of anhydrous dichloromethane. DCI (493 mg, 4.18 mmol, 0.8 eq) and bis(diisopropylamino)(2-cyanoethoxy)phosphine (1.65 g, 5.49 mmol, 1.05 eq) were added respectively. The reaction solution was purged with nitrogen 3 times, and stirred at 25 °C for 1 hour. After the reaction was completed, the reaction solution was washed with saturated aqueous sodium bicarbonate solution (2×30 mL). The organic phase was washed with saturated brine (20 mL), dried over anhydrous sodium sulfate, filtered, concentrated and purified by column chromatography (elution gradient: water/acetonitrile = 10/85) to give Compound NM103 as a white solid (2.7 g, yield 67.5%). MS-ESI (*m*/*z*) = 774 [M - H]⁺.

### Preparation Example 10: Synthesis of Compound NM104

In this preparation example, the synthetic scheme of Compound NM104 is shown as follows:

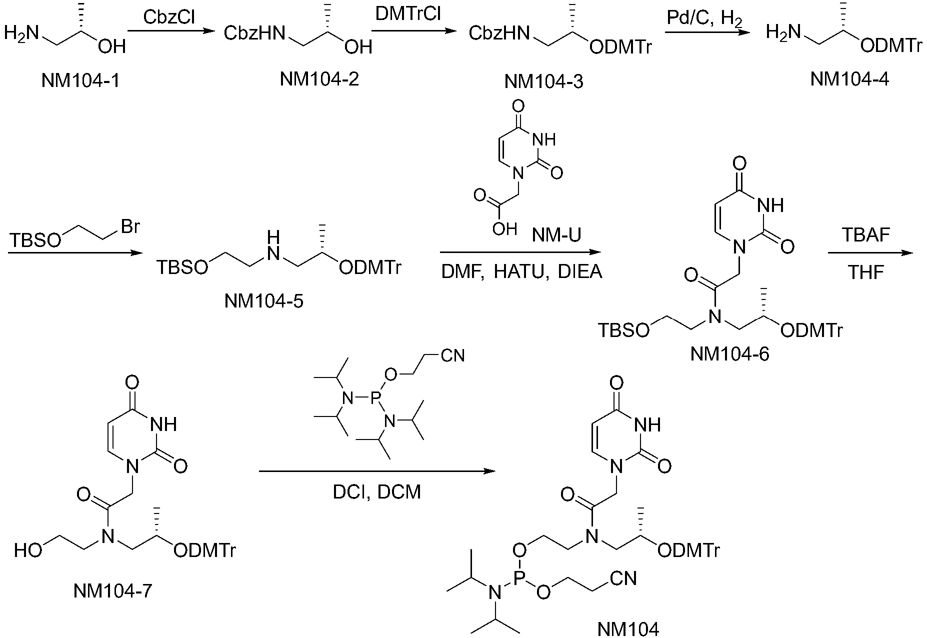

### (10-1) Synthesis of Compound NM104-2

Compound NM104-1 (10 g, 0.13 mol, 1 eq) was dissolved in 100 mL of a mixed solution of tetrahydrofuran and water (volume ratio of tetrahydrofuran to water of 1:4). Sodium bicarbonate (21.8 g, 0.26 mol, 2 eq) and benzyl chloroformate (23.3 g, 0.14 mol, 1.05 eq) were added sequentially at 0 °C. After the addition, the reaction solution was warmed to 25 °C, and allowed to react for 2 hours with stirring. After the reaction was completed, the reaction solution was extracted with ethyl acetate (3×100 mL). The organic phase was combined, washed with saturated brine (2×50 mL), dried over anhydrous sodium sulfate, filtered, concentrated and purified by column chromatography to give Compound NM104-2 as a white solid (20 g, yield 69.4%). MS-ESI (*m*/*z*) = 210.2 [M + H]⁺.

### (10-2) Synthesis of Compound NM104-3

Compound NM104-2 (20 g, 95.7 mmol, 1.0 eq) was dissolved in 200 mL of pyridine, and cooled to 0 °C in an ice bath. DMTrCl (4,4'-dimethoxytrityl chloride, 48.7 g, 143.6 mmol, 1.5 eq) was added in batches. The mixture was stirred at 0 °C for 1 hour, and quenched by adding methanol. After the reaction was completed, the reaction solution was concentrated to remove pyridine, added with saturated aqueous ammonium chloride solution (150 mL), and extracted with ethyl acetate (2×250 mL). The organic phase was combined. The organic phase was washed with saturated brine (150 mL), dried over anhydrous sodium sulfate, filtered, concentrated and purified by column chromatography (eluent: petroleum ether/ethyl acetate = 1/1, v/v) to give Compound NM104-3 as a yellow solid (37 g, yield 75.7%). MS-ESI (*m*/*z*) = 512.2 [M + H]⁺.

### (10-3) Synthesis of Compound NM104-4

Compound NM104-3 (35 g, 68.5 mmol, 1.0 eq) was dissolved in 350 mL of methanol, and 3.5 g of wet palladium on carbon (10%, 0.1 g/g) was added. The mixture was purged with hydrogen 3 times, and then stirred at 25 °C for 6 hours. After the reaction was completed, the reaction solution was filtered with suction, and the filtrate was concentrated to give Compound NM104-4 as a yellow solid (20 g, yield 77.5%). MS-ESI (*m*/*z*) = 378.3 [M + H]⁺.

### (10-4) Synthesis of Compound NM104-5

Compound NM104-4 (20 g, 52.9 mmol, 1 eq) was dissolved in 200 mL of acetonitrile. Potassium carbonate (14.6 g, 105.8 mmol, 2 eq) and (2-bromoethoxy)-tert-butyldimethylsilane (19.0 g, 79.4 mmol, 1.5 eq) were added sequentially, and stirred at 80 °C for 12 hours. After the reaction was completed, the reaction solution was cooled down to 25 °C, added with 100 mL of water, and extracted with ethyl acetate (3×150 mL). The organic phase was combined. The organic phase was washed with saturated aqueous sodium chloride solution (2×50 mL), dried over anhydrous sodium sulfate, filtered, concentrated and purified by column chromatography to give Compound NM104-5 as a white solid (24 g, yield 84.8%). MS-ESI (*m*/*z*) = 536.2 [M + H]⁺.

### (10-5) Synthesis of Compound NM104-6

Compound NM104-5 (10 g, 18.7 mmol, 1 eq) was dissolved in 100 mL of DMF. HATU (10.6 g, 28.1 mmol, 1.5 eq), Compound NM-U (4.8 g, 28.1 mmol, 1.5 eq) and DIEA (6.03 g, 46.75 mmol, 2.5 eq) were added sequentially, and the mixture was stirred at 25 °C for 2 hours. After the reaction was completed, the reaction solution was added with 100 mL of water and extracted with ethyl acetate (3×100 mL). The organic phase was combined, washed with saturated brine (5×50 mL), dried over anhydrous sodium sulfate, filtered, concentrated and purified by column chromatography to give Compound NM104-6 as a white solid (11 g, yield 85.9%). MS-ESI (*m*/*z*) = 688.2 [M + H]⁺.

### (10-6) Synthesis of Compound NM104-7

Compound NM104-6 (10 g, 14.5 mmol, 1 eq) was dissolved in 100 mL of tetrahydrofuran, and a 1 M tetrahydrofuran solution of tetrabutylammonium fluoride (21.8 mL, 21.8 mmol, 1.5 eq) was added. The mixture was stirred at 25 °C for 2 hours. After the reaction was completed, the reaction solution was added with 100 mL of water and extracted with ethyl acetate (3×100 mL). The organic phase was combined. The organic phase was washed with water (4×50 mL), dried over anhydrous sodium sulfate, filtered, concentrated and purified by column chromatography to give Compound NM104-7 as a white solid (7.0 g, yield 83.9%). MS-ESI (*m*/*z*) = 574.2 [M + H]⁺.

### (10-7) Synthesis of Compound NM104

Compound NM104-7 (3.0 g, 5.23 mmol, 1.0 eq) was dissolved in 30 mL of anhydrous dichloromethane. DCI (493 mg, 4.18 mmol, 0.8 eq) and bis(diisopropylamino)(2-cyanoethoxy)phosphine (1.65 g, 5.49 mmol, 1.05 eq) were added respectively. The mixture was purged with nitrogen 3 times, and stirred at 25 °C for 1 hour. After the reaction was completed, the reaction solution was washed by adding saturated aqueous sodium bicarbonate solution (2×30 mL). The organic phase was washed with saturated brine (20 mL), dried over anhydrous sodium sulfate, filtered, concentrated and purified by column chromatography (eluent: water/acetonitrile = 10/90, v/v) to give Compound NM104 as a white solid (2.45 g, yield 60.6%). MS-ESI (*m*/*z*) = 774 [M - H]⁺.

### Preparation Example 11: Synthesis of Compound NM105

In this preparation example, the synthetic scheme of Compound NM105 is shown as follows:

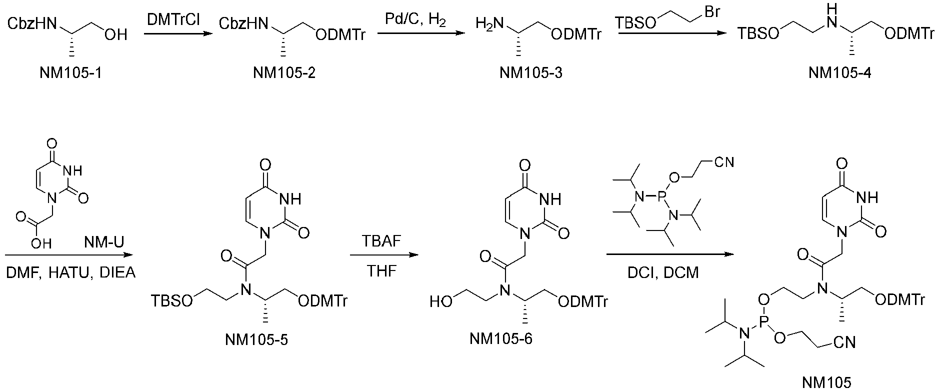

### (11-1) Synthesis of Compound NM105-2

Compound NM105-1 (20 g, 95.7 mmol, 1.0 eq) was dissolved in 200 mL of pyridine, and cooled to 0 °C in an ice bath. DMTrCl (4,4'-dimethoxytrityl chloride, 48.7 g, 143.6 mmol, 1.5 eq) was added in batches, and stirred at 0 °C for 1 hour. The reaction was quenched by adding methanol. After the reaction was completed, the reaction solution was concentrated to remove pyridine, added with saturated aqueous ammonium chloride solution (150 mL), and extracted with ethyl acetate (2×250 mL). The organic phase was combined. The organic phase was washed with saturated brine (150 mL), dried over anhydrous sodium sulfate, filtered, concentrated and purified by column chromatography (eluent: petroleum ether/ethyl acetate = 1/1, v/v) to give Compound NM105-2 as a yellow solid (32.5 g, yield 66.3%). MS-ESI (*m*/*z*) = 512.2 [M + H]⁺.

### (11-2) Synthesis of Compound NM105-3

Compound NM105-2 (30 g, 58.6 mmol, 1.0 eq) was dissolved in 300 mL of methanol, 3.0 g of palladium on carbon (10%, 0.1 g/g) was added. The mixture was purged with hydrogen 3 times, and the mixture was then stirred at 25 °C for 6 hours. After the reaction was completed, the mixture was filtered with suction, and the filtrate was concentrated to give Compound NM105-3 as a yellow solid (20 g, yield 90.9%). MS-ESI (*m*/*z*) = 378.3 [M - H]⁺.

### (11-3) Synthesis of Compound NM105-4

Compound NM105-3 (20 g, 52.9 mmol, 1 eq) was dissolved in 200 mL of acetonitrile. Potassium carbonate (14.6 g, 105.8 mmol, 2 eq) and (2-bromoethoxy)-tert-butyldimethylsilane (19.0 g, 79.4 mmol, 1.5 eq) were added sequentially, and stirred at 80 °C for 12 hours. After the reaction was completed, the reaction solution was cooled to 25 °C, added with 100 mL of water, and extracted with ethyl acetate (3×150 mL), and the organic phase was combined. The organic phase was washed with saturated aqueous sodium chloride solution (2×50 mL), dried over anhydrous sodium sulfate, filtered, concentrated and purified by column chromatography to give Compound NM105-4 as a white solid (19 g, yield 67.1%). MS-ESI (*m*/*z*) = 536.2 [M + H]⁺.

### (11-4) Synthesis of Compound NM105-5

Compound NM105-4 (10 g, 18.7 mmol, 1 eq) was dissolved in 100 mL of DMF. HATU (10.6 g, 28.1 mmol, 1.5 eq), Compound NM-U (4.8 g, 28.1 mmol, 1.5 eq) and DIEA (6.03 g, 46.75 mmol, 2.5 eq) were added sequentially, and stirred at 25 °C for 2 hours. After the reaction was completed, the reaction solution was added with 100 mL of water, and extracted with ethyl acetate (3×100 mL). The organic phase was combined, washed with saturated brine (5×50 mL), dried over anhydrous sodium sulfate, filtered, concentrated and purified by column chromatography to give Compound NM105-5 as a white solid (9.4 g, yield 73.4%). MS-ESI (*m*/*z*) = 688.2 [M + H]⁺.

### (11-5) Synthesis of Compound NM105-6

Compound NM105-5 (10 g, 14.5 mmol, 1 eq) was dissolved in 100 mL of tetrahydrofuran, and a 1 M tetrahydrofuran solution of tetrabutylammonium fluoride (21.8 mL, 21.8 mmol, 1.5 eq) was added, and stirred at 25 °C for 2 hours. After the reaction was completed, the reaction solution was added with 100 mL of water and extracted with ethyl acetate (3×100 mL), and the organic phase was combined. The organic phase was washed with water (4×50 mL), dried over anhydrous sodium sulfate, filtered, concentrated and purified by column chromatography to give Compound NM105-6 as a white solid (6.1 g, yield 73.1%). MS-ESI (*m*/*z*) = 574.2 [M + H]⁺.

### (11-6) Synthesis of Compound NM105

Compound NM105-6 (3.0 g, 5.23 mmol, 1.0 eq) was dissolved in 30 mL of anhydrous dichloromethane. DCI (493 mg, 4.18 mmol, 0.8 eq) and bis(diisopropylamino)(2-cyanoethoxy)phosphine (1.65 g, 5.49 mmol, 1.05 eq) were added respectively. The mixture was purged with nitrogen 3 times, and stirred at 25 °C for 1 hour. After the reaction was completed, the reaction solution was washed with saturated aqueous sodium bicarbonate solution (2×30 mL), washed with saturated brine (20 mL), dried over anhydrous sodium sulfate, filtered, concentrated and purified by column chromatography (eluent: water/acetonitrile = 10/90, v/v) to give Compound NM105 as a white solid (2.23 g, yield 55.8%). MS-ESI (*m*/*z*) = 774 [M + H]⁺.

### Synthesis of Ligands

### Preparation Example 12: Synthesis of Compound CR01008 and Compound CR01008Z

### (12-1) Synthesis of Compound CR01008

In this preparation example, the synthetic scheme of Compound CR01008 is shown as follows:

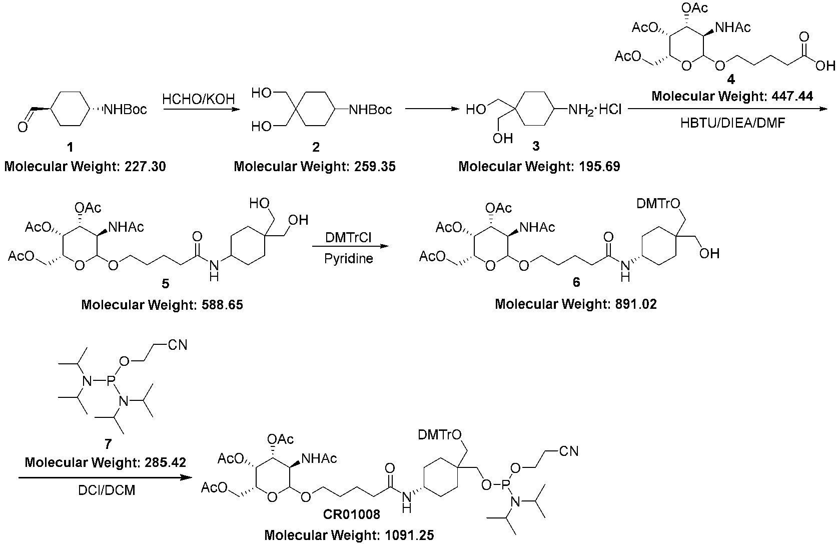

### (12-1-1) Synthesis of Compound 2

Compound 1 (trans-4-(Boc-amino)cyclohexylcarbaldehyde, 10.0 g, 1.0 eq) and an aqueous solution of formaldehyde (8.9 g, 37 wt%, 2.4 eq) were dissolved in 33 mL of methanol. 13 mL of a 45.3 wt% KOH aqueous solution was added dropwise. After the dropwise addition, the mixture was stirred at 25 °C for 30 minutes, warmed to 60 °C, and refluxed at 60 °C for 2 hours. After the reaction was completed, the reaction solution was cooled to room temperature, then evaporated to dryness under reduced pressure to give a crude product as a white solid. The crude product was added with a small amount of water, slurried, and filtered to give Compound 2 as a white solid (9 g, yield 78.9%). MS-ESI (m/z) = 260 [M + H]⁻.

### (12-1-2) Synthesis of Compound 3

Compound 2 (9 g, 1 eq) was dissolved in 70 mL of 1,4-dioxane, and a 1,4-dioxane solution of hydrogen chloride (45 mL, 4 M) was added. The mixture was stirred at 25 °C for 1 hour. After the reaction was completed, the reaction solution was evaporated to dryness under reduced pressure to give Compound 3 as a white solid (6.8 g, yield 100%).

### (12-1-3) Synthesis of Compound 5

Compound 3 (1.8 g, 2.0 eq), Compound 4 (5-[[(2R,3R,4R,5R,6R)-3-acetamido-4,5-diacetoxy-6-(acetoxymethyl)-2-tetrahydropyranyl]oxy]pen -tanoic acid, 2.1 g, 1.0 eq) and DIEA (N,N-diisopropylethylamine, 3.5 g, 6.0 eq) were dissolved in 15 mL of DMF. HBTU (1.9 g, 1.1 eq) was added. The mixture was stirred at 25 °C for 3 hours under N₂ atmosphere. After the reaction was completed, the reaction solution was evaporated to dryness under reduced pressure, followed by reversed-phase purification (22 vol% aqueous acetonitrile ), to give Compound 5 as a white solid (1.78 g, yield 64.4%). MS-ESI (m/z) = 589 [M + H]⁻.

### (12-1-4) Synthesis of Compound 6

Compound 5 (1.54 g, 1.0 eq) was dissolved in 15 mL of pyridine. The reaction system was cooled to 0 °C using an ice-water bath, and DMTrCl (4,4'-dimethoxytrityl chloride, 1.32 g, 1.5 eq) was added at 0 °C. The reaction solution was allowed to react at 25 °C for 3 hours, and was then quenched by adding 15 mL of methanol. After the reaction was completed, the reaction solution was evaporated to dryness under reduced pressure, followed by reversed-phase purification (60 vol% aqueous acetonitrile), to give Compound 6 as a yellow solid (1 g, yield 42.7%). MS-ESI (m/z) = 891 [M + H]⁺.

### (12-1-5) Synthesis of Compound CR01008

Compound 6 (1.08 g, 1.0 eq) was dissolved in 20 mL of anhydrous dichloromethane, and DCI (115 mg, 0.8 eq) and Compound 7 (bis(diisopropylamino)(2-cyanoethoxy)phosphine, 732 mg, 2.1 eq) were added respectively. The mixture was purged with nitrogen 3 times, and stirredat 25 °C for 2 hours. After the reaction was completed, the reaction solution was added with 20 mL of saturated aqueous sodium bicarbonate solution and extracted with 20 mL of dichloromethane for 3 times (3×20 mL), and the organic phase was combined. The organic phase was evaporated to dryness under reduced pressure, followed by reversed-phase purification (72 vol% aqueous acetonitrile) and vacuum drying for 12 hours, to give Compound CR01008 as a white powder (1 g, yield 76.0%). MS-ESI (m/z) = 1091 [M - Na]⁺.

1H NMR (400 MHz, DMSO-d6) δ 1.05 (d, J = 6.7 Hz, 6H).1.14 (d, J = 6.7 Hz, 6H), 1.37 - 1.17 (m, 5H), 1.60 - 1.40 (m, 6H),1.68 - 1.62 (m, 1H),1.80 (s, 3H),1.80 (s, 3H),1.92 (s, 3H), 2.02 (s, 5H),2.13 (s, 3H),2.71 (t, J = 5.9 Hz, 2H), 2.79 (d, J = 8.4 Hz, 1H), 2.87 (d, J = 8.4 Hz, 1H),3.36 (s, 1H), 3.58 - 3.39 (m, 3H), 3.69 - 3.60 (m, 2H), 3.75 (s, 7H), 3.90 (dt, J = 11.2, 8.8 Hz, 1H), 4.05 (s, 3H),4.51 (d, J = 8.4 Hz, 1H), 4.99 (dd, J = 11.3, 3.4 Hz, 1H), 5.24 (d, J = 3.4 Hz, 1H), 5.78 (s, 1H), 6.93 - 6.87 (m, 4H),7.35 - 7.21 (m, 7H), 7.44 - 7.37 (m, 2H), 7.66 (d, J = 7.8 Hz, 1H), 7.84 (d, J = 9.2 Hz, 1H).

### (12-2) Synthesis of Compound CR01008Z

In this preparation example, the synthetic scheme of Compound CR01008Z is shown as follows:

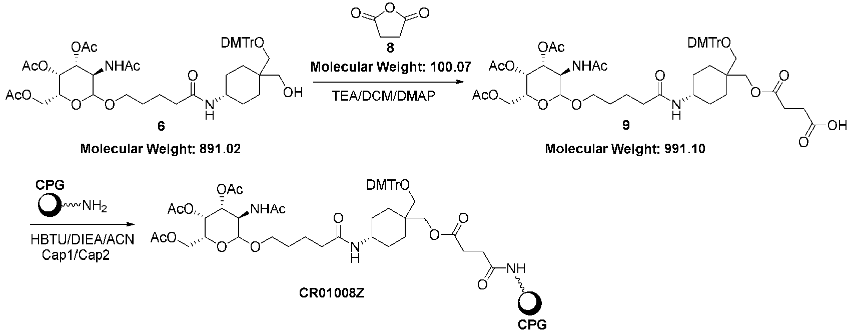

### (12-2-1) Synthesis of Compound 9

Compound 6 (500 mg) was dissolved in 10 mL of dichloromethane, and Compound 8 (succinic anhydride, 112 mg), DMAP (6.8 mg) and TEA (226.2 mg) were added. The mixture was purged with nitrogen 3 times, stirred at 25 °C for 16 hours, and then purified via flash, to give Compound 9 (300 mg, yield 53.6%). MS-ESI (m/z) = 1013 [M - Na]⁺.

### (12-2-2) Synthesis of Compound CR01008Z

Compound 9 (50 mg), amino CPG (1.25 g, 80 µmol/g, 0.1 mmol), HBTU (27 mg) and DIEA (12 mg) were added to a 20 mL sample vial, and shaken on a shaker for 16 hours. After the reaction was completed, the reaction solution was filtered to obtain a filter cake. The filter cake was first washed once with 10 mL of acetonitrile (1×10 mL) and then dried under vacuum. The dried filter cake, DMAP (3 mg), Cap1 (10 mL, 200 V) and Cap2 (1 mL, 20 V) were added to a 20 mL sample vial, and shaken on a shaker for 6 hours. After the reaction was completed, the reaction solution was filtered to obtain a filter cake. The filter cake was first washed once with 10 mL of acetonitrile (1×10 mL) and then dried under vacuum, to give Compound CR01008Z (1.03 g, loading capacity: 20-30 µmol/g).

Wherein, Cap1 and Cap2 are capping reagents; Cap1 is a 20 vol% pyridine/acetonitrile mixed solution of N-methylimidazole, with a volume ratio of pyridine to acetonitrile of 3:5; Cap2 is a 20 vol% acetonitrile solution of acetic anhydride.

### Preparation Example 13: Synthesis of Compound CR01013 and Compound CR01013Z

### (13-1) Preparation of Compound CR01013

In this preparation example, the synthetic scheme of Compound CR01013 is shown as follows:

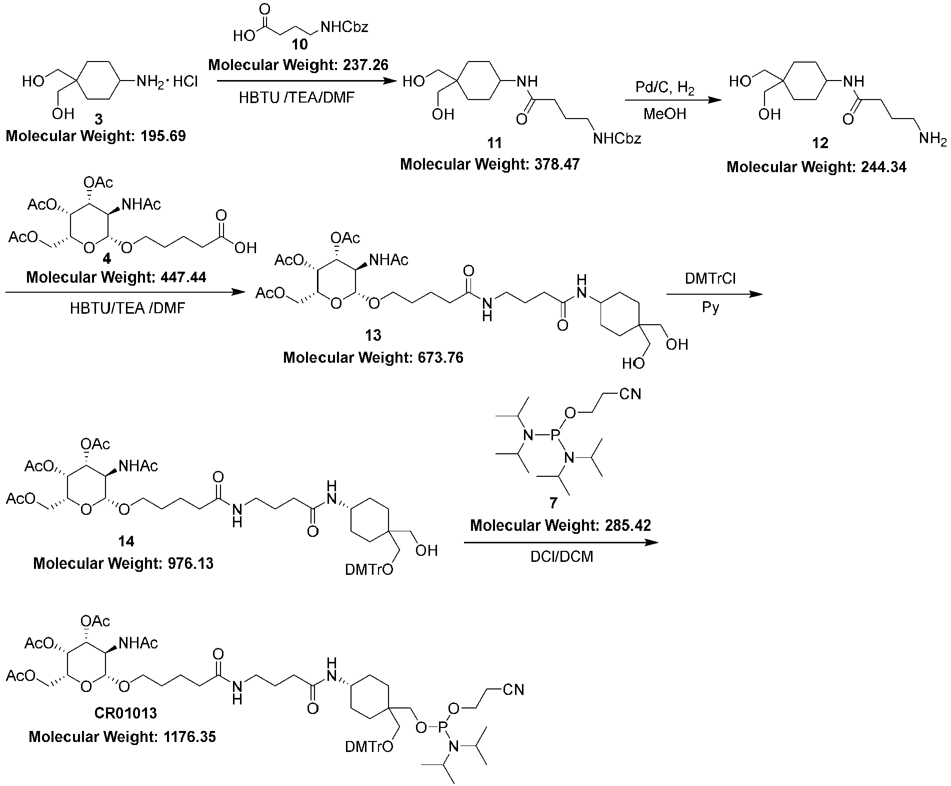

### (13-1-1) Synthesis of Compound 11

Compound 3 (3.6 g) was dissolved in 36 mL of DMF, and then TEA (5.62 g), Compound 10 (N-benzyloxycarbonyl-4-aminobutyric acid, 5.28 g) and HBTU (8.43 g) were added. The mixture was stirred at 25 °C for 16 hours. After the reaction was completed, the reaction solution was added into 200 mL of saturated aqueous sodium bicarbonate solution, and extracted with 100 mL of ethyl acetate three times (3×100 mL), and the organic phase was combined. The organic phase was first washed once with 50 mL of saturated aqueous sodium chloride solution (1×50 mL), then dried over anhydrous sodium sulfate, and evaporated to dryness under reduced pressure, followed by purification via normal-phase column chromatography (eluent: dichloromethane/methanol = 10/1, v/v), to give Compound 11 as a white solid (2.3 g, yield 33.0%). ESI MS (m/z) = 379.5 [M + H]⁺.

### (13-1-2) Synthesis of Compound 12

Compound 11 (2.3 g) was dissolved in 23 mL of methanol, and wet palladium on carbon (230 mg, loading capacity: 10 wt%) was added. The mixture was purged with hydrogen three times. The reaction system was stirred at 25 °C for 16 hours under hydrogen atmosphere (15 psi). After the reaction was completed, the reaction solution was filtered to obtain the filtrate, which was evaporated to dryness under reduced pressure to give Compound 12 as a yellow oil (1.48 g, yield 99.8%).

### (13-1-3) Synthesis of Compound 13

Compound 12 (1.48 g) was dissolved in 15 mL of DMF. Triethylamine (1.22 g), Compound 4 (1.35 g) and HBTU (3.45 g) were added. The mixture was stirred at 25 °C for 16 hours. After the reaction was completed, the reaction solution was added into 150 mL of saturated aqueous sodium bicarbonate solution and extracted with 50 mL of ethyl acetate three times (3×50 mL), and the organic phase was combined. The organic phase was first washed once with 30 mL of saturated aqueous sodium chloride solution (1×30 mL), then dried over anhydrous sodium sulfate, evaporated to dryness under reduced pressure, and purified via reversed-phase column chromatography (C18 chromatographic column, eluent: water/acetonitrile = 5/1, v/v), to give Compound 13 as a white solid (1.3 g, yield 31.8%). ESI-MS (m/z) = 674.3 [M + H]⁻.

### (13-1-4) Synthesis of Compound 14

Compound 13 (1.1 g) was dissolved in 11 mL of pyridine. The reaction system was cooled to 0 °C using an ice-water bath, and DMTrCl (813 mg) was added in batches at 0 °C. The reaction system was stirred at 0 °C for 1 hour. After the reaction was completed, the reaction solution was quenched by adding methanol, the solvent was distilled off, and the resulting product was purified via reversed-phase column chromatography (eluent: water/acetonitrile = 1/4, v/v) to give Compound 14 as a white solid (800 mg, yield 50.3%). ESI-MS (m/z) = 976.5 [M + H]⁺.

### (13-1-5) Synthesis of Compound CR01013

At 25 °C, Compound 14 (550 mg) was dissolved in 5 mL of dichloromethane (DCM). 4,5-Dicyanoimidazole (53.2 mg) and Compound 7 (2-cyanoethyl N,N,N',N'-tetraisopropylphosphorodiamidite, 255.4 mg) were added. The mixture was purged with nitrogen three times, and the reaction system was stirred at 25 °C for 1 hour under nitrogen atmosphere. After the reaction was completed, the reaction solution was first washed twice with 5 mL of saturated aqueous sodium bicarbonate solution (2×5 mL) and then washed once with 30 mL of saturated aqueous sodium chloride solution (1×30 mL). The organic phase was separated, dried over anhydrous sodium sulfate. The organic phase was evaporated to dryness under reduced pressure, and purified via normal-phase column chromatography (eluent: dichloromethane/methanol = 20/1, v/v) to give Compound CR01013 as a white solid (532 mg, yield 80.4%). ESI-MS (m/z) = 1176.7 [M + H]⁺.

¹H NMR (400 MHz, DMSO-d6) δ 0.95 - 1.05 (d, J = 6.7 Hz, 5H), 1.06 - 1.15 (q, J = 7.6 Hz, 8H), 1.15 - 1.21 (t, J = 7.2 Hz, 14H), 1.72 - 1.80 (s, 3H), 1.84 - 1.92 (s, 3H), 1.94 - 2.07 (d, J = 16.0 Hz, 7H), 2.07 - 2.14 (s, 3H), 2.64 - 2.72 (q, J = 5.8 Hz, 2H), 2.74 - 2.89 (d, J = 8.5 Hz, 2H), 3.35 - 3.56 (m, 4H), 3.57 - 3.70 (m, 4H), 3.71 - 3.77 (s, 6H), 3.81 - 3.93 (m, 1H), 3.96 - 4.09 (d, J = 6.4 Hz, 3H), 6.82 - 6.97 (d, J = 8.7 Hz, 4H), 7.17 - 7.27 (t, J = 8.7 Hz, 5H), 7.27 - 7.34 (t, J = 7.6 Hz, 2H), 7.34 - 7.43 (d, J = 7.5 Hz, 2H).

### (13-2) Preparation of Compound CR01013Z

In this preparation example, the synthetic scheme of Compound CR01013Z is shown as follows:

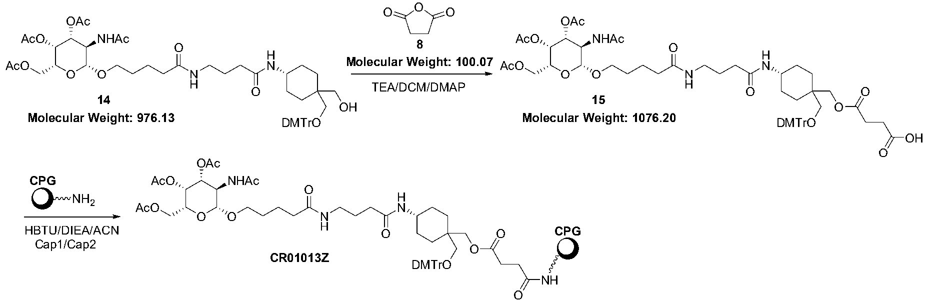

### (13-2-1) Synthesis of Compound 15

At 25 °C, Compound 14 (100 mg, 0.10 mmol) was dissolved in 2 mL of dichloromethane. Triethylamine (25.9 mg, 0.25 mmol), DMAP (1.25 mg, 0.01 mmol) and Compound 8 (succinic anhydride, 15.4 mg, 0.15 mmol) were added. The reaction system was stirred at 25 °C for 16 hours. After the reaction was completed, the solvent in the reaction solution was distilled off, and the resulting product was purified via reversed-phase column chromatography (C18 chromatographic column, eluent: water/acetonitrile = 2/1, v/v) to give Compound 15 as a yellow oil (110 mg, 0.10 mmol, yield 100%). ESI-MS (m/z) = 1099.3 [M + Na]⁺.

### (13-2-2) Synthesis of Compound CR01013Z

Compound 15 (50 mg, 0.04 mmol) was dissolved in 10 mL of acetonitrile. HBTU (24.2 mg, 0.06 mmol), DIEA (11.0 mg, 0.08 mmol) and amino CPG (1.06 g, loading capacity: 80 µmol/g) were added. The reaction system was stirred at 25 °C for 16 hours. After the reaction was completed, the reaction solution was filtered to give a filter cake. The filter cake was successively washed with 50 mL of dichloromethane twice (2×50 mL), with 50 mL of acetonitrile three times (3×50 mL) and with 50 mL of ethyl acetate once (1×50 mL), and then dried under vacuum. Cap1 (4.8 mL), Cap2 (0.54 mL) and DMAP (2.59 mg) were added to the dried filter cake. The reaction system was stirred at 25 °C for 5 hours. After the reaction was completed, the reaction solution was filtered to obtain a filter cake. The filter cake was washed three times with 50 mL of acetonitrile (3×50 mL) and dried under vacuum to give Compound CR01013Z (900 mg, loading capacity: 20-30 µmol/g).

Wherein Cap1 and Cap2 are capping reagents; Cap1 is a 20 vol% pyridine/acetonitrile mixed solution of N-methylimidazole with a volume ratio of pyridine to acetonitrile of 3:5; Cap2 is a 20 vol% acetonitrile solution of acetic anhydride.

### Compound L96-PS

Compound L96-PS was purchased from Asymchem Laboratories (Tianjin) Co., Ltd., with a loading capacity of 120±12 µmol/g (detection method: UV/HPLC). The structural formula of Compound L96-PS is shown below: 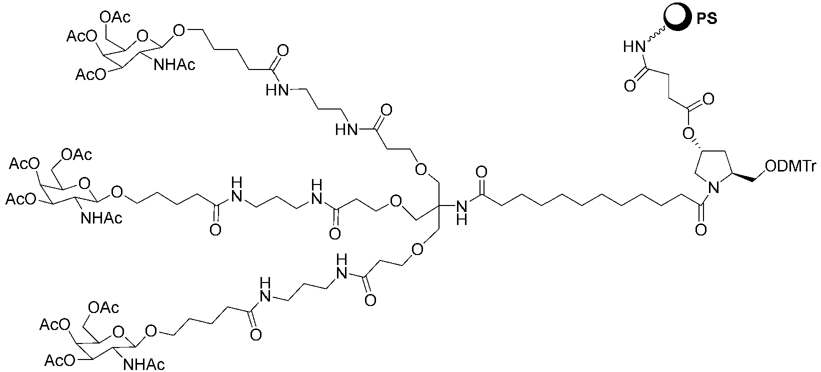 wherein, PS represents polystyrene resin solid-phase support.

### Synthesis of siRNA Conjugates

Unless otherwise specified, all siRNA sequences used in the present disclosure were commissioned to Suzhou Beixin Biotechnology Co., Ltd. for synthesis; all PCR primer synthesis used in the present disclosure were commissioned to Beijing Tsingke Biotech Co., Ltd..

### Preparation Example 14: Synthesis of siRNA Conjugates

### (14-1) Synthesis of Sense Strand (SS)

By means of the phosphoramidite nucleic acid solid-phase synthesis method, the cycle was initiated using the above-mentioned compounds linked to solid-phase supports (such as CR01008Z, CR01013Z, L96-PS), and nucleoside monomers were linked one by one in the 3'-5' direction according to the nucleotide sequence (during the synthesis process, Compound CR01008 and Compound CR01013 are each regarded as one nucleoside monomer). Each linkage of a nucleoside monomer involves a four-step reaction: deprotection, coupling, capping, and oxidation or sulfuration. The synthesis conditions were given as follows:

Each of nucleoside monomers was prepared as a 0.1 M acetonitrile solution of the nucleoside monomer.

The conditions for deprotection were the same for each step. The conditions for deprotection: temperature was 25 °C; reaction time was 70 seconds; the deprotection reagent was a solution of dichloroacetic acid in dichloromethane (3 vol%); the molar ratio of dichloroacetic acid to the 4,4'-dimethoxytrityl protecting group on the solid-phase support was 5:1.

The conditions for coupling were the same for each step. The conditions for coupling: temperature was 25 °C; the molar ratio of the nucleic acid sequence linked to the solid-phase support to the nucleoside monomer was 1:10; the molar ratio of the nucleic acid sequence linked to the solid-phase support to the coupling reagent was 1:65; reaction time was 600 seconds; the coupling reagent was a 0.5 M solution of 5-ethylthio-1H-tetrazole in acetonitrile; the sulfurization reagent was a 0.2 mol/L solution of xanthane hydride in a mixture of acetonitrile and pyridine (volume ratio of acetonitrile to pyridine of 1:1).

The conditions for capping were the same for each step. The conditions for capping: temperature was 25 °C; reaction time was 2 minutes; the capping reagent solution was a mixed solution of Cap1 and Cap2 at a molar ratio of 1:1; Cap1 was a 20 vol% solution of N-methylimidazole in a mixture of pyridine/acetonitrile with a volume ratio of pyridine to acetonitrile of 3:5; Cap2 was a 20 vol% solution of acetic anhydride in acetonitrile; the molar ratio of N-methylimidazole in the capping reagent Cap1, acetic anhydride in the capping reagent Cap2, and the nucleic acid sequence linked to the solid-phase support was 1:1:1.

The conditions for oxidation were the same for each step. The conditions for oxidation: temperature was 25 °C; reaction time was 3 seconds; the oxidation reagent was a 0.05 M iodine solution; the molar ratio of iodine to the nucleic acid sequence linked to the solid-phase support in the coupling reaction was 30:1; the oxidation reaction was carried out in a water/pyridine mixed solvent (volume ratio of water to pyridine of 1:9). The conditions for sulfuration: temperature was 25 °C; reaction time was 360 seconds; the sulfurization reagent was a 0.2 M solution of xanthane hydride in pyridine; the molar ratio of the sulfurization reagent to the nucleic acid sequence linked to the solid-phase support in the coupling reaction was 4:1; the sulfurization reaction was carried out in a water/pyridine mixed solvent (volume ratio of water to pyridine of 1:9).

After the coupling of the last nucleoside monomer was completed, the nucleic acid sequence linked to the solid-phase support was sequentially cleaved, deprotected, purified, and desalted, followed by lyophilization to give the sense strand. Wherein,

The conditions for cleavage and deprotection were as follows: the synthesized nucleotide sequence linked to the solid-phase support was added to 25 wt% ammonia solution with a amount of 0.5 mL/µmol, and allowed to react at 55 °C for 16 hours. The solvent was removed, and the solution was concentrated to dryness under vacuum. After the ammonia solution treatment, the product was dissolved in 0.4 mL/µmol N-methylpyrrolidone relative to the amount of single-stranded nucleic acid, and 0.3 mL/µmol triethylamine and 0.6 mL/µmol triethylamine trihydrofluoride were then added to remove the 2'-O-TBDMS protection groups on the ribose.

The conditions for purification and desalination: the nucleic acid was purified using a preparative ion chromatography purification column (Source 15Q) with gradient elution of NaCl. Specifically: eluent 1 was 20 mM sodium phosphate (pH=8.1), and the solvent was a water/acetonitrile mixed solution (volume ratio of water to acetonitrile of 9:1); eluent 2 was 1.5 M sodium chloride and 20 mM sodium phosphate (pH=8.1), and the solvent was a water/acetonitrile mixed solution (volume ratio of water to acetonitrile of 9:1); and the elution gradient was eluent 1:eluent 2 = (100:0) - (50:50). The product eluates were collected and combined, and then desalted using a reversed-phase chromatography purification column. The desalination conditions included desalting with a Sephadex column packed with Sephadex G25, eluting with deionized water.

Detection: purity detection was performed using ion-exchange chromatography (IEX-HPLC); molecular weight detection was performed using liquid chromatography-mass spectrometry (LC-MS). The measured value of molecular weight was compared with the theoretical value; if the measured value was consistent with rge theoretical value, it indicated that the compound was conjugated to the 3' end of the sense strand of the siRNA.

During the synthesis of the sense strand, three clusters of CR01008 (denoted as (CR01008×3) or (CR01008)×3) and three clusters of CR01013 (denoted as (CR01013×3) or (CR01013)×3) were obtained, respectively.

Wherein the structural formula of the three clusters of CR01008 is shown below:

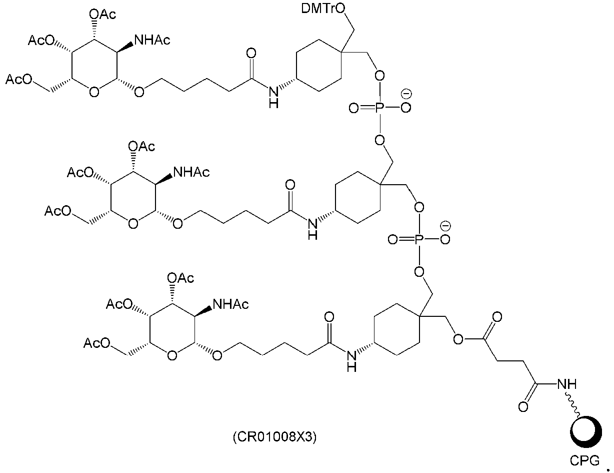

The structural formula of the three clusters of CR01013 is shown below:

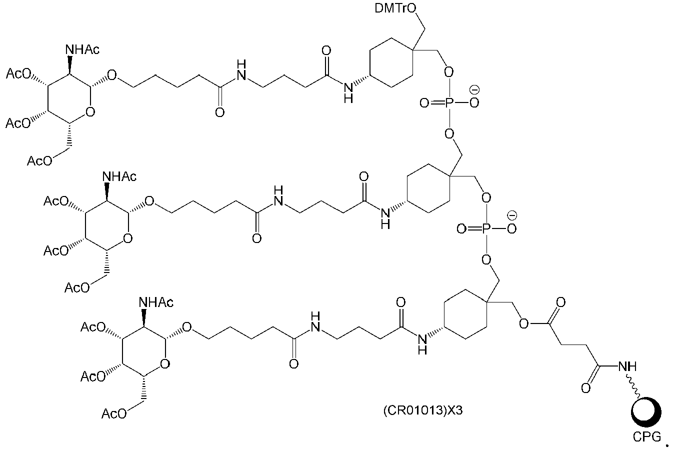

### (14-2) Synthesis of Antisense Strand (AS)

The antisense strand was synthesized using a universal solid-phase support. The conditions for deprotection, coupling, capping, oxidation or sulfuration, the conditions for cleavage and deprotection, as well as the conditions for purification and desalination in the solid-phase synthesis method of the antisense strand were the same as those for the synthesis of the sense strand in step (14-1).

Detection: purity detection was performed using ion-exchange chromatography (IEX-HPLC); molecular weight detection was performed using liquid chromatography-mass spectrometry (LC-MS). The measured value of molecular weight was compared with the theoretical value; if the measured value was consistent with the theoretical value, it indicated that the siRNA antisense strand was obtained.

### (14-3) Synthesis of siRNA Conjugates

The sense strand synthesized in step (14-1) and the antisense strand synthesized in step (14-2) were mixed at an equimolar ratio, dissolved in water for injection, and then heated to 95 °C, slowly cooled to room temperature and maintained at room temperature for 10 minutes, allowing the sense strand and antisense strand to form a double-stranded structure via hydrogen bonds, thus affording the siRNA conjugates with the sense and antisense strands shown in Table 5.

The structural formula of the siRNA conjugate with L96 conjugated to the 3' end of the sense strand is shown below: 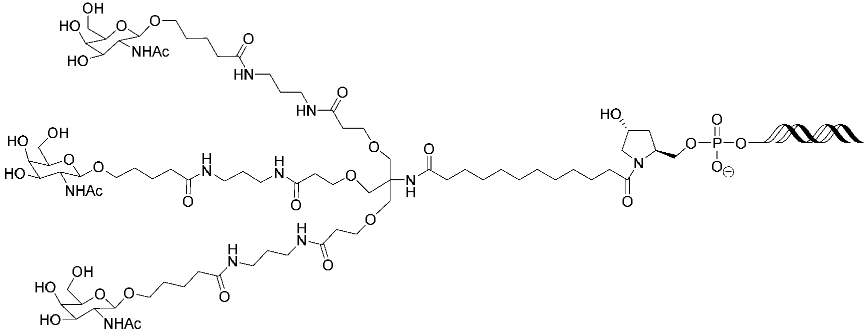 wherein, 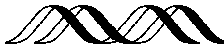 represents siRNA. Wherein L96 is conjugated to the 3' end of the sense strand of the siRNA via phosphodiester linkage.

The structural formula of the siRNA conjugate with (CR01008×3) conjugated to the 3' end of the sense strand is shown below: 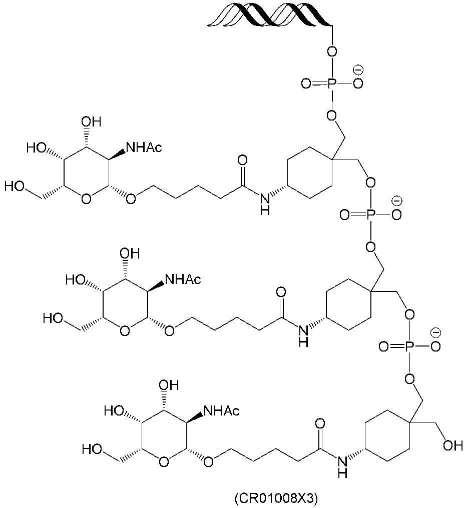 wherein, 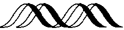 represents siRNA. (CR01008×3) is conjugated to the 3' end of the sense strand of the siRNA via phosphodiester linkage.

The structural formula of the siRNA conjugate with (CR01008×3) conjugated to the 3' end of the sense strand is shown below: 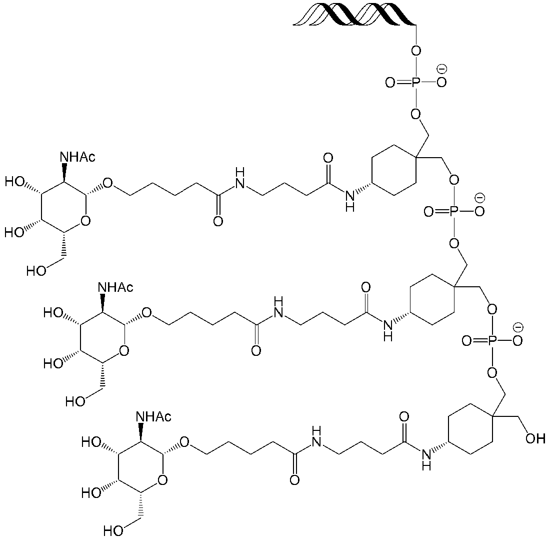 wherein, 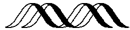 represents siRNA. (CR01013×3) is conjugated to the 3' end of the sense strand of the siRNA via phosphodiester linkage.

In the siRNA conjugates prepared in the specific Examples of the present disclosure, the unmodified nucleotide sequences forming the siRNA conjugates are shown in the following table:

**Table 4 Information on Unmodified Nucleotide Sequences Forming siRNA Conjugates**

| Number | Sense strand (5'-3') | SEQ ID | Antisense strand (5'-3') | SEQ ID |
|---|---|---|---|---|
| RZ502017 | CAGACAGACAAGACCAUCU | NO.1 | AGAUGGUCUUGUCUGUCUGUU | NO.4 |
| RZ502025 | CAGACAGACAAGACCAUCU | NO.1 | AGAUGGUCUUGUCUGUCUGUU | NO.4 |
| RZ502051 | CAGACAGACAAGACCAUCU | NO.1 | AGAUGGUCUUGUCUGUCUGUU | NO.4 |
| RZ597002 | CCAAGAGCACCAAGAACUA | NO.2 | UAGUUCUUGGUGCUCUUGGUU | NO.5 |
| RZ597007 | CCAAGAGCACCAAGAACUA | NO.2 | UAGUUCUUGGUGCUCUUGGUU | NO.5 |
| RZ597112 | CCAAGAGCACCAAGAACUA | NO.2 | UAGUUCUUGGUGCUCUUGGUU | NO.5 |
| RZ597102 | CCAAGAGCACCAAGAACUA | NO.2 | UAGUUCUUGGUGCUCUUGGUU | NO.5 |
| RZ597103 | CCAAGAGCACCAAGAACUA | NO.2 | UAGUUCUUGGUGCUCUUGGUU | NO.5 |
| RZ597113 | CCAAGAGCACCAAGAACUA | NO.2 | UAGUUCUUGGUGCUCUUGGUU | NO.5 |
| RZ597129 | CCAAGAGCACCAAGAACUA | NO.2 | UAGUUCUUGGUGCUCUUGGUU | NO.5 |
| RZ597130 | CCAAGAGCACCAAGAACUA | NO.2 | UAGUUCUUGGUGCUCUUGGUU | NO.5 |
| RZ597131 | CCAAGAGCACCAAGAACUA | NO.2 | UAGUUCUUGGUGCUCUUGGUU | NO.5 |
| RZ597132 | CCAAGAGCACCAAGAACUA | NO.2 | UAGUUCUUGGUGCUCUUGGUU | NO.5 |
| RZ597133 | CCAAGAGCACCAAGAACUA | NO.2 | UAGUUCUUGGUGCUCUUGGUU | NO.5 |
| RZ597134 | CCAAGAGCACCAAGAACUA | NO.2 | UAGUUCUUGGUGCUCUUGGUU | NO.5 |
| RZ597135 | CCAAGAGCACCAAGAACUA | NO.2 | UAGUUCUUGGUGCUCUUGGUU | NO.5 |
| RZ597136 | CCAAGAGCACCAAGAACUA | NO.2 | UAGUUCUUGGUGCUCUUGGUU | NO.5 |
| RZ597137 | CCAAGAGCACCAAGAACUA | NO.2 | UAGUUCUUGGUGCUCUUGGUU | NO.5 |
| RZ597138 | CCAAGAGCACCAAGAACUA | NO.2 | UAGUUCUUGGUGCUCUUGGUU | NO.5 |
| RZ597139 | CCAAGAGCACCAAGAACUA | NO.2 | UAGUUCUUGGUGCUCUUGGUU | NO.5 |
| RZ597140 | CCAAGAGCACCAAGAACUA | NO.2 | UAGUUCUUGGUGCUCUUGGUU | NO.5 |
| RZ597141 | CCAAGAGCACCAAGAACUA | NO.2 | UAGUUCUUGGUGCUCUUGGUU | NO.5 |
| RZ597142 | CCAAGAGCACCAAGAACUA | NO.2 | UAGUUCUUGGUGCUCUUGGUU | NO.5 |
| RZ597143 | CCAAGAGCACCAAGAACUA | NO.2 | UAGUUCUUGGUGCUCUUGGUU | NO.5 |
| RZ597144 | CCAAGAGCACCAAGAACUA | NO.2 | UAGUUCUUGGUGCUCUUGGUU | NO.5 |
| RZ597145 | CCAAGAGCACCAAGAACUA | NO.2 | UAGUUCUUGGUGCUCUUGGUU | NO.5 |
| RZ597146 | CCAAGAGCACCAAGAACUA | NO.2 | UAGUUCUUGGUGCUCUUGGUU | NO.5 |
| RZ597147 | CCAAGAGCACCAAGAACUA | NO.2 | UAGUUCUUGGUGCUCUUGGUU | NO.5 |
| RZ597148 | CCAAGAGCACCAAGAACUA | NO.2 | UAGUUCUUGGUGCUCUUGGUU | NO.5 |
| RZ597149 | CCAAGAGCACCAAGAACUA | NO.2 | UAGUUCUUGGUGCUCUUGGUU | NO.5 |
| RZ592007 | AUGUGAAAGUCAUCGACAA | NO.3 | UUGUCGAUGACUUUCACAUUC | NO.6 |
| RZ592008 | AUGUGAAAGUCAUCGACAA | NO.3 | UUGUCGAUGACUUUCACAUUC | NO.6 |
| RZ592009 | AUGUGAAAGUCAUCGACAA | NO.3 | UUGUCGAUGACUUUCACAUUC | NO.6 |
| RZ592010 | AUGUGAAAGUCAUCGACAA | NO.3 | UUGUCGAUGACUUUCACAUUC | NO.6 |
| RZ592011 | AUGUGAAAGUCAUCGACAA | NO.3 | UUGUCGAUGACUUUCACAUUC | NO.6 |
| RZ592012 | AUGUGAAAGUCAUCGACAA | NO.3 | UUGUCGAUGACUUUCACAUUC | NO.6 |
| RZ592013 | AUGUGAAAGUCAUCGACAA | NO.3 | UUGUCGAUGACUUUCACAUUC | NO.6 |
| RZ592014 | AUGUGAAAGUCAUCGACAA | NO.3 | UUGUCGAUGACUUUCACAUUC | NO.6 |
| RZ592015 | AUGUGAAAGUCAUCGACAA | NO.3 | UUGUCGAUGACUUUCACAUUC | NO.6 |
| RZ592016 | AUGUGAAAGUCAUCGACAA | NO.3 | UUGUCGAUGACUUUCACAUUC | NO.6 |
| RZ592017 | AUGUGAAAGUCAUCGACAA | NO.3 | UUGUCGAUGACUUUCACAUUC | NO.6 |
| RZ592018 | AUGUGAAAGUCAUCGACAA | NO.3 | UUGUCGAUGACUUUCACAUUC | NO.6 |
| RZ592019 | AUGUGAAAGUCAUCGACAA | NO.3 | UUGUCGAUGACUUUCACAUUC | NO.6 |
| RZ592020 | AUGUGAAAGUCAUCGACAA | NO.3 | UUGUCGAUGACUUUCACAUUC | NO.6 |
| RZ592021 | AUGUGAAAGUCAUCGACAA | NO.3 | UUGUCGAUGACUUUCACAUUC | NO.6 |
| RZ592022 | AUGUGAAAGUCAUCGACAA | NO.3 | UUGUCGAUGACUUUCACAUUC | NO.6 |
| RZ592023 | AUGUGAAAGUCAUCGACAA | NO.3 | UUGUCGAUGACUUUCACAUUC | NO.6 |
| RZ592024 | AUGUGAAAGUCAUCGACAA | NO.3 | UUGUCGAUGACUUUCACAUUC | NO.6 |
| RZ592025 | AUGUGAAAGUCAUCGACAA | NO.3 | UUGUCGAUGACUUUCACAUUC | NO.6 |
| RZ592026 | AUGUGAAAGUCAUCGACAA | NO.3 | UUGUCGAUGACUUUCACAUUC | NO.6 |
| RZ592027 | AUGUGAAAGUCAUCGACAA | NO.3 | UUGUCGAUGACUUUCACAUUC | NO.6 |
| RZ597156 | CCAAGAGCACCAAGAACUA | NO.2 | UAGUUCUUGGUGCUCUUGGUU | NO.5 |
| RZ597157 | CCAAGAGCACCAAGAACUA | NO.2 | UAGUUCUUGGUGCUCUUGGUU | NO.5 |
| RZ597158 | CCAAGAGCACCAAGAACUA | NO.2 | UAGUUCUUGGUGCUCUUGGUU | NO.5 |
| RZ597159 | CCAAGAGCACCAAGAACUA | NO.2 | UAGUUCUUGGUGCUCUUGGUU | NO.5 |
| RZ597160 | CCAAGAGCACCAAGAACUA | NO.2 | UAGUUCUUGGUGCUCUUGGUU | NO.5 |
| RZ597161 | CCAAGAGCACCAAGAACUA | NO.2 | UAGUUCUUGGUGCUCUUGGUU | NO.5 |
| RZ597162 | CCAAGAGCACCAAGAACUA | NO.2 | UAGUUCUUGGUGCUCUUGGUU | NO.5 |
| RZ597163 | CCAAGAGCACCAAGAACUA | NO.2 | UAGUUCUUGGUGCUCUUGGUU | NO.5 |
| RZ597164 | CCAAGAGCACCAAGAACUA | NO.2 | UAGUUCUUGGUGCUCUUGGUU | NO.5 |
| RZ597165 | CCAAGAGCACCAAGAACUA | NO.2 | UAGUUCUUGGUGCUCUUGGUU | NO.5 |
| RZ597166 | CCAAGAGCACCAAGAACUA | NO.2 | UAGUUCUUGGUGCUCUUGGUU | NO.5 |
| RZ597167 | CCAAGAGCACCAAGAACUA | NO.2 | UAGUUCUUGGUGCUCUUGGUU | NO.5 |

In the siRNA conjugates prepared in the specific Examples of the present disclosure, the information on the modified nucleotide sequences of the siRNA conjugates is shown in the following table:

**Table 5 Sequence Information for siRNA Conjugates**

| Number | Sense Strand (5'-3') | SEQ ID | Antisense Strand (5'-3') | SEQ ID |
|---|---|---|---|---|
| RZ50201 7 | CmsAmsGmAmCmAmGfAdCfAmAmGm AmCmCmAmUmCmUm_L96 | NO.11 | AmsGfsAmUmGmGfUmCmUmUmGmUmCmU fGmUfCmUmGmsUmsUm | NO.12 |
| RZ50202 5 | CmsAmsGmAmCmAmGfAdCfAmAmGm AmCmCmAmUmCmUm_L96 | NO.13 | AmsGfsAmUmGmGf(NM023)CmUmUmGmUm CmUfGmUfCmUmGmsUmsUm | NO. 14 |
| RZ50205 1 | CmsAmsGmAmCmAmGfAdCfAmAmGm AmCmCmAmUmCmUm_L96 | NO.15 | AmsGfsAmUmGmGf(NM022)CmUmUmGmUm CmUfGmUfCmUmGmsUmsUm | NO.16 |
| RZ59700 2 | CmsCmsAmAmGmAmGfCfAfCmCmAm AmGmAmAmCmUmAm_L96 | NO.17 | UmsAfsGmUmUmCfUmUmGmGmUmGmCmU fCmUfUmGmGmsUmsUm | NO.18 |
| RZ59700 7 | CmsCmsAmAmGmAmGfCfAfCmCmAm AmGmAmAmCmUmAm_L96 | NO.19 | UmsAfsGmUmUmCf(NM023)UmGmGmUmGm CmUfCmUfUmGmGmsUmsUm | NO.20 |
| RZ59711 2 | CmsCmsAmAmGmAmGfCfAfCmCmAm AmGmAmAmCmUmAm_L96 | NO.21 | UmsAfsGmUmUmCf(NM022)UmGmGmUmGm CmUfCmUfUmGmGmsUmsUm | NO.22 |
| RZ59710 2 | CmsCmsAmAmGmAmGfCfAfCmCmAm AmGmAmAmCmUmAm_L96 | NO.23 | UmsAfsGmUmUmCf(NM036)UmGmGmUmGm CmUfCmUfUmGmGmsUmsUm | NO.24 |
| RZ59710 3 | CmsCmsAmAmGmAmGfCfAfCmCmAm AmGmAmAmCmUmAm_L96 | NO.25 | UmsAfsGmUmUmCf(NM037)UmGmGmUmGm CmUfCmUfUmGmGmsUmsUm | NO.26 |
| RZ59711 3 | CmsCmsAmAmGmAmGfCfAfCmCmAm AmGmAmAmCmUmAm_L96 | NO.27 | UmsAfsGmUmUmCf(SNB)UmGmGmUmGmC mUfCmUfUmGmGmsUmsUm | NO.28 |
| RZ59712 9 | CmsCmsAmAmGmAmGfCfAfCfCmAmA mGmAmAmCmUmAm_(CR01008)×3 | NO.29 | UmsAfsGmUmUmCfUmUmGfGmUmGmCmUf CmUfUmGmGmsUmsUm | NO.30 |
| RZ59713 0 | CmsCmsAmAmGmAmGfCfAfCfCmAmA mGmAmAmCmUmAm_(CR01008)×3 | NO.31 | UmsAfs(NM085)UmUmCfUmUmGfGmUmGm CmUfCmUfUmGmGmsUmsUm | NO.32 |
| RZ59713 1 | CmsCmsAmAmGmAmGfCfAfCfCmAmA mGmAmAmCmUmAm_(CR01008)×3 | NO.33 | UmsAfsGm(NM023)UmCfUmUmGfGmUmGm CmUfCmUfUmGmGmsUmsUm | NO.34 |
| RZ59713 2 | CmsCmsAmAmGmAmGfCfAfCfCmAmA mGmAmAmCmUmAm_(CR01008)×3 | NO.35 | UmsAfsGmUm(NM023)CfUmUmGfGmUmGm CmUfCmUfUmGmGmsUmsUm | NO.36 |
| RZ597133 | CmsCmsAmAmGmAmGfCfAfCfCmAmA mGmAmAmCmUmAm_(CR01008)×3 | NO.37 | UmsAfsGmUmUm(NM086)UmUmGfGmUmGm CmUfCmUfUmGmGmsUmsUm | NO.38 |
| RZ59713 4 | CmsCmsAmAmGmAmGfCfAfCfCmAmA mGmAmAmCmUmAm_(CR01008)×3 | NO.39 | UmsAfsGmUmUmCf(NM023)UmGfGmUmGm CmUfCmUfUmGmGmsUmsUm | NO.40 |
| RZ59713 5 | CmsCmsAmAmGmAmGfCfAfCfCmAmA mGmAmAmCmUmAm_(CR01008)×3 | NO.41 | UmsAfsGmUmUmCfUm(NM023)GfGmUmGm CmUfCmUfUmGmGmsUmsUm | NO.42 |
| RZ59713 6 | CmsCmsAmAmGmAmGfCfAfCfCmAmA mGmAmAmCmUmAm_(CR01008)×3 | NO.43 | UmsAfsGmUmUmCfUmUmGfGmUmGmCmUf C(moe)UfUmGmGmsUmsUm | NO.44 |
| RZ59713 7 | CmsCmsAmAmGmAmGfCfAfCfCmAmA mGmAmAmCmUmAm_(CR01008)×3 | NO.45 | UmsAfs(NM085)UmUmCfUmUmGfGmUmGm CmUfC(moe)UfUmGmGmsUmsUm | NO.46 |
| RZ59713 8 | CmsCmsAmAmGmAmGfCfAfCfCmAmA mGmAmAmCmUmAm_(CR01008)×3 | NO.47 | UmsAfsGm(NM023)UmCfUmUmGfGmUmGm CmUfC(moe)UfUmGmGmsUmsUm | NO.48 |
| RZ59713 9 | CmsCmsAmAmGmAmGfCfAfCfCmAmA mGmAmAmCmUmAm_(CR01008)×3 | NO.49 | UmsAfsGmUm(NM023)CfUmUmGfGmUmGm CmUfC(moe)UfUmGmGmsUmsUm | NO.50 |
| RZ59714 0 | CmsCmsAmAmGmAmGfCfAfCfCmAmA mGmAmAmCmUmAm_(CR01008)×3 | NO.51 | UmsAfsGmUmUm(NM086)UmUmGfGmUmGm CmUfC(moe)UfUmGmGmsUmsUm | NO.52 |
| RZ59714 1 | CmsCmsAmAmGmAmGfCfAfCfCmAmA mGmAmAmCmUmAm_(CR01008)×3 | NO.53 | UmsAfsGmUmUmCf(NM023)UmGfGmUmGm CmUfC(moe)UfUmGmGmsUmsUm | NO.54 |
| RZ59714 2 | CmsCmsAmAmGmAmGfCfAfCfCmAmA mGmAmAmCmUmAm_(CR01008)×3 | NO.55 | UmsAfsGmUmUmCfUm(NM023)GfGmUmGm CmUfC(moe)UfUmGmGmsUmsUm | NO.56 |
| RZ59714 3 | CmsCmsAmAmGmAmGfCfAfCfCmAmA mGmAmAmCmUmAm_(CR01008)×3 | NO.57 | UmsAfsGmUmUmCfUmUmGmGmUmGfCmUf C(moe)UfUmGmGmsUmsUm | NO.58 |
| RZ59714 4 | CmsCmsAmAmGmAmGfCfAfCfCmAmA mGmAmAmCmUmAm_(CR01008)×3 | NO.59 | UmsAfs(NM085)UmUmCfUmUmGmGmUmGf CmUfC(moe)UfUmGmGmsUmsUm | NO.60 |
| RZ59714 5 | CmsCmsAmAmGmAmGfCfAfCfCmAmA mGmAmAmCmUmAm_(CR01008)×3 | NO.61 | UmsAfsGm(NM023)UmCfUmUmGmGmUmGf CmUfC(moe)UfUmGmGmsUmsUm | NO.62 |
| RZ59714 6 | CmsCmsAmAmGmAmGfCfAfCfCmAmA mGmAmAmCmUmAm_(CR01008)×3 | NO.63 | UmsAfsGmUm(NM023)CfUmUmGmGmUmGf CmUfC(moe)UfUmGmGmsUmsUm | NO.64 |
| RZ59714 7 | CmsCmsAmAmGmAmGfCfAfCfCmAmA mGmAmAmCmUmAm_(CR01008)×3 | NO.65 | UmsAfsGmUmUm(NM086)UmUmGmGmUmGf CmUfC(moe)UfUmGmGmsUmsUm | NO.66 |
| RZ59714 8 | CmsCmsAmAmGmAmGfCfAfCfCmAmA mGmAmAmCmUmAm_(CR01008)×3 | NO.67 | UmsAfsGmUmUmCf(NM023)UmGmGmUmGf CmUfC(moe)UfUmGmGmsUmsUm | NO.68 |
| RZ59714 9 | CmsCmsAmAmGmAmGfCfAfCfCmAmA mGmAmAmCmUmAm_(CR01008)×3 | NO.69 | UmsAfsGmUmUmCfUm(NM023)GmGmUmGf CmUfC(moe)UfUmGmGmsUmsUm | NO.70 |
| RZ59200 7 | AmsUmsGmUmGmAmAfAfGfUfCmAmU mCmGmAmCmAmAm_(CR01008)×3 | NO.71 | UmsUfsGmUmCmGfAmUmGfAmCmUmUmUf CmAfCmAmUmsUmsCm | NO.72 |
| RZ59200 8 | AmsUmsGmUmGmAmAfAfGfUfCmAmU mCmGmAmCmAmAm_(CR01008)×3 | NO.73 | UmsUfs(NM085)UmCmGfAmUmGfAmCmUm UmUfCmAfCmAmUmsUmsCm | NO.74 |
| RZ59200 9 | AmsUmsGmUmGmAmAfAfGfUfCmAmU mCmGmAmCmAmAm_(CR01008)×3 | NO.75 | UmsUfsGm(NM023)CmGfAmUmGfAmCmUm UmUfCmAfCmAmUmsUmsCm | NO.76 |
| RZ59201 0 | AmsUmsGmUmGmAmAfAfGfUfCmAmU mCmGmAmCmAmAm_(CR01008)×3 | NO.77 | UmsUfsGmUm(NM086)GfAmUmGfAmCmUm UmUfCmAfCmAmUmsUmsCm | NO.78 |
| RZ59201 1 | AmsUmsGmUmGmAmAfAfGfUfCmAmU mCmGmAmCmAmAm_(CR01008)×3 | NO.79 | UmsUfsGmUmCm(NM085)AmUmGfAmCmUm UmUfCmAfCmAmUmsUmsCm | NO.80 |
| RZ59201 2 | AmsUmsGmUmGmAmAfAfGfUfCmAmU mCmGmAmCmAmAm_(CR01008)×3 | NO.81 | UmsUfsGmUmCmGf(NM084)UmGfAmCmUm UmUfCmAfCmAmUmsUmsCm | NO.82 |
| RZ59201 3 | AmsUmsGmUmGmAmAfAfGfUfCmAmU mCmGmAmCmAmAm_(CR01008)×3 | NO.83 | UmsUfsGmUmCmGfAm(NM023)GfAmCmUm UmUfCmAfCmAmUmsUmsCm | NO.84 |
| RZ59201 4 | AmsUmsGmUmGmAmAfAfGfUfCmAmU mCmGmAmCmAmAm_(CR01008)×3 | NO.85 | UmsUfsGmUmCmGfAmUmGfAmCmUmUmUf C(moe)AfCmAmUmsUmsCm | NO.86 |
| RZ59201 5 | AmsUmsGmUmGmAmAfAfGfUfCmAmU mCmGmAmCmAmAm_(CR01008)×3 | NO.87 | UmsUfs(NM085)UmCmGfAmUmGfAmCmUm UmUfC(moe)AfCmAmUmsUmsCm | NO.88 |
| RZ59201 6 | AmsUmsGmUmGmAmAfAfGfUfCmAmU mCmGmAmCmAmAm_(CR01008)×3 | NO.89 | UmsUfsGm(NM023)CmGfAmUmGfAmCmUm UmUfC(moe)AfCmAmUmsUmsCm | NO.90 |
| RZ59201 7 | AmsUmsGmUmGmAmAfAfGfUfCmAmU mCmGmAmCmAmAm_(CR01008)×3 | NO.91 | UmsUfsGmUm(NM086)GfAmUmGfAmCmUm UmUfC(moe)AfCmAmUmsUmsCm | NO.92 |
| RZ59201 8 | AmsUmsGmUmGmAmAfAfGfUfCmAmU mCmGmAmCmAmAm_(CR01008)×3 | NO.93 | UmsUfsGmUmCm(NM085)AmUmGfAmCmUm UmUfC(moe)AfCmAmUmsUmsCm | NO.94 |
| RZ59201 9 | AmsUmsGmUmGmAmAfAfGfUfCmAmU mCmGmAmCmAmAm_(CR01008)×3 | NO.95 | UmsUfsGmUmCmGf(NM084)UmGfAmCmUm UmUfC(moe)AfCmAmUmsUmsCm | NO.96 |
| RZ59202 0 | AmsUmsGmUmGmAmAfAfGfUfCmAmU mCmGmAmCmAmAm_(CR01008)×3 | NO.97 | UmsUfsGmUmCmGfAm(NM023)GfAmCmUm UmUfC(moe)AfCmAmUmsUmsCm | NO.98 |
| RZ59202 1 | AmsUmsGmUmGmAmAfAfGfUfCmAmU mCmGmAmCmAmAm_(CR01008)×3 | NO.99 | UmsUfsGmUmCmGfAmUmGmAmCmUfUmUf C(moe)AfCmAmUmsUmsCm | NO.100 |
| RZ59202 2 | AmsUmsGmUmGmAmAfAfGfUfCmAmU mCmGmAmCmAmAm_(CR01008)×3 | NO.101 | UmsUfs(NM085)UmCmGfAmUmGmAmCmUf UmUfC(moe)AfCmAmUmsUmsCm | NO.102 |
| RZ59202 3 | AmsUmsGmUmGmAmAfAfGfUfCmAmU mCmGmAmCmAmAm_(CR01008)×3 | NO.103 | UmsUfsGm(NM023)CmGfAmUmGmAmCmUf UmUfC(moe)AfCmAmUmsUmsCm | NO.104 |
| RZ59202 4 | AmsUmsGmUmGmAmAfAfGfUfCmAmU mCmGmAmCmAmAm_(CR01008)×3 | NO.105 | UmsUfsGmUm(NM086)GfAmUmGmAmCmUf UmUfC(moe)AfCmAmUmsUmsCm | NO.106 |
| RZ59202 5 | AmsUmsGmUmGmAmAfAfGfUfCmAmU mCmGmAmCmAmAm_(CR01008)×3 | NO.107 | UmsUfsGmUmCm(NM085)AmUmGmAmCmUf UmUfC(moe)AfCmAmUmsUmsCm | NO.108 |
| RZ59202 6 | AmsUmsGmUmGmAmAfAfGfUfCmAmU mCmGmAmCmAmAm_(CR01008)×3 | NO.109 | UmsUfsGmUmCmGf(NM084)UmGmAmCmUf UmUfC(moe)AfCmAmUmsUmsCm | NO.110 |
| RZ59202 7 | AmsUmsGmUmGmAmAfAfGfUfCmAmU mCmGmAmCmAmAm_(CR01008)×3 | NO.111 | UmsUfsGmUmCmGfAm(NM023)GmAmCmUf UmUfC(moe)AfCmAmUmsUmsCm | NO.112 |
| RZ59715 6 | CmsCmsAmAmGmAmGfCfAfCfCmAmA mGmAmAmCmUmAm_(CR01008)×3 | NO.113 | UmsAfsGm(NM102)UmCfUmUmGfGmUmGm CmUfC(moe)UfUmGmGmsUmsUm | NO.114 |
| RZ59715 7 | CmsCmsAmAmGmAmGfCfAfCfCmAmA mGmAmAmCmUmAm_(CR01008)×3 | NO.115 | UmsAfsGmUm(NM102)CfUmUmGfGmUmGm CmUfC(moe)UfUmGmGmsUmsUm | NO.116 |
| RZ59715 8 | CmsCmsAmAmGmAmGfCfAfCfCmAmA mGmAmAmCmUmAm_(CR01008)×3 | NO.117 | UmsAfsGmUmUmCf(NM102)UmGfGmUmGm CmUfC(moe)UfUmGmGmsUmsUm | NO.118 |
| RZ59715 9 | CmsCmsAmAmGmAmGfCfAfCfCmAmA mGmAmAmCmUmAm_(CR01008)×3 | NO.119 | UmsAfsGm(NM103)UmCfUmUmGfGmUmGm CmUfC(moe)UfUmGmGmsUmsUm | NO.120 |
| RZ59716 0 | CmsCmsAmAmGmAmGfCfAfCfCmAmA mGmAmAmCmUmAm_(CR01008)×3 | NO.121 | UmsAfsGmUm(NM103)CfUmUmGfGmUmGm CmUfC(moe)UfUmGmGmsUmsUm | NO.122 |
| RZ597161 | CmsCmsAmAmGmAmGfCfAfCfCmAmA mGmAmAmCmUmAm_(CR01008)×3 | NO.123 | UmsAfsGmUmUmCf(NM103)UmGfGmUmGm CmUfC(moe)UfUmGmGmsUmsUm | NO.124 |
| RZ59716 2 | CmsCmsAmAmGmAmGfCfAfCfCmAmA mGmAmAmCmUmAm_(CR01008)×3 | NO.125 | UmsAfsGm(NM104)UmCfUmUmGfGmUmGm CmUfC(moe)UfUmGmGmsUmsUm | NO.126 |
| RZ59716 3 | CmsCmsAmAmGmAmGfCfAfCfCmAmA mGmAmAmCmUmAm_(CR01008)×3 | NO.127 | UmsAfsGmUm(NM104)CfUmUmGfGmUmGm CmUfC(moe)UfUmGmGmsUmsUm | NO.128 |
| RZ59716 4 | CmsCmsAmAmGmAmGfCfAfCfCmAmA mGmAmAmCmUmAm_(CR01008)×3 | NO.129 | UmsAfsGmUmUmCf(NM104)UmGfGmUmGm CmUfC(moe)UfUmGmGmsUmsUm | NO.130 |
| RZ59716 5 | CmsCmsAmAmGmAmGfCfAfCfCmAmA mGmAmAmCmUmAm_(CR01008)×3 | NO.131 | UmsAfsGm(NM105)UmCfUmUmGfGmUmGm CmUfC(moe)UfUmGmGmsUmsUm | NO.132 |
| RZ59716 6 | CmsCmsAmAmGmAmGfCfAfCfCmAmA mGmAmAmCmUmAm_(CR01008)×3 | NO.133 | UmsAfsGmUm(NM105)CfUmUmGfGmUmGm CmUfC(moe)UfUmGmGmsUmsUm | NO.134 |
| RZ59716 7 | CmsCmsAmAmGmAmGfCfAfCfCmAmA mGmAmAmCmUmAm_(CR01008)×3 | NO.135 | UmsAfsGmUmUmCf(NM105)UmGfGmUmGm CmUfC(moe)UfUmGmGmsUmsUm | NO.136 |

Illustrative explanation: "CmsAmsGmAmCmAmGfAdCfAmAmGmAmCmCmAmUmCmUm_L96" represents that L96 is conjugated to the 3' end of the modified version of the nucleotide sequence shown in SEQ ID NO. 1 via phosphodiester linkage. Wherein, in the direction from the 5' end to the 3' end, the modified version of the nucleotide sequence shown in SEQ ID NO. 1 is CmsAmsGmAmCmAmGfAdCfAmAmGmAmCmCmAmUmCmUm.

Unless otherwise specified, the meanings of the base compositions and modifications in the synthetic sequences of the present disclosure are as follows: uppercase letters A, U, G, C, T represent the base compositions of nucleotides, respectively; lowercase letter m indicates that the nucleotide represented by the adjacent uppercase letter to the left of the letter m is a 2'-O-methyl modified (also referred to as 2'-methoxy modified) nucleotide; lowercase letter f indicates that the nucleotide represented by the adjacent uppercase letter to the left of the letter f is a 2'-fluoro modified nucleotide; lowercase letter d indicates that the nucleotide represented by the adjacent uppercase letter to the left of the letter d is a 2'-deoxy modified nucleotide; the combined identifier (moe) indicates that the nucleotide represented by the adjacent uppercase letter to the left of the combined identifier (moe) is a 2'-O-methoxyethyl modified nucleotide; lowercase letter s indicates that a phosphorothioate linkage exists between the two adjacent nucleotides to the left and right of the letter s.
(NM022), (NM023), (NM036), (NM037), (NM084), (NM085), (NM086), (NM102), (NM103), (NM104), (NM105) and (SNB) each represent one nucleotide.

Wherein, after the aforementioned "siRNA conjugate synthesis", the structural formula of Compound NM022 in the above sequence is shown as (NM022): 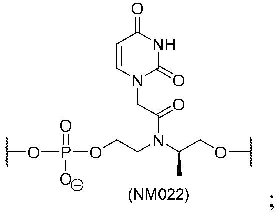
after the aforementioned "siRNA conjugate synthesis", the structural formula of Compound NM023 in the above sequence is shown as (NM023): 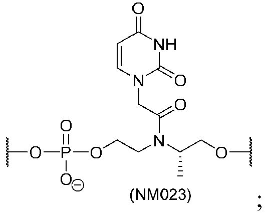
after the aforementioned "siRNA conjugate synthesis", the structural formula of Compound NM036 in the above sequence is shown as (NM036): 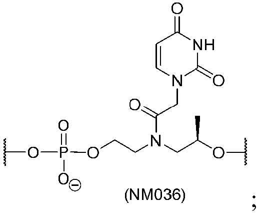
after the aforementioned "siRNA conjugate synthesis", the structural formula of Compound NM037 in the above sequence is shown as (NM037): 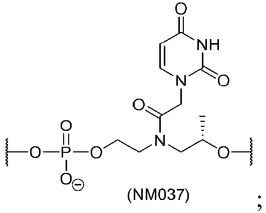
after the aforementioned "siRNA conjugate synthesis", the structural formula of Compound NM084 in the above sequence is shown as (NM084): 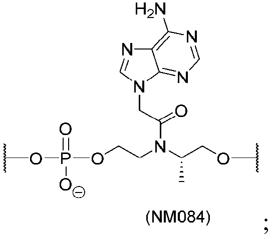
after the aforementioned "siRNA conjugate synthesis", the structural formula of Compound NM085 in the above sequence is shown as (NM085): 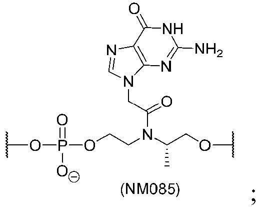
after the aforementioned "siRNA conjugate synthesis", the structural formula of Compound NM086 in the above sequence is shown as (NM086): 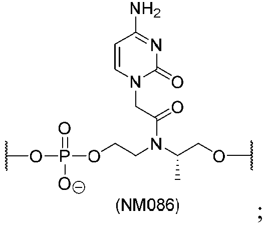
after the aforementioned "siRNA conjugate synthesis", the structural formula of Compound NM102 in the above sequence is shown as (NM102): 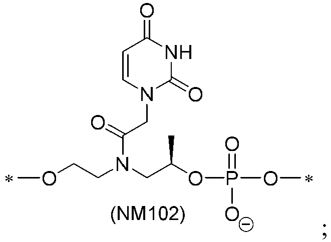
after the aforementioned "siRNA conjugate synthesis", the structural formula of Compound NM103 in the above sequence is shown as (NM103): 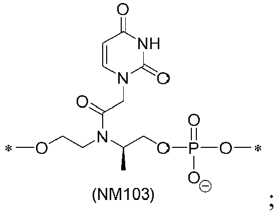
after the aforementioned "siRNA conjugate synthesis", the structural formula of Compound NM104 in the above sequence is shown as (NM104): 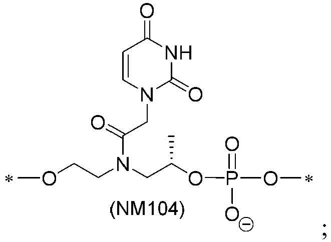
after the aforementioned "siRNA conjugate synthesis", the structural formula of Compound NM105 in the above sequence is shown as (NM105): 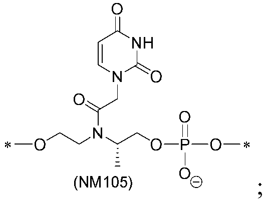
the structural formula of (SNB) is: 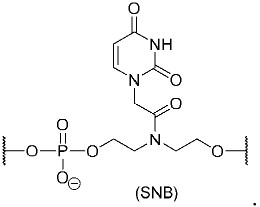 (SNB) is derived from the nucleoside analog SNB, and the structural formula of SNB is

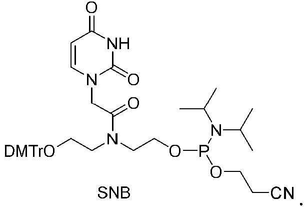

**Table 6 Detection Results of siRNA Conjugates**

| siRNA conjugate ID | Sense strand | | | Antisense strand | | | siRNA conjugate | | |
|---|---|---|---|---|---|---|---|---|---|
| | Theoretical molecular weight | Actual molecular weight | Purity % | Theoretical molecular weight | Actual molecular weight | Purity % | Theoretical molecular weight | Actual molecular weight | Purity % |
| RZ502017 | 8084.3 | 8084.8 | 97.9 | 6955.6 | 6956.7 | 96.8 | 15039.9 | 15041.5 | 93.3 |
| RZ502025 | 8084.3 | 8084.6 | 97.9 | 6968.5 | 6968.8 | 95.3 | 15052.8 | 15053.4 | 93.0 |
| RZ502051 | 8084.3 | 8084.9 | 95.8 | 6968.5 | 6968.8 | 98.4 | 15052.8 | 15053.7 | 93.5 |
| RZ597002 | 8125.3 | 8126 | 96.8 | 6932.6 | 6932.9 | 95.7 | 15057.9 | 15058.9 | 96.9 |
| RZ597007 | 8125.3 | 8126.7 | 97.8 | 6945.5 | 6945.5 | 98.0 | 15070.8 | 15072.2 | 95.1 |
| RZ597112 | 8125.3 | 8126.7 | 98.7 | 6945.5 | 6946 | 97.8 | 15070.8 | 15072.7 | 96.4 |
| RZ597102 | 8125.3 | 8126.7 | 97.8 | 6946.6 | 6946.2 | 98.9 | 15072 | 15072.9 | 90.5 |
| RZ597103 | 8125.3 | 8126.7 | 97.8 | 6946.6 | 6946.4 | 97.0 | 15072 | 15073.1 | 91.2 |
| RZ597113 | 8125.3 | 8126.5 | 98.9 | 6930.4 | 6932.3 | 97.3 | 15055.7 | 15058.8 | 91.1 |

It can be seen from the data shown in Table 6 that, the siRNA conjugates shown in Table 5 were obtained in the present disclosure, with high purity.

### Biological Assays

Unless otherwise specified, all the experimental animals, C57BL/6J mice, used in the present disclosure were purchased from Beijing SPF Biotechnology Co., Ltd.; all the serum biochemical detections in the present disclosure were commissioned to Beijing Sinogenetic Biotechnology Co., Ltd.; all the pathological sectioning, staining and slide reading in the present disclosure were commissioned to Wuhan Servicebio Technology Co., Ltd.

### Evaluation Method for Inhibitory Activity of siRNA Conjugates toTarget Genes in Mice

C57BL/6J mice, 6-8 weeks of age, were randomly grouped by body weight (all females). The drug dosage for mice in each group was calculated based on body weight, and a single administration was performed via subcutaneous injection in the abdominal region. Each siRNA conjugate was dissolved in PBS at the corresponding concentration (calculated based on siRNA) for administration, with the administration volume being 5 mL (based on siRNA)/kg (based on the mouse body weight). The PBS control group was given 5 mL/kg (based on the mouse body weight) of PBS solution (without the drug conjugates). The day of administration was designated as Day 0 (denoted as D0). At predetermined time points after administration, 5 mice in each group were sacrificed respectively. Each sacrificed mouse was subjected to gross dissection, and liver tissues of each sacrificed mouse were collected. The liver tissue was cut into small pieces of approximately 2 mm³ and preserved in RNALater.

Liver tissue samples at different time points from different experimental groups were taken from the aforementioned RNAlater. The liver tissue samples were homogenized for 60 seconds in a Tissuelyser II Automatic Tissue Homogenizer, and then the total RNA was extracted using an Automatic Nucleic Acid Extractor (purchased from Zhejiang Hanwei Technology Co., Ltd.) and a Nucleic Acid Extraction Kit (purchased from Zhejiang Hanwei Technology Co., Ltd.) according to the standard operating procedures for total RNA extraction.

1 µg of the aforementioned total RNA was taken, and a 20 µL reverse transcription system was prepared and the reverse transcription reaction was completed using a reverse transcription kit (Promega Corporation, Reverse Transcription System, A3500) with Oligo (dT)15 reverse transcription primers, in accordance with the method described in the reverse transcription kit instructions. After the reaction was completed, 80 µL of RNase-Free water was added to the reverse transcription system to obtain a cDNA solution. Subsequently, a real-time fluorescent quantitative PCR kit (ABI Corporation, SYBR^{™} Select Master Mix, Catalog number: 4472908) was used to detect the expression level of target gene mRNA in liver tissues. In this real-time fluorescent quantitative PCR method, primers targeting the target gene and primers targeting the internal reference gene were used to detect the target gene and the internal reference gene, respectively. A 20 µL Real-time PCR reaction system was prepared for each PCR detection well in accordance with the method described in the real-time fluorescent quantitative PCR kit instructions. Each reaction system contained 5 µL of the cDNA solution obtained from the aforementioned reverse transcription reaction, 10 µL of SYBR^{™} Select Master Mix, 0.5 µL of 10 µM upstream primer, 0.5 µL of 10 µM downstream primer, and 4 µL of RNase-Free H₂O. The prepared reaction system was placed in a real-time fluorescent quantitative PCR instrument (ABI Corporation, StepOnePlus^{™}), and Real-time PCR amplification was performed using a three-step method. The amplification program was as follows: pre-denaturation at 95 °C for 10 min, followed by denaturation at 95 °C for 30 s, annealing at 60 °C for 30 s, and extension at 72 °C for 30 s; the denaturation, annealing, and extension process was repeated for 40 cycles. In this real-time fluorescent quantitative PCR method, the ΔΔCt method was used for relative quantitative calculation of the mRNA expression level and inhibition rate of the target gene in each test group. The calculation method is as follows:
ΔCt (test group) = Ct (target gene in test group) - Ct (internal reference gene in test group) 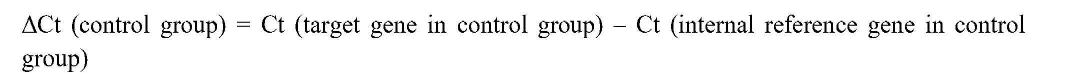 ΔCt (control group) = Ct (target gene in control group) - Ct (internal reference gene in control group) $ΔΔCt \left(test group\right) = ΔCt \left(test group\right) - ΔCt \left(control group average\right)$ $ΔΔCt \left(control group\right) = ΔCt \left(control group\right) - ΔCt \left(control group average\right)$

Wherein, the ΔCt (control group average) is the arithmetic mean of the ΔCt (control group) for each of the 5 mice sacrificed at the same time point in the control group. Therefore, each mouse in both the test group and the control group corresponds to a ΔΔCt value.

Using the control group as the baseline, the target gene mRNA expression level of the test group was normalized, and the target gene mRNA residual expression level of the control group was defined as 100%. 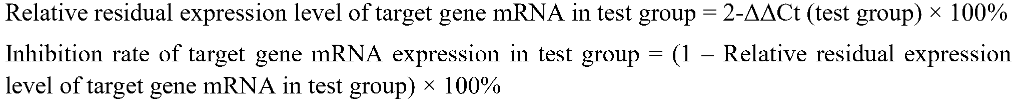 Relative residual expression level of target gene mRNA in test group = 2-ΔΔCt (test group) × 100% Inhibition rate of target gene mRNA expression in test group = (1 - Relative residual expression level of target gene mRNA in test group) × 100%

Unless otherwise specified, the in vivo activity experimental data were expressed as X̅±SD, and all experimental data were plotted and analyzed using GraphPad prism 8.0 software.

In the present disclosure, unless otherwise specified, all in vivo activity experimental data used in the present disclosure were expressed as X̅±SD ( X̅±STDEV), and all experimental data were plotted and analyzed using GraphPad prism 8.0 software.

### Example 1 Evaluation of Inhibitory Activity of siRNA Conjugates Against Target Gene Complement Component 3 (CC3) in Mice

This example evaluated the inhibitory activity of siRNA conjugate RZ502025 comprising the NM023 group in the antisense strand, siRNA conjugate RZ502051 comprising the NM022 group, and control conjugate RZ502017 without the aforementioned groups against the target gene CC3 in mice using the method for evaluating target gene inhibitory activity in mice. The NM022 group and the NM023 group are isomers of each other.

C57BL/6J mice, 6-8 weeks of age, were randomly divided into 4 groups by body weight, with 10 mice per group. Mice in each group were administered with the aforementioned siRNA conjugates via subcutaneous injection in the abdominal region. Wherein each mouse in the PBS control group was given a dose with an administration volume of 5 ml/kg; each mouse in the siRNA conjugate experimental groups was given a dose of 3 mg/kg (based on siRNA) with an administration volume of 5 mL/kg. The day of administration was designated as Day 0 (D0). After administration, 5 mice in each group were sacrificed on Day 7 (D7) and 5 mice in each group were sacrificed on Day 21 (D21), respectively. Gross dissection was performed on the animals, and liver tissues were collected for RNA extraction, reverse transcription reaction and Real-time PCR detection. The relative quantitative calculation of target gene mRNA in each test group was performed according to the aforementioned ΔΔCt method.

**Table 7 Primer Sequence Information in Example 1**

| Target site | | Primer type | Primer Sequence (5'-3') |
|---|---|---|---|
| Target gene | CC3 | Upstream primer | CCAGCTCCCCATTAGCTCTG (SEQ ID NO.7) |
| | | Downstream primer | GCACTTGCCTCTTTAGGAAGTC (SEQ ID NO.8) |
| Internal reference gene | GAPDH | Upstream primer | TGCACCACCAACTGCTTAG (SEQ ID NO.9) |
| | | Downstream primer | GGATGCAGGGATGATGTTC (SEQ ID NO.10) |

**Table 8 Inhibitory Activity against Target Gene in Mice After Administration of the siRNA Conjugates in Example 1**

| Group | D7 | | D21 | |
|---|---|---|---|---|
| | Mean % inhibition rate | +SD value | Mean % inhibition rate | ±SD value |
| PBS | 0.00 | 15.93 | 0.00 | 30.82 |
| RZ502017 | 65.10 | 13.92 | 42.25 | 10.31 |
| RZ502025 | 92.18 | 1.29 | 45.45 | 8.40 |
| RZ502051 | 88.26 | 10.51 | 45.73 | 17.91 |

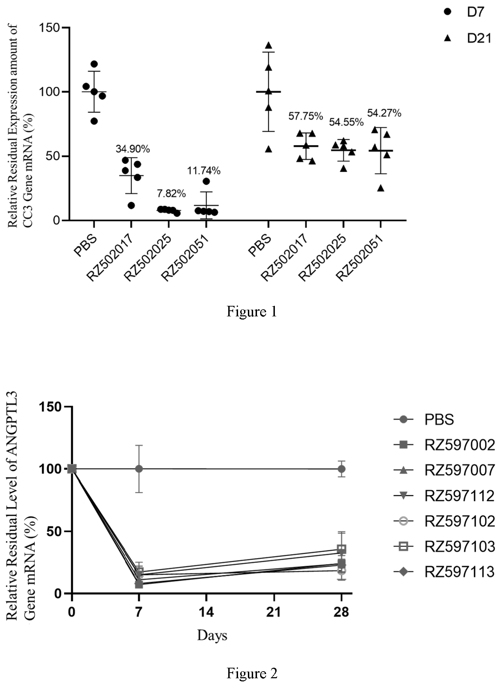

The results of Example 1 are shown in Figure 1 and Table 8. Conjugate RZ502025 comprising the NM023 group in the antisense strand and conjugate RZ502051 comprising the NM022 group exhibited superior activity on D7 and D21 as compared to the control conjugate RZ502017 without such groups.

### Example 2 Evaluation of Inhibitory Activity of siRNA Conjugates Against Target Gene Angiopoietin-like Protein 3 (ANGPTL3) in Mice

This example evaluated the inhibitory activity of siRNA conjugate RZ597007 comprising the NM023 group, siRNA conjugate RZ597112 comprising the NM022 group, siRNA conjugate RZ597102 comprising the NM036 group, siRNA conjugate RZ597103 comprising the NM037 group, reference conjugate RZ597113 comprising the SNB group, and reference conjugate RZ597002 without such groups against the target gene ANGPTL3 in mice using the method for evaluating target gene inhibitory activity in mice. Wherein, the NM036 group differs from the NM023 group in the position of methyl on the open-ring structure; the NM037 group differs from the NM022 group in the position of methyl on the open-ring structure; and the NM036 group and the NM037 group are isomers of each other.

C57BL/6J mice, 6-8 weeks of age, were randomly divided into 7 groups by body weight, with 10 mice per group. Mice in each group were administered the aforementioned siRNA conjugates via subcutaneous administration in the abdominal region. Wherein each mouse in the PBS control group was given a dose with an administration volume of 5 mL/kg; each mouse in the siRNA conjugate experimental groups was given a dose of 3 mg/kg (based on siRNA) with an administration volume of 5 mL/kg. The day of administration was designated as D0. After administration, 5 mice in each group were sacrificed on D7 and D28, respectively. Gross dissection was performed on the animals, and liver tissues were collected, cut into several small pieces of 2 mm³, and preserved in RNALater. The methods for RNA extraction, reverse transcription reaction and Real-time PCR detection were described above, and the relative quantitative calculation of target gene mRNA in each test group was performed according to the aforementioned ΔΔCt method.

**Table 9 Primer Sequence Information in Example 2**

| Target site | | Primer type | Primer Sequence (5'-3') |
|---|---|---|---|
| Target gene | ANGPTL3 | Upstream primer | GAGGAGCAGCTAACCAACTTAAT (SEQ ID NO.11) |
| | | Downstream primer | TCTGCATGTGCTGTTGACTTAAT (SEQ ID NO.12) |
| Internal reference gene | GAPDH | Upstream primer | TGCACCACCAACTGCTTAG (SEQ ID NO.9) |
| | | Downstream primer | GGATGCAGGGATGATGTTC (SEQ ID NO.10) |

**Table 10 Inhibitory Activity of the Target Gene in Mice After Administration of siRNA Conjugates in Example 2**

| Group | D7 | | D28 | |
|---|---|---|---|---|
| | Mean % inhibition rate | ±SD value | Mean % inhibition rate | +SD value |
| PBS | 0.00 | 18.95 | 0.00 | 6.49 |
| RZ597002 | 92.78 | 1.15 | 75.87 | 3.60 |
| RZ597007 | 88.97 | 3.40 | 75.99 | 6.49 |
| RZ597112 | 84.89 | 2.09 | 67.34 | 15.85 |
| RZ597102 | 84.94 | 10.14 | 81.51 | 7.84 |
| RZ597103 | 82.69 | 4.69 | 64.28 | 14.15 |
| RZ597113 | 91.95 | 3.16 | 77.10 | 11.59 |

The results of Example 2 are shown in Figure 2 and Table 10. The conjugate RZ597007 comprising the NM023 group in the antisense strand exhibited the activity substantially comparable to that of the control conjugate RZ597113 comprising the SNB group and the control conjugate RZ597002 without such groups at both D7 and D28. At D7 and D28, the inhibitory activity of conjugate RZ597112 comprising the NM022 group differed by 8%-10% from that of the control conjugates. At D7, the conjugate RZ597102 comprising the NM036 group in the antisense strand exhibited the inhibitory activity substantially comparable to that of the control conjugates RZ597113 and RZ597002, while at D28, its inhibitory activity was superior to that of the control conjugates. The inhibitory activity of conjugate RZ597103 comprising the NM037 group in the antisense strand was weaker than that of the control conjugates at both D7 and D28. In summary, it indicates that, compared to the control sequences, the siRNA conjugate comprising the NM036 group can maintain superior inhibitory activity, the conjugate comprising the NM023 group can maintain comparable inhibitory activity, the NM023 configuration was superior to the NM022 configuration, and the conjugate comprising the NM037 group ranked next.

### Example 3 Toxicological Evaluation of siRNA Conjugates in Mice

This example evaluated the nature and extent of toxic reactions in ICR mice of conjugate RZ597007 comprising the NM023 group in the antisense strand, conjugate RZ597112 comprising the NM022 group, conjugate RZ597102 comprising the NM036 group, conjugate RZ597103 comprising the NM037 group, as well as reference conjugate RZ597113 comprising the SNB group and reference conjugate RZ597002 without such groups, using subcutaneous injection administration.

ICR mice aged 6-8 weeks were randomly divided into groups by body weight, with 3 mice per group. Each test group was administered the drug conjugate at the aforementioned dose, and a PBS control group was additionally included. The drug dosage was calculated for all mice based on body weight, and a single dose was administered by abdominal subcutaneous injection. Each drug conjugate was administered as a PBS solution at a concentration of 60 mg/mL (calculated based on siRNA), with a does volume of 10 mL /kg (mouse body weight), i.e., the dose of each drug conjugate was 600 mg/kg mouse body weight (calculated based on siRNA). The PBS control group was given the same volume of PBS solution (without drug conjugate), and observations were conducted for 7 days. The day of administration was designated as Day 0 (denoted as D0). During the test period, clinical observations were performed at least once daily after the initial administration; on Day 7 of the test (denoted as D7), blood was collected from all animals (mice were fasted for no less than 12 hours with free access to water, before sampling and detection) for blood biochemical detection. Blood samples (non-anticoagulated) were collected, placed at room temperature for approximately 30 min, and centrifuged at 2000 g for 10 min at 4 °C after blood coagulation, and blood biochemical detection was performed using an automatic biochemical analyzer (Mindray BS-430). The calculation formula for the change rate of each serum biochemical indicator: Change rate = (test group - blank control group (PBS)) / blank control group.

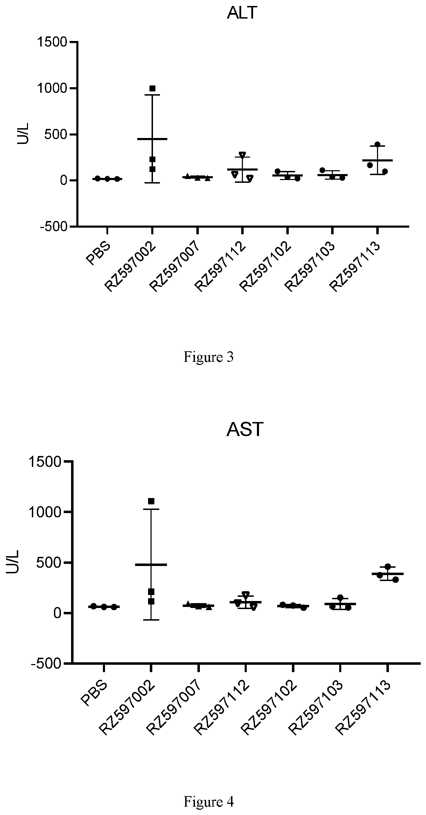

Figures 3 and 4 are scatter plots showing the serum ALT and AST concentrations in mice after administration of 600 mg/kg of RZ597007, RZ597112, RZ597102, RZ597103, and reference conjugates RZ597002 and RZ597113, as well as the blank control group PBS, respectively. As shown in Figures 3 and 4, compared to the blank control group, after administration of conjugate RZ597002 without such groups, the serum ALT level in mice increased from 18.10 U/L of the blank control group to 450.10 U/L, with a change rate of 23.9-fold; the AST concentration was also significantly elevated, increasing from 62.60 U/L to 478.90 U/L, with a change rate of 6.7-fold, exhibiting an obvious hepatotoxic reaction.

However, after administration of the siRNA conjugates of this example, the serum ALT and AST concentrations decreased to varying degrees, indicating that the siRNA conjugates of this example have a certain effect of reducing hepatotoxicity. Specifically, after administration of the conjugate comprising the NM023 group (RZ597007), the serum ALT concentration in mice decreased to 34.30 U/L, resulting in a reduction of the ALT change rate of this conjugate from 23.9-fold (RZ597002) to 0.9-fold; and the AST concentration decreased to 74.40 U/L, resulting in a reduction of the AST change rate of this conjugate from 6.7-fold (RZ597002) to 0.2-fold, which was substantially comparable to that of the PBS group. After administration of the conjugate comprising the NM036 group (RZ597102), the serum ALT change rate in mice was 1.9-fold and the AST change rate was 0.1-fold. After administration of the conjugate comprising the NM037 group (RZ597103), the serum ALT change rate in mice was 2.2-fold and the AST change rate was 0.5-fold. Neither of these two groups showed obvious increases in ALT and AST levels. The NM023, NM036 and NM037 groups have an obvious effect of reducing hepatotoxicity.

After administration of the conjugate comprising the NM022 group (RZ597112), the serum ALT change rate in mice was 5.5-fold and the AST change rate was 0.7-fold. Compared to the control conjugate (RZ597002) at 23.9-fold (ALT) and 6.7-fold (AST), it still exhibited a certain effect of alleviating hepatotoxicity.

However, for the reference conjugate RZ597113 comprising the SNB group, the serum ALT and AST change rates in mice were 11.1-fold and 5.2-fold, respectively. Although it exhibited a certain effect of alleviating hepatotoxicity compared to the control conjugate (RZ597002), it still exhibited a greater degree of hepatotoxic reaction compared to the other siRNA conjugates in this example.

**Table 11 Results of Blood Biochemical Detection in Mice After Administration of siRNA Conjugates of in Example 3**

| Test indicator | | Alanine aminotransferase (ALT) | Aspartate aminotransferase (AST) | Total bilirubin (TBIL) | Urea | Creatinine (Crea) | Triglycerides (TG) | Total cholesterol (TC) |
|---|---|---|---|---|---|---|---|---|
| PBS | Mean Value | 18.1 | 62.6 | 0.87 | 5.02 | 27.5 | 4.44 | 14.92 |
| | ±SD | 2.85 | 3.8 | 0.34 | 0.88 | 1.25 | 1.53 | 0.33 |
| RZ597002 | Mean Value | 450.1 | 478.9 | 1.42 | 5.32 | 30.2 | 1.61 | 4.76 |
| | ±SD | 477.54 | 548.39 | 1.37 | 0.56 | 6.64 | 0.27 | 0.5 |
| RZ597007 | Mean Value | 34.3 | 74.4 | 0.77 | 5 | 35.3 | 1.24 | 4.03 |
| | ±SD | 10.95 | 16.79 | 0.33 | 0.28 | 3.46 | 0.43 | 0.07 |
| RZ597112 | Mean Value | 116.9 | 108.2 | 0.87 | 6.77 | 38.9 | 1.7 | 4.21 |
| | ±SD | 135.99 | 60.52 | 0.31 | 0.6 | 7.25 | 0.3 | 0.77 |
| RZ597102 | Mean Value | 52 | 69 | 0.63 | 5.73 | 30.9 | 1.41 | 4.28 |
| | ±SD | 42.01 | 15.06 | 0.32 | 0.81 | 11.11 | 0.27 | 1.76 |
| RZ597103 | Mean Value | 58.5 | 91.1 | 0.87 | 4.19 | 34.4 | 1.78 | 3.97 |
| | ±SD | 45.98 | 53.17 | 0.15 | 0.45 | 4.03 | 0.18 | 0.5 |
| RZ597113 | Mean Value | 218.5 | 388.3 | 0.89 | 5.72 | 32.3 | 1.77 | 4.06 |
| | ±SD | 153.61 | 65.47 | 0.12 | 1.32 | 1.51 | 0.29 | 0.18 |

On Day 7 of the experiment (denoted as D7), all animals were subjected to dissection. Prior to dissection, the mice to be dissected were fasted for at least 12 hours with free access to water, anesthetized with a RWD R540IE small animal anesthesia machine, subjected to blood collection, and then euthanized by exsanguination via the abdominal aorta, followed by gross anatomical observation. The livers of all animals were preserved in 4% cell tissue fixative and subjected to histopathological examination (using hematoxylin-eosin staining). The severity of hepatocellular degeneration in the pathological sections was evaluated and graded using a four-grade classification system (reference: [US] Peter Mann et al., International Harmonization of Nomenclature and Diagnostic Criteria for Pathological Changes in Rats and Mice (INHAND) [M]. Translated by Yang Lifeng, Zhou Xiangmei & Zhao Deming. Beijing: China Agricultural Press, 2019), and relatively compared.

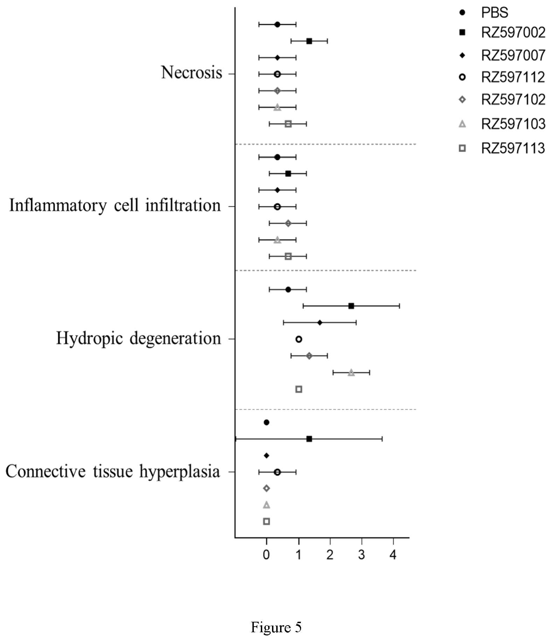

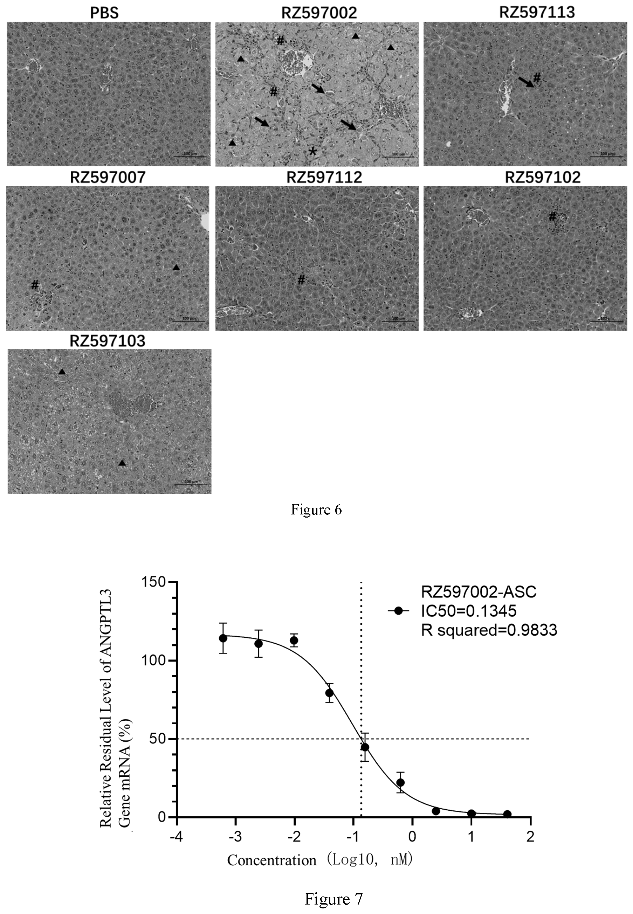

As shown in the pathological section results in Figures 5 and 6, compared with the blank control group, among the 3 mice administered with the reference conjugate RZ597002 without any such substituted groups: 1 mouse exhibited moderate to severe hepatocellular degeneration, specifically manifested as hydropic degeneration in a large number of hepatocytes, severe cellular swelling, loose and pale-stained cytoplasm (A), irregular arrangement of hepatocytes, frequent hepatocellular necrosis, karyolysis (arrow), connective tissue hyperplasia visible around numerous venous blood vessels and hepatic sinusoids (*), disordered hepatic lobule structure, accompanied by scattered lymphocyte infiltration (#); 1 mouse exhibited mild to moderate hepatocellular degeneration, swelling in a large number of hepatocytes, loose and pale-stained cytoplasm, spotty hepatocyte necrosis, and a small amount of granulocyte and lymphocyte infiltration; and 1 mouse exhibited mild hepatocellular degeneration, with hydropic degeneration in partial hepatocytes and a small amount of hepatocellular necrosis. In summary, they exhibited more severe hepatocellular degeneration than those in the blank control group. Among the 3 mice administered with the reference conjugate RZ597113 comprising the SNB group: 2 mice exhibited slight to mild hepatocellular degeneration, specifically manifested as hydropic degeneration in a small number of hepatocytes, spotty necrosis of partial hepatocytes, and punctate lymphocyte infiltration; and 1 mouse showed slight hepatocellular degeneration, manifested as slight swelling in partial hepatocytes, and loose, pale-stained cytoplasm.

After administration of the conjugates comprising the substituted groups NM023 (RZ597007), NM022 (RZ597112), NM036 (RZ597102) and NM037 (RZ597103), each indicator of hepatocellular degeneration in the pathological sections was reduced. In particular, each siRNA conjugate showed lower grades for hepatocellular necrosis levels than the reference conjugates RZ597113 and RZ597002 (Figures 5 and 6). Specifically, both groups of mice administered with the conjugate comprising the NM022 group (RZ597112) and the conjugate comprising the NM036 group (RZ597102) showed slight hepatocellular degeneration. In each group, only 1 mouse exhibited a small amount of spotty hepatocellular necrosis, slight lymphocyte infiltration, and a small amount of hydropic degeneration of hepatocytes, while the remaining 2 mice showed only slight hydropic degeneration of hepatocytes without hepatocyte necrosis. Among the 3 mice administered with the conjugate comprising the NM023 group (RZ597007), 2 mice exhibited slight to mild hepatocellular degeneration, manifested as partial hepatocyte swelling, of which 1 was accompanied by a small amount of spotty hepatocellular necrosis, and the other 1 exhibited slight hydropic degeneration of hepatocytes. The 3 mice administered with the conjugate comprising the NM037 group (RZ597103) exhibited slight to mild hepatocellular degeneration, manifested as partial hepatocyte swelling, of which 1 was accompanied by a small amount of spotty hepatocellular necrosis. In addition, in the blank control group, 1 mouse also exhibited a very small amount of spotty hepatocellular necrosis, partial inflammatory cell infiltration and hydropic degeneration; and the slight hepatocellular degeneration observed was assessed to be attributable to the animal's own cellular metabolism .

In summary, compared with the conjugate RZ597002 without such groups and the reference conjugate RZ597113, the siRNA conjugates of the present disclosure can reduce the hepatotoxic reactions due to off-target effects to a certain extent, exhibiting higher safety. Among them, the conjugate RZ597102 comprising the NM036 group demonstrated better safety while maintaining superior inhibitory activity.

### Method for Evaluating the in Vitro Cell Inhibitory Activity of siRNA Conjugates Against Target Genes

Plasmid construction: the siRNA seed region with the on-target/off-target sequences was designed and respectively inserted into the multiple cloning site downstream of the hRluc gene in the psi-CHECK2 plasmid, to construct the antisense strand on-target plasmid (ASC) and off-target plasmid (ASM).

Cell culture and transfection: Cells were routinely cultured in DMEM complete medium under the conditions of 37 °C and 5% CO₂. When the density of HEK293T cells in a 75 cm² cell culture flask reached 80-90%, the medium was discarded and the cells were washed with 0.25% trypsin. After the cells were digested with trypsin, the digestion was terminated with medium. The cells were resuspended, centrifuged at 800 rpm for 5 min, resuspended in fresh medium for counting, and diluted to a cell density of 8×10³ cells/well, and seeded into a 96-well cell plate at 100 µL/well, and transfection was performed after 24 hours of culture.

Preparation of siRNA gradient dilutions: Each of the conjugates was prepared into a 20 µM stock solution (calculated as siRNA) with PBS. Then, the stock solution was serially diluted with PBS to working solutions at concentrations of 4000 nM, 1000 nM, 250 nM, 62.5 nM, 15.625 nM, 3.906 nM, 0.9766 nM, 0.2441 nM, and 0.06104 nM, respectively. Preparation of transfection mixtures: 0.2 µL of Lipo2000 was added into 9.8 µL of Opti-MEM for each well to prepare 10 µL of Lipo2000 mixture, which was placed at room temperature for 5 min. For each well, 10 ng of ASC/ASM plasmid was added, i.e., 0.05 µL of the plasmid stock solution at a concentration of 200 ng/ul was added into 8.95 µL of Opti-MEM to prepare 9 µL of plasmid mixture. 1 µL of siRNA working solution, 9 µL of plasmid mixture and 10 µL of Lipo2000 were mixed to obtain the transfection mixture. Meanwhile, transfection control wells at 0 nM were set up, i.e., 1 µL of PBS, 9 µL of plasmid mixture and 10 µL of Lipo2000 were mixed to obtain 20 µL of control transfection mixture (control). After mixing thoroughly by pipetting, the mixture was incubated at room temperature for 20 min before subsequent transfection.

The complete medium in the culture wells was aspirated and discarded. Each well was replaced with 80 µL of Opti-MEM medium, and then 20 µL of the transfection mixture was added. Each concentration of each conjugate was tested in triplicate wells. After cultivation in a 37 °C, 5% CO₂ incubator for 4 h, 100 µL of DMEM medium containing 10% fetal bovine serum was supplemented to each well. The culture plate was placed in a 37 °C, 5% CO₂ cell incubator and further cultured for 24 h.

Detection and analysis: the medium in the 96-well plate was completely aspirated. 150 µL of Firefly reaction solution (prepared at a 1:1 ratio, for each well, i.e., 75 µL of Firefly substrate and 75 µL of DMEM /(well)) was added to each well, placed and shaken on a shaker protected from light for 10 min to allow the reaction. 120 µL of the reaction solution was aspirated to a detection plate, and the plate was read on a microplate reader to obtain the Firefly values. Then, 60 µL of stop solution (prepared at a 1:100 ratio, for each well, i.e., 1 µL of Renilla substrate and 100 µL of Buffer /(well), freshly prepared immediately before used) was added to the detection plate, placed and shaken on a shaker protected from light for 10 min to allow the reaction. The plate was read on a microplate reader to obtain the Renilla values; the normalized ratio was calculated as follows: Ratio = Renilla / Firefly.

Calculation of the remaining inhibitory activity rate: [1-Mean(Ratio_{siRNA conjugate}/Ratio_{control}) ]*100%= inhibited activity (%); Wherein, Ratio_{control} is the mean value of the Ratios of the three replicate wells in the control wells (without siRNA conjugates); Ratio_{siRNA conjugate} represents the mean value of the Ratios of the three replicate wells in the test wells (containing siRNA conjugate); inhibited activity represents the remaining inhibitory activity; Mean(Ratio_{siRNA}/Ratio_{control}) refers to the mean value of (Ratio_{siRNA conjugate}/Ratio_{control}) *100% in the three replicate wells.

IC50 curve fitting was performed using the log(inhibitor) vs. response- Variable slope (four parameters) four - parameter fitting model in GraphPad prism 8.0. The calculation formula for the IC50 concentration value is: X=10^(LogIC50-Log((Top-Bottom)/(50-Bottom)-1)/HillSlope). All parameters in the formula can be obtained from the analysis result table of GraphPad prism 8.0.

IC60 curve fitting was performed using the log(inhibitor) vs. response- Variable slope (four parameters) four - parameter fitting model in GraphPad prism 8.0. The calculation formula for the IC60 concentration value is: X=10^(LogIC50-Log((Top-Bottom)/(60-Bottom)-1)/HillSlope). All parameters in the formula can be obtained from the analysis result table of GraphPad prism 8.0.

IC75 curve fitting was performed using the log(inhibitor) vs. response- Variable slope (four parameters) four - parameter fitting model in GraphPad prism 8.0. The calculation formula for the IC75 concentration value is: X=10^(LogIC50-Log((Top-Bottom)/(75-Bottom)-1)/HillSlope). All parameters in the formula can be obtained from the analysis result table of GraphPad prism 8.0.

### Example 4 Evaluation of the Inhibitory Activity of siRNA Conjugates Against the Target Gene Angiopoietin-like Protein 3 (ANGPTL3) in HEK293T Cells

This example evaluated the inhibitory activity of siRNA conjugate RZ597007 comprising the NM023 group, siRNA conjugate RZ597112 comprising the NM022 group, siRNA conjugate RZ597102 comprising the NM036 group, siRNA conjugate RZ597103 comprising the NM037, reference conjugate RZ597113 comprising the SNB, and reference conjugate RZ597002 without such substituted groups against the target gene ANGPTL3 in HEK293T cells using the in vitro cell method for evaluating the inhibitory activity against the target gene. Wherein, the NM022 and NM023 groups are isomers of each other; the NM036 and NM037 groups are isomers of each other; the NM036 group differs from the NM023 group in the position of methyl on the open-ring structure.

In accordance with the aforementioned in vitro cell method for evaluating the inhibitory activity against the target gene, on-target assays and off-target assays of the siRNA conjugates were performed respectively, and the IC50 values of the on-target assays and the IC75 values of the off-target assays were calculated.

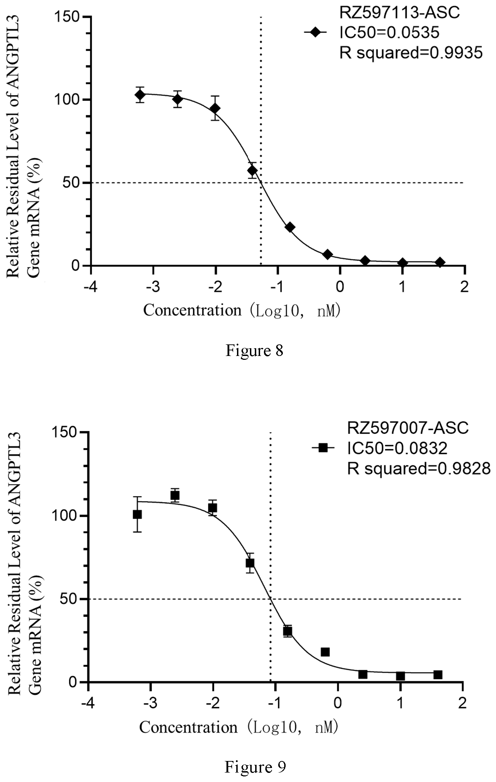

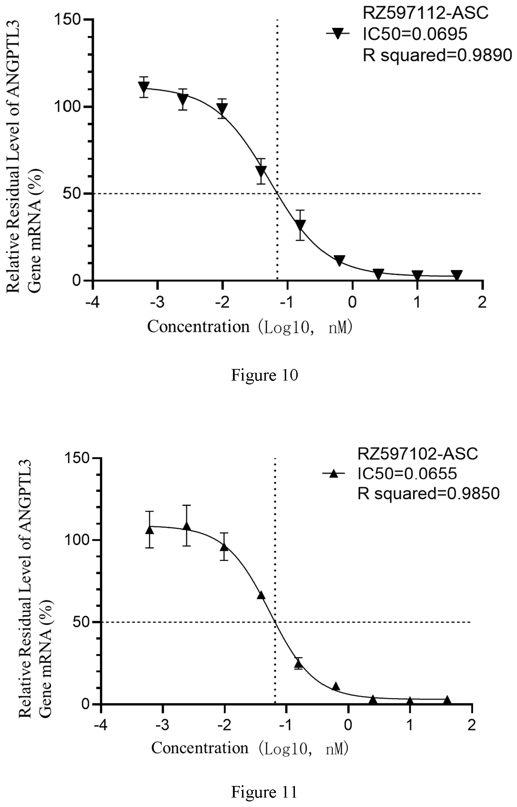

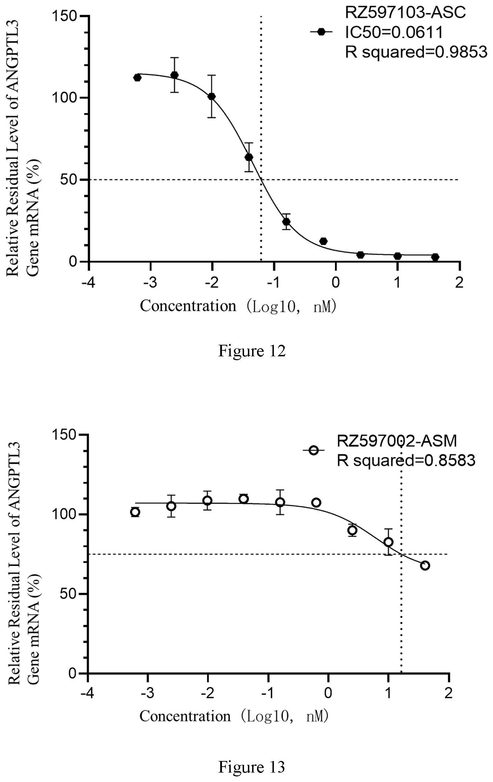

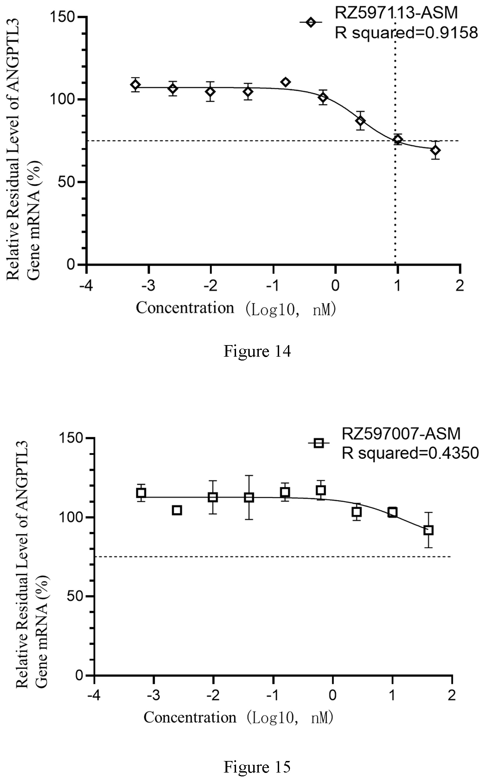

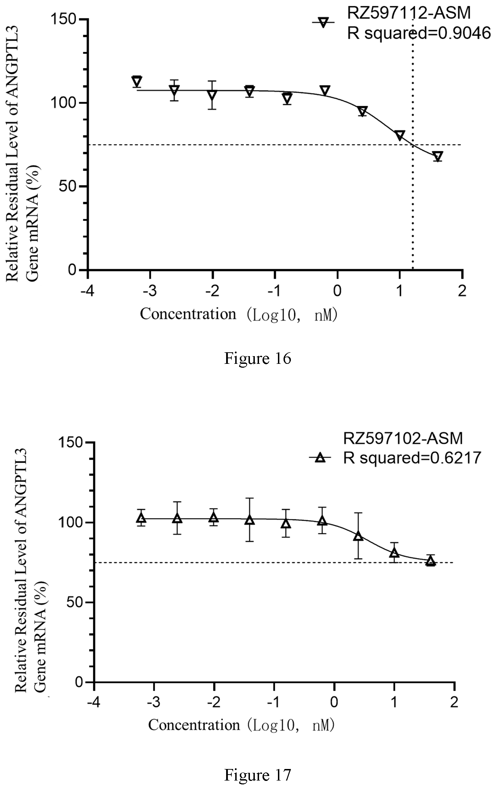

The results of Example 4 showed that in the on-target (ASC) evaluation, the IC50 values of siRNA conjugates RZ597007, RZ597112, RZ597102 and RZ597103 were 0.0832 nM, 0.0695 nM, 0.0655 nM and 0.0611 nM, respectively, with activity superior to that of the reference conjugate RZ597002 without such substituted groups (0.1345 nM); and being substantially comparable to that of the reference conjugate RZ597113 comprising the SNB group (0.0535 nM). However, the calculation of IC75 values in the off-target (ASM) activity evaluation found that RZ597007 and RZ597102 had almost no off-target activity; compared with RZ597002 (16.3539 nM) and RZ597113 (9.0746 nM), RZ597112 had an IC75 value of 16.1609 nM, which is superior to RZ597113 in reducing off-target effects; RZ597103 had an IC75 value of 37.6409 nM, showing a certain trend of preventing off-target effects (Figures 7-18, Table 12).

**Table 12 Evaluation of IC50 and IC75 Values of siRNA Conjugates Against the Target Gene ANGPTL3 in HEK293T Cells**

| Group | ASC IC50(nM) | ASM IC75(nM) |
|---|---|---|
| RZ597002 | 0.1345 | 16.3539 |
| RZ597113 | 0.0535 | 9.0746 |
| RZ597007 | 0.0832 | / |
| RZ597112 | 0.0695 | 16.1609 |
| RZ597102 | 0.0655 | / |
| RZ597103 | 0.0611 | 37.6409 |
| Note: "/" indicates that no IC75 value was obtained in the IC75 curve fitting. | | |

### Example 5 Evaluation of the Inhibitory Activity of siRNA Conjugates Against the Target Gene ANGPTL3 in HEK293T Cells

This example evaluated the inhibitory activity of siRNA conjugate RZ597157 comprising the NM102 group at position 5 of the antisense strand, siRNA conjugate RZ597161 comprising the NM103 group at position 7 of the antisense strand, and reference conjugate RZ597136 without any substituted groups against the target gene ANGPTL3 in HEK293T cells using the in vitro cell method for evaluating the inhibitory activity against the target gene.

In accordance with the aforementioned in vitro cell method for evaluating the inhibitory activity against the target gene, on-target assays and off-target assays of the siRNA conjugates were performed, and the IC50 values of the on-target assays and the IC60 values of the off-target assays were calculated.

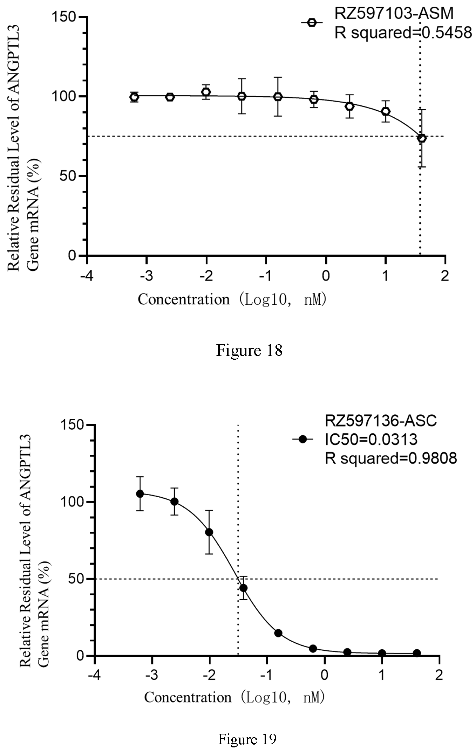

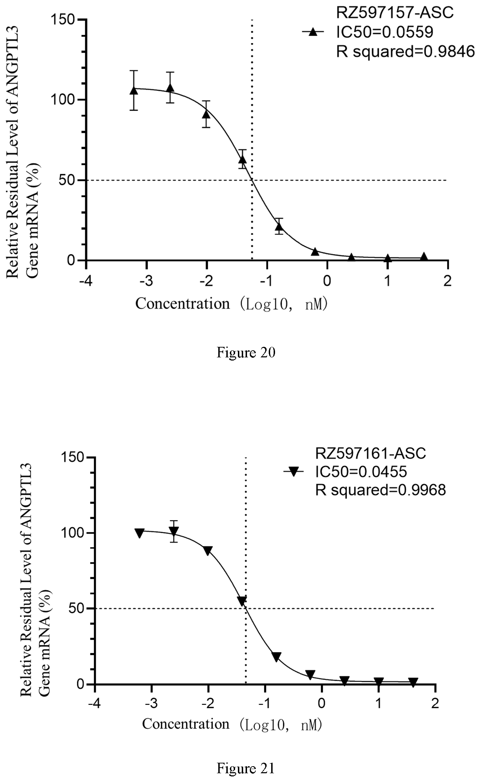

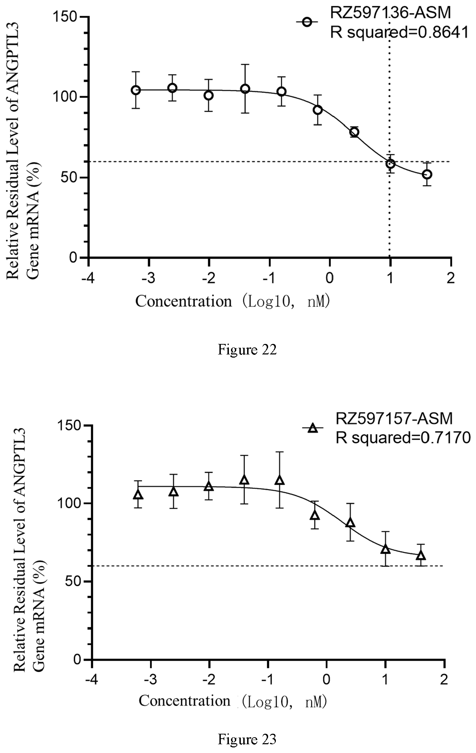

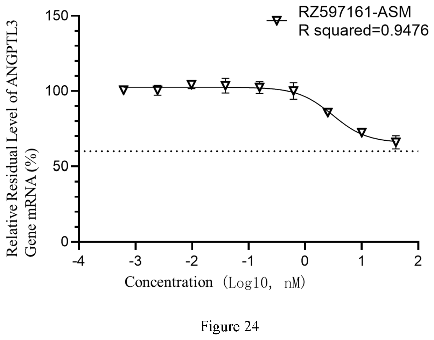

The results of Example 5 showed that the IC50 values for the on-target (ASC) activity evaluation of siRNA conjugates RZ597157 and RZ597161 were 0.0559 nM and 0.0455 nM, respectively, which were substantially comparable to that of the reference conjugate RZ597136 (0.0313 nM); no IC60 values were obtained by fitting in the off-target (ASM) activity evaluation of RZ597157 and RZ597161, while that of the reference conjugate RZ597136 was 9.5713 nM, indicating that RZ597157 and RZ597161 showed a certain trend of preventing off-target effects (Figures 19-24, Table 13).

**Table 13 Evaluation of IC50 and IC60 Values of siRNA Conjugates Against the Target Gene ANGPTL3 in HEK293T Cells**

| Group | ASC IC50(nM) | ASM IC60(nM) |
|---|---|---|
| RZ597136 | 0.0313 | 9.5713 |
| RZ597157 | 0.0559 | / |
| RZ597161 | 0.0455 | / |
| Note: "/" indicates that no IC60 value was obtained in the IC60 curve fitting. | | |

It can be understood that the above embodiments are merely exemplary embodiments used to illustrate the principles of the present disclosure, but the present disclosure is not limited thereto. For the skilled in the art, various modifications and improvements can be made without departing from the spirit and essence of the present disclosure, and such modifications and improvements are also considered to be within the protection scope of the present disclosure.

## Claims

1. A nucleotide analog, **characterized in that**, it is selected from the structure set forth in the following formula (II-i), or a stereoisomer thereof, or a pharmaceutically acceptable salt thereof: 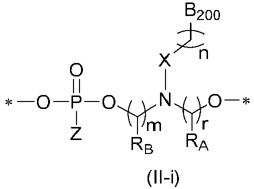
wherein, B₂₀₀ is selected from a base or a modified base;
Z is selected from hydroxyl or sulfhydryl;
n is selected from 1, 2 or 3;
X is selected from 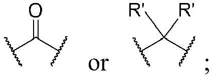 each R' is independently selected from optionally substituted C₁₋₃ alkyl, and the optionally substituted C₁₋₃ alkyl contains substituent(s) independently selected from halogen, C₁₋₃ alkoxy, hydroxyl or amino;
m is selected from 1, 2, 3 or 4;
r is selected from 1, 2, 3 or 4;
each R_{A} and each R_{B} are independently selected from H or optionally substituted C₁₋₃ alkyl, and there is at least one optionally substituted C₁₋₃ alkyl in R_{A} and R_{B}; if the optionally substituted C₁₋₃ alkyl contains substituent(s), the substituent(s) are independently selected from halogen, C₁₋₃ alkoxy, hydroxyl or amino;
each * independently represents a site of covalent bond attachment;
optionally, the nucleotide analog set forth in the formula (II-i) has the structure shown in the following formula (200), or a stereoisomer thereof, or a pharmaceutically acceptable salt thereof: 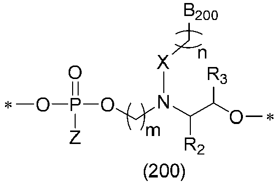
wherein, R₂ and R₃ are independently selected from H or optionally substituted C₁₋₃ alkyl; and at least one of R₂ and R₃ is selected from optionally substituted C₁₋₃ alkyl; if the optionally substituted C₁₋₃ alkyl contains substituent(s), the substituent(s) are independently selected from halogen, C₁₋₃ alkoxy, hydroxyl or amino; and the remaining substituents are as defined above.

2. The nucleotide analog of claim 1, **characterized in that**, the base is selected from uracil U, thymine T, cytosine C, adenine A or guanine G;
optionally, the modified base is selected from 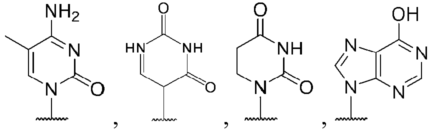 and 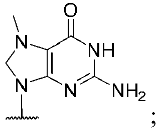
optionally, B₂₀₀ is selected from any one of the following structures:

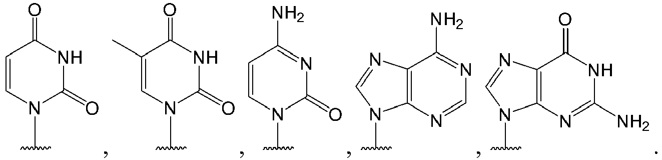

3. The nucleotide analog of any one of claims 1-2, **characterized in that** n is selected from 1 or 2; optionally, n is selected from 1.

4. The nucleotide analog of any one of claims 1-3, **characterized in that** m is selected from 1, 2 or 3; optionally, m is selected from 2.

5. The nucleotide analog of any one of claims 1-4, **characterized in that** X is selected from

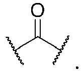

6. The nucleotide analog of any one of claims 1-5, **characterized in that** R₂ and R₃ are independently selected from H or optionally substituted C₁₋₃ alkyl; and R₂ and R₃ are not simultaneously H or optionally substituted C₁₋₃ alkyl;
optionally, R₂ and R₃ are independently selected from H or C₁₋₃ alkyl; and R₂ and R₃ are not simultaneously H or C₁₋₃ alkyl;
optionally, R₂ and R₃ are independently selected from H or methyl; and R₂ and R₃ are not simultaneously H or methyl.

7. The nucleotide analog of any one of claims 1-6, **characterized in that** the nucleotide analog has the structure set forth in formula (201), or a stereoisomer thereof, or a pharmaceutically acceptable salt thereof:

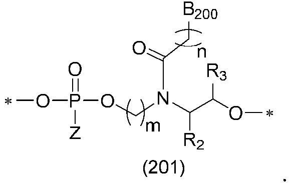

8. The nucleotide analog of any one of claims 1-7, **characterized in that** the nucleotide analog has any one of the following structures, or a stereoisomer thereof, or a pharmaceutically acceptable salt thereof: 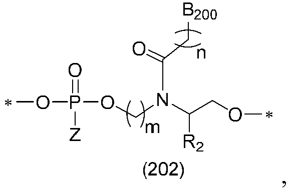 wherein R₂ is selected from optionally substituted C₁₋₃ alkyl; 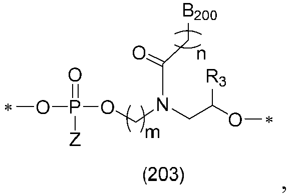 wherein R₃ is selected from optionally substituted C₁₋₃ alkyl.

9. The nucleotide analog of any one of claims 1-8, **characterized in that** the nucleotide analog has any one of the following structures, or a stereoisomer thereof, or a pharmaceutically acceptable salt thereof: 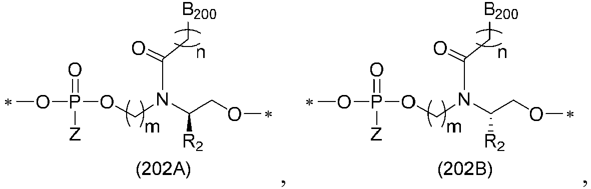 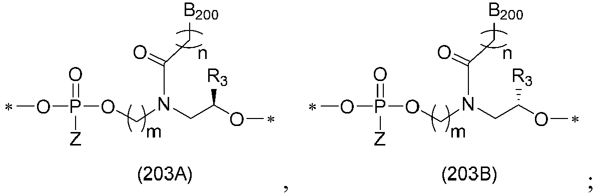 wherein R₂ and R₃ are independently selected from optionally substituted C₁₋₃ alkyl.

10. The nucleotide analog of any one of claims 1-9, **characterized in that** the nucleotide analog is selected from any one of the following structures, or a stereoisomer thereof, or a pharmaceutically acceptable salt thereof:

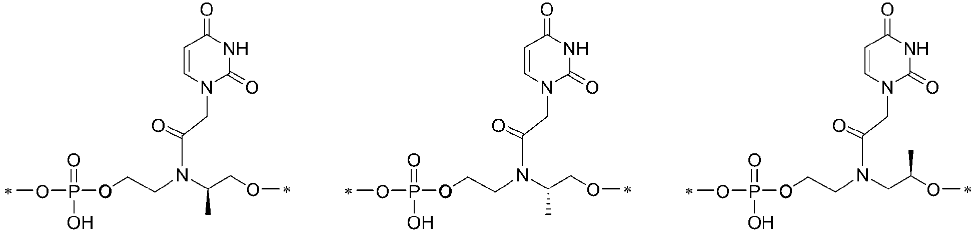

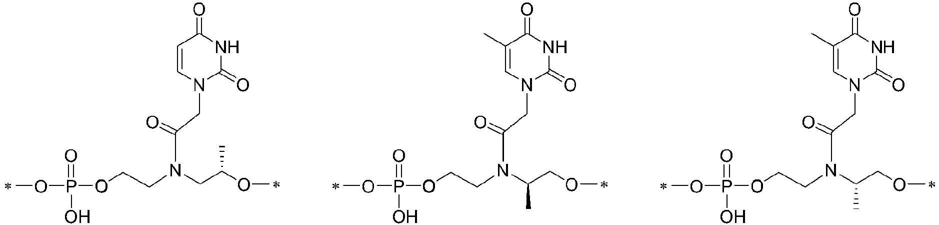

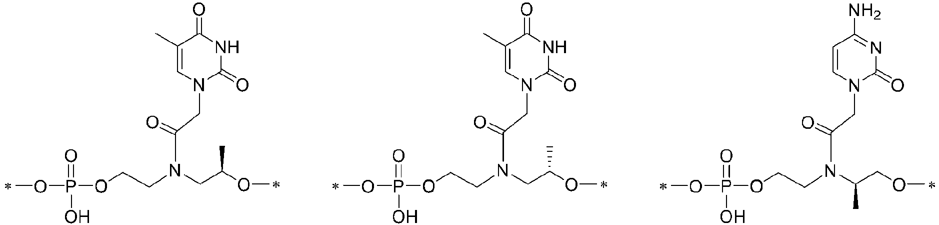

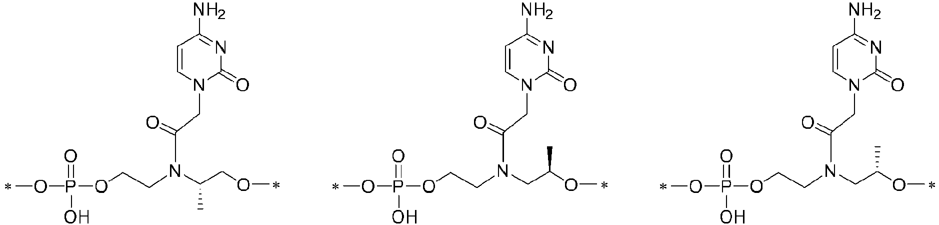

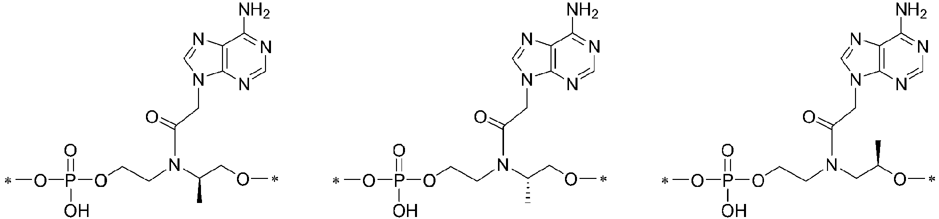

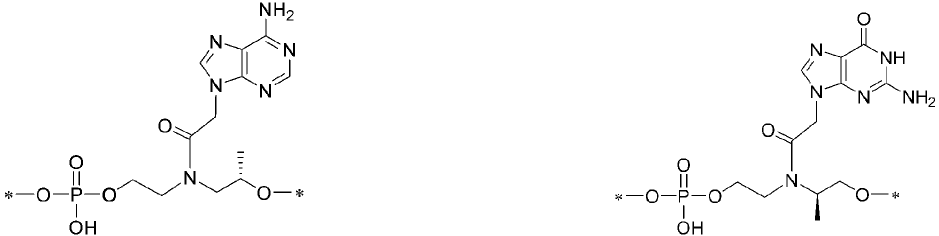

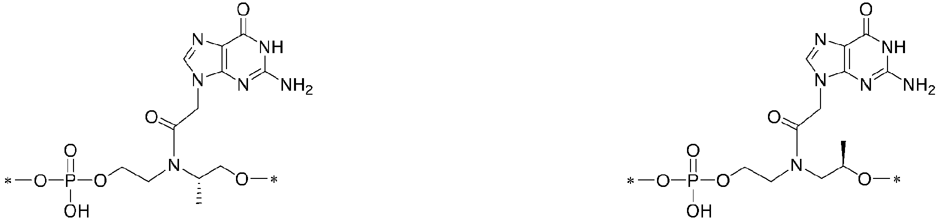

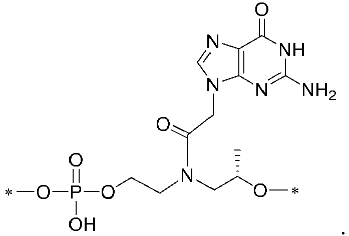

11. The nucleotide analog of claim 1, **characterized in that** the nucleotide analog set forth in the formula (II-i) has the structure set forth in the following formula (500), or a stereoisomer thereof, or a pharmaceutically acceptable salt thereof: 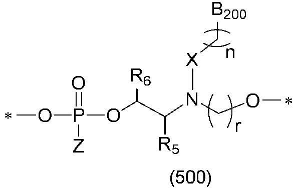
wherein, B₂₀₀, Z, n, X and * are as defined in any one of claims 1-10;
r is selected from 1, 2, 3 or 4;
R₅ and R₆ are independently selected from H or optionally substituted C₁₋₃ alkyl; and at least one of R₅ and R₆ is selected from optionally substituted C₁₋₃ alkyl; if the optionally substituted C₁₋₃ alkyl contains substituent(s), the substituent(s) are independently selected from halogen, C₁₋₃ alkoxy, hydroxyl or amino.

12. The nucleotide analog of claim 11, **characterized in that** r is selected from 1, 2 or 3;
optionally, r is selected from 2;
optionally, R₅ and R₆ are independently selected from H or optionally substituted C₁₋₃ alkyl; and R₅ and R₆ are not simultaneously H or optionally substituted C₁₋₃ alkyl;
optionally, R₅ and R₆ are independently selected from H or C₁₋₃ alkyl; and R₅ and R₆ are not simultaneously H or C₁₋₃ alkyl;
optionally, R₅ and R₆ are independently selected from H or methyl; and R₅ and R₆ are not simultaneously H or methyl;
optionally, the nucleotide analog has the structure set forth in formula (501), or a stereoisomer thereof, or a pharmaceutically acceptable salt thereof: 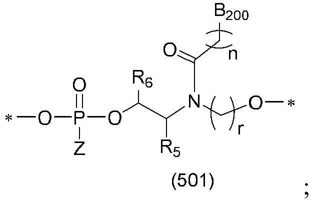
optionally, the nucleotide analog has any one of the following structures, or a stereoisomer thereof, or a pharmaceutically acceptable salt thereof: 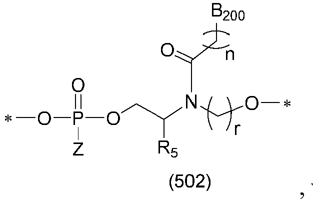
wherein R₅ is selected from optionally substituted C₁₋₃ alkyl; 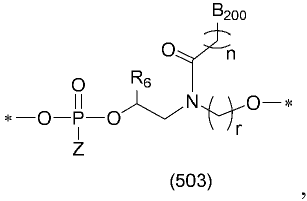
wherein R₆ is selected from optionally substituted C₁₋₃ alkyl;
optionally, the nucleotide analog has any one of the following structures, or a stereoisomer thereof, or a pharmaceutically acceptable salt thereof: 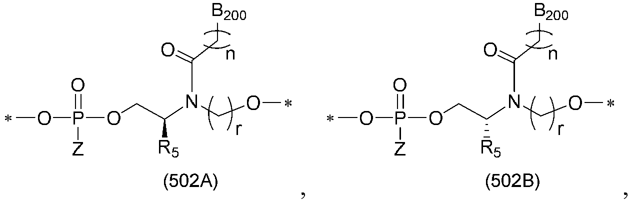 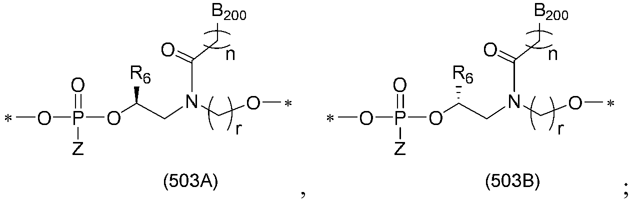
wherein R₅ and R₆ are independently selected from optionally substituted C₁₋₃ alkyl;
optionally, the nucleotide analog is selected from any one of the following structures, or a stereoisomer thereof, or a pharmaceutically acceptable salt thereof:

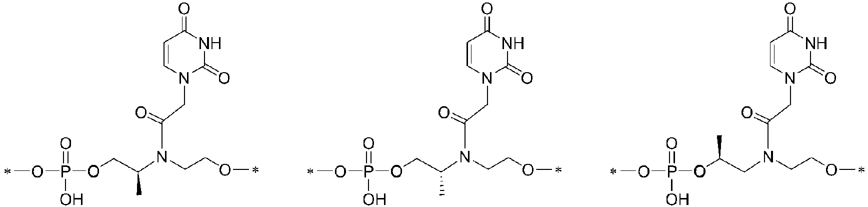

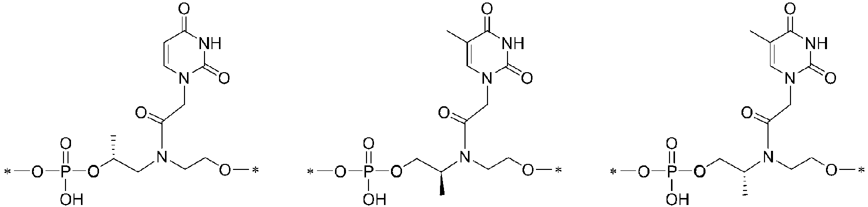

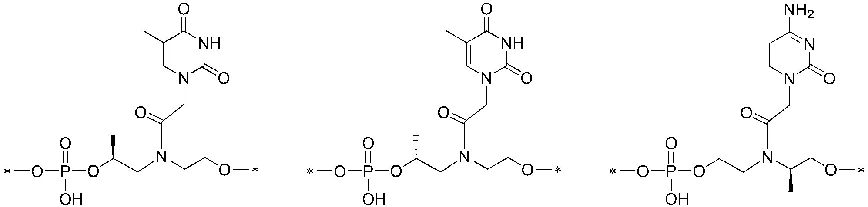

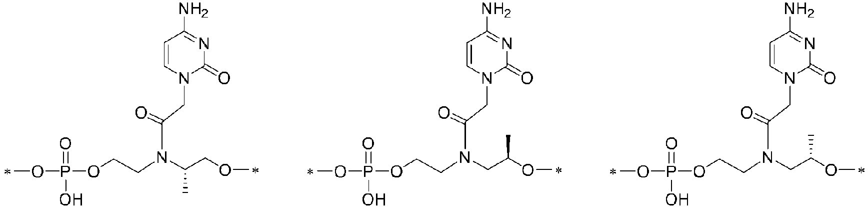

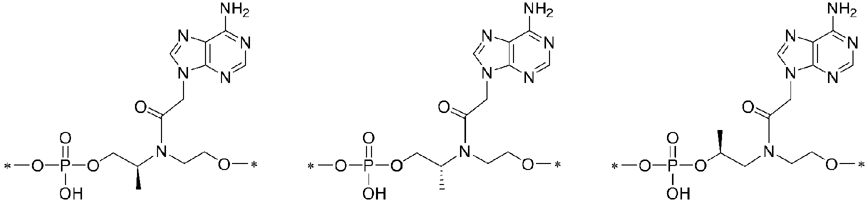

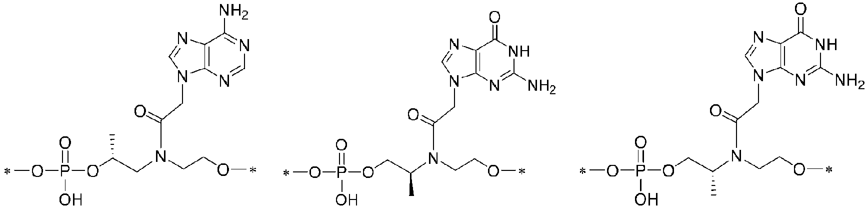

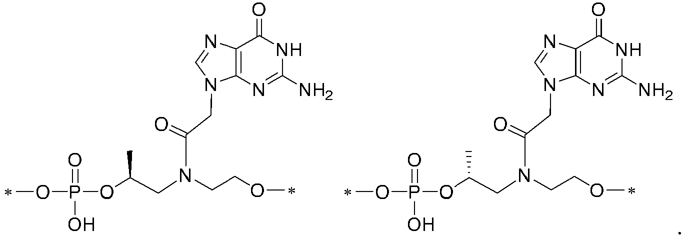

13. A double-stranded oligonucleotide, **characterized in that** the double-stranded oligonucleotide comprises a sense strand and an antisense strand, each strand having 17-35 nucleotides; in the direction from the 5' end to the 3' end, at least one nucleotide at positions 2-8 of the antisense strand is substituted by the nucleotide analog of any one of claims 1-12.

14. The double-stranded oligonucleotide of claim 13, **characterized in that**, in the direction from the 5' end to the 3' end, at least one nucleotide at positions 3-8 of the antisense strand is substituted by the nucleotide analog;
optionally, in the direction from the 5' end to the 3' end, any one of the nucleotides at position 3, position **4,** position 5, position 6, position 7 or position 8 of the antisense strand is substituted by the nucleotide analog;
optionally, in the direction from the 5' end to the 3' end, any one of the nucleotides at position 5, position 6, position 7 or position 8 of the antisense strand is substituted by the nucleotide analog;
optionally, in the direction from the 5' end to the 3' end, the nucleotide at position 3 of the antisense strand is substituted by the nucleotide analog;
optionally, in the direction from the 5' end to the 3' end, the nucleotide at position 4 of the antisense strand is substituted by the nucleotide analog;
optionally, in the direction from the 5' end to the 3' end, the nucleotide at position 5 of the antisense strand is substituted by the nucleotide analog;
optionally, in the direction from the 5' end to the 3' end, the nucleotide at position 6 of the antisense strand is substituted by the nucleotide analog;
optionally, in the direction from the 5' end to the 3' end, the nucleotide at position 7 of the antisense strand is substituted by the nucleotide analog;
optionally, in the direction from the 5' end to the 3' end, the nucleotide at position 8 of the antisense strand is substituted by the nucleotide analog.

15. The double-stranded oligonucleotide of any one of claims 13-14, **characterized in that** the Base in each of the nucleotide analogs is the same as the base in the nucleotide it substitutes.

16. The double-stranded oligonucleotide of any one of claims 13-15, **characterized in that**, in the nucleotide sequence of the sense strand and in the direction from the 5' end to the 3' end, the nucleotide at position 5 is selected from 2'-O-methyl modified nucleotides or 2'-O-methoxyethyl modified nucleotides, at least three nucleotides at positions 7-10 are 2'-fluoro modified nucleotides, and the nucleotides at the remaining positions are independently selected from 2'-O-methyl modified nucleotides;
and/or, in the nucleotide sequence of the antisense strand and in the direction from the 5' end to the 3' end, at least one nucleotide at positions 3-8 is independently selected from the nucleotide analogs, at least four nucleotides at positions 2, 6, 9, 12, 14 and 16 are selected from 2'-fluoro modified nucleotides, the nucleotide at position 15 is selected from 2'-O-methoxyethyl modified nucleotides or 2'-O-methyl modified nucleotides, and the nucleotides at the remaining positions are independently selected from 2'-O-methyl modified nucleotides; wherein, when the nucleotide at position 6 is selected from the nucleotide analogs, the nucleotides at positions 2, 9, 12, 14 and 16 are selected from 2'-fluoro modified nucleotides; when the nucleotide at position 6 is selected from 2'-fluoro modified nucleotides, at least one nucleotide at positions 3-5 and 7-8 is independently selected from the nucleotide analogs;
optionally, in the nucleotide sequence of the sense strand and in the direction from the 5' end to the 3' end, at least three nucleotides at positions 7-10 are 2'-fluoro modified nucleotides, and the nucleotides at the remaining positions are independently selected from 2'-O-methyl modified nucleotides; and/or, in the nucleotide sequence of the antisense strand and in the direction from the 5' end to the 3' end, at least one nucleotide at positions 3-8 is independently selected from the nucleotide analog, at least four nucleotides at positions 2, 6, 9, 12, 14 and 16 are selected from 2'-fluoro modified nucleotides, the nucleotide at position 15 is selected from 2'-O-methoxyethyl modified nucleotides or 2'-O-methyl modified nucleotides, and the nucleotides at the remaining positions are independently selected from 2'-O-methyl modified nucleotides; wherein, when the nucleotide at position 6 is selected from the nucleotide analogs, the nucleotides at positions 2, 9, 12, 14 and 16 are selected from 2'-fluoro modified nucleotides; when the nucleotide at position 6 is selected from 2'-fluoro modified nucleotides, at least one nucleotide at positions 3-5 and 7-8 is independently selected from the nucleotide analogs.

17. The double-stranded oligonucleotide of any one of claims 13-16, **characterized in that**, in the nucleotide sequence of the sense strand and in the direction from the 5' end to the 3' end, at least three nucleotides at positions 7-10 are 2'-fluoro modified nucleotides, and the nucleotides at the remaining positions are independently selected from 2'-O-methyl modified nucleotides;
in the nucleotide sequence of the antisense strand and in the direction from the 5' end to the 3' end, at least one nucleotide at positions 3-8 is independently selected from the nucleotide analogs, at least three nucleotides at positions 2, 6, 14 and 16 are selected from 2'-fluoro modified nucleotides, and the nucleotide at position 9 or position 12 is selected from 2'-fluoro modified nucleotides, the nucleotide at position 15 is selected from 2'-O-methoxyethyl modified nucleotides or 2'-O-methyl modified nucleotides, and the nucleotides at the remaining positions are independently selected from 2'-O-methyl modified nucleotides; wherein, when the nucleotide at position 6 is selected from the nucleotide analogs, the nucleotides at positions 2, 9, 12, 14 and 16 are selected from 2'-fluoro modified nucleotides; when the nucleotide at position 6 is selected from 2'-fluoro modified nucleotides, at least one nucleotide at positions 3-5 and 7-8 is independently selected from the nucleotide analogs.

18. The double-stranded oligonucleotide of any one of claims 13-17, **characterized in that** the modifications of the sense strand and the antisense strand in the double-stranded oligonucleotide are selected from one of the following (1) to (24):
(1) in the nucleotide sequence of the sense strand and in the direction from the 5' end to the 3' end, the nucleotides at positions 7-10 are independently selected from 2'-fluoro modified nucleotides, and the nucleotides at the remaining positions are independently selected from 2'-O-methyl modified nucleotides;
in the nucleotide sequence of the antisense strand and in the direction from the 5' end to the 3' end, the nucleotide at position 3 is selected from the nucleotide analogs, the nucleotides at positions 2, 6, 9, 14 and 16 are independently selected from 2'-fluoro modified nucleotides, and the nucleotides at the remaining positions are independently selected from 2'-O-methyl modified nucleotides;
(2) in the nucleotide sequence of the sense strand and in the direction from the 5' end to the 3' end, the nucleotides at positions 7-10 are independently selected from 2'-fluoro modified nucleotides, and the nucleotides at the remaining positions are independently selected from 2'-O-methyl modified nucleotides;
in the nucleotide sequence of the antisense strand and in the direction from the 5' end to the 3' end, the nucleotide at position 4 is selected from the nucleotide analogs, the nucleotides at positions 2, 6, 9, 14 and 16 are independently selected from 2'-fluoro modified nucleotides, and the nucleotides at the remaining positions are independently selected from 2'-O-methyl modified nucleotides;
(3) in the nucleotide sequence of the sense strand and in the direction from the 5' end to the 3' end, the nucleotides at positions 7-10 are independently selected from 2'-fluoro modified nucleotides, and the nucleotides at the remaining positions are independently selected from 2'-O-methyl modified nucleotides;
in the nucleotide sequence of the antisense strand and in the direction from the 5' end to the 3' end, the nucleotide at position 5 is selected from the nucleotide analogs, the nucleotides at positions 2, 6, 9, 14 and 16 are independently selected from 2'-fluoro modified nucleotides, and the nucleotides at the remaining positions are independently selected from 2'-O-methyl modified nucleotides;
(4) in the nucleotide sequence of the sense strand and in the direction from the 5' end to the 3' end, the nucleotides at positions 7-10 are independently selected from 2'-fluoro modified nucleotides, and the nucleotides at the remaining positions are independently selected from 2'-O-methyl modified nucleotides;
in the nucleotide sequence of the antisense strand and in the direction from the 5' end to the 3' end, the nucleotide at position 6 is selected from the nucleotide analogs, the nucleotides at positions 2, 9, 14 and 16 are independently selected from 2'-fluoro modified nucleotides, and the nucleotides at the remaining positions are independently selected from 2'-O-methyl modified nucleotides;
(5) in the nucleotide sequence of the sense strand and in the direction from the 5' end to the 3' end, the nucleotides at positions 7-10 are independently selected from 2'-fluoro modified nucleotides, and the nucleotides at the remaining positions are independently selected from 2'-O-methyl modified nucleotides;
in the nucleotide sequence of the antisense strand and in the direction from the 5' end to the 3' end, the nucleotide at position 7 is selected from the nucleotide analogs, the nucleotides at positions 2, 6, 9, 14 and 16 are independently selected from 2'-fluoro modified nucleotides, and the nucleotides at the remaining positions are independently selected from 2'-O-methyl modified nucleotides;
(6) in the nucleotide sequence of the sense strand and in the direction from the 5' end to the 3' end, the nucleotides at positions 7-10 are independently selected from 2'-fluoro modified nucleotides, and the nucleotides at the remaining positions are independently selected from 2'-O-methyl modified nucleotides;
in the nucleotide sequence of the antisense strand and in the direction from the 5' end to the 3' end, the nucleotide at position 8 is selected from the nucleotide analogs, the nucleotides at positions 2, 6, 9, 14 and 16 are independently selected from 2'-fluoro modified nucleotides, and the nucleotides at the remaining positions are independently selected from 2'-O-methyl modified nucleotides;
(7) in the nucleotide sequence of the sense strand and in the direction from the 5' end to the 3' end, the nucleotides at positions 7-10 are independently selected from 2'-fluoro modified nucleotides, and the nucleotides at the remaining positions are independently selected from 2'-O-methyl modified nucleotides;
in the nucleotide sequence of the antisense strand and in the direction from the 5' end to the 3' end, the nucleotide at position 3 is selected from the nucleotide analogs, the nucleotides at positions 2, 6, 9, 14 and 16 are independently selected from 2'-fluoro modified nucleotides, the nucleotide at position 15 is selected from 2'-O-methoxyethyl modified nucleotides, and the nucleotides at the remaining positions are independently selected from 2'-O-methyl modified nucleotides;
(8) in the nucleotide sequence of the sense strand and in the direction from the 5' end to the 3' end, the nucleotides at positions 7-10 are independently selected from 2'-fluoro modified nucleotides, and the nucleotides at the remaining positions are independently selected from 2'-O-methyl modified nucleotides;
in the nucleotide sequence of the antisense strand and in the direction from the 5' end to the 3' end, the nucleotide at position 4 is selected from the nucleotide analogs, the nucleotides at positions 2, 6, 9, 14 and 16 are independently selected from 2'-fluoro modified nucleotides, the nucleotide at position 15 is selected from 2'-O-methoxyethyl modified nucleotides, and the nucleotides at the remaining positions are independently selected from 2'-O-methyl modified nucleotides;
(9) in the nucleotide sequence of the sense strand and in the direction from the 5' end to the 3' end, the nucleotides at positions 7-10 are independently selected from 2'-fluoro modified nucleotides, the nucleotide at position 15 is selected from 2'-O-methoxyethyl modified nucleotides, and the nucleotides at the remaining positions are independently selected from 2'-O-methyl modified nucleotides;
in the nucleotide sequence of the antisense strand and in the direction from the 5' end to the 3' end, the nucleotide at position 5 is selected from the nucleotide analogs, the nucleotides at positions 2, 6, 9, 14 and 16 are independently selected from 2'-fluoro modified nucleotides, the nucleotide at position 15 is selected from 2'-O-methoxyethyl modified nucleotides, and the nucleotides at the remaining positions are independently selected from 2'-O-methyl modified nucleotides;
(10) in the nucleotide sequence of the sense strand and in the direction from the 5' end to the 3' end, the nucleotides at positions 7-10 are independently selected from 2'-fluoro modified nucleotides, the nucleotide at position 15 is selected from 2'-O-methoxyethyl modified nucleotides, and the nucleotides at the remaining positions are independently selected from 2'-O-methyl modified nucleotides;
in the nucleotide sequence of the antisense strand and in the direction from the 5' end to the 3' end, the nucleotide at position 6 is selected from the nucleotide analogs, the nucleotides at positions 2, 9, 14 and 16 are independently selected from 2'-fluoro modified nucleotides, the nucleotide at position 15 is selected from 2'-O-methoxyethyl modified nucleotides, and the nucleotides at the remaining positions are independently selected from 2'-O-methyl modified nucleotides;
(11) in the nucleotide sequence of the sense strand and in the direction from the 5' end to the 3' end, the nucleotides at positions 7-10 are independently selected from 2'-fluoro modified nucleotides, the nucleotide at position 15 is selected from 2'-O-methoxyethyl modified nucleotides, and the nucleotides at the remaining positions are independently selected from 2'-O-methyl modified nucleotides;
in the nucleotide sequence of the antisense strand and in the direction from the 5' end to the 3' end, the nucleotide at position 7 is selected from the nucleotide analogs, the nucleotides at positions 2, 6, 9, 14 and 16 are independently selected from 2'-fluoro modified nucleotides, the nucleotide at position 15 is selected from 2'-O-methoxyethyl modified nucleotides, and the nucleotides at the remaining positions are independently selected from 2'-O-methyl modified nucleotides;
(12) in the nucleotide sequence of the sense strand and in the direction from the 5' end to the 3' end, the nucleotides at positions 7-10 are independently selected from 2'-fluoro modified nucleotides, and the nucleotides at the remaining positions are independently selected from 2'-O-methyl modified nucleotides;
in the nucleotide sequence of the antisense strand and in the direction from the 5' end to the 3' end, the nucleotide at position 8 is selected from the nucleotide analogs, the nucleotides at positions 2, 6, 9, 14 and 16 are independently selected from 2'-fluoro modified nucleotides, the nucleotide at position 15 is selected from 2'-O-methoxyethyl modified nucleotides, and the nucleotides at the remaining positions are independently selected from 2'-O-methyl modified nucleotides;
(13) in the nucleotide sequence of the sense strand and in the direction from the 5' end to the 3' end, the nucleotides at positions 7-10 are independently selected from 2'-fluoro modified nucleotides, and the nucleotides at the remaining positions are independently selected from 2'-O-methyl modified nucleotides;
in the nucleotide sequence of the antisense strand and in the direction from the 5' end to the 3' end, the nucleotide at position 3 is selected from the nucleotide analogs, the nucleotides at positions 2, 6, 12, 14 and 16 are independently selected from 2'-fluoro modified nucleotides, and the nucleotides at the remaining positions are independently selected from 2'-O-methyl modified nucleotides;
(14) in the nucleotide sequence of the sense strand and in the direction from the 5' end to the 3' end, the nucleotides at positions 7-10 are independently selected from 2'-fluoro modified nucleotides, and the nucleotides at the remaining positions are independently selected from 2'-O-methyl modified nucleotides;
in the nucleotide sequence of the antisense strand and in the direction from the 5' end to the 3' end, the nucleotide at position 4 is selected from the nucleotide analogs, the nucleotides at positions 2, 6, 12, 14 and 16 are independently selected from 2'-fluoro modified nucleotides, and the nucleotides at the remaining positions are independently selected from 2'-O-methyl modified nucleotides;
(15) in the nucleotide sequence of the sense strand and in the direction from the 5' end to the 3' end, the nucleotides at positions 7-10 are independently selected from 2'-fluoro modified nucleotides, and the nucleotides at the remaining positions are independently selected from 2'-O-methyl modified nucleotides;
in the nucleotide sequence of the antisense strand and in the direction from the 5' end to the 3' end, the nucleotide at position 5 is selected from the nucleotide analogs, the nucleotides at positions 2, 6, 12, 14 and 16 are independently selected from 2'-fluoro modified nucleotides, and the nucleotides at the remaining positions are independently selected from 2'-O-methyl modified nucleotides;
(16) in the nucleotide sequence of the sense strand and in the direction from the 5' end to the 3' end, the nucleotides at positions 7-10 are independently selected from 2'-fluoro modified nucleotides, and the nucleotides at the remaining positions are independently selected from 2'-O-methyl modified nucleotides;
in the nucleotide sequence of the antisense strand and in the direction from the 5' end to the 3' end, the nucleotide at position 6 is selected from the nucleotide analogs, the nucleotides at positions 2, 12, 14 and 16 are independently selected from 2'-fluoro modified nucleotides, and the nucleotides at the remaining positions are independently selected from 2'-O-methyl modified nucleotides;
(17) in the nucleotide sequence of the sense strand and in the direction from the 5' end to the 3' end, the nucleotides at positions 7-10 are independently selected from 2'-fluoro modified nucleotides, and the nucleotides at the remaining positions are independently selected from 2'-O-methyl modified nucleotides;
in the nucleotide sequence of the antisense strand and in the direction from the 5' end to the 3' end, the nucleotide at position 7 is selected from the nucleotide analogs, the nucleotides at positions 2, 6, 12, 14 and 16 are independently selected from 2'-fluoro modified nucleotides, and the nucleotides at the remaining positions are independently selected from 2'-O-methyl modified nucleotides;
(18) in the nucleotide sequence of the sense strand and in the direction from the 5' end to the 3' end, the nucleotides at positions 7-10 are independently selected from 2'-fluoro modified nucleotides, and the nucleotides at the remaining positions are independently selected from 2'-O-methyl modified nucleotides;
in the nucleotide sequence of the antisense strand and in the direction from the 5' end to the 3' end, the nucleotide at position 8 is selected from the nucleotide analogs, the nucleotides at positions 2, 6, 12, 14 and 16 are independently selected from 2'-fluoro modified nucleotides, and the nucleotides at the remaining positions are independently selected from 2'-O-methyl modified nucleotides;
(19) in the nucleotide sequence of the sense strand and in the direction from the 5' end to the 3' end, the nucleotides at positions 7-10 are independently selected from 2'-fluoro modified nucleotides, and the nucleotides at the remaining positions are independently selected from 2'-O-methyl modified nucleotides;
in the nucleotide sequence of the antisense strand and in the direction from the 5' end to the 3' end, the nucleotide at position 3 is selected from the nucleotide analogs, the nucleotides at positions 2, 6, 12, 14 and 16 are independently selected from 2'-fluoro modified nucleotides, the nucleotide at position 15 is selected from 2'-O-methoxyethyl modified nucleotides, and the nucleotides at the remaining positions are independently selected from 2'-O-methyl modified nucleotides;
(20) in the nucleotide sequence of the sense strand and in the direction from the 5' end to the 3' end, the nucleotides at positions 7-10 are independently selected from 2'-fluoro modified nucleotides, and the nucleotides at the remaining positions are independently selected from 2'-O-methyl modified nucleotides;
in the nucleotide sequence of the antisense strand and in the direction from the 5' end to the 3' end, the nucleotide at position 4 is selected from the nucleotide analogs, the nucleotides at positions 2, 6, 12, 14 and 16 are independently selected from 2'-fluoro modified nucleotides, the nucleotide at position 15 is selected from 2'-O-methoxyethyl modified nucleotides, and the nucleotides at the remaining positions are independently selected from 2'-O-methyl modified nucleotides;
(21) in the nucleotide sequence of the sense strand and in the direction from the 5' end to the 3' end, the nucleotides at positions 7-10 are independently selected from 2'-fluoro modified nucleotides, the nucleotide at position 15 is selected from 2'-O-methoxyethyl modified nucleotides, and the nucleotides at the remaining positions are independently selected from 2'-O-methyl modified nucleotides;
in the nucleotide sequence of the antisense strand and in the direction from the 5' end to the 3' end, the nucleotide at position 5 is selected from the nucleotide analogs, the nucleotides at positions 2, 6, 12, 14 and 16 are independently selected from 2'-fluoro modified nucleotides, the nucleotide at position 15 is selected from 2'-O-methoxyethyl modified nucleotides, and the nucleotides at the remaining positions are independently selected from 2'-O-methyl modified nucleotides;
(22) in the nucleotide sequence of the sense strand and in the direction from the 5' end to the 3' end, the nucleotides at positions 7-10 are independently selected from 2'-fluoro modified nucleotides, the nucleotide at position 15 is selected from 2'-O-methoxyethyl modified nucleotides, and the nucleotides at the remaining positions are independently selected from 2'-O-methyl modified nucleotides;
in the nucleotide sequence of the antisense strand and in the direction from the 5' end to the 3' end, the nucleotide at position 6 is selected from the nucleotide analogs, the nucleotides at positions 2, 12, 14 and 16 are independently selected from 2'-fluoro modified nucleotides, the nucleotide at position 15 is selected from 2'-O-methoxyethyl modified nucleotides, and the nucleotides at the remaining positions are independently selected from 2'-O-methyl modified nucleotides;
(23) in the nucleotide sequence of the sense strand and in the direction from the 5' end to the 3' end, the nucleotides at positions 7-10 are independently selected from 2'-fluoro modified nucleotides, the nucleotide at position 15 is selected from 2'-O-methoxyethyl modified nucleotides, and the nucleotides at the remaining positions are independently selected from 2'-O-methyl modified nucleotides;
in the nucleotide sequence of the antisense strand and in the direction from the 5' end to the 3' end, the nucleotide at position 7 is selected from the nucleotide analogs, the nucleotides at positions 2, 6, 12, 14 and 16 are independently selected from 2'-fluoro modified nucleotides, the nucleotide at position 15 is selected from 2'-O-methoxyethyl modified nucleotides, and the nucleotides at the remaining positions are independently selected from 2'-O-methyl modified nucleotides;
(24) in the nucleotide sequence of the sense strand and in the direction from the 5' end to the 3' end, the nucleotides at positions 7-10 are independently selected from 2'-fluoro modified nucleotides, and the nucleotides at the remaining positions are independently selected from 2'-O-methyl modified nucleotides;
in the nucleotide sequence of the antisense strand and in the direction from the 5' end to the 3' end, the nucleotide at position 8 is selected from the nucleotide analogs, the nucleotides at positions 2, 6, 12, 14 and 16 are independently selected from 2'-fluoro modified nucleotides, the nucleotide at position 15 is selected from 2'-O-methoxyethyl modified nucleotides, and the nucleotides at the remaining positions are independently selected from 2'-O-methyl modified nucleotides.

19. The double-stranded oligonucleotide of any one of claims 13-18, **characterized in that** the double-stranded oligonucleotide is selected from siRNA.

20. A double-stranded oligonucleotide conjugate, **characterized in that** the conjugate comprises the double-stranded oligonucleotide of any one of claims 13-19 and one or more ligands capable of binding to a cell surface receptor;
optionally, the ligand is selected from asialoglycoprotein receptor ligands (ASGPR ligands); optionally, the ASGPR ligands comprise galactose, galactose derivatives or galactose clusters;
the galactose derivatives comprise galactose derivatives with affinity for the asialoglycoprotein receptor equal to or exceeding that of galactose;
the galactose clusters comprise molecules having 2-4 terminal galactoses and/or galactose derivatives;
optionally, the galactose derivatives are selected from galactosamine, N-formylgalactosamine, N-acetylgalactosamine, N-propionylgalactosamine, N-n-butyrylgalactosamine and N-isobutyrylgalactosamine.

21. The conjugate of claim 20, **characterized in that** the number of the ligands is selected from one, and the ligand is conjugated to the 3' end of the sense strand of the double-stranded oligonucleotide.

22. The conjugate of any one of claims 20-21, **characterized in that** each of the ligand is selected from the structure set forth in formula (300), or a stereoisomer thereof, or a pharmaceutically acceptable salt thereof: 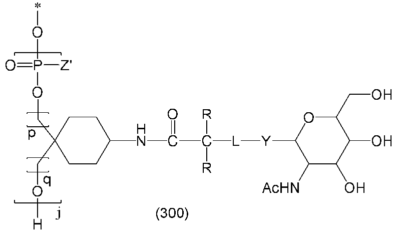
wherein, * represents the site of covalent attachment of the ligand to the sense strand or the antisense strand;
j is selected from 1, 2, 3 or 4;
each Z' is independently selected from hydroxyl or sulfhydryl;
each p is independently selected from 1, 2 or 3;
each q is independently selected from 1, 2 or 3;
each R is independently selected from H, optionally substituted C₁₋₆ alkyl or optionally substituted C₁₋₆ alkoxy;
each L is independently selected from optionally substituted C₂₋₂₀ alkylene or 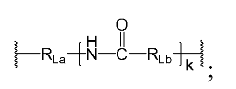 R_{La} and R_{Lb} are independently selected from optionally substituted C₁₋₁₀ alkylene, and k is selected from 1, 2, 3, 4 or 5;
each Y is independently selected from O, S or NH;
optionally, m is selected from 1, 2 or 3;
optionally, m is selected from 3;
optionally, Z' is selected from hydroxyl;
optionally, p is selected from 1;
optionally, q is selected from 1;
optionally, R is selected from H;
optionally, each L is independently selected from optionally substituted C₂₋₁₀ alkylene or 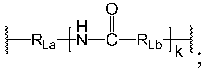 wherein R_{La} and R_{Lb} are independently selected from optionally substituted C₁₋₁₀ alkylene, and k is selected from 1, 2 or 3;
optionally, k is selected from 1;
optionally, each L is independently selected from 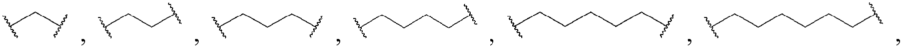 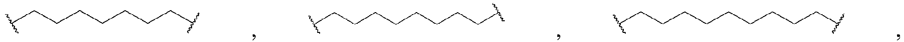 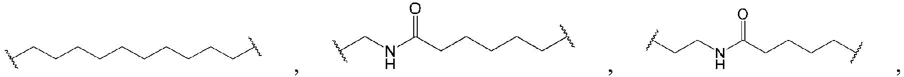 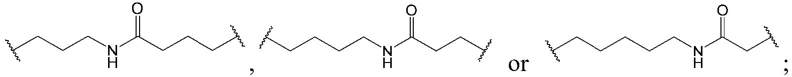
optionally, each L is independently selected from 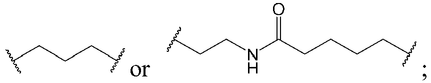
optionally, each L is independently selected from 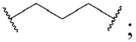
optionally, each L is independently selected from 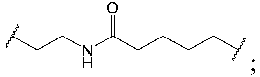
optionally, Y is selected from O.

23. The conjugate of claims 20-22, **characterized in that** each of the ligand is independently selected from the structure set forth in formula (301), or a stereoisomer thereof, or a pharmaceutically acceptable salt thereof:

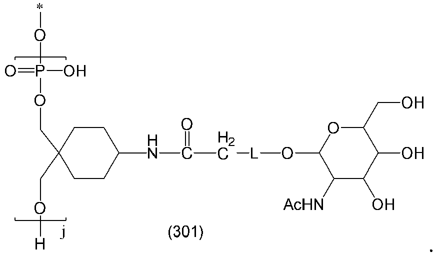

24. The conjugate of any one of claims 20-23, **characterized in that** each of the ligand is independently selected from any one of the following structures, or a stereoisomer thereof, or a pharmaceutically acceptable salt thereof:

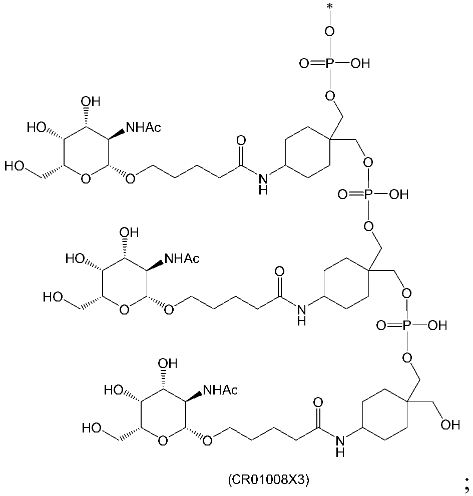

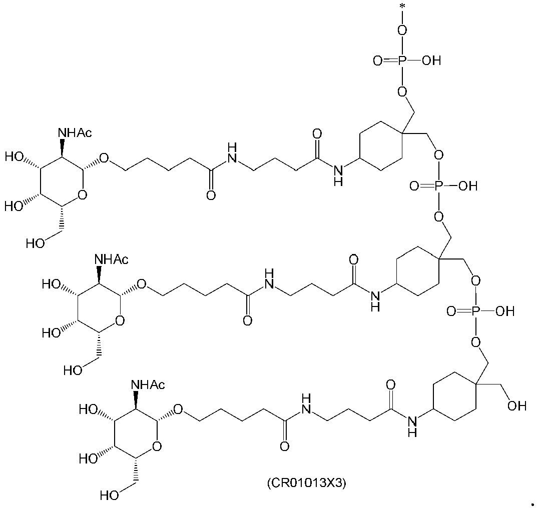

25. The conjugate of any one of claims 20-24, **characterized in that** each of the ligand is independently selected from the structure set forth in formula (CR01008×3), or a stereoisomer thereof, or a pharmaceutically acceptable salt thereof:

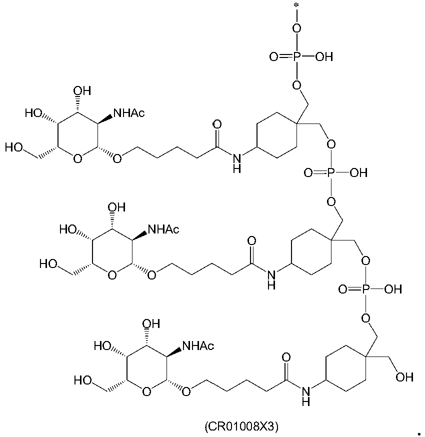

26. A composition, **characterized in that** the composition comprises the double-stranded oligonucleotide of any one of claims 13-19 and/or the double-stranded oligonucleotide conjugate of any one of claims 20-25.

27. A pharmaceutical composition, **characterized in that** the pharmaceutical composition comprises the double-stranded oligonucleotide of any one of claims 13-19, and/or the double-stranded oligonucleotide conjugate of any one of claims 20-25, and/or the composition of claim 26.

28. A nucleoside analog, **characterized in that** it is selected from the structure set forth in formula (I-i), or a stereoisomer thereof, or a pharmaceutically acceptable salt thereof: 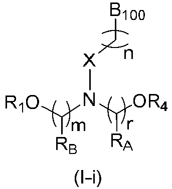
wherein, B₁₀₀ is as defined for B₂₀₀ in claim 1; if B₁₀₀ contains amino, the amino is protected by an amino protecting group;
n is selected from 1, 2 or 3;
X is selected from 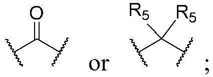 each R₅ is independently selected from optionally substituted C₁₋₃ alkyl, and the optionally substituted C₁₋₃ alkyl contains substituent(s) independently selected from halogen, C₁₋₃ alkoxy, hydroxyl or amino;
R₁ is selected from H or a hydroxyl protecting group;
optionally, the hydroxyl protecting group is selected from trityl, 4-methoxytrityl, 4,4'-dimethoxytrityl or 4,4',4"-trimethoxytrityl;
R₄ is selected from H or 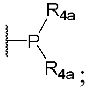 each R₄ₐ is independently selected from 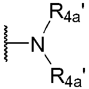 or C₁₋₆ alkoxy comprising a cyano substituent, and at least one R₄ₐ is selected from 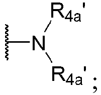 each R₄ₐ' is independently selected from optionally substituted C₁₋₆ alkyl;
m is selected from 1, 2, 3 or 4;
r is selected from 1, 2, 3 or 4;
each R_{A} and each R_{B} are independently selected from H or optionally substituted C₁₋₃ alkyl; and there is at least one optionally substituted C₁₋₃ alkyl in R_{A} and R_{B}; if the optionally substituted C₁₋₃ alkyl contains substituent(s), the substituent(s) are independently selected from halogen, C₁₋₃ alkoxy, hydroxyl or amino;
optionally, the nucleoside analog set forth in the formula (I-i) has the structure set forth in formula (100), or a stereoisomer thereof, or a pharmaceutically acceptable salt thereof: 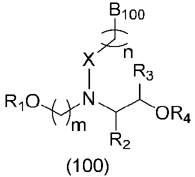
wherein, R₂ and R₃ are independently selected from H or optionally substituted C₁₋₃ alkyl; and at least one of R₂ and R₃ is selected from optionally substituted C₁₋₃ alkyl; if the optionally substituted C₁₋₃ alkyl contains substituent(s), the substituent(s) are independently selected from halogen, C₁₋₃ alkoxy, hydroxyl or amino; and the remaining substituents are defined as above.

29. The nucleoside analog of claim 28, **characterized in that** the hydroxyl protecting group is selected from 4,4'-dimethoxytrityl.

30. The nucleoside analog of any one of claims 28-29, **characterized in that** R₄ₐ^{'} is selected from isopropyl, and the C₁₋₆ alkoxy comprising a cyano substituent is selected from

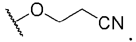

31. The nucleoside analog of any one of claims 28-30, **characterized in that** 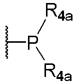 is selected from 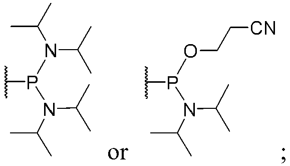 optionally, 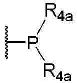 is selected from

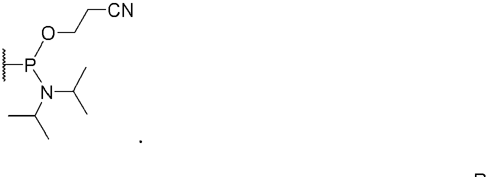

32. The nucleoside analog of any one of claims 28-31, **characterized in that** 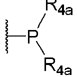 is selected from

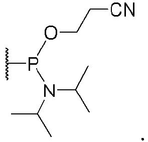

33. The nucleoside analog of any one of claims 28-32, **characterized in that** the nucleoside analog is selected from any one of the following structures, or a stereoisomer thereof, or a pharmaceutically acceptable salt thereof:

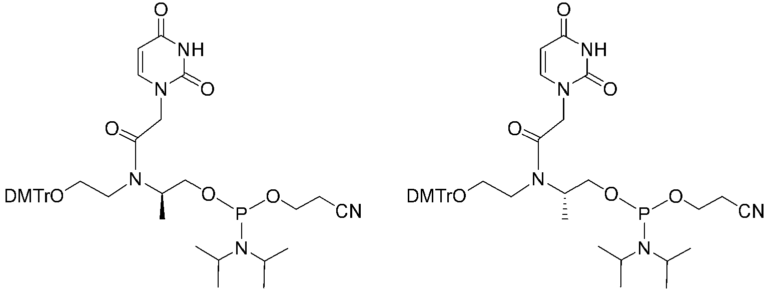

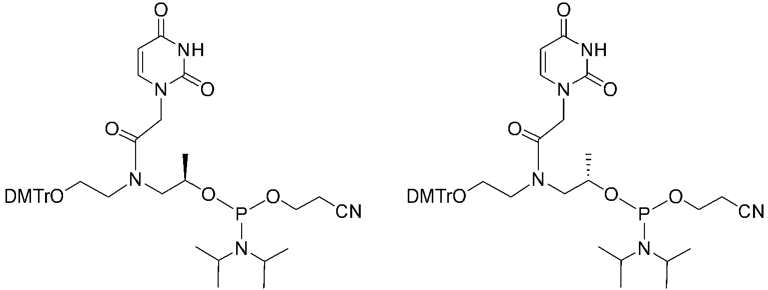

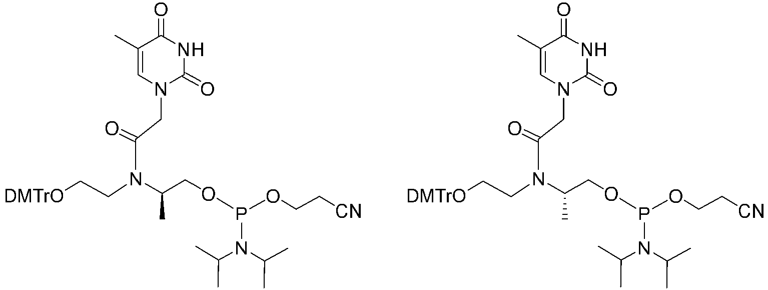

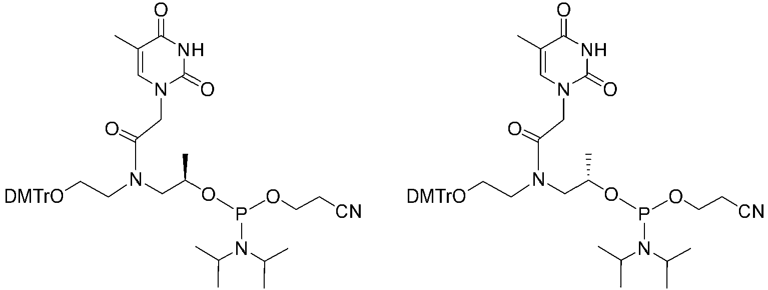

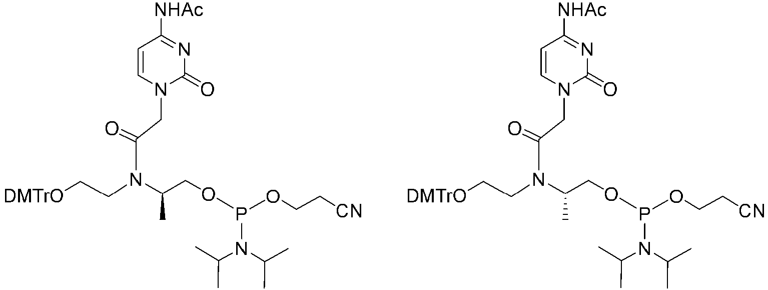

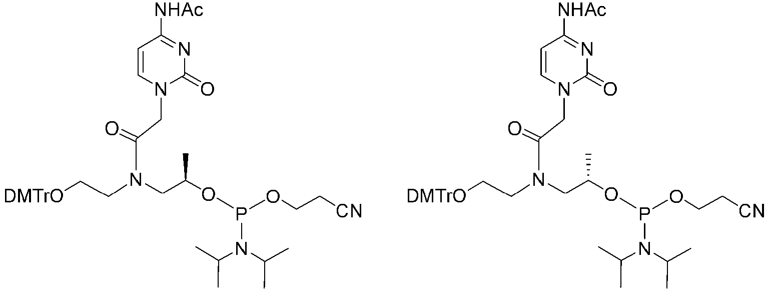

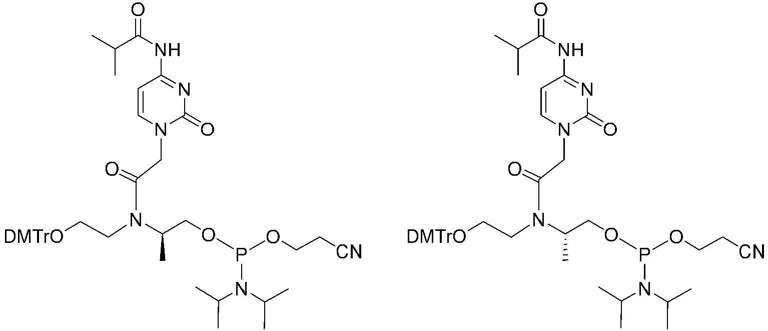

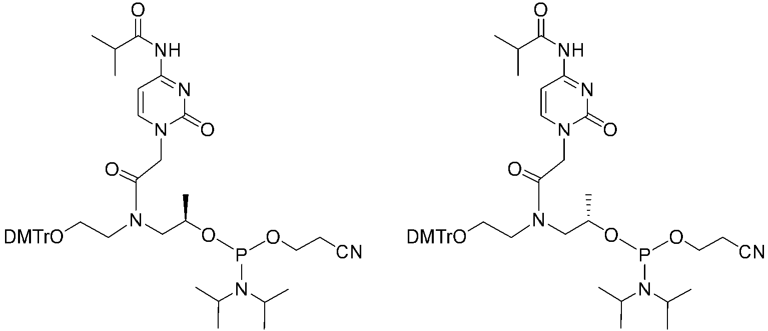

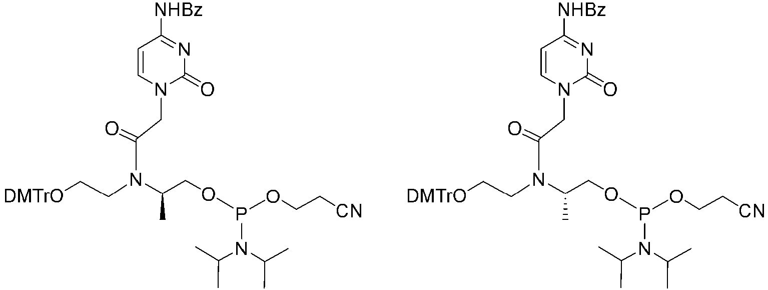

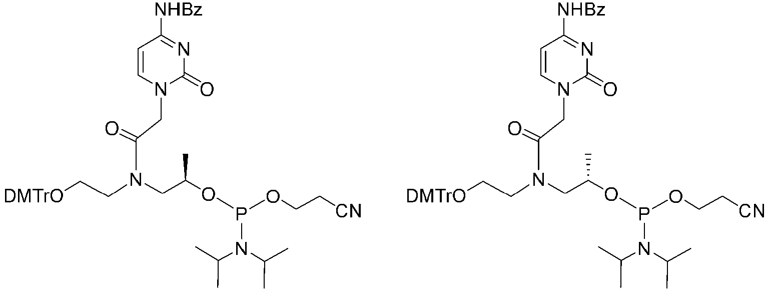

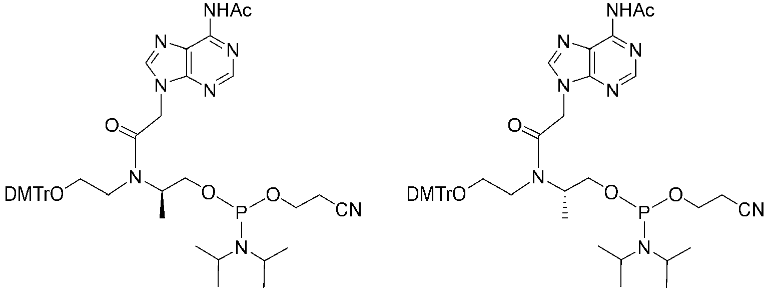

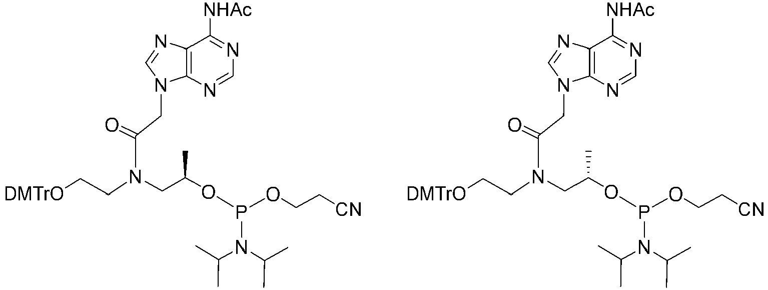

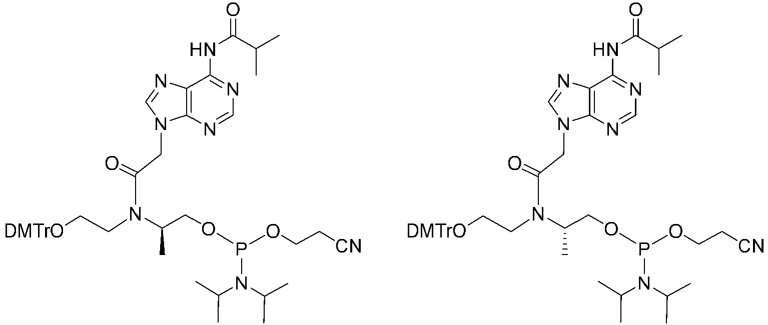

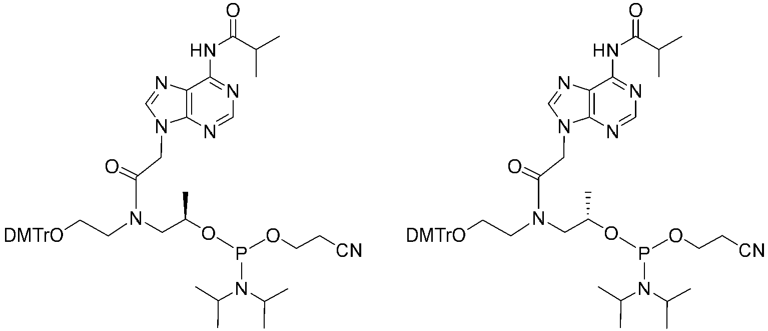

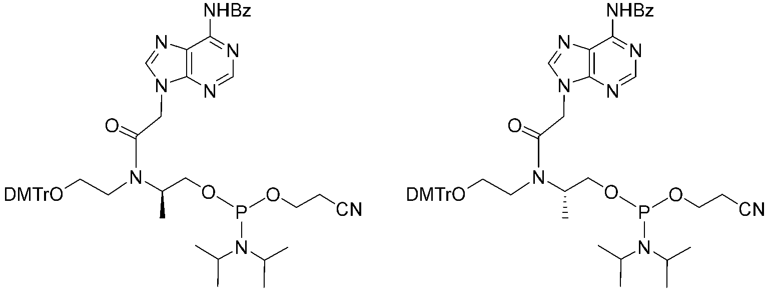

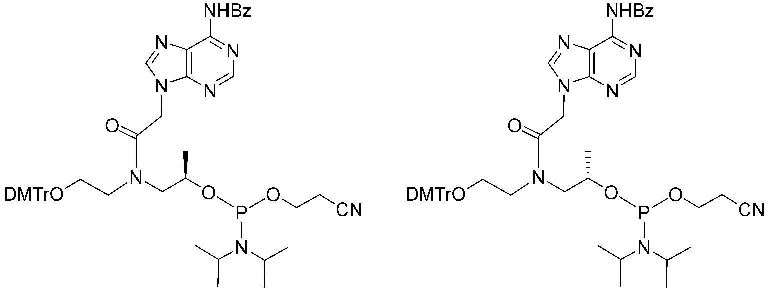

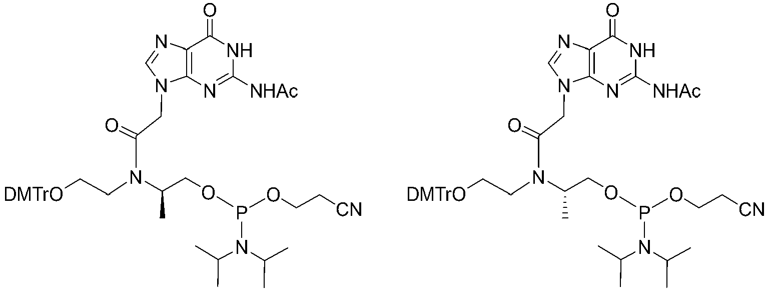

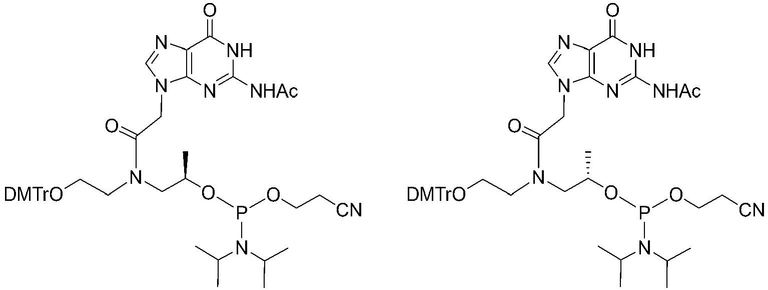

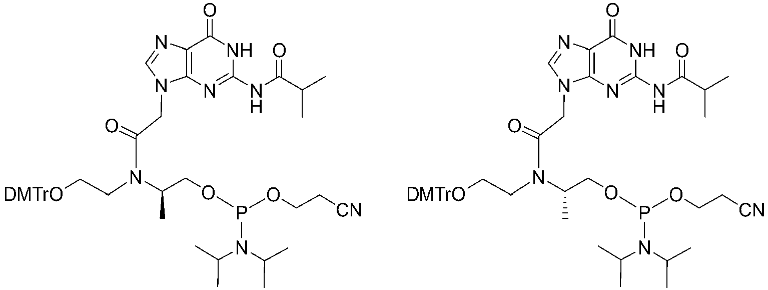

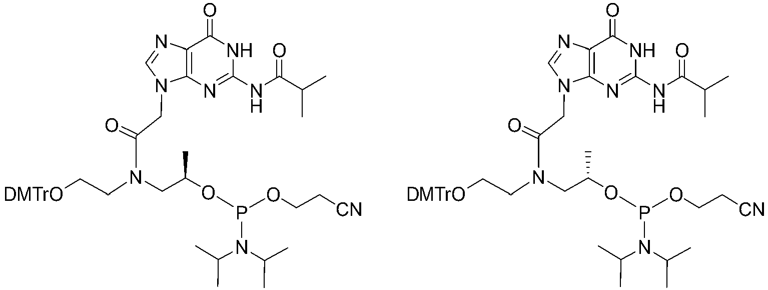

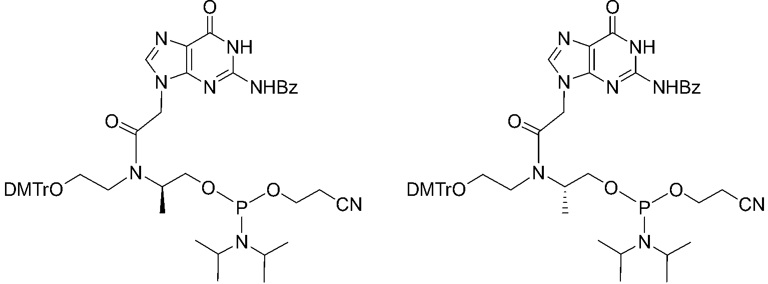

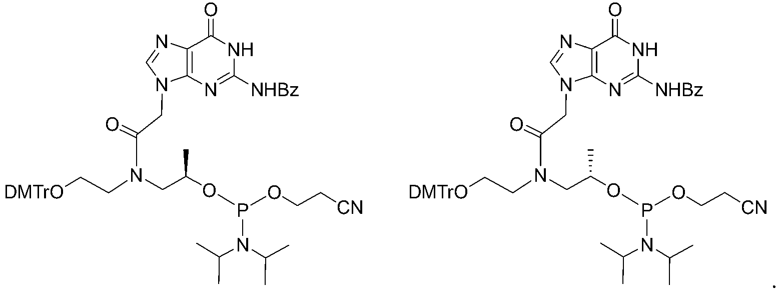

34. The nucleoside analog of claim 28, **characterized in that** the nucleoside analog set forth in the formula (I-i) has the structure set forth in the following formula (400), or a stereoisomer thereof, or a pharmaceutically acceptable salt thereof: 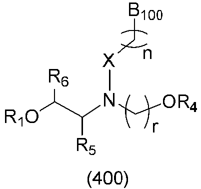
wherein R₁, R₄, B₁₀₀, n, X and * are as defined in any one of claims 11-12;
r is selected from 1, 2, 3 or 4;
R₅ and R₆ are independently selected from H or optionally substituted C₁₋₃ alkyl; and at least one of R₅ and R₆ is selected from optionally substituted C₁₋₃ alkyl; if the optionally substituted C₁₋₃ alkyl contains substituent(s), the substituent(s) are independently selected from halogen, C₁₋₃ alkoxy, hydroxyl or amino.

35. The nucleoside analog of claim 34, **characterized in that** r is selected from 1, 2 or 3;
optionally, r is selected from 2;
optionally, R₅ and R₆ are independently selected from H or optionally substituted C₁₋₃ alkyl; and R₅ and R₆ are not simultaneously H or optionally substituted C₁₋₃ alkyl;
optionally, R₅ and R₆ are independently selected from H or C₁₋₃ alkyl; and R₅ and R₆ are not simultaneously H or C₁₋₃ alkyl;
optionally, R₅ and R₆ are independently selected from H or methyl; and R₅ and R₆ are not simultaneously H or methyl;
optionally, the nucleoside analog has the structure set forth in formula (401), or a stereoisomer thereof, or a pharmaceutically acceptable salt thereof: 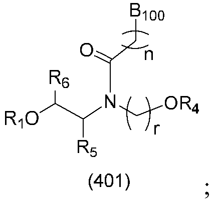
optionally, the nucleoside analog has any one of the following structures, or a stereoisomer thereof, or a pharmaceutically acceptable salt thereof: 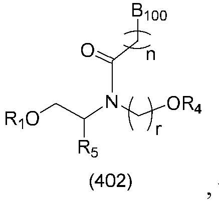
wherein R₅ is selected from optionally substituted C₁₋₃ alkyl; 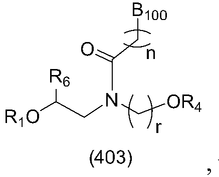
wherein R₆ is selected from optionally substituted C₁₋₃ alkyl;
optionally, the nucleoside analog has any one of the following structures, or a stereoisomer thereof, or a pharmaceutically acceptable salt thereof: 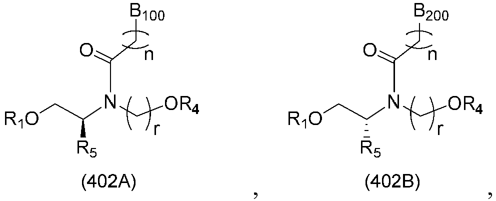 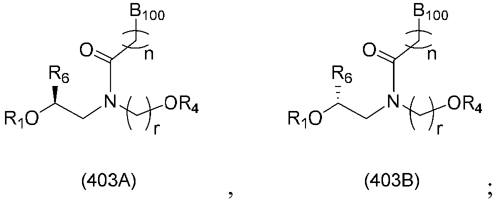
wherein R₅ and R₆ are independently selected from optionally substituted C₁₋₃ alkyl;
optionally, the nucleoside analog is selected from any one of the following structures, or a stereoisomer thereof, or a pharmaceutically acceptable salt thereof:

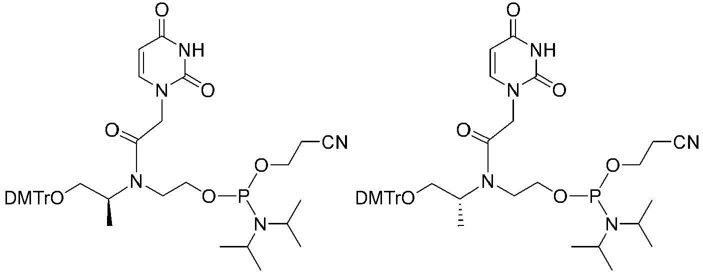

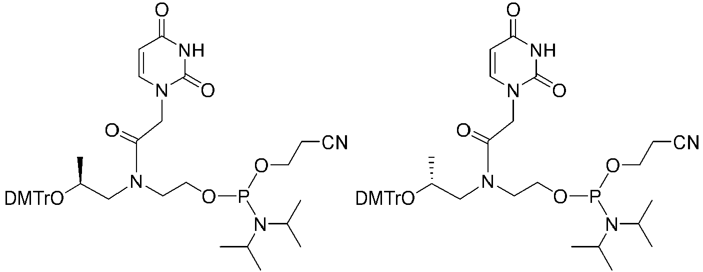

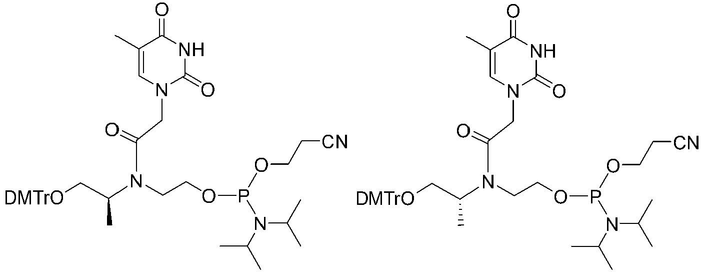

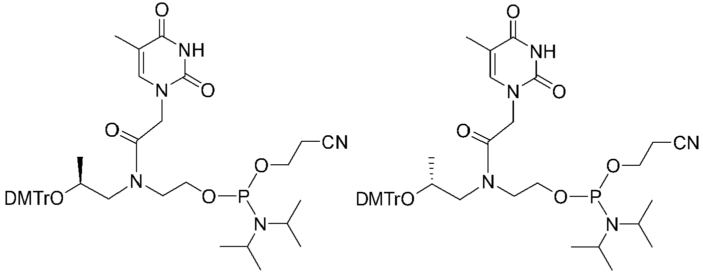

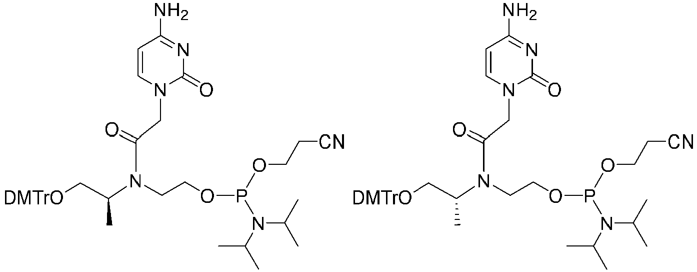

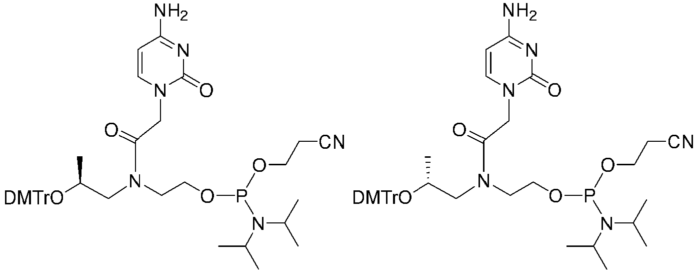

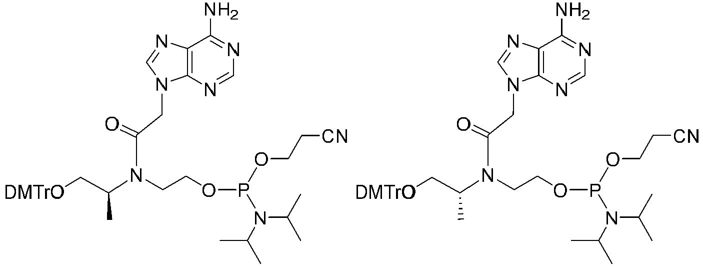

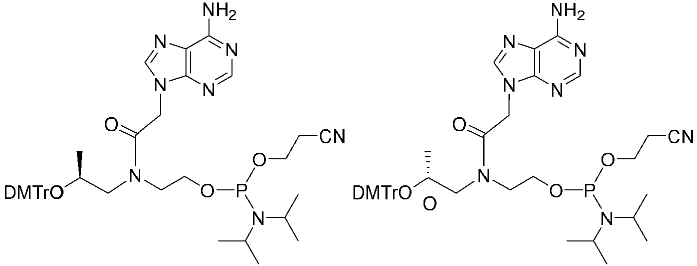

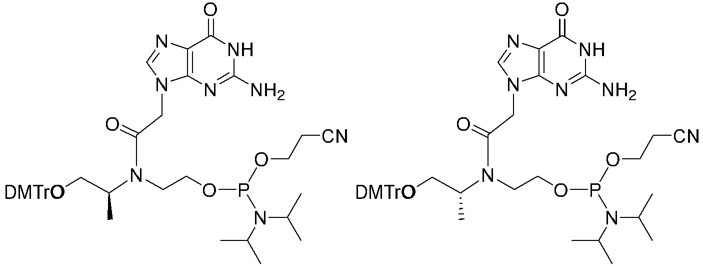

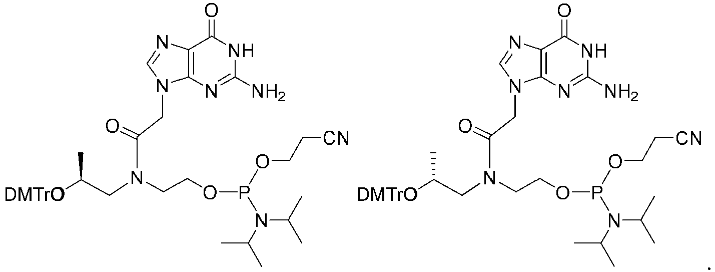

36. Use of the nucleotide analog of any one of claims 1-12, and/or the double-stranded oligonucleotide of any one of claims 13-19, and/or the double-stranded oligonucleotide conjugate of any one of claims 20-25, and/or the composition of claim 26, and/or the pharmaceutical composition of claim 27, and/or the nucleoside analog of any one of claims 28-35 in the preparation of a medicament for treating and/or preventing diseases or conditions associated with dysregulation of mRNA expression level of a specific gene.

37. A method for regulating the expression of a specific gene in a target cell, **characterized in that** the method comprises:
contacting the double-stranded oligonucleotide of any one of claims 13-19, and/or the double-stranded oligonucleotide conjugate of any one of claims 20-25, and/or the composition of claim 26, and/or the pharmaceutical composition of claim 27 with the target cell.

38. A method for preventing and/or treating a disease or condition associated with dysregulation of mRNA expression level of a specific gene in a target cell in a subject, **characterized in that** the method comprises:
administering to the subject a pharmaceutically acceptable dose of the double-stranded oligonucleotide of any one of claims 13-19, and/or the double-stranded oligonucleotide conjugate of any one of claims 20-25, and/or the composition of claim 26, and/or the pharmaceutical composition of claim 27.

39. A kit comprising the double-stranded oligonucleotide of any one of claims 13-19, and/or the double-stranded oligonucleotide conjugate of any one of claims 20-25, and/or the composition of claim 26, and/or the pharmaceutical composition of claim 27.
